(19) Europäisches Patentamt / European Patent Office / Office européen des brevets

(11) **EP 4 748 844 A1**

(12) **EUROPEAN PATENT APPLICATION**
published in accordance with Art. 153(4) EPC

(43) Date of publication:
**27.05.2026 Bulletin 2026/22**

(21) Application number: **24842368.3**

(22) Date of filing: **16.07.2024**

(51) International Patent Classification (IPC):
***C07D 513/04*** *(2006.01)* ***A61K 31/5415*** *(2006.01)*
***A61P 3/10*** *(2006.01)*

(52) Cooperative Patent Classification (CPC):
**A61K 31/404; A61K 31/407; A61K 31/4162; A61K 31/437; A61K 31/454; A61K 31/538; A61K 31/5415; A61K 31/542; A61P 1/00; A61P 1/04; A61P 3/10; A61P 9/00; A61P 17/00; A61P 17/06; A61P 19/02;** (Cont.)

(86) International application number:
**PCT/CN2024/105679**

(87) International publication number:
**WO 2025/016379 (23.01.2025 Gazette 2025/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.07.2023 CN 202310886773**

(71) Applicant: **SHANGHAI INSTITUTE OF MATERIA MEDICA, CHINESE ACADEMY OF SCIENCES**
**Shanghai 201203 (CN)**

(72) Inventors:
- **DUAN, Wenhu**
  **Shanghai 201203 (CN)**
- **GENG, Meiyu**
  **Shanghai 201203 (CN)**
- **ZHAN, Zhengsheng**
  **Shanghai 201203 (CN)**
- **XIE, Zuoquan**
  **Shanghai 201203 (CN)**
- **LI, Wenxin**
  **Shanghai 201203 (CN)**
- **WANG, Xiyuan**
  **Shanghai 201203 (CN)**
- **ZHOU, Hongfei**
  **Shanghai 201203 (CN)**
- **DING, Jian**
  **Shanghai 201203 (CN)**

(74) Representative: **Mewburn Ellis LLP**
**Aurora Building**
**Counterslip**
**Bristol BS1 6BX (GB)**

# (54) AZETIDINE COMPOUND AND MEDICAL USE THEREOF

(57) The present invention relates to an azetidine compound and a medical use thereof. Specifically, the compound has the structure as shown in formula (I), and can be used as a secretion regulator for type I interferons, especially as a cGAS/STING signal pathway-targeted inhibitor, and can be used for preparing drugs for preventing and/or treating inflammatory diseases and autoimmune diseases.

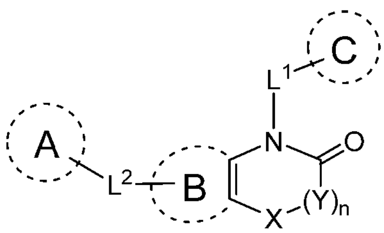

( I )

EP 4 748 844 A1

(52) Cooperative Patent Classification (CPC): (Cont.)
**A61P 21/00; A61P 25/00; A61P 27/02;
A61P 29/00; C07D 209/40; C07D 401/14;
C07D 403/12; C07D 403/14; C07D 405/14;
C07D 409/14; C07D 413/12; C07D 413/14;
C07D 417/12; C07D 417/14; C07D 471/04;
C07D 495/04; C07D 513/04**

## Description

### FIELD OF THE INVENTION

The present invention belongs to the field of medicinal chemistry and pharmacotherapy, specifically, the present invention relates to an azetidine compound and a pharmaceutical use thereof, as well as a pharmaceutical composition of the compound and a use as a secretion regulator of type I interferons, especially as a cGAS/STING signal pathway-targeted inhibitor, in the preparation of drugs for preventing and/or treating inflammatory diseases and autoimmune diseases.

### BACKGROUND OF THE INVENTION

STING, also known as transmembrane protein 173 (TMEM173), MPYS, MITA, or ERIS, is a protein encoded by the human TMEM173 gene. The cGAS/STING signaling pathway plays an important role in innate immune processes. When the DNA receptor cGAS (cyclic GMP-AMP synthase) detects the DNA of pathogen, it induces the production of cGAMP (cyclic GMP-AMP). Subsequently, cGAMP activates the stimulator of interferon genes (STNG) to recruit TANK binding kinase 1 (TBK1) downstream of STING, which induces the secretion of type I interferon and cytokines by phosphorylating interferon regulatory factor 3 (IRF3), and activates the acquired immune system through a series of cascade reactions, activating T cells to exert antitumor immune effects. However, abnormal activation of the innate immune system can induce various diseases, and the molecular mechanism of the cGAS/STING signaling pathway provides new idea for targeted drug development.

The cGAS/STING signaling pathway can be precisely regulated through physiological processes and drug methods, including protein post-translational modifications (phosphorylation, ubiquitination, etc.), small molecule antagonists (such as H-151, C-176), and agonists (such as DMXAA, MSA-2). When the cGAS/STING signaling pathway is abnormally activated or overactivated, it can cause inflammatory diseases and autoimmune diseases such as AGS syndrome, systemic lupus erythematosus (SLE), and amyotrophic lateral sclerosis (ALS). Therefore, unlike the immune killing effect of cGAS/STING activators on tumor cells, targeted inhibition of the cGAS/STING signaling pathway can be used for the treatment of such inflammatory diseases and autoimmune diseases.

Although the research on small molecule compounds targeted-inhibiting the cGAS/STING signaling pathway is still in its infancy, it has always been a hot topic in the field of medical physiology. In 2018, Haag *et al.* reported a class of nitrofuran and indolylurea compounds in *Nature* (Nature 2018, 559, 269-273.) that covalently bind to Cys91 on STING proteins to block palmitoylation induced by STING activation, thereby interfering with their assembly into multimeric complexes in the Golgi apparatus and inhibiting downstream signaling pathways. In addition, this type of inhibitor can also inhibit the secretion of inflammatory cytokines mediated by STING protein in mice cells to alleviate the pathological characteristics of mouse auto inflammatory diseases. In 2019, Lama *et al.* published a series of indolopyridine compounds (Nat. Commun. 2019, 10, 2261.) in *Nature Communication,* which can effectively inhibit cGAS activity in human bone marrow-derived macrophages (BMDM). In 2021, the team led by Wang Chen from China Pharmaceutical University reported the sulfonamide compound SN-011, which binds to the cyclic dinucleotide binding domain of STING protein and exhibits STING inhibitory activity. The compound can strongly inhibit inflammation diseases and autoimmune diseases in Trex1$^{-/-}$ mice (Proc. Natl. Acad. Sci. USA 2021, 118, e2105465118). Therefore, small molecule inhibitors targeting the cGAS/STING signaling pathway are expected to become important therapeutic drugs for autoimmune diseases.

In recent years, well-known pharmaceutical companies such as Novartis, Bayer, and Eli Lilly have joined the research and development of small molecule inhibitors targeting the cGAS/STING signaling pathway. Most of these research compounds are still at the stage of cellular activity validation, and there are currently no small molecule inhibitors in the clinical research stage. Therefore, there is an urgent need to develop small molecule inhibitors targeting the cGAS/STING signaling pathway with high activity and favorable druggability, in order to develop therapeutic drugs for autoimmune diseases.

### SUMMARY OF THE INVENTION

The object of the present invention is to provide small molecule inhibitors targeting the cGAS/STING signaling pathway with high activity and favorable druggability, which can be used as therapeutic drugs for autoimmune diseases.

In the first aspect of the present invention, provided is a compound represented by formula (I), or a prodrug, an enantiomer, a diastereomer, a racemate, and mixtures thereof, or a pharmaceutically acceptable salt thereof,

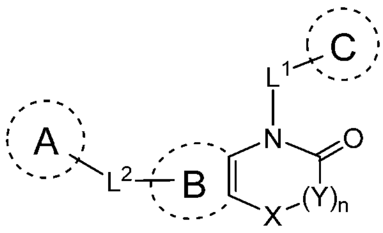

( I )

wherein, ring B is substituted or unsubstituted group selected from the group consisting of 6-membered aryl, 6-membered heteroaryl, and 5-membered heteroaryl, wherein the heteroatoms in the heteroaryl are selected from N, S and O; the number of heteroatoms is 1-3;

ring A and ring C are each independently substituted or unsubstituted group selected from the group consisting of 3-8-membered heterocyclyl, C3-C8 cycloalkyl, 5-membered heteroaryl, 6-membered aryl, 6-membered heteroaryl, and

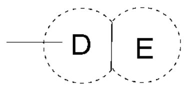,

wherein the substituted refers to being substituted by 1-5 $R^1$ groups; wherein, each $R^1$ is independently selected from the group consisting of hydrogen, halogen, carboxyl, hydroxyl, cyano, amino, -COO-C1-C6 alkyl, nitro, - CO-(substituted or unsubstituted 3-8-membered heterocyclyl), -CO-(substituted or unsubstituted C3-C8 cycloalkyl), substituted or unsubstituted C6-C12 aryl-C1-C6-alkyl, substituted or unsubstituted 5-12 membered heteroaryl-C1-C6-alkyl, substituted or unsubstituted C1-C6 alkyl, substituted or unsubstituted C2-C6 alkenyl, substituted or unsubstituted C2-C6 alkynyl, substituted or unsubstituted C1-C6 alkoxy, substituted or unsubstituted C1-C6 alkylacyl, substituted or unsubstituted aminoacyl, substituted or unsubstituted C1-C6 alkylamido, substituted or unsubstituted C1-C4 alkylamino, substituted or unsubstituted C6-C12 arylamino, substituted or unsubstituted 3-8-membered heterocyclyl, substituted or unsubstituted C3-C8 cycloalkyl, substituted or unsubstituted 5-12 membered heteroaryl, and substituted or unsubstituted C6-C12 aryl; the substitution in $R^1$ refers to being substituted by one or more groups selected from the group consisting of halogen, carboxyl, hydroxyl, cyano, amino, nitro, benzyl, C1-C6 alkyl-OH, C1-C6 alkylamino, C1-C6 alkyl, halogenated C1-C6 alkyl, C1-C6 alkoxy, halogenated C1-C6 alkoxy, C3-C8 cycloalkyl, and 3-8-membered heterocyclyl;

ring D and ring E are each independently C5-C6 cycloalkane, benzene ring, 5-6 membered heterocycloalkane, or 5-6 membered heteroaromatic ring;

$L^1$ is selected from the group consisting of -substituted or unsubstituted C1-C6 alkylene-, - (substituted or unsubstituted C1-C6 alkylene)-sulfonyl-, substituted or unsubstituted monocyclic or bicyclic C3-C6 cycloalkyl, -substituted or unsubstituted C2-C6 alkenylene-, -substituted or unsubstituted C2-C6 alkynylene-, -substituted or unsubstituted C1-C6 alkylene-O-, -substituted or unsubstituted C1-C6 alkylene-OCO-, -substituted or unsubstituted monocyclic or bicyclic C6-C12 aryl, substituted or unsubstituted monocyclic or bicyclic 5-10 membered heteroaryl, substituted or unsubstituted monocyclic or bicyclic 3-8 membered heterocyclyl,

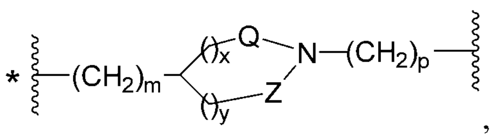, and 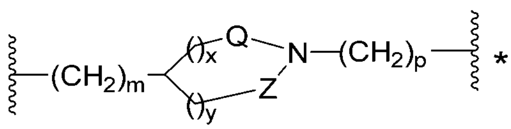;

wherein, m and p are each independently 0-3; x and y are each independently selected from 0-4; wherein "*" represents the connection to ring C;

$L^2$ is selected from the group consisting of

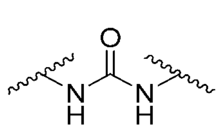, 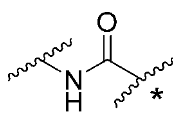, 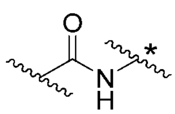,

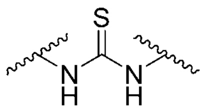, 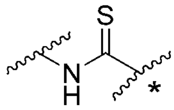, and 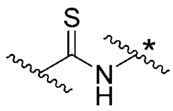,

wherein "*" represents the connection to ring B;
X and Y are each independently selected from the group consisting of S, SO, $SO_2$, O, $NR^2$, and $CR^3R^4$;
Q and Z are each independently selected from the group consisting of CO and a chemical bond;
with the proviso that when ring B is a 6-membered aryl, and $L^1$ is not

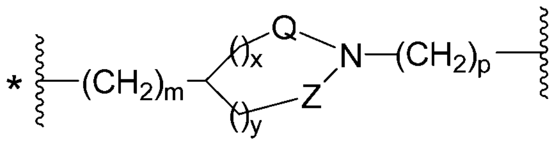, or 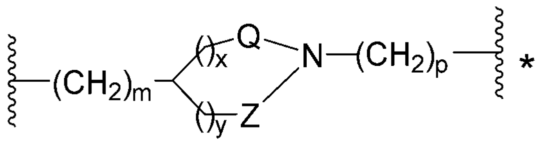,

$L^2$ is

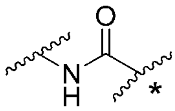, or 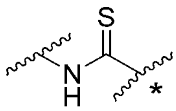,

wherein the "*" in

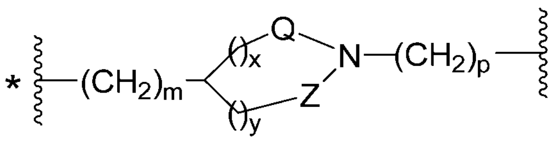,

and

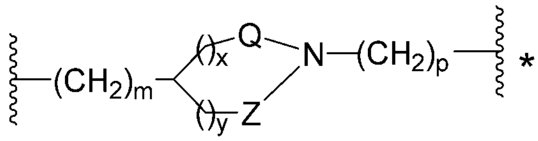

represents the connection to ring C, and the "*" in

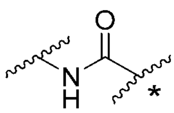 and 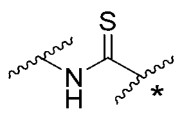

represents the connection to ring B;
$R^2$, $R^3$, and $R^4$ are each independently selected from the group consisting of hydrogen atom, halogen, carboxyl, hydroxyl, cyano, amino, nitro, benzyl, C1-C6 alkyl-OH, C1-C6 alkylamino, C1-C6 alkyl, halogenated C1-C6 alkyl, C1-C6 alkoxy, halogenated C1-C6 alkoxy, C3-C8 cycloalkyl, and 3-8-membered heterocyclyl; or $R^3$, $R^4$ and the C atom to which they are attached form a 3-5 membered cycloalkyl or heterocyclyl; or $R^3$, $R^4$ and the C atom to which they are attached form a C2-C6 alkenyl;
n is 0, or 1, wherein, when n is 0, X is directly connected to C=O;
the substituted refers to being substituted by one or more groups selected from the group consisting of halogen, carboxyl, hydroxyl, cyano, amino, nitro, benzyl, C1-C6 alkyl-OH, C1-C6 alkylamino, C1-C6 alkyl, halogenated C1-C6 alkyl, C1-C6 alkoxy, halogenated C1-C6 alkoxy, C3-C8 cycloalkyl, and 3-8-membered heterocyclyl.

In another preferred embodiment, ring B is selected from the following structures:

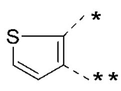 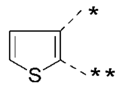 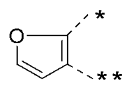 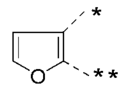 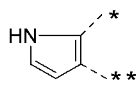 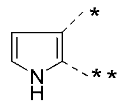

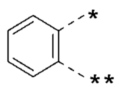 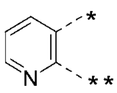 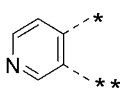 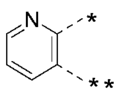 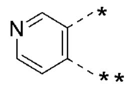 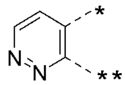

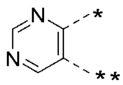 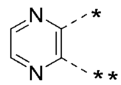

,

wherein, "*" represents the connection to C atom adjacent to N, and "**" represents the connection to C atom adjacent to X; $L^2$ is connected to any unsubstituted site of ring B.

In another preferred embodiment, the compound is not

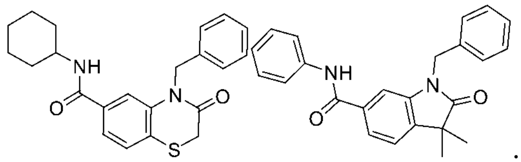

.

In another preferred embodiment, the compound has the structure shown in formulas II-VII:

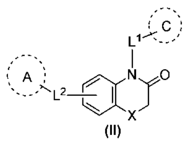
(II)
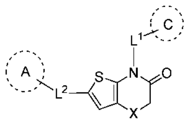
(III)
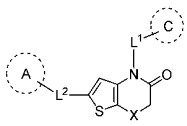
(IV)
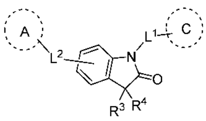
(V)
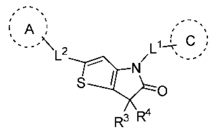
(VI)
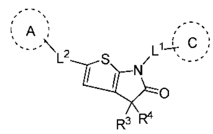
(VII)

wherein, the definitions of ring A, ring C, $R^3$, $R^4$, X, $L^1$, and $L^2$ are as described herein.

In another preferred embodiment, the compound has the structure shown in formulas II-VII:

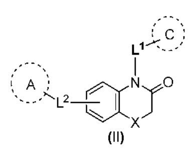
(II)
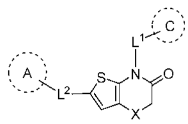
(III)
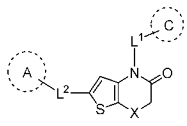
(IV)
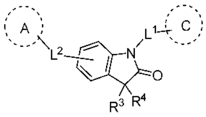
(V)
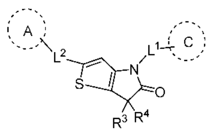
(VI)
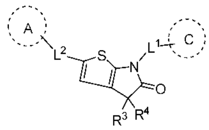
(VII)

wherein, the definitions of ring A, ring C, $L^1$, and $L^2$ are as described herein;
$R^3$ and $R^4$ are each independently C1-C6 alkyl, or halogen, or $R^3$, $R^4$ and the C atom to which they are attached form a 3-5 membered cycloalkyl or heterocyclyl; or $R^3$, $R^4$ and the C atom to which they are attached form a C2-C6 alkenyl;
X is selected from the group consisting of O and S atoms;
preferably, $R^3$ and $R^4$ are each independently methyl, ethyl, n-propyl, isopropyl, n-butyl, isobutyl, *tert-butyl,* fluorine, chlorine, or bromine, or $R^3$, $R^4$ and the C atom to which they are attached form cyclopropyl, cyclobutyl, cyclopentyl, or cyclohexyl; or $R^3$, $R^4$ and the C atom to which they are attached form vinyl, propenyl, or allyl;
X is selected from the group consisting of O and S atoms, preferably S atom.

In another preferred embodiment, the compound has the structure shown in formulas II-VII:

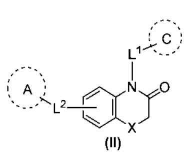
(II)
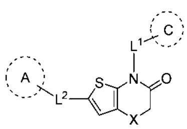
(III)
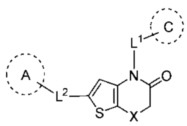
(IV)
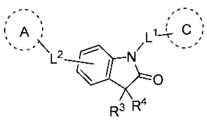
(V)
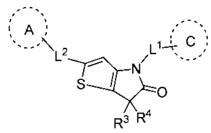
(VI)
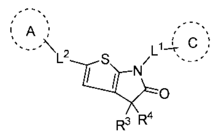
(VII)

wherein, the definitions of ring A, ring C, $L^1$, and $L^2$ are as described herein;
$R^3$ and $R^4$ are methyl;
X is selected from the group consisting of O and S atoms.

In another preferred embodiment, in formula II or formula V,
$L^1$ is selected from the group consisting of -substituted or unsubstituted C1-C6 alkylene-, - substituted or unsubstituted C2-C6 alkenylene-, -substituted or unsubstituted C2-C6 alkynylene-, -substituted or unsubstituted C1-C6 alkylene-O-,

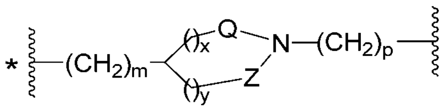,

and

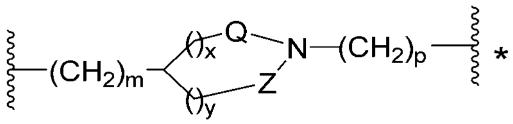;

with the proviso that when $L^1$ is not

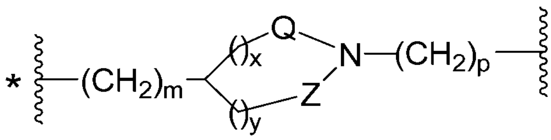, or 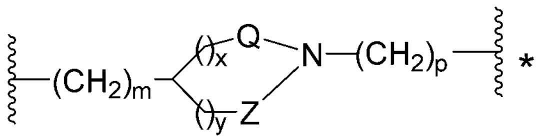,

$L^2$ is

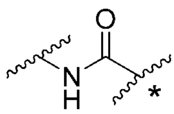, or 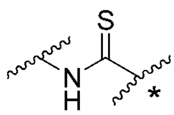,

wherein the "*" in

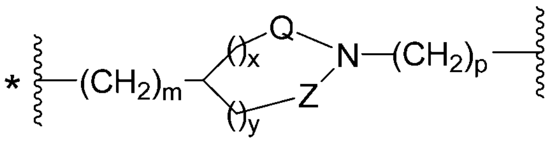

and

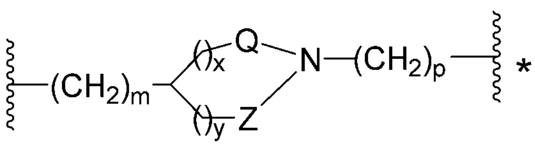

represents the connection to ring C, and the "*" in

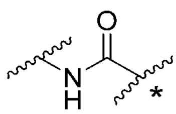 and 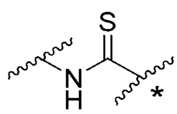

represents the connection to the mother nucleus, and the other end is connected to ring A.

In another preferred embodiment, in formula II or formula V,

$L^1$ is selected from the group consisting of -substituted or unsubstituted C1-C6 alkylene-(preferably -substituted or unsubstituted C1-C3 alkylene-),

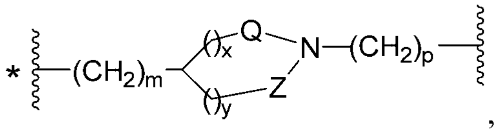

,

and

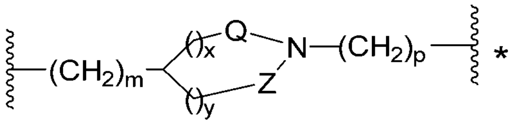

;

with the proviso that when $L^1$ is not

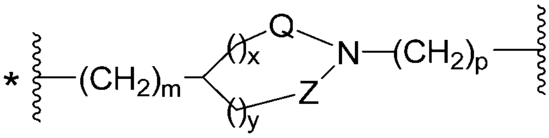

, or

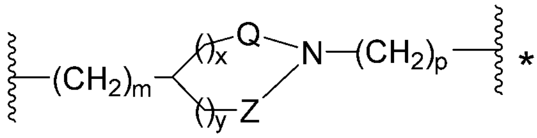

,

$L^2$ is

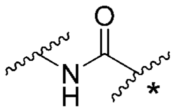

,

wherein the "*" in

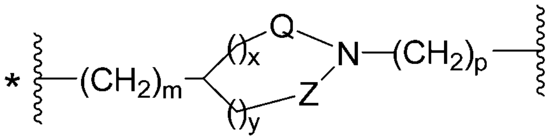

and

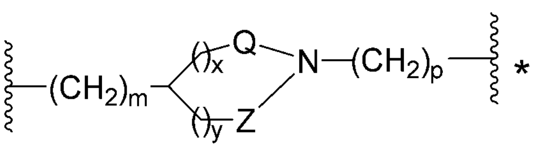

represents the connection to ring C, and the "*" in

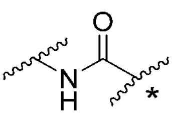

and

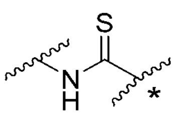

represents the connection to the mother nucleus, and the other end is connected to ring A.

In another preferred embodiment, in formula II or formula V,

$L^1$ is selected from the group consisting of -substituted or unsubstituted C1-C6 alkylene-, - substituted or unsubstituted C2-C6 alkenylene-, -substituted or unsubstituted C2-C6 alkynylene-, and -substituted or unsubstituted C1-C6 alkylene-O-;

$L^2$ is selected from the group consisting of

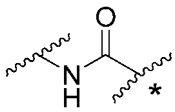 and 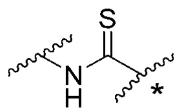,

wherein the "*" represents the connection to the mother nucleus, and the other end is connected to ring A.

In another preferred embodiment, in formula II or formula V,

$L^1$ is -substituted or unsubstituted C1-C6 alkylene- (preferably -substituted or unsubstituted C1-C3 alkylene-);

$L^2$ is

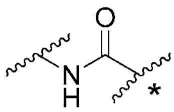,

wherein the "*" represents the connection to the mother nucleus, and the other end is connected to ring A.

In another preferred embodiment, in formula II or formula V, $L^1$ is selected from the group consisting of

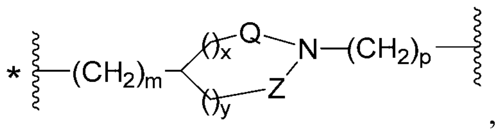, and 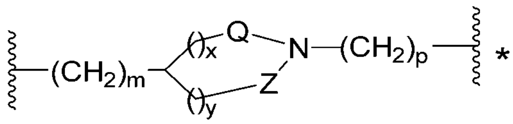,

wherein the "*" represents the connection to ring C.

In another preferred embodiment, in formula II or formula V, $L^1$ is

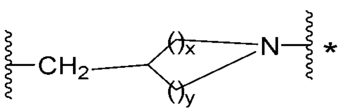, 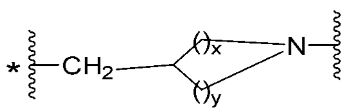, 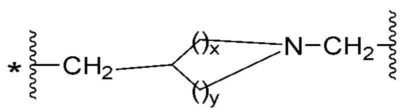,

or

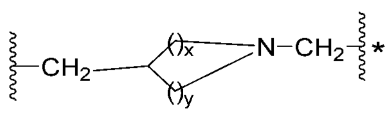,

wherein, x and y are each independently 0-2; wherein the "*" represents the connection to ring C.

In another preferred embodiment, in formula III or formula IV or formula VI or formula VII, $L^1$ is selected from the group consisting of -substituted or unsubstituted C1-C6 alkylene-, - substituted or unsubstituted C2-C6 alkenylene-, -substituted or unsubstituted C2-C6 alkynylene-, and -substituted or unsubstituted C1-C6 alkylene-O-.

In another preferred embodiment, in formula III or formula IV or formula VI or formula VII, $L^1$ is -substituted or unsubstituted C1-C6 alkylene- (preferably -substituted or unsubstituted C1-C3 alkylene-).

In another preferred embodiment, ring A is

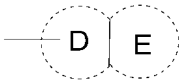.

In another preferred embodiment, when ring A is

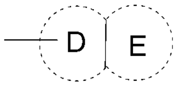,

ring D and ring E are each independently substituted or unsubstituted groups selected from the group consisting of benzene ring and 5-6 membered heteroaromatic ring.

In another preferred embodiment, when ring A is

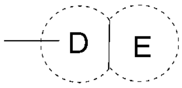,

ring D and ring E are each independently substituted or unsubstituted groups selected from the group consisting of benzene ring, pyrrole ring, furan ring, and thiophene ring.

In another preferred embodiment, when ring A is

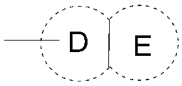,

one of ring D and ring E is a substituted or unsubstituted benzene ring, and the other is a substituted or unsubstituted group selected from the group consisting of pyrrole ring, furan ring, and thiophene ring.

In another preferred embodiment, ring A is substituted or unsubstituted group selected from the group consisting of

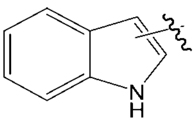, 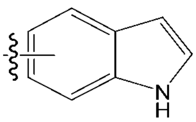, 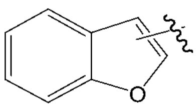, and 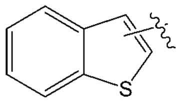.

In another preferred embodiment, ring A is substituted or unsubstituted group selected from the group consisting of

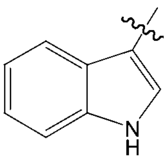, 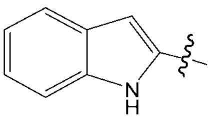, 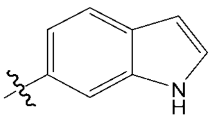, 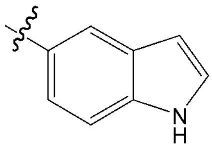,

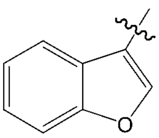, 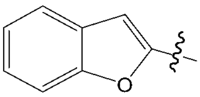, 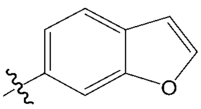, 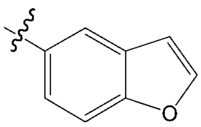, 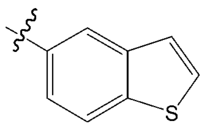,

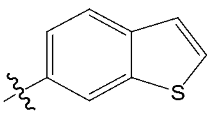, 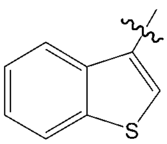, and 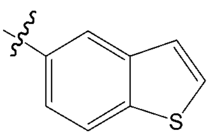.

In another preferred embodiment, ring A is substituted or unsubstituted group selected from the group consisting of

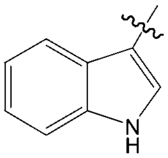, 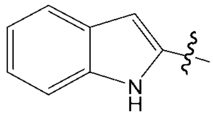, 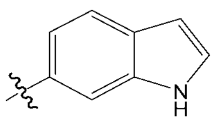, 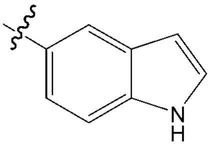,

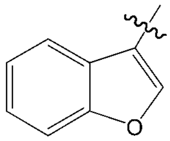, and 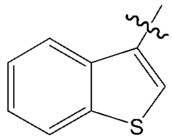.

In another preferred embodiment, ring A is halogen substituted (preferably F substituted) or unsubstituted group selected from the group consisting of

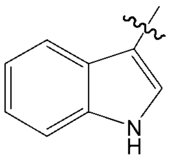, 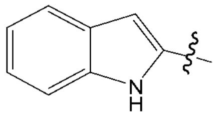,

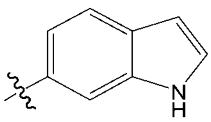, and 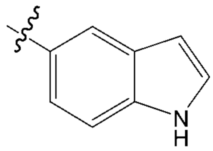.

In another preferred embodiment, ring C is substituted or unsubstituted group selected from the group consisting of 3-6-membered heterocyclyl, C3-C6 cycloalkyl, 6-membered aryl, 6-membered heteroaryl, 5-membered heteroaryl, and

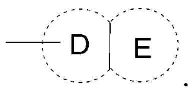.

In another preferred embodiment, ring C is substituted or unsubstituted group selected from the group consisting of C3-C6 cycloalkyl, 6-membered aryl, 6-membered heteroaryl, 5-membered heteroaryl, and

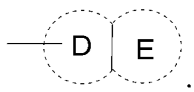.

In another preferred embodiment, when ring C is

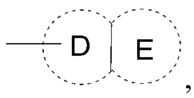,

ring D and ring E are each independently substituted or unsubstituted groups selected from the group consisting of benzene ring, 5-6 membered heterocycloalkane, and 5-6 membered heteroaromatic ring.

In another preferred embodiment, when ring C is

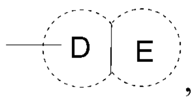,

ring D and ring E are each independently substituted or unsubstituted groups selected from the group consisting of benzene ring, 5-membered heterocycloalkane, and 5-membered heteroaromatic ring.

In another preferred embodiment, when ring C is

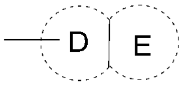,

one of ring D and ring E is a substituted or unsubstituted benzene ring, and the other is a substituted or unsubstituted group selected from the group consisting of 5-membered heterocycloalkane, and 5-membered heteroaromatic ring.

In another preferred embodiment, when ring C is

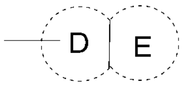,

one of ring D and ring E is a substituted or unsubstituted benzene ring, and the other is a substituted or unsubstituted group selected from the group consisting of dioxolane, dioxane, thiophene, furan, pyrrole, pyrazole, imidazole, oxazole, and isoxazole.

In another preferred embodiment, ring C is substituted or unsubstituted group selected from the group consisting of phenyl, thienyl, furyl, pyrrolyl, pyridinyl, pyrazolyl, imidazolyl, oxazolyl, isoxazolyl, cyclopropylalkyl, cyclobutanyl, cyclopentyl, benzofuryl, benzothienyl, indolyl,

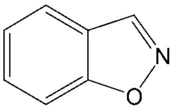, 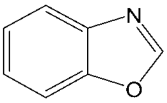, 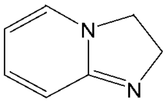, and 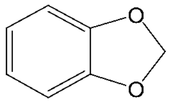.

In another preferred embodiment, ring C is substituted or unsubstituted group selected from the group consisting of

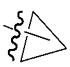, 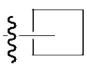, 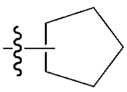, 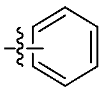, 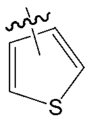, 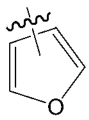, 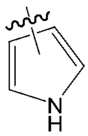, 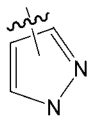,

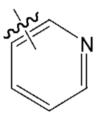, 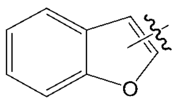, 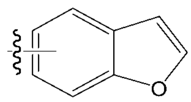, 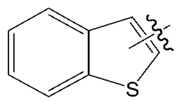, 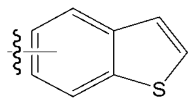, 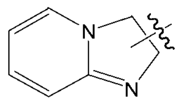,

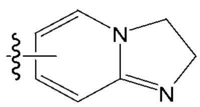, 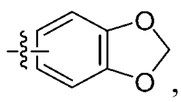, 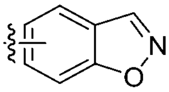, and 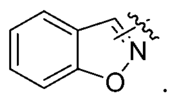.

In another preferred embodiment, $R^1$ is selected from the group consisting of halogen, hydroxyl, amino, C1-C6 alkyl, C2-C6 alkenyl, C1-C6 haloalkyl, C1-C6 alkoxy, C1-C6 alkylhydroxyl, C1-C4 alkylamino, and C6 aryl.

In another preferred embodiment, $R^1$ is selected from the group consisting of halogen, C1-C6 alkyl, C1-C6 haloalkyl, C1-C6 alkoxy, and C6 aryl.

In another preferred embodiment, $R^1$ is selected from the group consisting of fluorine, chlorine, bromine, methyl, ethyl, n-propyl, isopropyl, n-butyl, isobutyl, *tert-butyl,* vinyl, propenyl, allyl, trifluoromethyl, trichloromethyl, pentafluoroethyl, methoxy, ethoxy, n-propoxy, isopropoxy, n-butoxy, isobutoxy, tert-butoxy, and phenyl.

In another preferred embodiment, $L^1$ is selected from the group consisting of -substituted or unsubstituted C1-C6 alkylene-, -substituted or unsubstituted C2-C6 alkenylene-, -substituted or unsubstituted C1-C6 alkylene-O-,

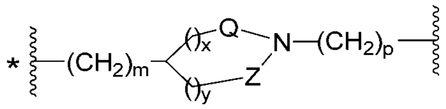
,

and

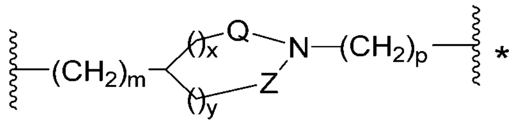
;

wherein, m and p are each independently 0-3; x and y are each independently selected from 0-4; Q and Z are each independently selected from the group consisting of CO and a chemical bond; wherein "*" represents the connection to ring C.

In another preferred embodiment, $L^1$ is selected from the group consisting of -substituted or unsubstituted C1-C3 alkylene-.

In another preferred embodiment, when $L^1$ is -substituted or unsubstituted C1-C6 alkylene-(preferably -substituted or unsubstituted C1-C3 alkylene-), $L^2$ is selected from the group consisting of

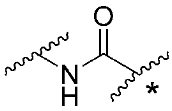
, 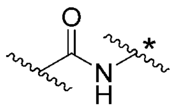
, 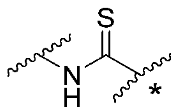
, and 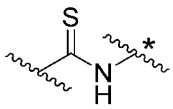
,

wherein the "*" represents the connection to ring B.

In another preferred embodiment, $L^1$ is selected from the group consisting of

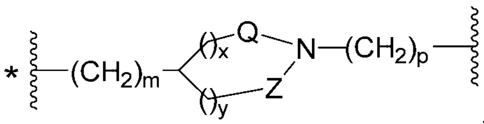
, and 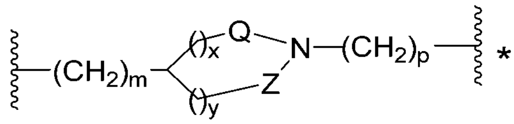
,

wherein the "*" represents the connection to ring C.

In another preferred embodiment, m and p are each independently 0,1, 2, or 3.

In another preferred embodiment, x and y are each independently 0,1, or 2.

In another preferred embodiment, $L^1$ is selected from the group consisting of

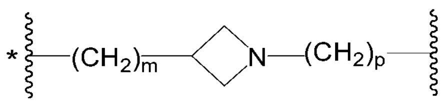
, 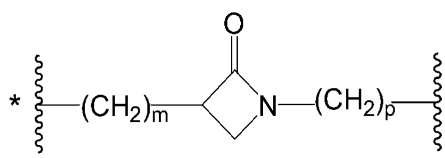
,

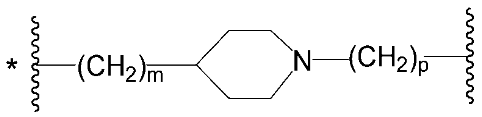
, 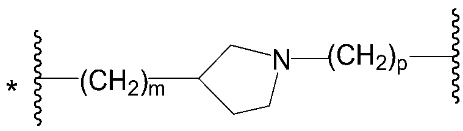
,

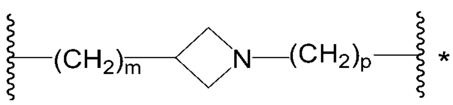
, 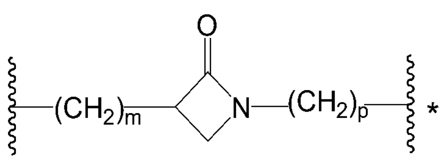
,

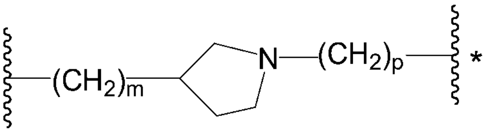, and 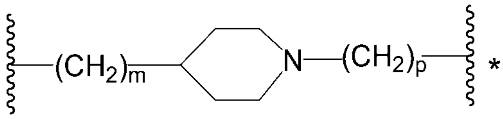,

wherein the "*" represents the connection to ring C.

In another preferred embodiment, $L^1$ is

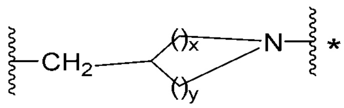,

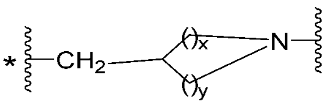, 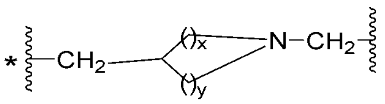, or 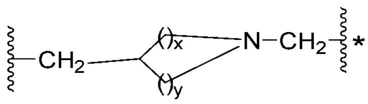,

wherein the "*" represents the connection to ring C.

In another preferred embodiment, $R^2$, $R^3$, and $R^4$ are each independently selected from the group consisting of hydrogen atom, halogen, hydroxyl, amino, C1-C4 alkyl-OH, C1-C4 alkylamino, C1-C4 alkyl, halogenated C1-C4 alkyl, C1-C4 alkoxy, and halogenated C1-C4 alkoxy; or $R^3$, $R^4$ and the C atom to which they are attached form a 3-5 membered cycloalkyl or heterocyclyl; or $R^3$, $R^4$ and the C atom to which they are attached form a C2-C4 alkenyl.

In another preferred embodiment, $R^2$, $R^3$, and $R^4$ are each independently selected from the group consisting of hydrogen atom, halogen, C1-C4 alkyl, halogenated C1-C4 alkyl, and C1-C4 alkoxy; or $R^3$, $R^4$ and the C atom to which they are attached form a 3-5 membered cycloalkyl or heterocyclyl; or $R^3$, $R^4$ and the C atom to which they are attached form a C2-C4 alkenyl.

In another preferred embodiment, $R^2$, $R^3$, and $R^4$ are each independently selected from the group consisting of C1-C6 alkyl, and halogen; or $R^3$, $R^4$ and the C atom to which they are attached form a 3-5 membered cycloalkyl or heterocyclyl; or $R^3$, $R^4$ and the C atom to which they are attached form a C2-C4 alkenyl.

In another preferred embodiment, $R^2$, $R^3$, and $R^4$ are each independently selected from the group consisting of methyl, ethyl, *n*-propyl, isopropyl, *n*-butyl, isobutyl, *tert-butyl,* fluorine, chlorine, and bromine, or $R^3$, $R^4$ and the C atom to which they are attached form cyclopropyl, cyclobutyl, cyclopentyl, or cyclohexyl; or $R^3$, $R^4$ and the C atom to which they are attached form vinyl, propenyl, or allyl.

In another preferred embodiment, $R^3$ and $R^4$ are each independently selected from the group consisting of methyl, ethyl, and chlorine, or $R^3$, $R^4$ and the C atom to which they are attached form cyclopropyl, or vinyl.

In another preferred embodiment, $R^3$, and $R^4$ are methyl.

In another preferred embodiment, X is S, O, or $CR^3R^4$, wherein $R^3$ and $R^4$ are each independently selected from the group consisting of methyl, ethyl, and chlorine, or $R^3$, $R^4$ and the C atom to which they are attached form cyclopropyl, or vinyl.

In another preferred embodiment, X is S, or $CR^3R^4$, wherein $R^3$, and $R^4$ are methyl.

In another preferred embodiment, n is 0 or 1, wherein when n is 0, X is directly connected to C=O, when n is 1, Y is $CR^3R^4$, wherein $R^3$ and $R^4$ are H.

In another preferred embodiment, the definitions of ring A, ring B, ring C, X, Y, $L^1$, $L^2$ and n are each independently the corresponding groups in compound **I-1** to compound **I-129.**

In another preferred embodiment, the compound has the structure shown in formulas II-VII:

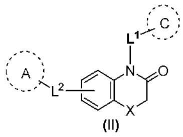
(II)

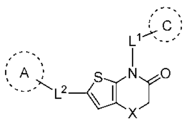
(III)

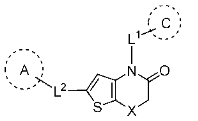
(IV)

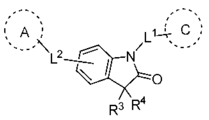
(V)

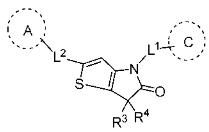
(VI)

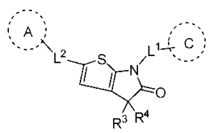
(VII)

wherein, ring A is

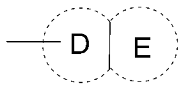,

wherein ring D and ring E are each independently substituted or unsubstituted groups selected from the group consisting of benzene ring and 5-6 membered heteroaromatic ring; the substituted refers to being substituted by 1-5 $R^1$ groups, wherein, $R^1$ is selected from the group consisting of hydrogen, halogen, hydroxyl, amino, C1-C6 alkyl, C2-C6 alkenyl, C1-C6 haloalkyl, C1-C6 alkoxy, C1-C6 alkylhydroxyl, and C1-C4 alkylamino;

ring C is substituted or unsubstituted group selected from the group consisting of 3-6 membered heterocyclyl, C3-C6 cycloalkyl, 6-membered aryl, 6-membered heteroaryl, 5-membered heteroaryl, and

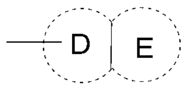,

wherein ring D and ring E are each independently substituted or unsubstituted groups selected from the group consisting of benzene ring, 5-6 membered heterocycloalkane, and 5-6 membered heteroaromatic ring; the substituted refers to being substituted by 1-5 $R^1$ groups, wherein, $R^1$ is selected from the group consisting of hydrogen, halogen, hydroxyl, amino, C1-C6 alkyl, C2-C6 alkenyl, C1-C6 haloalkyl, C1-C6 alkoxy, C1-C6 alkylhydroxyl, C1-C4 alkylamino, and C6 aryl;

$L^1$ is selected from the group consisting of -substituted or unsubstituted C1-C6 alkylene-, - substituted or unsubstituted C2-C6 alkenylene-, -substituted or unsubstituted C1-C6 alkylene-O-,

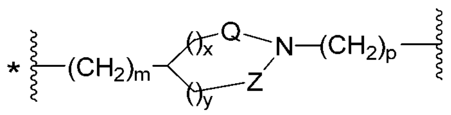, and 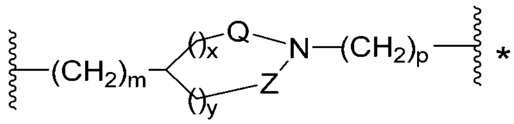;

wherein, m and p are each independently 0, 1, 2, or 3; x and y are each independently selected from 0, 1, 2, 3, or 4; Q and Z are each independently selected from the group consisting of CO and a chemical bond; wherein "*" represents the connection to ring C;

$L^2$ is selected from the group consisting of

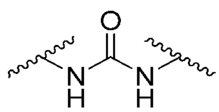, 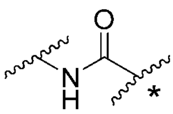, 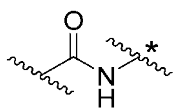,

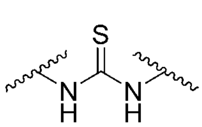, 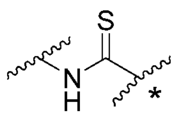, and 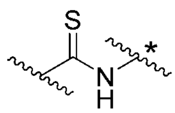,

wherein the "*" in

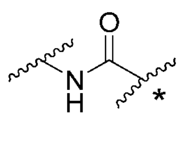,

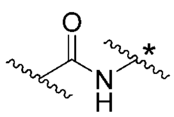, 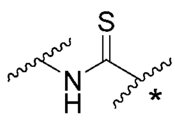, or 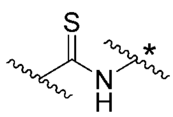

represents the connection to the mother nucleus and the other end is connected to ring A;

$R^3$ and $R^4$ are each independently selected from the group consisting of C1-C6 alkyl, and halogen; or $R^3$, $R^4$ and the C atom to which they are attached form a 3-5 membered cycloalkyl or heterocyclyl; or $R^3$, $R^4$ and the C atom to which they are attached form a C2-C6 alkenyl;

X is selected from the group consisting of O and S atoms;

with the proviso that, in formula II or formula V, when $L^1$ is not

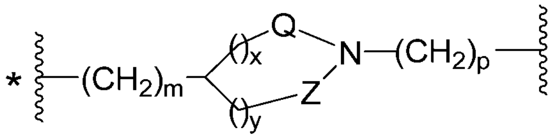
or
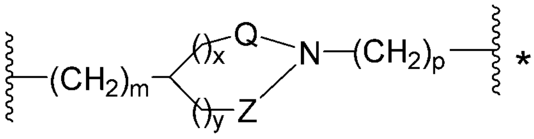
,

$L^2$ is

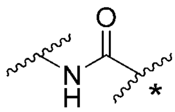
or
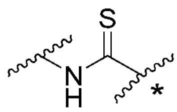
,

wherein the "*" in

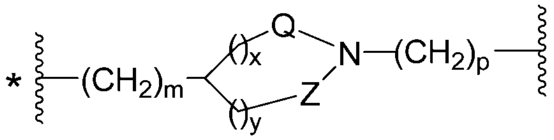

and

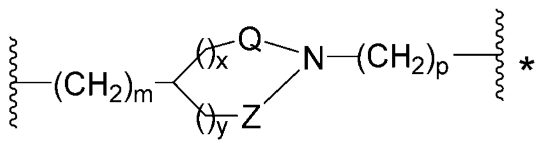

represents the connection to ring C, and the "*" in

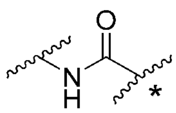
and
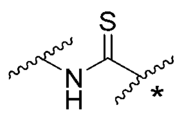

represents the connection to the mother nucleus and the other end is connected to ring A;

the substituted refers to being substituted by one or more groups selected from the group consisting of halogen, carboxyl, hydroxyl, cyano, amino, nitro, benzyl, C1-C6 alkyl-OH, C1-C6 alkylamino, C1-C6 alkyl, halogenated C1-C6 alkyl, C1-C6 alkoxy, halogenated C1-C6 alkoxy, C3-C8 cycloalkyl, and 3-8-membered heterocyclyl.

In another preferred embodiment, the compound has the structure shown in formulas II-VII:

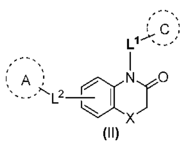
(II)
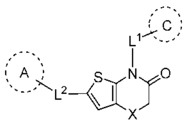
(III)
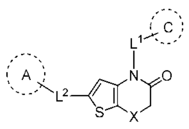
(IV)
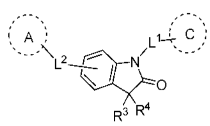
(V)
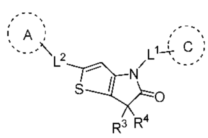
(VI)
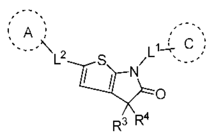
(VII)

wherein, ring A is

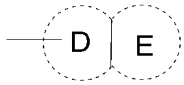,

wherein one of ring D and ring E is a substituted or unsubstituted benzene ring, and the other is a substituted or unsubstituted group selected from the group consisting of pyrrole ring, furan ring, and thiophene ring; the substituted refers to being substituted by 1-5 $R^1$ groups, wherein, $R^1$ is halogen, C1-C6 alkyl, C1-C6 haloalkyl, or C1-C6 alkoxy;
ring C is substituted or unsubstituted group selected from the group consisting of 3-6-membered heterocyclyl, C3-C6 cycloalkyl, 6-membered aryl, 6-membered heteroaryl, 5-membered heteroaryl, and

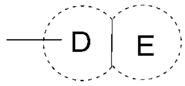,

wherein one of ring D and ring E is a substituted or unsubstituted benzene ring, and the other is a substituted or unsubstituted group selected from the group consisting of 5-membered heterocycloalkane, and 5-membered heteroaromatic ring; the substituted refers to being substituted by 1-5 $R^1$ groups, wherein, $R^1$ is selected from the group consisting of halogen, C1-C6 alkyl, C1-C6 haloalkyl, C1-C6 alkoxy, and C6 aryl;
$L^1$ is selected from the group consisting of - C1-C6 alkylene-,

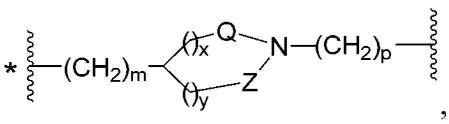, and 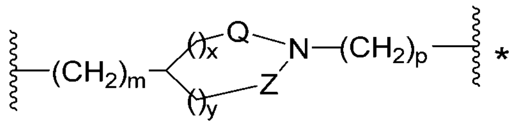;

wherein, m and p are each independently 0, 1, or 2; x and y are each independently selected from 0, 1, or 2; Q and Z are each independently selected from the group consisting of CO and a chemical bond; wherein "*" represents the connection to ring C;
$L^2$ is selected from the group consisting of

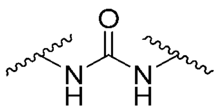, 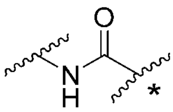, 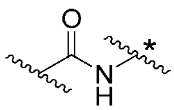,

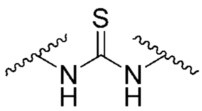, 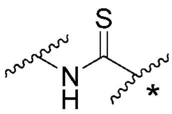, and 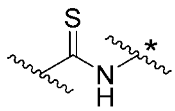,

wherein the "*" in

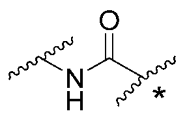,

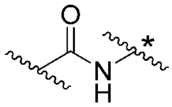, 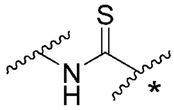, or 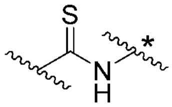

represents the connection to the mother nucleus;
$R^3$ and $R^4$ are each independently C1-C6 alkyl;
X is S atom;

with the proviso that, in formula II or formula V, when $L^1$ is not

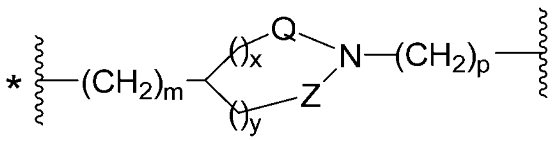

or

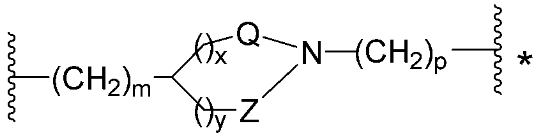

,

$L^2$ is

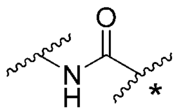

or

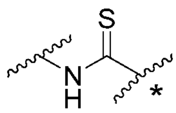

,

wherein the "*" in

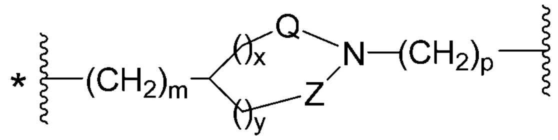

and

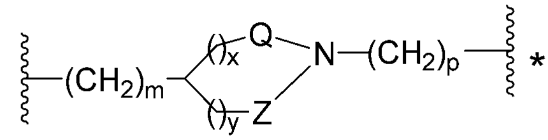

represents the connection to ring C, and the "*" in

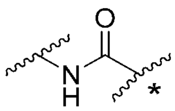

and

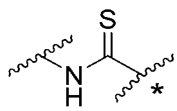

represents the connection to the mother nucleus and the other end is connected to ring A.

In another preferred embodiment, the compound has the structure shown in formula II or formula IV or formula V or formula VI:

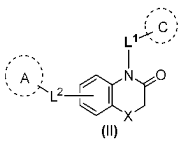

(II)

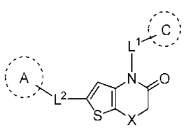

(IV)

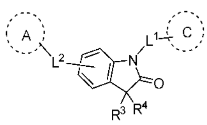

(V)

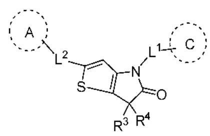

(VI)

wherein, ring A is substituted or unsubstituted group selected from the group consisting of

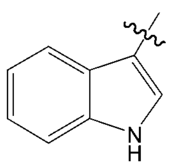

,

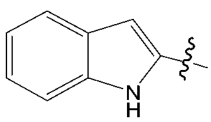

,

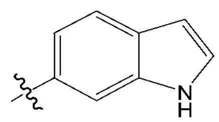

, and

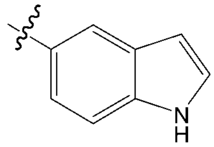

;

the substituted refers to being substituted by 1-5 $R^1$ groups, wherein, $R^1$ is selected from the group consisting of

halogen, C1-C6 alkyl, C1-C6 haloalkyl, and C1-C6 alkoxy;
ring C is substituted or unsubstituted group selected from the group consisting of 3-6-membered heterocyclyl, C3-C6 cycloalkyl, 6-membered aryl, 6-membered heteroaryl, 5-membered heteroaryl, and

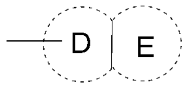,

wherein one of ring D and ring E is a substituted or unsubstituted benzene ring, and the other is a substituted or unsubstituted group selected from the group consisting of 5-membered heterocycloalkane, and 5-membered heteroaromatic ring; the substituted refers to being substituted by 1-5 $R^1$ groups, wherein, $R^1$ is selected from the group consisting of halogen, C1-C6 alkyl, C1-C6 haloalkyl, C1-C6 alkoxy, and C6 aryl;
$L^1$ is selected from the group consisting of -C1-C6 alkylene-,

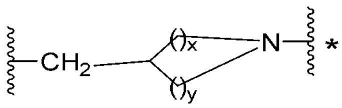,

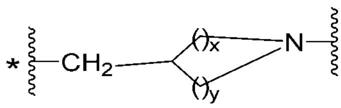, 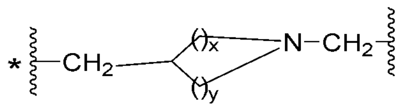

, and

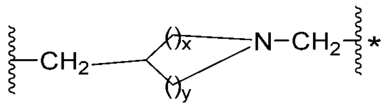,

wherein, x and y are each independently selected from 0, 1, or 2; wherein "*" represents the connection to ring C;
$L^2$ is selected from the group consisting of

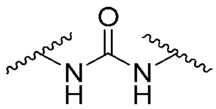 and 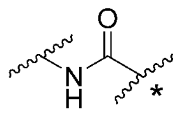,

the "*" in

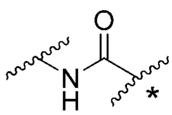

represents the connection to the mother nucleus, and the other end is connected to ring A;
$R^3$ and $R^4$ are each independently C1-C6 alkyl;
X is S atom;
with the proviso that, in formula II or formula V, when $L^1$ is not

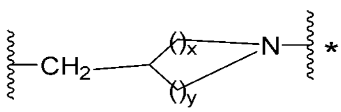,

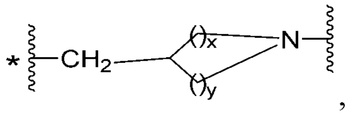, 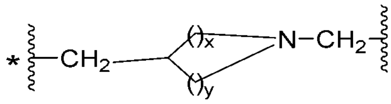, or 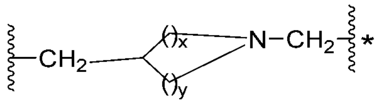,

$L^2$ is

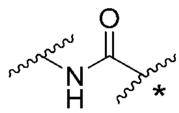,

wherein the "*" in

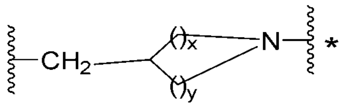, 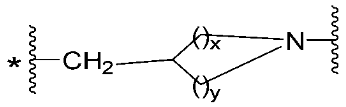,

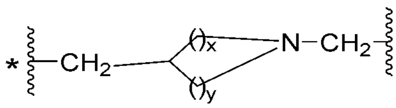, or 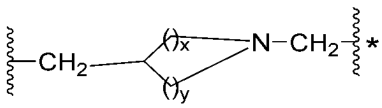

represents the connection to ring C, and the "*" in

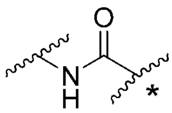

represents the connection to the mother nucleus and the other end is connected to ring A.

In another preferred embodiment, the compound has the following structure:

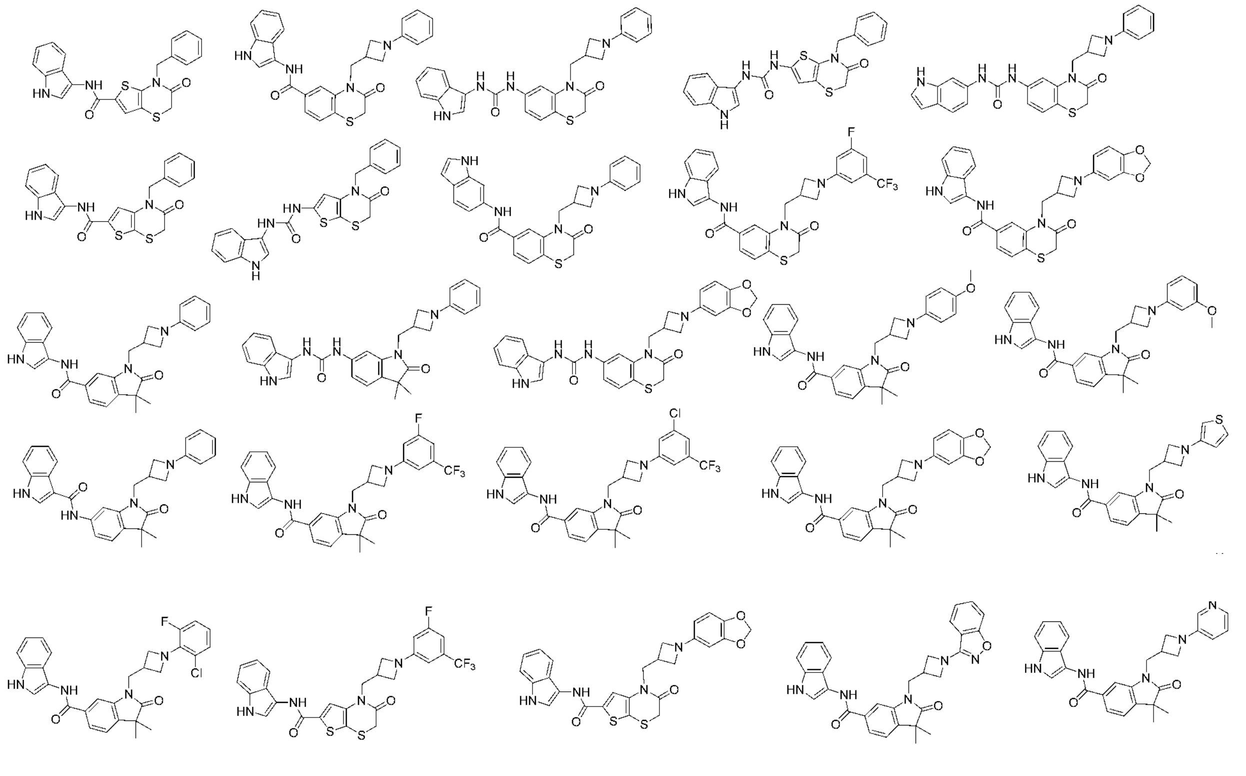

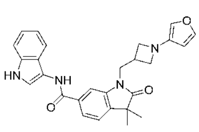
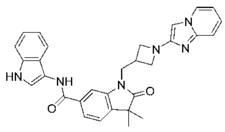
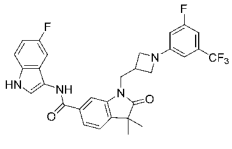
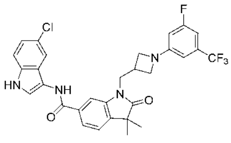
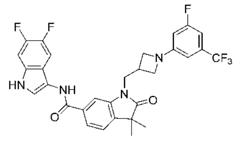

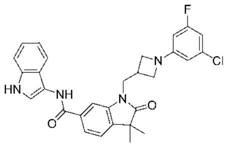
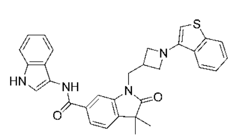
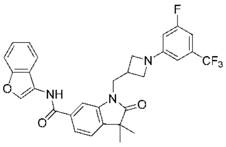
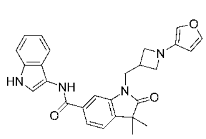
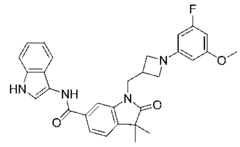

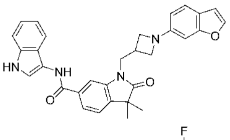
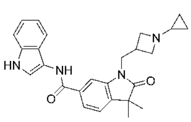
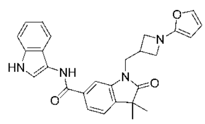
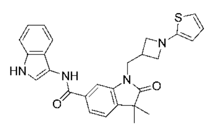
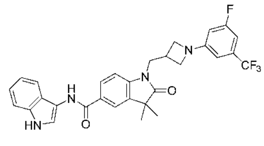

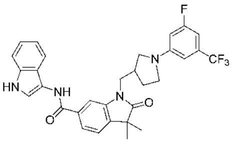
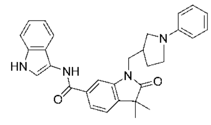
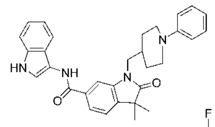
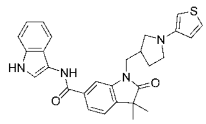
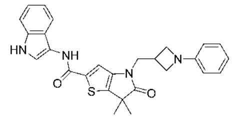

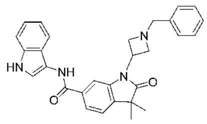
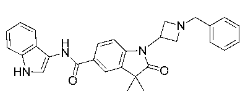
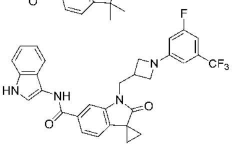
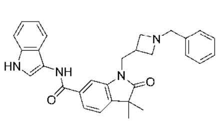
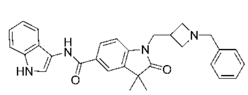

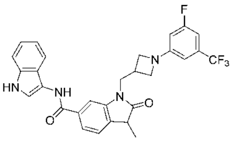
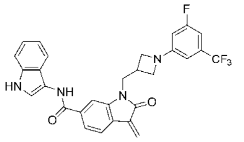
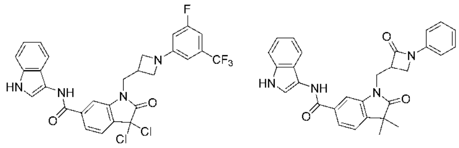

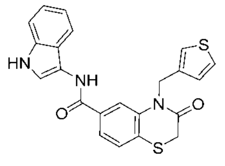
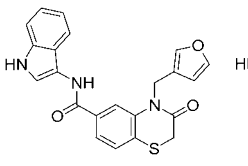
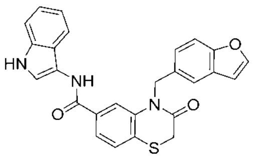
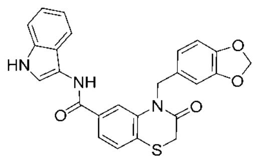
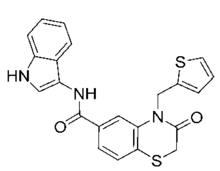

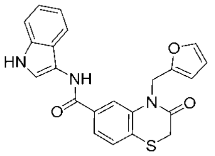
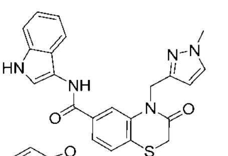
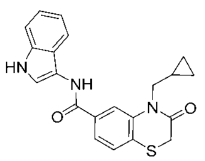
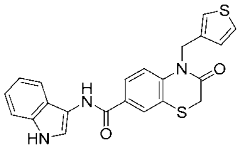
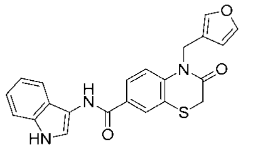

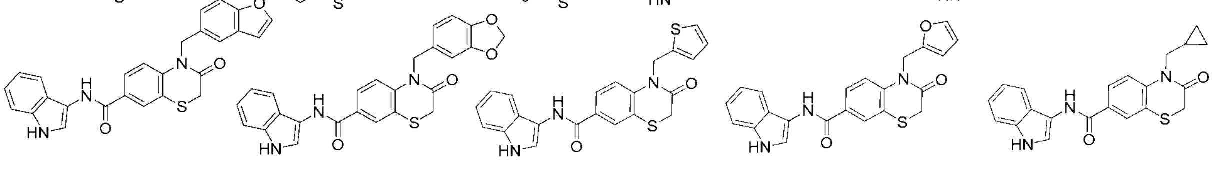

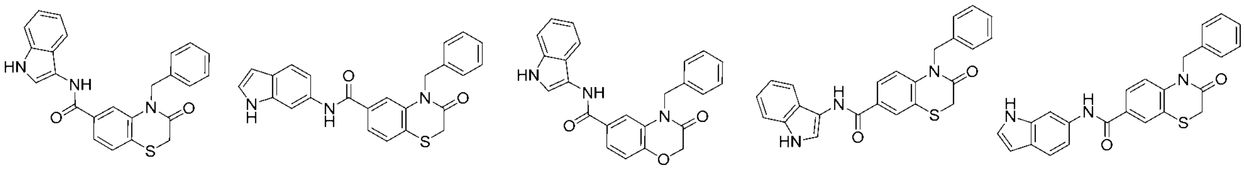

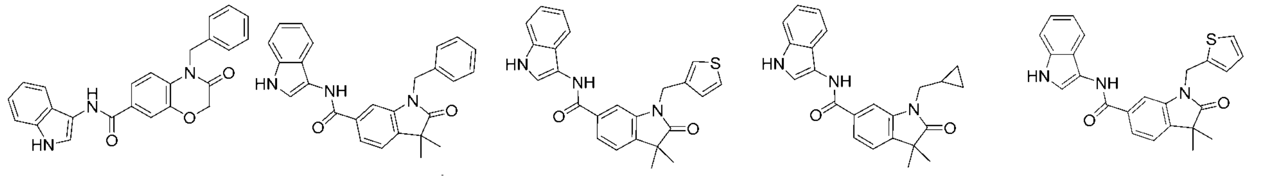

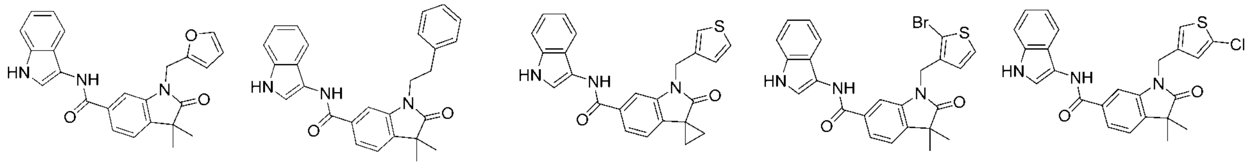

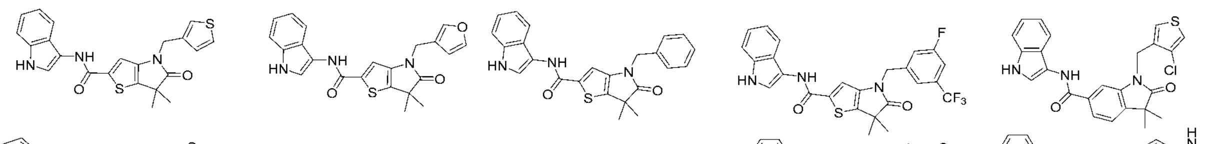

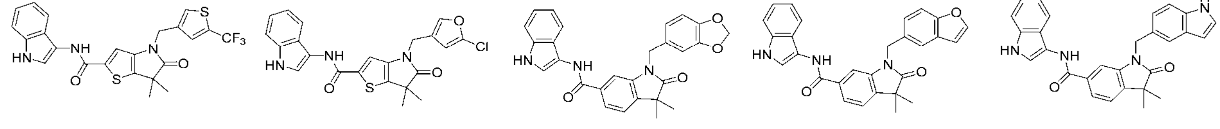

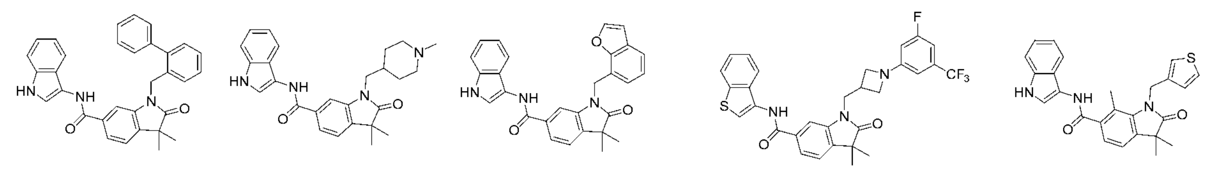

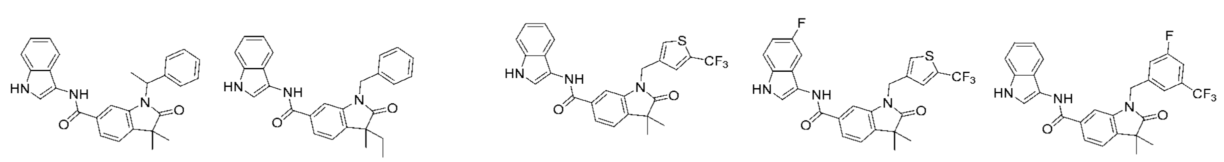

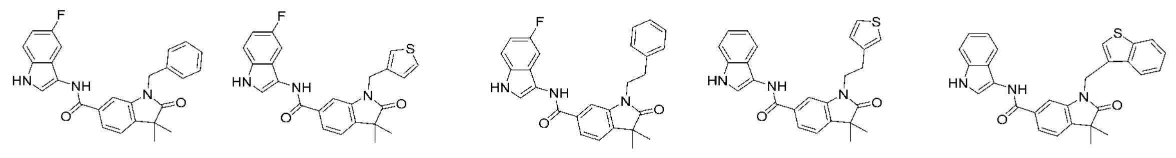

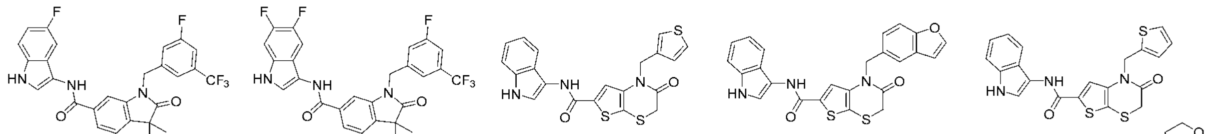

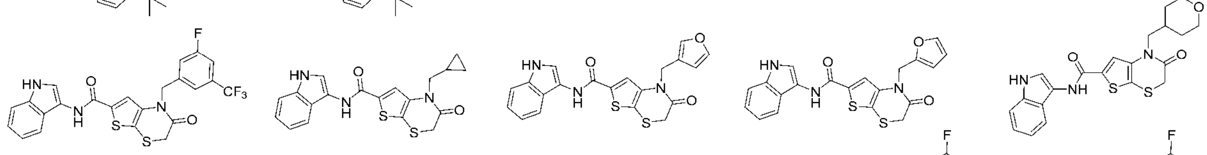

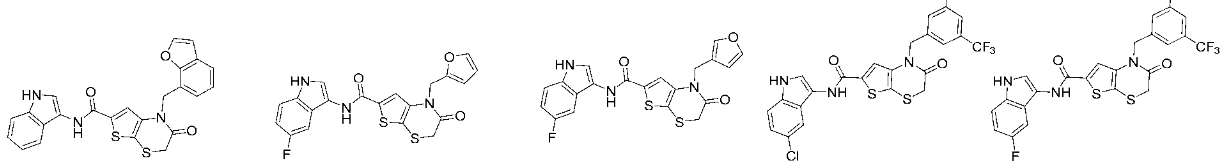

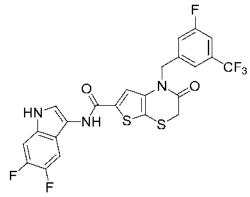 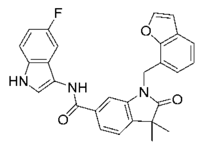 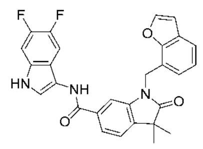 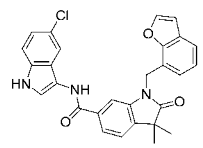 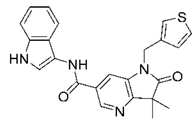 .

In the second aspect of the present invention, provided is a method for preparing the compound as described in the first aspect of the present invention, or the prodrug, the enantiomer, the diastereomer, the racemate, and the mixtures thereof, or the pharmaceutically acceptable salt thereof, the method comprises the following steps:

method 1:

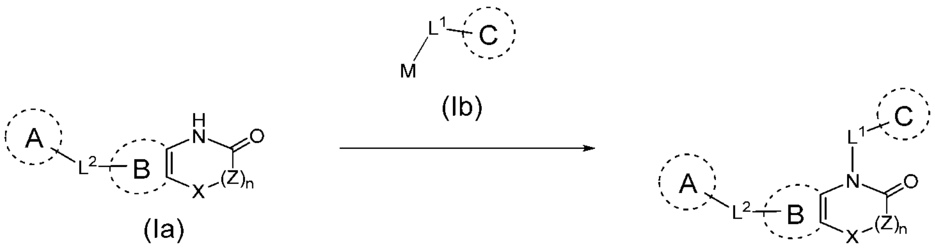
(Ib)
(Ia)
( I )

1) intermediate (Ia) undergoes nucleophilic substitution reaction with intermediate (Ib) in the presence of sodium hydroxide, sodium carbonate, or cesium carbonate to obtain the compound shown in formula (I); wherein, M is selected from the group consisting of chlorine, bromine, iodine, or methylsulfonate group (MsO);

method 2:

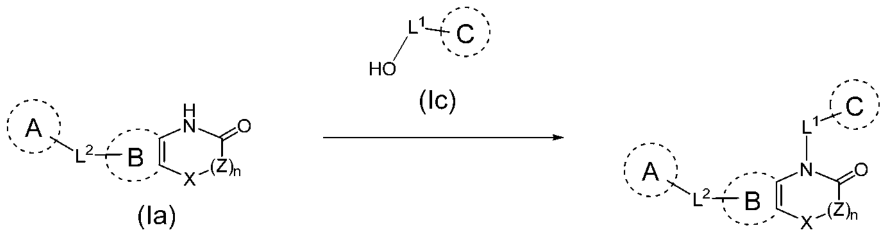
(Ic)
(Ia)
( I )

2) intermediate (Ia) and intermediate (Ic) undergo Mitsunobu reaction in the presence of triphenylphosphine and diisopropyl azodicarboxylate to obtain the compound shown in formula (I).

In the third aspect of the present invention, provided is a pharmaceutical composition comprising a therapeutically effective amount of one or more compounds selected from the group consisting of the compound as described in the first aspect, or the prodrug, the enantiomer, the diastereomer, the racemate, and the mixtures thereof, or the pharmaceutically acceptable salt thereof; and optionally pharmaceutically acceptable carriers.

In the fourth aspect of the present invention, provided is use of the compound as described in the first aspect, or the prodrug, the enantiomer, the diastereomer, the racemate, and the mixtures thereof, or the pharmaceutically acceptable salt thereof, or the pharmaceutical composition as described in the second aspect, for the preparation of cGAS/STING pathway targeted inhibitors; or for the preparation of drugs for preventing and/or treating inflammatory diseases and/or autoimmune diseases.

In another preferred embodiment, the inflammatory disease is selected from the group consisting of inflammatory bowel disease, Crohn's disease, ulcerative colitis, intestinal malabsorption syndrome, irritable bowel syndrome, uveitis, mucositis, diabetes, cardiovascular disease and neurodegenerative disease.

In another preferred embodiment, the autoimmune disease is selected from the group consisting of Singleton-Merten syndrome (SMS), Aicardi-Goutières syndrome (AGS), systemic lupus erythematosus (SLE), amyotrophic lateral sclerosis (ALS), familial chilblain lupus erythematosus (FCL), multiple sclerosis, autoimmune colitis, retinal vasculopathy with cerebral leukoencephalopathy (RVCL), STING-associated vasculopathy with onset in infancy (SAVI), scleroderma,

psoriasis, Sjogren's syndrome, and rheumatoid arthritis.

In the fifth aspect of the present invention, provided is a cGAS/STING pathway-targeted inhibitor, comprising the compound as described in the first aspect of the present invention, or the prodrug, the enantiomer, the diastereomer, the racemate, and the mixtures thereof, or the pharmaceutically acceptable salt thereof.

In the sixth aspect of the present invention, provided is a method for inhibiting the activity of STING protein, wherein the compound as described in the first aspect is contacted with cells capable of expressing STING protein, and the secretion of interferon can be inhibited by inhibiting the activity of STING protein.

In another preferred embodiment, the method is an *in vitro,* non-diagnostic, and non-therapeutic method.

In another preferred embodiment, the cells are of human and/or mouse origin.

In another preferred embodiment, the cells are THP1-Blue-ESG cells, THP1-Dual cells, and/or Raw-Lucia cells containing interferon stimulated gene(ISG) reporter system.

In the seventh aspect of the present invention, provided is a method for preventing and/or treating inflammatory diseases and/or autoimmune diseases, comprising the steps of: administering the compound as described in the first aspect of the present invention, or the prodrug, the enantiomer, the diastereomer, the racemate, and the mixtures thereof, or the pharmaceutically acceptable salt thereof to a subject in need thereof.

In another preferred embodiment, the subject is a human or non-human mammal, such as a mouse, rat, rabbit, chimpanzee, pig, dog, or sheep.

It should be understood that within the scope of the present invention, the above-described technical features of the present invention and the technical features described in detail below (e.g., examples) may be combined with each other to constitute a new or preferred technical solution. Limited by space, it will not be repeated here.

## DESCRIPTION OF THE DRAWINGS

Figure 1 shows the secretion levels of cytokines CXCL10 and IL6 in plasma after injection administration in Example 133.

Figure 2 shows the changes in cytokine gene expression in mice kidney tissues after injection administration in Example 133.

Figure 3 shows the changes in cytokine gene expression in mice heart tissues after injection administration in Example 133.

Figure 4 shows the secretion levels of cytokines CXCL10 and IL6 in mice plasma after oral administration in Example 134.

Figure 5 shows the changes in cytokine gene expression in mice kidney tissues after oral administration in Example 134.

Figure 6 shows the changes in cytokine gene expression in mice heart tissues after oral administration in Example 134.

Figure 7 shows the survival curve of mice in Example 135.

Figure 8 shows the changes in cytokine gene expression in mice kidney tissues in Example 135.

Figure 9 shows the changes in cytokine gene expression in mice heart tissues in Example 135.

Figure 10 shows the changes in cytokine gene expression in mice heart tissues in Example 136.

## DETAILED DESCRIPTION OF THE INVENTION

The inventor of the present invention has conducted extensive and in-depth research and developed a dihydroisoquinoline compound that can be used as a cGAS/STING signal pathway-targeted inhibitor for the treatment of inflammatory diseases and autoimmune diseases. On this basis, the present invention has been completed.

### Terminologys

Unless otherwise defined, all technical and scientific terms used herein have the same meanings as commonly understood by one of ordinary skill in the art to which this invention belongs.

As used herein, the terms "comprise", "include" and "contain" are used interchangeably, and they encompass not only closed-ended definitions but also semi-closed and open-ended definitions. In other words, the terms include the meanings of "consist of" and "consist essentially of".

In the present invention, the term "halogen" refers to F, Cl, Br or I.

In the present invention, unless specifically indicated otherwise, all terms used have the ordinary meanings well known to those skilled in the art.

In the present invention, the term "C1-C6" means having 1, 2, 3, 4, 5 or 6 carbon atoms; "C1-C8" means having 1, 2, 3, 4, 5, 6, 7 or 8 carbon atoms, and the like.

The term "5-12-membered" means having 5 to 12 ring atoms, and the like.

In the present invention, the term "alkyl" refers to a saturated linear or branched hydrocarbon moiety. For example, the term "C1-C6 alkyl" refers to a straight-chain or branched alkyl group having 1 to 6 carbon atoms, including but not limited to methyl, ethyl, propyl, isopropyl, butyl, isobutyl, sec-butyl, *tert*-butyl, pentyl, hexyl and the like; preferably ethyl, propyl, isopropyl, butyl, isobutyl, sec-butyl and *tert*-butyl.

In the present invention, the term "alkoxy" refers to an -O-(alkyl) group. For example, the term "C1-C6 alkoxy" refers to a straight-chain or branched alkoxy group having 1 to 6 carbon atoms, including but not limited to methoxy, ethoxy, propoxy, isopropoxy, butoxy and the like.

In the present invention, the term "alkenyl" refers to a straight-chain or branched hydrocarbon moiety containing at least one double bond. For example, the term "C2-C6 alkenyl" refers to a straight-chain or branched alkenyl group having 2 to 6 carbon atoms with one double bond, including but not limited to vinyl, propenyl, butenyl, isobutenyl, pentenyl, hexenyl and the like.

In the present invention, the term "alkynyl" refers to a straight-chain or branched alkynyl group containing one triple bond. For example, the term "C2-C6 alkynyl" refers to a straight-chain or branched alkynyl group having 2 to 6 carbon atoms with one triple bond, including but not limited to ethynyl, propynyl, butynyl, isobutynyl, pentynyl, hexynyl and the like.

In the present invention, the term "cycloalkyl" refers to a saturated cyclic hydrocarbon moiety. For example, the term "C3-C10 cycloalkyl" refers to a cyclic alkyl group having 3 to 10 carbon atoms in the ring, including but not limited to cyclopropyl, cyclobutyl, cyclopentyl, cyclohexyl, cycloheptyl, cyclooctyl, cyclodecyl and the like. The terms "C3-C8 cycloalkyl", "C3-C7 cycloalkyl" and "C3-C6 cycloalkyl" have similar meanings.

The term "heterocyclyl" refers to a saturated or partially saturated cyclic group containing heteroatoms selected from N, S and O, which can be monocyclic or bicyclic (e.g., bridged or spirocyclic). The heterocyclyl group is preferably a 3-8-membered heterocyclyl, more preferably a 4-6-membered heterocyclyl, and even more preferably a 5-6-membered heterocyclyl. Examples of heterocyclyl groups include but are not limited to oxetanyl, azetidinyl, tetrahydro-2H-pyranyl, piperidinyl, piperazinyl, tetrahydrofuranyl, morpholinyl, pyrrolidinyl,

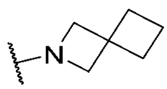

,

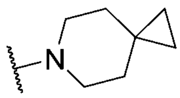

and the like.

In the present invention, the term "aryl" refers to a hydrocarbon moiety containing one or more aromatic rings. For example, the term "C6-C12 aryl" refers to an aromatic cyclic group having 6 to 12 carbon atoms with no heteroatoms in the ring, such as phenyl, naphthyl and the like. The term "C6-C12 aryl" has the similar meaning. Examples of aryl groups include but are not limited to phenyl (Ph), naphthyl, pyrenyl, anthryl, phenanthryl and the like.

The term "heteroaryl" refers to a cyclic aromatic group containing 1 to 3 heteroatoms selected from N, S and O, which can be monocyclic or fused-ring. In the present invention, the heteroaryl group is preferably a 5-12-membered heteroaryl, more preferably 5-10-membered, and even more preferably 5-8-membered. Examples of heteroaryl groups include but are not limited to pyridyl, pyridazinyl, pyrimidinyl, pyrazinyl, triazinyl, pyrrolyl, pyrazolyl, imidazolyl, (1,2,3)-triazolyl, (1,2,4)-triazolyl, tetrazolyl, furyl, thienyl, isoxazolyl, thiazolyl, oxazolyl, carbazolyl, indolyl, indazolyl, benzothienyl, benzofuranyl, benzimidazolyl, benzotriazolyl, benzothiazolyl, benzothiadiazolyl, benzoxazolyl, isomeric quinolyl, phthalazinyl, quinoxalinyl, quinazolinyl, cinnolinyl, naphthyridinyl, tetrazolyl and the like. The heteroaryl ring may be fused to an aryl, heterocyclyl or cycloalkyl ring, wherein the ring attached to the parent structure is the heteroaryl ring. The heteroaryl group can be optionally substituted or unsubstituted. When substituted, the substituents are preferably one or more groups independently selected from alkyl, deuterated alkyl, haloalkyl, alkoxy, haloalkoxy, alkenyl, alkynyl, alkylthio, alkylamino, halogen, amino, nitro, hydroxyl, mercapto, cyano, cycloalkyl, heterocyclyl, aryl, heteroaryl, cycloalkylthio, oxo, amido, sulfonamido, formyl, formamido, carboxyl, carboxylate and the like. In the present invention, among the substituents, the terms "heteroaromatic ring", "aromatic heterocycle" and "heteroaryl" have the same meaning.

In the present invention, "amino" refers to a group with the structure -N(R)(R'), wherein R and R' can independently represent hydrogen, alkyl or substituted alkyl, cycloalkyl or substituted cycloalkyl, aryl or substituted aryl, heterocycle or substituted heterocycle as defined above. R and R' can be the same or different in a dialkylamine moiety. Examples of amino groups include $NH_2$, $NHCH_3$, $N(CH_3)_2$ and the like.

Unless otherwise stated, the alkyl, alkoxy, cycloalkyl, heterocyclyl and aryl groups described herein are substituted or unsubstituted. Possible substituents on alkyl, alkoxy, cycloalkyl, heterocyclyl and aryl groups include but are not limited to hydroxyl, amino, nitro, nitrile group, halogen, C1-C6 alkyl, C2-C10 alkenyl, C2-C10 alkynyl, C3-C20 cycloalkyl, C3-C20 cycloalkenyl, C1-C20 heterocycloalkyl, C1-C20 heterocycloalkenyl, C1-C6 alkoxy, aryl, heteroaryl, heteroaryloxy, C1-C10 alkylamino, C1-C20 dialkylamino, arylamino, diarylamino, C1-C10 alkylsulfamoyl, arylsulfamoyl, C1-C10 alkylimino, C1-C10 alkylsulfoimino, arylsulfoimino, mercapto, C1-C10 alkylthio, C1-C10 alkylsulfonyl, arylsulfonyl, acylamino, aminoacyl, aminothioacyl, guanidino, ureido, cyano, acyl, thioacyl, acyloxy, carboxyl, carboxylate and the like. On the other hand, cycloalkyl, heterocycloalkyl, heterocycloalkenyl, aryl and heteroaryl groups can also be fused to each other.

In the present invention, the term "substituted" means monosubstituted or polysubstituted, wherein polysubstituted includes disubstituted, trisubstituted, tetrasubstituted and pentasubstituted. "Disubstituted" means having two substituents, and the like.

In the present invention, the term "more" independently refers to 2, 3, 4 or 5.

The term "prodrug", also referred to as a precursor drug, drug precursor, and the like, refers to a compound obtained by chemical structural modification of a drug, which is inactive or has low activity *in vitro* and releases the active drug through enzymatic or non-enzymatic transformation *in vivo* to exert its pharmacological effect. Examples include ester forms of compounds containing carboxyl groups and the like.

The term "pharmaceutically acceptable salt" refers to a salt (including inner salts such as zwitterions) that has efficacy similar to the parent compound and is biologically or otherwise acceptable (e.g., non-toxic and harmless to the subject). Therefore, the embodiments of the present invention provide pharmaceutically acceptable salts of the compounds of the present invention. As used herein, the term "salt" refers to any acid salt formed with inorganic and/or organic acids, as well as any basic salt formed with inorganic and/or organic bases. Salts of the compounds of the present invention can be prepared by methods known to those of ordinary skill in the art, for example, by reacting a compound of the present invention with a certain amount of acid or base (e.g., an equivalent amount of acid or base) in a medium (e.g., a medium that allows the salt to precipitate; or using water as the medium followed by lyophilization).

**Active Ingredient**

As used herein, the terms "compound of the present invention" or "active ingredient of the present invention" are used interchangeably and refer to the compound of formula (I), or a pharmaceutically acceptable salt, a hydrate, a solvate, an isotopic compound (e.g., deuterated compound), or a prodrug thereof. The terms also encompass a racemate and an optical isomer.

The compound is understood to include its salts. As used herein, the term "salt" refers to an acid salt or basic salt formed with inorganic or organic acids and bases. Furthermore, when a compound of the present invention contains a basic moiety (including but not limited to pyridine or imidazole), or an acidic moiety (including but not limited to carboxylic acid), the possible zwitterion ("inner salt") formed thereby is included within the scope of the term "salt". Pharmaceutically acceptable (i.e., non-toxic and physiologically acceptable) salts are preferred, although other salts are also useful, e.g., for separation or purification steps in the preparation process. The compounds of the present invention may form salts. For example, compound I reacts with a suitable amount (e.g., an equivalent amount) of an acid or a base, and the salt is precipitated from the medium, or obtained by freeze-drying in an aqueous solution..

The basic moieties contained in the compounds of the present invention, including but not limited to amine, pyridine, or imidazole rings, may form salts with organic or inorganic acids. Typical acids capable of forming such salts include acetate (e.g., formed with acetic acid or trihaloacetic acid, such as trifluoroacetic acid), adipate, alginate, ascorbate, aspartate, benzoate, benzenesulfonate, bisulfate, borate, butyrate, citrate, camphorate, camphorsulfonate, cyclopentanepropionate, diglycolate, dodecylsulfate, ethane sulfonate, fumarate, glucoheptonate, glycerophosphate, hemisulfate, heptanoate, hexanoate, hydrochloride, hydrobromide, hydroiodide, isethionate (e.g., 2-hydroxyethanesulfonates), lactate, maleate, methanesulfonate, naphthalenesulfonate (e.g., 2-naphthalenesulfonates), nicotinate, nitrate, oxalate, pectate, persulfate, phenylpropionate (e.g., 3-phenylpropionates), phosphate, picrate, pivalate, propionate, salicylate, succinate, sulfate (e.g., formed with sulfuric acid), sulfonate, tartrate, thiocyanate, toluenesulfonates (e.g., p-toluenesulfonates), dodecanoate, and the like.

Certain compounds of the present invention may contain acidic moieties, including but not limited to carboxylic acids, which may form salts with various organic or inorganic bases. Typical salts formed with base include ammonium salt, alkali metal salt (e.g., sodium, lithium, potassium salts), alkaline earth metal salt (e.g., calcium, magnesium salts), and salts formed with organic bases (e.g., organic amine) such as benzathine, dicyclohexylamine, hydrabamine (salt formed with *N,N*-di(dehydroabietyl)ethylenediamine), *N*-methyl-D-glucamine, *N*-methyl-D-glucosamide, *tert*-butylamine, as well as salts formed with amino acids (e.g., arginine, lysine, etc.). Basic nitrogen-containing groups may be quaternized with halides such as small-molecule alkyl halide (e.g., chlorides, bromides, and iodides of methyl, ethyl, propyl, and butyl), dialkyl sulfate (e.g., dimethyl sulfate, diethyl sulfate, dibutyl sulfate, and dipentyl sulfate), long-chain alkyl halide (e.g.,

chlorides, bromides, and iodides of decyl, lauryl, myristyl, and cetyl), and aralkyl halide (e.g., bromides of benzyl, and phenyl), etc.

Prodrugs and solvates of the compounds of the present invention are also encompassed herein. The term "prodrug" as used herein refers to a compound that, in the treatment of a relevant disease, undergoes chemical transformation via metabolic or chemical processes to produce the compound, salt, or solvate of the present invention. The compounds of the present invention include solvates such as hydrates.

The compounds, salts, or solvates of the present invention may exist in tautomeric forms (e.g., amides and imino ethers). All such tautomers are part of the present invention.

All stereoisomers of the compounds (e.g., those asymmetric carbon atoms that may exist due to various substitutions), including enantiomeric and diastereomeric forms, are contemplated within the scope of the present invention. Individual stereoisomers of the compounds of the present invention may exist substantially free of other isomers (e.g., as pure or substantially pure optical isomers with specific activity), or may be present as mixtures (e.g., racemates), or mixtures with all or a portion of the other stereoisomers.. Chiral centers in the present invention have either S or R configuration as defined by the 1974 Recommendations of the International Union of Pure and Applied Chemistry (IUPAC). Racemic forms can be resolved by physical methods such as fractional crystallization, or by derivatization into diastereoisomers, followed by separation and crystallization, or by separation using chiral column chromatography. Individual optical isomers can be obtained from racemates by suitable methods including, but not limited to, conventional approaches such as salt formation with optically active acids followed by recrystallization.

The compounds of the present invention, obtained by preparation, isolation, and purification sequentially, have a weight content of 90% or higher, e.g., 95% or higher, 99% or higher ("highly pure" compounds), as described herein. Such "highly pure" compounds of the present invention are also part of the present invention.

All configurational isomers of the compounds of the present invention are encompassed herein, whether in the form of mixtures, pure, or highly pure forms. The definition of the compounds of the present invention includes both cis (Z) and trans (E) olefin isomers, as well as cis and trans isomers of carbocyclic and heterocyclic rings.

Throughout the specification, groups and substituents may be selected to provide stable moieties and compounds.

Certain compounds of the present invention may exist in specific geometric or stereoisomeric forms. The present invention encompasses all such compounds, including cis and trans isomers, R and S enantiomers, diastereomers, (D)-isomers, (L)-isomers, racemic mixtures, and other mixtures thereof. Additionally, asymmetric carbon atoms may be present in substituents such as alkyl groups. All isomers and mixtures thereof are included in the present invention.

According to the present invention, mixtures of isomers may have various ratios of the isomers. For example, a mixture containing only two isomers may have the following ratios: 50:50, 60:40, 70:30, 80:20, 90:10, 95:5, 96:4, 97:3, 98:2, 99:1, or 100:0. All such isomer ratios are within the scope of the present invention. Similar ratios readily understood by those of ordinary skill in the art, as well as ratios for more complex isomer mixtures, are also encompassed herein.

The present invention also includes isotopically labeled compounds that are identical to the compounds disclosed herein. However, in reality, substitution of one or more atoms with atoms having a different atomic weight or mass number generally occurs.. Examples of isotopes that can be incorporated into the compounds of the present invention include isotopes of hydrogen, carbon, nitrogen, oxygen, phosphorus, sulfur, fluorine, and chlorine, such as $^{2}H$, $^{3}H$, $^{13}C$, $^{11}C$, $^{14}C$, $^{15}N$, $^{18}O$, $^{17}O$, $^{31}P$, $^{32}P$, $^{35}S$, $^{18}F$, and $^{36}Cl$, respectively. Compounds of the present invention, or enantiomers, diastereomers, isomers, or pharmaceutically acceptable salts or solvates thereof, wherein the compounds contain the aforementioned isotopes or other isotopic atoms, are all within the scope of the present invention. Certain isotopically labeled compounds of the present invention, e.g., those incorporating radioactive isotopes such as $^{3}H$ and $^{14}C$, are also within the scope, which are useful in tissue distribution studies of drugs and substrates. Tritiated ($^{3}H$) and carbon-14 ($^{14}C$) isotopes are particularly preferred due to their ease of preparation and detection. Furthermore, substitution with heavier isotopes such as deuterium ($^{2}H$) offers advantages in certain therapeutic applications due to its greater metabolic stability, e.g., increased *in vivo* half-life or reduced dosage requirements, and thus may be preferred in some cases. Isotopically labeled compounds of the present invention can be prepared by conventional methods by substituting non-isotopically labeled reagents with readily available isotopically labeled reagents using the procedures disclosed in the Examples.

If a specific enantiomer of a compound of the present invention is to be synthesized, the compound can be prepared by asymmetric synthesis, or by derivatization with a chiral auxiliary reagent, followed by separation of the resulting diastereomeric mixture and removal of the chiral auxiliary reagent to yield the pure enantiomer. Alternatively, if the molecule contains a basic functional group (e.g., an amino group) or an acidic functional group (e.g., a carboxyl group), it can react with a suitable optically active acid or base to form a diastereomeric salt, which is then separated by conventional means such as fractional crystallization or chromatography, and the pure enantiomer obtained.

As described herein, the compounds of the present invention may be substituted with any number of substituents or functional groups to broaden their scope. In general, the term "substituted", whether appearing before or after the term "optionally", in the general formulas of the present invention includes the formula of substitutent, which means that a

hydrogen radical is replaced by a substituent of the designated structure. When multiple positions in a specific structure are substituted with multiple specified substituents, the substituents at each position may be the same or different. The term "substituted" as used herein includes all permissible substitutions of organic compounds. In a broad sense, permissible substituents include acyclic, cyclic, branched, unbranched, carbocyclic, heterocyclic, aromatic, and non-aromatic organic compounds. In the present invention, heteroatoms such as nitrogen may bear hydrogen substituent or any permissible organic substituents as described above to satisfy their valency. Furthermore, the present invention is not intended to limit the permissible substitutions of organic compounds in any way. The present invention contemplates that combinations of substituents and variable groups are suitable for treating diseases as a form of stable compound. The term "stable" as used herein refers to a stable compound that maintains the integrity of its chemical structure for a sufficient period to enable its detection and, preferably, for a sufficient period to be effective, and are used herein for the aforementioned purposes.

Metabolites of the compounds of the present invention and the pharmaceutically acceptable salts thereof, as well as prodrugs that can be converted *in vivo* into the structures of the compounds of the present invention and the pharmaceutically acceptable salts thereof, are also included in the claims of the present invention.

## Preparation methods of compounds

The following schemes and examples describe the method for preparing the compound of formula I. Raw materials and intermediates are purchased from commercial sources, or prepared by known steps, or specified by other manner. In some cases, the order of steps in executing the reaction scheme can be changed to facilitate the reaction or avoid unwanted side reaction products.

Usually, in the preparation process, each reaction is carried out in an inert solvent at room temperature to reflux temperature (such as 0 °C to 150 °C, preferably 10 °C to 100 °C). The reaction time is usually 0.1-60 hours, preferably 0.5-48 hours.

Preferably, the compound of formula (I) of the present invention can be prepared by the following two methods:

method 1:

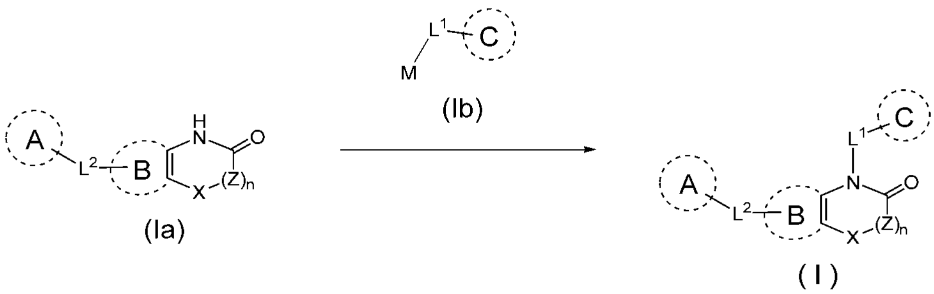

method 2:

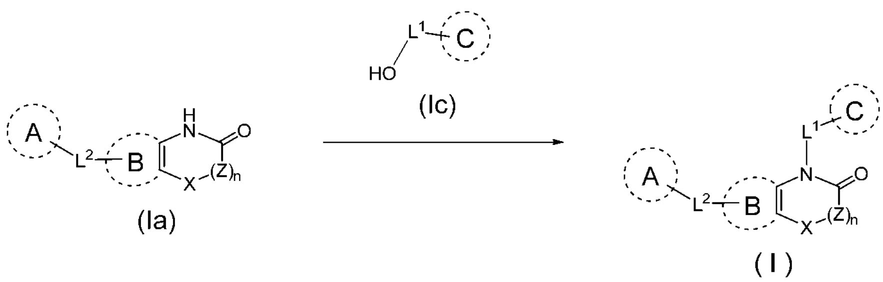

1) intermediate (Ia) undergoes nucleophilic substitution reaction with intermediate (Ib) in the presence of sodium hydroxide, sodium carbonate, or cesium carbonate to obtain the compound shown in formula (I); wherein, M is selected from the group consisting of chlorine, bromine, iodine, or methylsulfonate group (MsO);

2) intermediate (Ia) and intermediate (Ic) undergo Mitsunobu reaction in the presence of triphenylphosphine and diisopropyl azodicarboxylate to obtain the compound shown in formula (I).

**Pharmaceutical Compositions and Methods of Administration**

The compounds of the present invention exhibit excellent inhibitory activity against STING protein. Therefore, the compounds of the present invention, or stereoisomers or optical isomers, pharmaceutically acceptable salts, prodrugs or solvates thereof, as well as pharmaceutical compositions comprising the compound of the present invention as the main active ingredient, can be used for preventing and/or treating (stabilizing, alleviating or curing) inflammatory diseases and autoimmune diseases associated with STING protein. The inflammatory diseases and autoimmune diseases are selected from the group consisting of Singleton-Merten syndrome (SMS), Aicardi-Goutieres syndrome (AGS), systemic lupus erythematosus (SLE), amyotrophic lateral sclerosis (ALS), familial chilblain lupus (FCL), retinal vasculopathy with cerebral leukodystrophy (RVCL), STING-associated vasculopathy with onset in infancy (SAVI), scleroderma, psoriasis, Sjögren's syndrome, rheumatoid arthritis, inflammatory bowel disease, multiple sclerosis, Crohn's disease, ulcerative colitis, autoimmune colitis, malabsorption syndrome, irritable bowel syndrome, uveitis, mucositis, diabetes, cardiovascular diseases and neurodegenerative diseases.

The pharmaceutical compositions of the present invention comprise the compound of the present invention in a safe and effective amount, and pharmaceutically acceptable excipients or carriers. The term "safe and effective amount" refers to an amount of the compound sufficient to significantly ameliorate a disease condition without causing severe adverse side effects. Generally, the pharmaceutical composition contains 1-2000 mg of the compound of the present invention per dose, more preferably 10-200 mg of the compound of the present invention per dose. Preferably, said "one dose" is one capsule or tablet.

The term "pharmaceutically acceptable carriers" mean one or more compatible solid or liquid filler or gel substances that are suitable for human use and must have sufficient purity and sufficiently low toxicity. "Compatibility" herein refers to the ability of the components of a composition to blend with the compounds of the invention and with each other without significantly reducing the efficacy of the compounds. Examples of pharmaceutically acceptable carriers include cellulose and its derivatives (such as sodium carboxymethyl cellulose, sodium ethyl cellulose, cellulose acetate, etc.), gelatin, talc, solid lubricants (such as stearic acid, magnesium stearate), calcium sulfate, vegetable oil (such as soybean oil, sesame oil, peanut oil, olive oil, etc.), polyols (such as propylene glycol, glycerin, mannitol, sorbitol, etc.), emulsifier (such as Tween®), wetting agents (such as sodium dodecyl sulfate), colorants, flavoring agents, stabilizers, antioxidants, preservatives, pyrogen-free water, etc.

The administration modes of the compounds or pharmaceutical compositions of the present invention are not particularly limited, and representative modes of administration include (but are not limited to) oral, parenteral (intravenous, intramuscular or subcutaneous).

Solid dosage forms for oral administration include capsules, tablets, pills, powders and granules. In these solid dosage forms, the active compound is mixed with at least one conventional inert excipient (or carrier), such as sodium citrate or dicalcium phosphate, or mixed with the following components:(a) fillers or compatibilizers, e.g., starch, lactose, sucrose, glucose, mannitol and silicic acid; (b) binders, e.g., hydroxymethylcellulose, alginate, gelatin, polyvinylpyrrolidone, sucrose, and gum arabic; (c) humectants, e.g., glycerol; (d) disintegrants, e.g., agar, calcium carbonate, potato starch or tapioca starch, alginic acid, certain composite silicates, and sodium carbonate; (e) dissolution-retarding agents, e.g., paraffin; (f) absorption accelerators, e.g., quaternary amine compounds; (g) wetting agents, e.g., cetyl alcohol and glycerol monostearate; (h) adsorbents, e.g., kaolin; and (i) lubricants, e.g., talc, calcium stearate, magnesium stearate, solid polyethylene glycol, sodium dodecyl sulfate, and the mixtures thereof. In capsules, tablets and pills, dosage forms may also contain buffers.

Solid dosage forms such as tablets, dragees, capsules, pills, and granules can be prepared using coating and shell materials, such as enteric coatings and other materials known in the art. They may contain opacifying agents, and the release of the active compound or compounds in such compositions can be delayed in a certain part of the digestive tract. Examples of embedding components that can be employed are polymeric substances and waxes. If desired, the active compound can also be in micro-encapsulated form with one or more of the above-mentioned excipients.

Liquid dosage forms for oral administration include pharmaceutically acceptable emulsions, solutions, suspensions, syrups or tinctures. In addition to the active compounds, the liquid dosage forms may contain inert diluents conventionally used in the art such as water or other solvents, solubilizers and emulsifiers, such as ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil, or the mixtures thereof and the like.

In addition to these inert diluents, the compositions may also contain auxiliaries such as wetting agents, emulsifiers, suspending agents, sweeteners, flavoring agents and spices.

In addition to the active compound, the suspensions may comprise suspending agents, such as ethoxylated isooctadecanol, polyoxyethylene sorbitol and dehydrated sorbitol esters, microcrystalline cellulose, methanolic aluminum, agar, and the mixtures thereof.

Compositions for parenteral injection may comprise physiologically acceptable sterile aqueous or non-aqueous solutions, dispersions, suspensions or emulsions, and sterile powders for redissolved into sterile injectable solutions or

dispersions. Suitable aqueous and non-aqueous carriers, diluents, solvents or excipients include water, ethanol, polyols, and suitable mixtures thereof.

The compounds of the present invention may be administered alone or in combination with other pharmaceutically acceptable compounds.

When administered in combination, the pharmaceutical composition further comprises one or more (2, 3, 4, or more) other pharmaceutically acceptable compounds. One or more (2, 3, 4, or more) of said other pharmaceutically acceptable compounds may be used simultaneously, separately or sequentially with the compounds of the present invention for preventing and/or treating diseases related with the activity or expression level of STING protein.

When using the pharmaceutical composition, a safe and effective amount of the compound of the present invention is administered to a mammal (e.g., a human) in need of treatment, wherein the dosage upon administration is a pharmaceutically effective dosage. For a human weighing 60 kg, the daily dosage is usually 1 to 2000 mg, preferably 20 to 500 mg. Of course, the specific dosage should also take into account factors such as the route of administration and the patient's health status, which are within the skill of a skilled physician.

The term "safe and effective amount" refers to an amount of the active ingredient sufficient to significantly improve the condition without causing severe side effects. Generally, the pharmaceutical composition contains 1 to 2000 mg of active ingredient per dose, more preferably 10 to 200 mg of active ingredient per dose. Preferably, said "one dose" is one tablet.

**The main advantages of the present invention are as follows:**

1. The compounds of the present invention exhibit inhibitory activity against STING protein in human-derived cells and/or mouse-derived cells, and can be used as targeted inhibitors of the cGAS/STING signaling pathway;
2. The compounds of the present invention possess favorable pharmacological and pharmacokinetic properties.

The detailed experimental procedures in the following Examples further illustrate the present invention in specific terms. The exemplary compounds are depicted in their neutral forms in the Examples below. In some cases, compounds are isolated as salts depending on the method used for final purification and/or the intrinsic molecular properties. These examples are for illustrative purposes only and are not intended to limit the scope of this invention in any way. Unless otherwise defined, all technical and scientific terms used herein have the same meaning as commonly understood by one of ordinary skill in the art. In addition, any methods and materials similar or equivalent to those described herein can be applied to the methods of the present invention. The preferred embodiments and materials described herein are for illustrative purposes only.

The present invention is further illustrated by the following specific Examples. It should be understood that these Examples are for illustrative purposes only and are not intended to limit the scope of the present invention. The experimental methods in the following Examples that do not specify specific conditions are generally conducted according to conventional conditions or according to the conditions recommended by the manufacturer. Percentages and parts are calculated by weight unless otherwise indicated.

**Example**

**Example 1 4-benzyl-N-(1H-indol-3-yl)-3-oxo-3,4-dihydro-2H-thieno[3,2-b] [1,4]thiazin-6-carboxamide (I-1)**

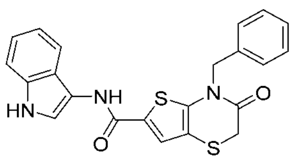

**Step 1: methyl 4-bromo-5-nitrothiophene-2-carboxylate (1-1)**

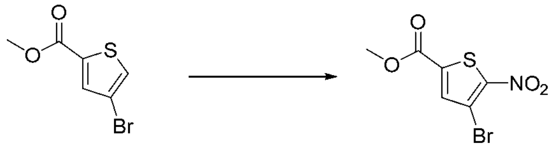

In an ice salt bath, 1 gram of methyl 4-bromothiophene-2-carboxylate was added to 5 milliliters of concentrated sulfuric acid. Then 0.5 milliliters of fuming nitric acid was dissolved in 5 milliliters of concentrated sulfuric acid and the mixture was slowly added dropwise to the reaction solution. After dripping, the mixture was allowed to react in an ice salt bath for 30 minutes. The reaction solution was slowly poured into ice water, washed with water three times, and dried to obtain 1.167 g of white solid methyl 4-bromo-5-nitrothiophene-2-carboxylate **(1-1)** with a yield of 97.25%. $^1$H NMR (400 MHz, DMSO-$d_6$) $\delta$ 8.00 (s, 1H), 3.90 (s, 3H).

**Step 2: methyl 4-((2-methoxy-2-carbonylethyl)thiol)-5-nitrothiophene-2-carboxylate (1-2)**

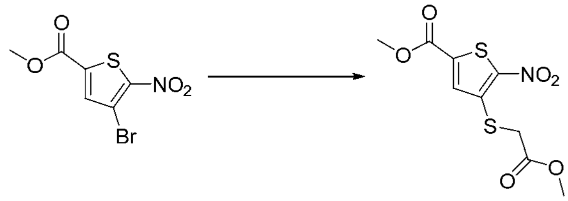

A mixture of 1 gram of methyl 4-bromo-5-nitrothiophene-2-carboxylate **(1-1),** 400 microliters of methyl mercaptoacetate, 1.6 milliliters of triethylamine, and 10 milliliters of tetrahydrofuran was stirred at room temperature for 4 hours. The reaction solution was poured into water, washed with water three times, and dried to obtain 1.013 g of yellow solid methyl 4-(2-methoxy-2-carbonylethyl)thiol-5-nitrothiophene-2-carboxylate **(1-2),** with a yield of 92.13%. $^1$H NMR (400 MHz, DMSO-$d_6$) $\delta$ 7.82 (s, 1H), 4.35 (s, 2H), 3.91 (s, 3H), 3.69 (s, 3H).

**Step 3: methyl 3-oxo-3,4-dihydro-2H-thieno[3,2-b][1,4]thiazin-6-carboxylate (1-3)**

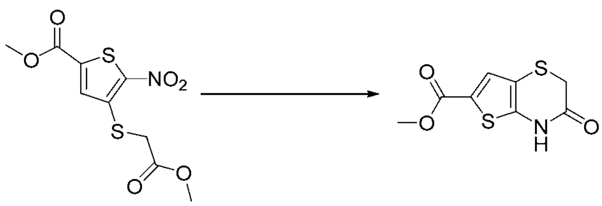

A mixture of 500 mg of methyl 4-((2-methoxy-2-carbonylethyl)thiol)-5-nitrothiophene-2-carboxylate **(1-2),** 480 mg of reduced iron powder, and 10 mL of acetic acid was heated and stirred at 80 °C under argon atmosphere for 2 hours. The reaction solution was concentrated to dryness, and the residue was subjected to column chromatography with dichloromethane: methanol=98:2 to obtain 360.3 mg of gray solid methyl 3-oxo-3,4-dihydro-2H-thieno[3,2-b][1,4]thiazin-6-carboxylate **(1-3)** with a yield of 91.68%. $^1$H NMR (400 MHz, DMSO-$d_6$) $\delta$ 10.75 (s, 1H), 7.30 (s, 1H), 3.80 (s, 3H), 3.63 (s, 2H).

**Step 4: methyl 4-benzyl-3-oxo-3,4-dihydro-2H-thieno[3,2-b][1,4]thiazin-6-carboxylate (1-4)**

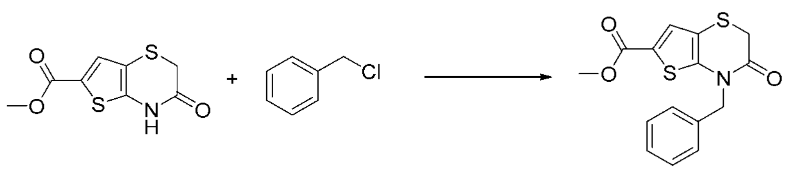

A mixture of 360 milligrams of methyl 3-oxo-3,4-dihydro-2H-thieno[3,2-b][1,4]thiazin-6-carboxylate (1-3), 220 microliters of benzyl chloride, 440 milligrams of potassium carbonate, and 6 milliliters of N, N-dimethylformamide was stirred at room temperature for 12 hours. The reaction solution was diluted with water, and extracted with ethyl acetate, and the organic layer was concentrated to dryness. The residue was subjected to column chromatography with dichloromethane: methanol = 98:2 to obtain 430.4 mg of methyl 4-benzyl-3-oxo-3,4-dihydro-2H-thieno[3,2-b][1,4]thiazin-6-carboxylate **(1-4)** with a yield of 85.91%. $^1$H NMR (400 MHz, DMSO-$d_6$) $\delta$ 7.66 (s, 1H), 7.32 (dd, $J$ = 8.0, 6.6 Hz, 2H), 7.22 (m, 1H), 7.18 (m, 2H), 5.18 (s, 2H), 3.85 (s, 2H), 3.76 (s, 3H).

**Step 5: 4-benzyl-3-oxo-3,4-dihydro-2H-thieno[3,2-b][1,4]thiazin-6-carboxylic acid (1-5)**

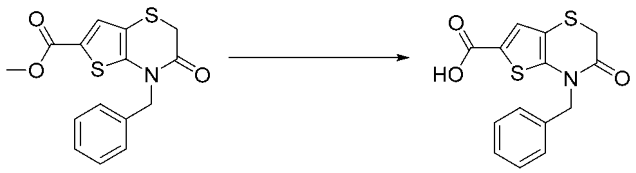

A mixture of 200 mg of methyl 4-benzyl-3-oxo-3,4-dihydro-2H-thieno[3,2-b][1,4]thiazin-6-carboxylate **(1-4),** 260 mg of lithium hydroxide monohydrate, and 4 mL of tetrahydrofuran was heated and stirred at 60 °C under argon atmosphere for 2 hours. 10% citric acid solution was slowly added dropwise to the reaction solution to adjust the pH to 2-3. After solid precipitation, the mixture was filtered and the filter cake was dried to obtain 256 mg of solid 4-benzyl-3-oxo-3,4-dihydro-2H-thieno[3,2-b][1,4]thiazin-6-carboxylic acid **(1-5)** with a yield of 100%. LCMS (ESI) *m*/*z* [M+H]$^+$: 306.0.

**Step 6: 4-benzyl-N-(1H-indol-3-yl)-3-oxo-3,4-dihydro-2H-thieno[3,2-b][1,4]thiazin-6-carboxamide (I-1)**

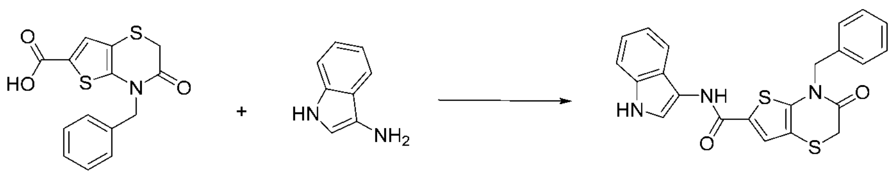

A mixture of 100 milligrams of 4-benzyl-3-oxo-3,4-dihydro-2H-thieno[3,2-b][1,4]thiazin-6-carboxylic acid **(1-5),** 40 milligrams of 3-aminoindole, 200 microliters of DIPEA, 150 milligrams of HATU, and 4 milliliters of N, N-dimethylformamide was stirred at room temperature for 12 hours. The reaction solution was diluted with water, and extracted with ethyl acetate, and the organic layer was concentrated to dryness. The residue was subjected to column chromatography with dichloromethane: ethyl acetate = 80:20 to obtain a yellow solid, 4-benzyl-N-(1H-indol-3-yl)-3-oxo-3,4-dihydro-2H-thieno [3,2-b][1,4]thiazin-6-carboxamide (**I-1**), 25.5 mg, with a yield of 20.08%. $^1$H NMR (400 MHz, DMSO-$d_6$) $\delta$ 10.99 (s, 1H), 10.09 (s, 1H), 8.05 (s, 1H), 7.71 (d, $J$ = 8.0 Hz, 1H), 7.64 (d, $J$ = 2.6 Hz, 1H), 7.41 (m, 6H), 7.11 (t, $J$ = 7.5 Hz, 1H), 7.02 (t, $J$ = 7.5 Hz, 1H), 5.12 (s, 2H), 3.83 (s, 2H).

**Example 2 N-(1H-indol-3-yl)-3-oxo-4-((1-phenylazetidin-3-yl)methyl)-3,4-dihydro-2H-benzo[b][1,4]thiazin-6-carboxamide (I-2)**

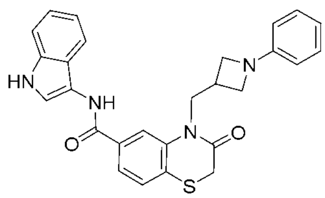

**Step 1: (1-phenylazetidin-3-yl)methanol (2-1)**

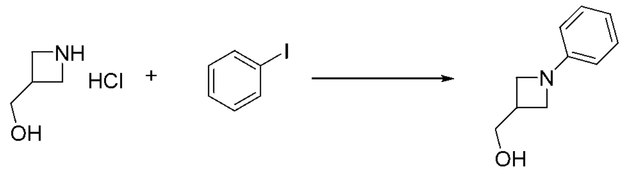

A mixture of 1 gram of 3-methylhydroxyazetidine hydrochloride, 1.81 milliliters of iodobenzene, 1.5 grams of cuprous iodide, 940 milligrams of L-proline, 3.4 grams of potassium carbonate, and 10 milliliters of dimethyl sulfoxide was heated and stirred at 90 °C under argon atmosphere for 12 hours. The reaction solution was diluted with water, and extracted with ethyl acetate, and the organic layer was concentrated to dryness. The residue was subjected to column chromatography with petroleum ether: ethyl acetate=50:50 to obtain 1.188 g of yellow oily solid (1-phenylazetidin-3-yl) methanol **(2-1)** with a yield of 90.0%. $^{1}$H NMR (400 MHz, DMSO-$d_6$) $\delta$ 7.17-7.06 (m, 2H), 6.63 (tt, *J* = 7.3, 1.2 Hz, 1H), 6.42-6.34 (m, 2H), 4.74 (t, *J* = 5.3 Hz, 1H), 3.79 (t, *J* = 7.5 Hz, 2H), 3.57 (dd, *J* = 6.5, 5.3 Hz, 2H), 3.50 (dd, *J* = 7.2, 5.4 Hz, 2H), 2.81-2.70 (m, 1H).

**Step 2: 3-(chloromethyl)-1-phenylazetidine (2-2)**

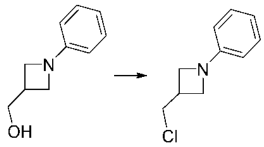

A mixture of 1.18 grams of (1-phenylazetidin-3-yl)methanol **(2-1),** 770 microliters of carbon tetrachloride, 2.1 grams of triphenylphosphine, and 10 milliliters of dichloromethane was stirred at room temperature for 12 hours. The reaction solution was concentrated to dryness, and the residue was subjected to column chromatography with petroleum ether: ethyl acetate = 99:1 to obtain 1 g of solid 3-(chloromethyl)-1-phenylazetidine **(2-2)** with a yield of 77.60%. $^{1}$H NMR (400 MHz, DMSO-$d_6$) $\delta$ 7.20-7.11 (m, 2H), 6.67 (tt, *J* = 7.3, 1.1 Hz, 1H), 6.47-6.40 (m, 2H), 3.89 (t, *J* = 7.5 Hz, 2H), 3.78 (d, *J* = 7.6 Hz, 2H), 3.49 (dd, *J* = 7.4, 5.7 Hz, 2H), 3.09 (tt, *J* = 7.7, 5.5 Hz, 1H).

**Step 3: methyl 3-oxo-4-((1-phenylazetidin-3-yl)methyl)-3,4-dihydro-2H-benzo[b][1,4]thiazin-6-carboxylate (2-3)**

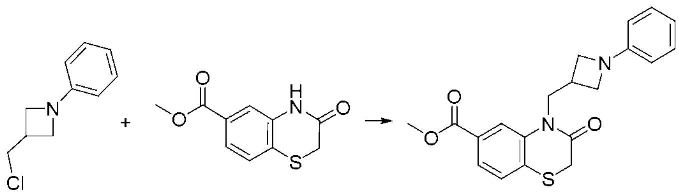

A mixture of 500 mg of methyl 3-carbonyl-3,4-dihydro-2H-1,4-benzothiazin-6-carboxylate, 450 mg of 3-(chloromethyl)-1-phenylazetidine, 1.85 g of potassium carbonate, and 10 mL of N, N-dimethylformamide was heated and stirred at 80 °C for 12 hours. The reaction solution was diluted with water, and extracted with ethyl acetate, and the organic layer was concentrated to dryness. The residue was subjected to column chromatography with dichloromethane: ethyl acetate = 95:5 to obtain 528.3 mg of methyl 3-oxo-4-((1-phenylazetidin-3-yl)methyl)-3,4-dihydro-2H-benzo[b][1,4]thiazin-6-carboxylate **(2-3)** as a white solid, with a yield of 64.03%. $^{1}$H NMR (400 MHz, DMSO-$d_6$) $\delta$ 7.84 (s, 1H), 7.66-7.56 (m, 2H), 7.14 (t, *J* = 7.7 Hz, 2H), 6.66 (t, *J* = 7.3 Hz, 1H), 6.38 (d, *J* = 7.9 Hz, 2H), 4.40 (d, *J* = 7.3 Hz, 2H), 3.85 (s, 3H), 3.76 (t, *J* = 7.4 Hz, 2H), 3.60 (s, 2H), 3.50 (t, *J* = 6.3 Hz, 2H), 2.85 (s, 1H).

**Step 4: 3-oxo-4-((1-phenylazetidin-3-yl)methyl)-3,4-dihydro-2H-benzo[b][1,4]thiazin-6-carboxylic acid (2-4)**

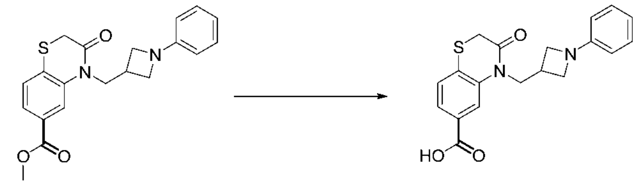

The preparation of 3-oxo-4-((1-phenylazetidin-3-yl)methyl)-3,4-dihydro-2H-benzo[b][1,4]thiazin-6-carboxylic acid **(2-4)** was the same as that of compound **1-5.** LCMS (ESI) *m/z* $[M+H]^+$: 355.1.

**Step 5: N-(1H-indol-3-yl)-3-oxo-4-((1-phenylazetidin-3-yl)methyl)-3,4-dihydro-2H-benzo[b] [1,4]thiazin-6-carboxamide (I-2)**

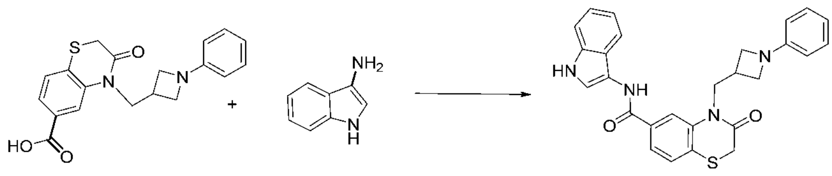

The preparation of (1H-indol-3-yl)-3-oxo-4-((1-phenylazetidin-3-yl)methyl)-3,4-dihydro-2H-benzo[b][1,4]thiazin-6-carboxamide (**I-2**) was the same as that of compound **I-1.** $^1$H NMR (400 MHz, DMSO-$d_6$) $\delta$ 11.06-10.90 (m, 1H), 10.24 (s, 1H), 7.92 (d, *J* = 1.7 Hz, 1H), 7.85-7.76 (m, 2H), 7.73 (dd, *J* = 8.1, 1.6 Hz, 1H), 7.60 (d, *J* = 8.0 Hz, 1H), 7.37 (d, *J* = 8.1 Hz, 1H), 7.18-7.08 (m, 3H), 7.05-6.98 (m, 1H), 6.66 (t, *J* = 7.3 Hz, 1H), 6.44-6.33 (m, 2H), 4.47 (d, *J* = 7.3 Hz, 2H), 3.81 (t, *J* = 7.4 Hz, 2H), 3.60 (s, 2H), 3.56 (dd, *J* = 7.1, 5.5 Hz, 2H), 2.96 (p, *J* = 6.7 Hz, 1H).

**Example 3 1-(1H-indol-3-yl)-3-(3-oxo-4-((1-phenylazetidin-3-yl)methyl)-3,4-dihydro-2H-benzo[b][1,4]thiazin-6-yl)urea (1-3)**

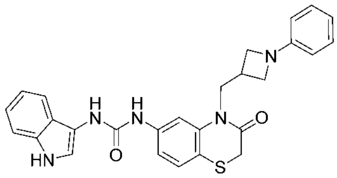

**Step 1: 3-oxo-4-((1-phenylazetidin-3-yl)methyl)-3,4-dihydro-2H-benzo[b][1,4]thiazin-6-carbonyl azide (3-1)**

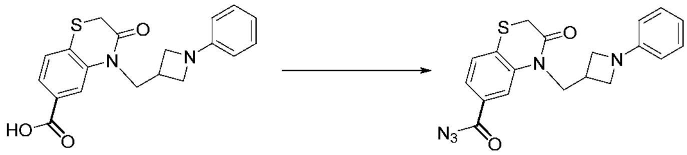

A mixture of 179 milligrams of 3-oxo-4-((1-phenylazetidin-3-yl)methyl)-3,4-dihydro-2H-benzo[b][1,4]thiazin-6-carboxylic acid **(2-4),** 95 microliters of triethylamine, 110 microliters of DPPA, and 5 milliliters of dichloromethane was stirred at room temperature for 12 hours. The reaction solution was concentrated to dryness, and the residue was subjected to column chromatography with petroleum ether: ethyl acetate = 90:10 to obtain 160 mg of solid 3-oxo-4-((1-phenylazetidin-3-yl)methyl)-3,4-dihydro-2H-benzo[b][1,4]thiazin-6-carbonyl azide **(3-1)** with a yield of 83.7%. $^1$H NMR (400 MHz, DMSO-$d_6$) $\delta$ 7.82 (d, *J* = 1.4 Hz, 1H), 7.63 (d, *J* = 1.8 Hz, 2H), 7.22-7.08 (m, 2H), 6.66 (t, *J* = 7.3 Hz, 1H), 6.43-6.30 (m, 2H), 4.40 (d, *J* = 7.4 Hz, 2H), 3.76 (t, *J* = 7.4 Hz, 2H), 3.62 (s, 2H), 3.50 (dd, *J* = 7.2, 5.3 Hz, 2H), 2.96-2.75 (m, 1H).

**Step 2: t-butyl (3-oxo-4-((1-phenylazetidin-3-yl)methyl)-3,4-dihydro-2H-benzo[b][1,4]thiazin-6-yl)carbamate (3-2)**

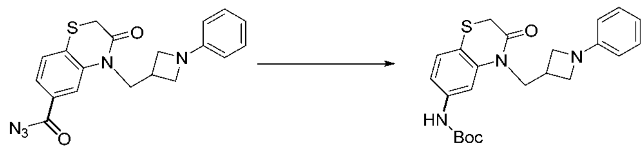

A mixture of 160 mg of 3-oxo-4-((1-phenylazetidin-3-yl)methyl)-3,4-dihydro-2H-benzo[b][1,4]thiazin-6-carbonyl azide **(3-1)** and 5 mL of *tert*-butanol was heated and stirred at 90 °C under argon atmosphere for 12 hours. The reaction solution was concentrated to dryness, and the residue was subjected to column chromatography with petroleum ether: ethyl acetate = 80:20 to obtain 78.1 mg of solid t-butyl (3-oxo-4-((1-phenylazetidin-3-yl)methyl)-3,4-dihydro-2H-benzo[b][1,4]thiazin-6-yl)carbamate **(3-2)** with a yield of 43.6%. $^1$H NMR (400 MHz, DMSO-$d_6$) $\delta$ 9.45 (s, 1H), 7.46 (s, 1H), 7.29 (d, *J* = 8.4 Hz, 1H), 7.22 (d, *J* = 8.7 Hz, 1H), 7.14 (t, *J* = 7.7 Hz, 2H), 6.66 (t, *J* = 7.3 Hz, 1H), 6.40 (d, *J* = 7.9 Hz, 2H), 4.21 (d, *J* = 7.2 Hz, 2H), 3.80 (t, *J* = 7.5 Hz, 2H), 3.59-3.51 (m, 2H), 3.47 (s, 2H), 2.94 (p, *J* = 6.9 Hz, 1H), 1.47 (s, 9H).

**Step 3: 6-amino-4-((1-phenylazetidin-3-yl)methyl)-2H-benzo[b][1,4]thiazin-3(4H)-one (3-3)**

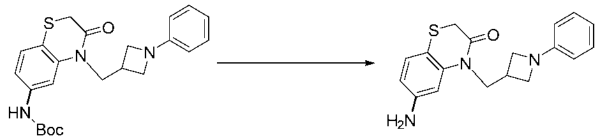

A mixture of 75 milligrams of t-butyl (3-oxo-4-((1-phenylazetidin-3-yl)methyl)-3,4-dihydro-2H-benzo[b][1,4]thiazin-6-yl)carbamate **(3-2)** and 5 milliliters of dioxane hydrochloride solution was stirred at room temperature for 12 hours. The reaction solution was concentrated to dryness to obtain solid 6-amino-4-((1-phenylazetidin-3-yl)methyl)-2H-benzo[b][1,4]thiazin-3(4H)-one **(3-3),** which was directly used in the next reaction step. LCMS (ESI) *m*/*z* [M+H]$^+$: 326.1.

**Step 4: 1-(1H-indol-3-yl)-3-(3-oxo-4-((1-phenylazetidin-3-yl)methyl)-3,4-dihydro-2H-benzo[b][1,4]thiazin-6-yl) urea (I-3)**

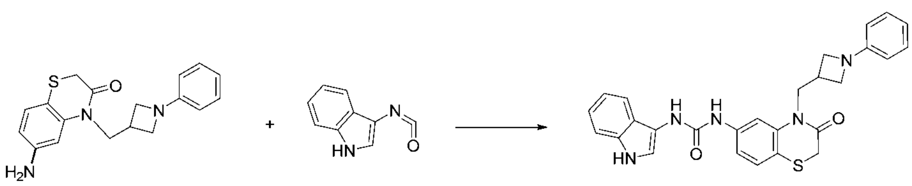

A mixture of 57 milligrams of 6-amino-4-((1-phenylazetidin-3-yl)methyl)-2H-benzo[b][1,4]thiazin-3(4H)-one **(3-3),** 27 milligrams of 3-isocyanato-1H-indole, 50 microliters of triethylamine, and 2 milliliters of tetrahydrofuran was stirred at room temperature for 12 hours. The reaction solution was concentrated to dryness, and the residue was subjected to column chromatography with dichloromethane: methanol = 95:5 to obtain 14.2 mg of solid 1-(1H-indol-3-yl)-3-(3-oxo-4-((1-phenylazetidin-3-yl)methyl)-3,4-dihydro-2H-benzo[b][1,4]thiazin-6-yl)urea (**I-3**) with a yield of 16.90%. $^1$H NMR (400 MHz, DMSO-$d_6$) $\delta$ 10.78 (s, 1H), 8.73 (s, 1H), 8.54 (s, 1H), 7.64 (d, *J* = 2.1 Hz, 1H), 7.54-7.45 (m, 2H), 7.32 (dd, *J* = 12.3, 8.2 Hz, 2H), 7.12 (dt, *J* = 20.6, 7.7 Hz, 4H), 7.01 (t, *J* = 7.4 Hz, 1H), 6.66 (t, *J* = 7.3 Hz, 1H), 6.40 (d, *J* = 8.0 Hz, 2H), 4.29 (d, *J* = 7.3 Hz, 2H), 3.81 (t, *J* = 7.4 Hz, 2H), 3.57 (t, *J*= 6.3 Hz, 2H), 3.49 (s, 2H), 2.97 (p, *J* = 6.9 Hz, 1H).

**Example 4 1-(4-benzyl-3-oxo-3,4-dihydro-2H-thieno[3,2-b][1,4]thiazin-6-yl)-3-(1H-indol-3-yl)urea (I-4)**

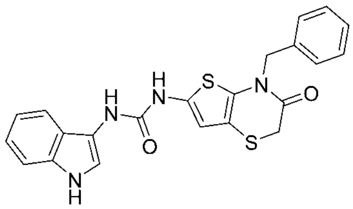

**Step 1: 4-benzyl-3-oxo-3,4-dihydro-2H-thieno[3,2-b][1,4]thiazin-6-carbonyl azide (4-1)**

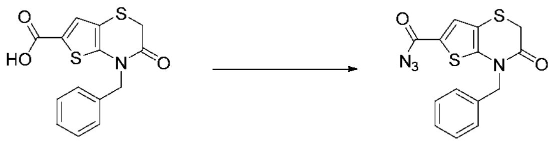

The preparation of 4-benzyl-3-oxo-3,4-dihydro-2H-thieno[3,2-b][1,4]thiazin-6-carbonyl azide **(4-1)** was the same as that of compound 3-1. $^1$H NMR (400 MHz, DMSO-$d_6$) $\delta$ 7.72 (s, 1H), 7.32 (t, *J* = 7.4 Hz, 2H), 7.25 (d, *J* = 7.2 Hz, 1H), 7.20 (d, *J* = 7.6 Hz, 2H), 5.19 (s, 2H), 3.89 (s, 2H).

**Step 2: *t*-butyl (4-benzyl-3-oxo-3,4-dihydro-2H-thieno[3,2-b][1,4]thiazin-6-yl)carbamate (4-2)**

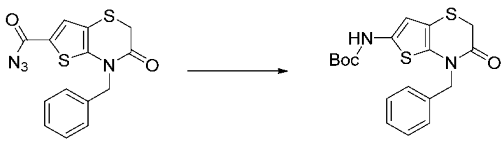

The preparation of *t*-butyl (4-benzyl-3-oxo-3,4-dihydro-2H-thieno[3,2-b][1,4]thiazin-6-yl)carbamate **(4-2)** was the same as that of compound **3-2,** which was directly used for the next reaction. LCMS (ESI) *m*/*z* [M+H]$^+$: 377.1.

**Step 3: 6-amino-4-benzyl-3-oxo-3,4-dihydro-2H-thieno[3,2-b][1,4]thiazin-3(4H)-one (4-3)**

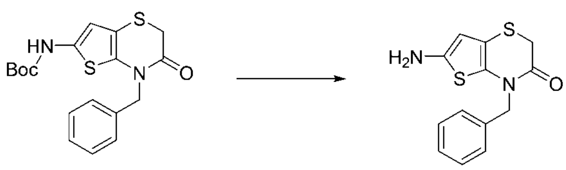

The preparation of 6-amino-4-benzyl-3-oxo-3,4-dihydro-2H-thieno[3,2-b][1,4]thiazin-3(4H)-one **(4-3)** was the same as that of compound **3-3,** which was directly used for the next reaction. LCMS (ESI) *m*/*z* [M+H]$^+$: 277.1.

**Step 4: 1-(4-benzyl-3-oxo-3,4-dihydro-2H-thieno[3,2-b][1,4]thiazin-6-yl)-3-(1H-indol-3-yl)urea (I-4)**

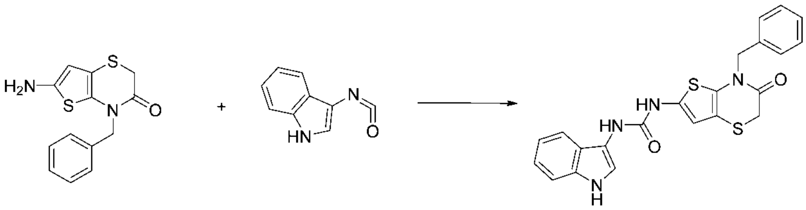

The preparation of 1-(4-benzyl-3-oxo-3,4-dihydro-2H-thieno[3,2-b][1,4]thiazin-6-yl)-3-(1H-indol-3-yl)urea **(I-4)** was the same as that of compound **I-3**. $^1$H NMR (400 MHz, $CD_3OD$) $\delta$ 7.45 (d, *J* = 7.9 Hz, 1H), 7.36-7.26 (m, 4H), 7.22 (d, *J* = 8.1 Hz, 3H), 7.12 (t, *J* = 7.6 Hz, 1H), 7.02 (t, *J* = 7.5 Hz, 1H), 6.34 (s, 1H), 5.06 (s, 2H), 3.61 (s, 2H).

**Example 5 1-(1H-indol-6-yl)-3-(3-oxo-4-((1-phenylazetidin-3-yl)methyl)-3,4-dihydro-2H-benzo[b][1,4]thiazin-6-yl)urea (1-5)**

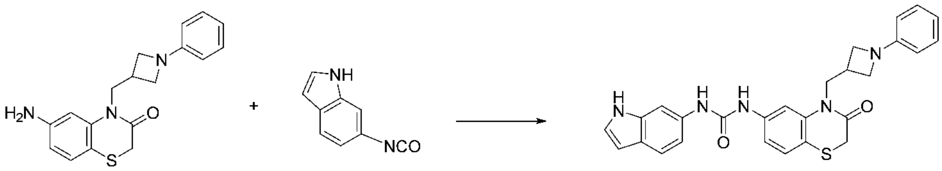

The preparation of 1-(1H-indol-6-yl)-3-(3-oxo-4-((1-phenylazetidin-3-yl)-3,4-dihydro-2H-benzo[b][1,4]thiazin-6-yl)urea (**I-5**) was the same as that of compound **I-3**. $^1$H NMR (400 MHz, $CD_3OD$) $\delta$ 7.68 (d, *J* = 30.7 Hz, 2H), 7.44 (d, *J* = 8.4 Hz, 1H), 7.28 (d, *J* = 8.3 Hz, 1H), 7.12 (dd, *J* = 15.6, 5.4 Hz, 3H), 7.04-6.93 (m, 1H), 6.93-6.83 (m, 1H), 6.67 (t, *J* = 7.2 Hz, 1H), 6.49-6.33 (m, 3H), 4.35 (d, *J* = 7.4 Hz, 2H), 3.83 (t, *J* = 7.5 Hz, 2H), 3.59 (q, *J* = 10.3, 6.6 Hz, 2H), 3.38 (s, 2H), 3.02 (dd, *J* = 14.3, 7.3 Hz, 1H).

**Example 6 1-benzyl-N-(1H-indol-3-yl)-2-oxo-2,3-dihydro-1H-thieno[2,3-b][1,4]thiazin-6-carboxamide (I-6)**

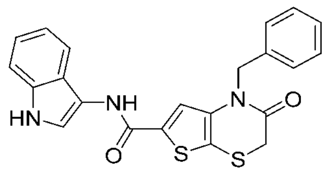

**Step 1: methyl 5-((2-methoxy-2-carbonylethyl)thiol)-4-nitrothiophene-2-carboxylate (6-1)**

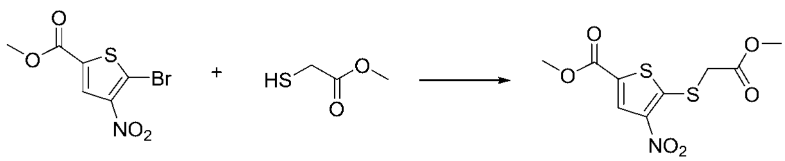

The preparation of methyl 5-((2-methoxy-2-carbonylethyl)thiol)-4-nitrothiophene-2-carboxylate **(6-1)** was the same as that of compound **1-2.** $^1$H NMR (400 MHz, DMSO-$d_6$) $\delta$ 8.17 (s, 1H), 4.38 (s, 2H), 3.86 (s, 3H), 3.73 (s, 3H).

**Step 2: methyl 2 -oxo-2,3-dihydro-1H-thieno[2,3-b] [1,4]thiazin-6-carboxylate (6-2)**

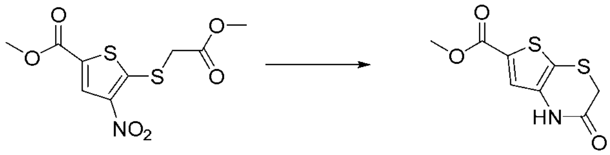

The preparation of methyl 2-oxo-2,3-dihydro-1H-thieno[2,3-b][1,4]thiazin-6-carboxylate **(6-2)** was the same as that of compound **1-3.** $^1$H NMR (400 MHz, DMSO-$d_6$) $\delta$ 11.29 (s, 1H), 7.56 (s, 1H), 3.77 (s, 3H), 3.54 (s, 2H).

**Step 3: methyl 1-benzyl-2-oxo-2,3-dihydro-1H-thieno[2,3-b][1,4]thiazin-6-carboxylate (6-3)**

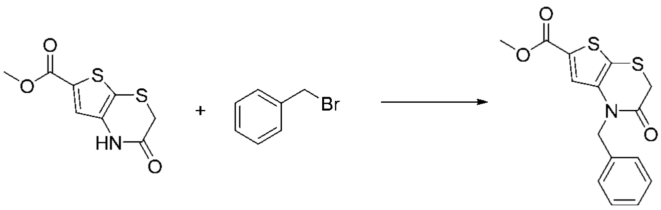

The preparation of methyl 1-benzyl-2-oxo-2,3-dihydro-1H-thieno[2,3-b][1,4]thiazin-6-carboxylate **(6-3)** was the same as that of compound **1-4.** $^1$H NMR (400 MHz, DMSO-$d_6$) $\delta$ 7.62 (s, 1H), 7.36 (dd, $J$ = 7.9, 6.6 Hz, 2H), 7.31-7.25 (m, 3H), 5.09 (s, 2H), 3.80 (s, 2H), 3.74 (s, 3H).

**Step 4: 1-benzyl-2-oxo-2,3-dihydro-1H-thieno[2,3-b][1,4]thiazin-6-carboxylic acid (6-4)**

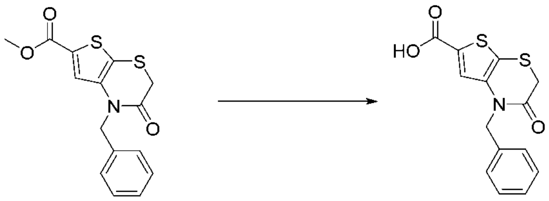

The preparation of 1-benzyl-2-oxo-2,3-dihydro-1H-thieno[2,3-b][1,4]thiazin-6-carboxylic acid **(6-4)** was the same as that of compound 1-5. LCMS (ESI) *m/z* $[M+H]^+$: 306.0.

**Step 5: 1-benzyl-N-(1H-indol-3-yl)-2-oxo-2,3-dihydro-1H-thieno[2,3-b][1,4]thiazin-6-carboxamide (I-6)**

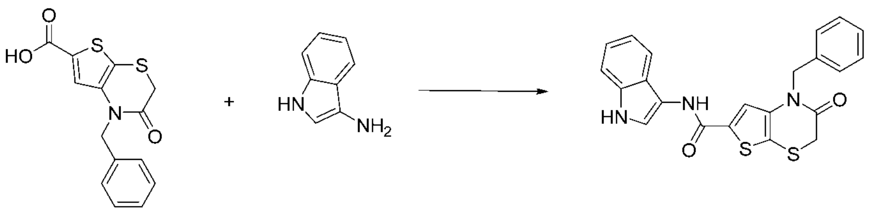

The preparation of 1-benzyl-N-(1H-indol-3-yl)-2-oxo-2,3-dihydro-1H-thieno[2,3-b][1,4]thiazin-6-carboxamide **(I-6)** was the same as that of compound **I-1.** $^1$H NMR (400 MHz, DMSO-$d_6$) $\delta$ 11.02 (s, 1H), 10.23 (s, 1H), 8.25 (s, 1H), 7.81 (s, 1H), 7.65 (d, $J$ = 2.6 Hz, 1H), 7.40-7.22 (m, 6H), 7.11 (t, $J$ = 7.5 Hz, 1H), 7.02 (t, $J$ = 7.5 Hz, 1H), 5.15 (s, 2H), 3.83 (s, 2H).

**Example 7 1-(1-benzyl-2-oxo-2,3-dihydro-1H-thieno[2,3-b][1,4]thiazin-6-yl)-3-(1H-indol-3-yl)urea (I-7)**

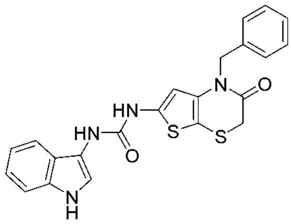

**Step 1: 1-benzyl-2-oxo-2,3-dihydro-1H-thieno[2,3-b] [1,4]thiazin-6-carbonyl azide (7-1)**

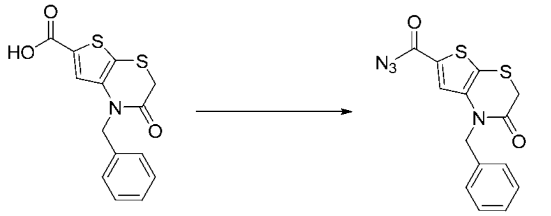

The preparation method of 1-benzyl-2-oxo-2,3-dihydro-1H-thieno[2,3-b][1,4]thiazin-6-carbonyl azide **(7-1)** was the same as that of compound **3-1.** $^1$H NMR (400 MHz, DMSO-*d$_6$*) $\delta$ 7.72 (s, 1H), 7.32 (t, *J* = 7.4 Hz, 2H), 7.25 (d, *J* = 7.2 Hz, 1H), 7.20 (d, *J* = 7.6 Hz, 2H), 5.19 (s, 2H), 3.89 (s, 2H).

**Step 2: t-butyl (1-benzyl-2-oxo-2,3-dihydro-1H-thieno[2,3-b][1,4]thiazin-6-yl)carbamate (7-2)**

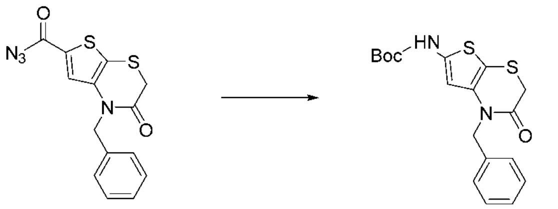

The preparation method of *t*-butyl (1-benzyl-2-oxo-2,3-dihydro-1H-thieno[2,3-b][1,4]thiazin-6-yl)carbamate **(7-2)** was the same as that of compound **3-2,** which was directly used for the next reaction. LCMS (ESI) *m/z* [M+H]$^+$: 377.1.

**Step 3: 6-amino-1-benzyl-1H-thieno[2,3-b][1,4]thiazin-2(3H)-one (7-3)**

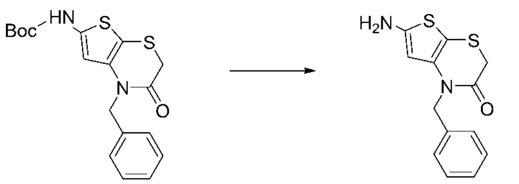

The preparation method of 6-amino-1-benzyl-1H-thieno[2,3-b][1,4]thiazin-2(3H)-one **(7-3)** was the same as that of compound **3-2,** which was directly used for the next reaction. LCMS (ESI) *m/z* [M+H]$^+$: 277.1.

**Step 4: 1-(1-benzyl-2-oxo-2,3-dihydro-1H-thieno[2,3-b][1,4]thiazin-6-yl)-3-(1H-indol-3-yl)urea (I-7)**

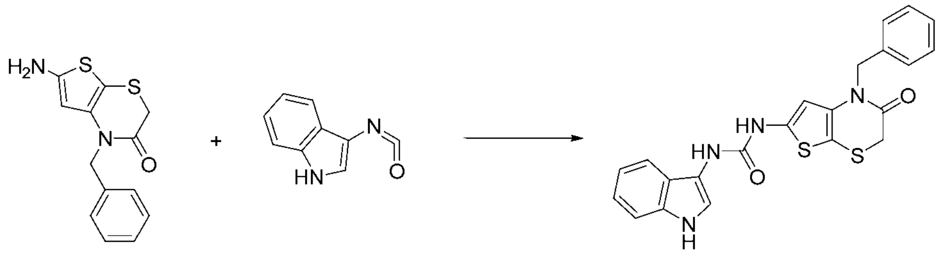

The preparation method of 1-(1-benzyl-2-oxo-2,3-dihydro-1H-thieno[2,3-b][1,4]thiazin-6-yl)-3-(1H-indol-3-yl) urea **(I-7)** was the same as that of compound **I-3**. $^{1}$H NMR (400 MHz, DMSO-$d_6$) $\delta$ 10.84 (s, 1H), 9.63 (s, 1H), 8.50 (s, 1H), 7.46-7.42 (m, 2H), 7.34 (dt, $J$ = 7.6, 3.4 Hz, 3H), 7.25 (dd, $J$ = 11.8, 7.2 Hz, 3H), 7.13-7.07 (m, 1H), 7.03-6.98 (m, 1H), 6.41 (s, 1H), 5.04 (s, 2H), 3.69 (s, 2H).

**Example 8 N-(1H-indol-6-yl)-3-oxo-4-((1-phenylazetidin-3-yl)methyl)-3,4-dihydro-2H-benzo[b][1,4]thiazin-6-carboxamide (I-8)**

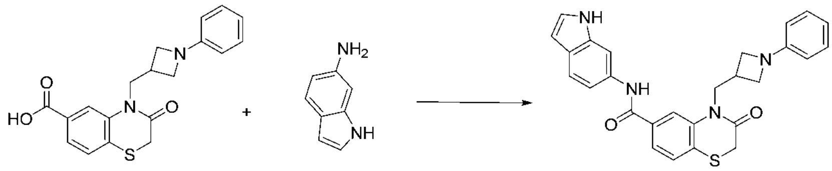

The preparation of N-(1H-indol-6-yl)-3-oxo-4-((1-phenylazetidin-3-yl)methyl)-3,4-dihydro-2H-benzo[b][1,4] thiazin-6-carboxamide **(I-8)** was the same as that of compound **I-3**. $^{1}$H NMR (400 MHz, DMSO-$d_6$) $\delta$ 11.08 (t, $J$ = 2.3 Hz, 1H), 10.20 (s, 1H), 8.05 (d, $J$ = 1.8 Hz, 1H), 7.89 (d, $J$ = 1.7 Hz, 1H), 7.70 (dd, $J$ = 8.1, 1.6 Hz, 1H), 7.60 (d, $J$ = 8.1 Hz, 1H), 7.49 (d, $J$ = 8.5 Hz, 1H), 7.30 (t, $J$ = 2.7 Hz, 1H), 7.26 (dd, $J$ = 8.5, 1.9 Hz, 1H), 7.18-7.10 (m, 2H), 6.69-6.62 (m, 1H), 6.46-6.33 (m, 3H), 4.46 (d, $J$ = 7.3 Hz, 2H), 3.79 (t, $J$ = 7.4 Hz, 2H), 3.59 (s, 2H), 3.54 (dd, $J$ = 7.2, 5.5 Hz, 2H), 2.93 (dq, $J$ = 15.5, 7.8 Hz, 1H).

**Example 9 4-((1-(3-fluoro-5-trifluoromethylphenyl)azetidin-3-yl)methyl)-N-(1H-indol-3-yl)-3-oxo-3,4-dihydro-2H-benzo[b][1,4]thiazin-6-carboxamide (I-9)**

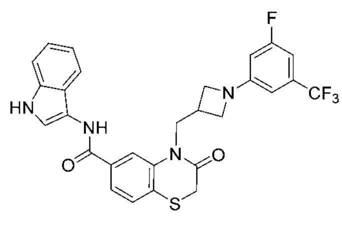

**Step 1: (1-(3-fluoro-5-(trifluoromethyl)phenyl)azetidin-3-yl)methanol (9-1)**

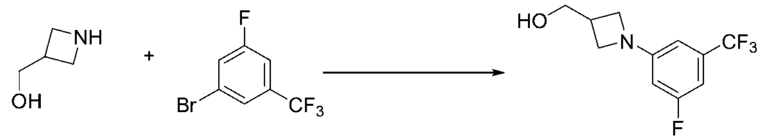

A mixture of 1 gram of 3-methylhydroxyazetidine hydrochloride, 1150 microliters of 3-bromo-5-fluorotrifluorotoluene, 680 milligrams of tris (dibenzylideneacetone) dipalladium, 1.38 grams of BINAP, 10 drops of triethylamine, 2.48 grams of potassium *tert*-butoxide, and 15 milliliters of toluene was heated and stirred at 110 °C under argon atmosphere for 12 hours. The reaction solution was concentrated to dryness, and the residue was subjected to column chromatography with petroleum ether: ethyl acetate = 60:40 to obtain 918.4 mg of an oily solid (1-(3-fluoro-5-(trifluoromethyl)phenyl) azetidin-3-yl)methanol **(9-1),** with a yield of 50.0%. $^1$H NMR (400 MHz, DMSO-$d_6$) $\delta$ 6.74 (dt, *J* = 8.9, 1.9 Hz, 1H), 6.52-6.42 (m, 2H), 4.81 (t, *J* = 5.3 Hz, 1H), 3.91 (t, *J* = 7.9 Hz, 2H), 3.63 (dd, *J* = 7.7, 5.4 Hz, 2H), 3.57 (t, *J* = 5.7 Hz, 2H), 2.80 (tt, *J* = 8.2, 5.8 Hz, 1H).

**Step 2: methyl 4-((1-(3-fluoro-5-(trifluoromethyl)phenyl)azetidin-3-yl)methyl)-3-oxo-3,4-dihydro-2H-benzo|b][1,4]thiophene-6-carboxylate (9-2)**

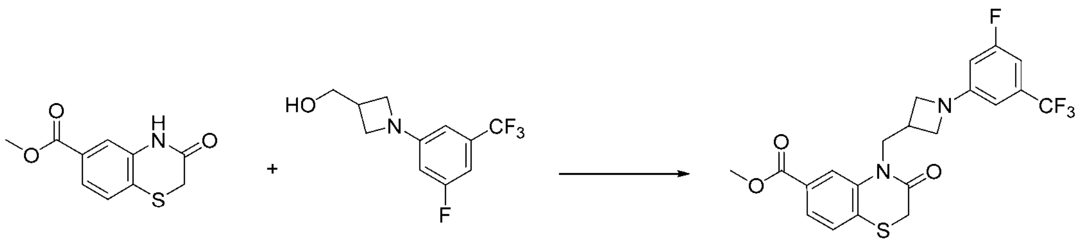

A mixture of 300 mg of methyl 3-carbonyl-3,4-dihydro-2H-1,4-benzothiazin-6-carboxylate, 395 mg of (1-(3-fluoro-5-(trifluoromethyl)phenyl)azetidin-3-yl)methanol **(9-1),** 387 mg of triphenylphosphine, 290 μL of DIAD, and 5 mL of tetrahydrofuran was stirred at room temperature for 12 hours. The reaction solution was concentrated to dryness, and the residue was subjected to column chromatography with petroleum ether: ethyl acetate=80:20 to obtain 465.3 mg of solid methyl 4-((1-(3-fluoro-5-(trifluoromethyl)phenyl)azetidin-3-yl)methyl)-3-oxo-3,4-dihydro-2H-benzo[b][1,4]thiophene-6-carboxylate **(9-2)** with a yield of 76.3%. $^1$H NMR (400 MHz, DMSO-$d_6$) $\delta$ 7.83 (s, 1H), 7.61 (q, *J*= 8.1 Hz, 2H), 6.77 (d, *J* = 8.8 Hz, 1H), 6.51-6.44 (m, 2H), 4.41 (d, *J* = 7.4 Hz, 2H), 3.90 (d, *J* = 7.9 Hz, 2H), 3.86 (d, *J* = 5.5 Hz, 3H), 3.62 (dd, *J* = 7.6, 5.2 Hz, 2H), 3.59 (s, 2H), 2.96-2.84 (m, 1H).

**Step 3: 4-((1-(3-fluoro-5-(trifluoromethyl)phenyl)azetidin-3-yl)methyl)-3-oxo-3,4-dihydro-2H-benzo[b][1,4]thiophene-6-carboxylic acid (9-3)**

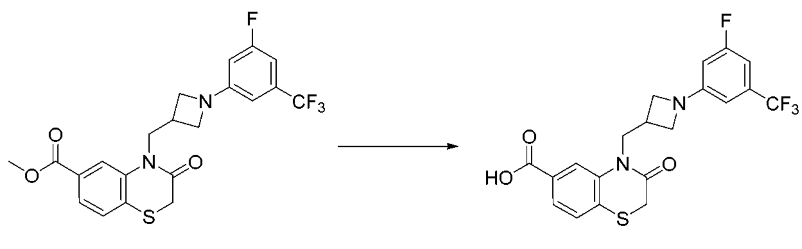

The preparation method of 4-((1-(3-fluoro-5-(trifluoromethyl)phenyl)azetidin-3-yl)methyl)-3-oxo-3,4-dihydro-2H-benzo[b][1,4]thiophene-6-carboxylic acid **(9-3)** was the same as that of **1-5.** LCMS (ESI) *m/z* [M+H]$^+$: 441.1.

**Step 4: 4-((1-(3-fluoro-5-(trifluoromethyl)phenyl)azetidin-3-yl)methyl)-N-(1H-indol-3-yl)-3-oxo-3,4-dihydro-2H-benzo[b][1,4]thiazin-6-carboxamide (I-9)**

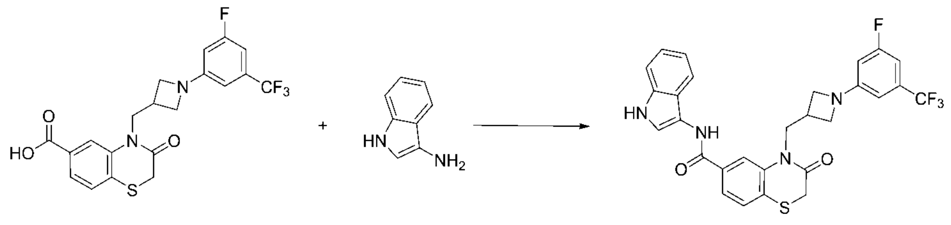

The preparation method of ((1-(3-fluoro-5-(trifluoromethyl)phenyl)azetidin-3-yl)methyl)-N-(1H-indol-3-yl)-3-oxo-3,4-dihydro-2H-benzo[b][1,4]thiazin-6-carboxamide **(I-9)**was the same as that of **I-1.** $^1$H NMR (400 MHz, DMSO-$d_6$) $\delta$ 10.96 (s, 1H), 10.23 (s, 1H), 7.93 (d, *J* = 1.7 Hz, 1H), 7.85-7.78 (m, 2H), 7.73 (dd, *J* = 8.1, 1.6 Hz, 1H), 7.60 (d, *J* = 8.0 Hz, 1H), 7.37 (dt, *J* = 8.2, 0.9 Hz, 1H), 7.16-7.09 (m, 1H), 7.00 (ddd, *J* = 8.0, 6.9, 1.0 Hz, 1H), 6.78 (d, *J* = 8.8 Hz, 1H), 6.53-6.46 (m, 2H), 4.49 (d, *J* = 7.3 Hz, 2H), 3.93 (t, *J* = 7.8 Hz, 2H), 3.68 (dd, *J* = 7.8, 5.4 Hz, 2H), 3.60 (s, 2H), 3.02 (d, *J* = 7.8 Hz, 1H).

**Example 10 4-((1-(benzo[d][1,3]dioxolan-5-yl)azetidin-3-yl)methyl)-N-(1H-indol-3-yl)-3-oxo-3,4-dihydro-2H-benzo[b][1,4]thiazin-6-carboxamide (I-10)**

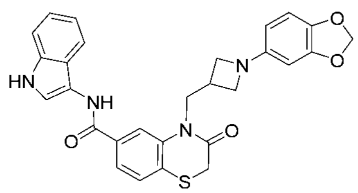

**Step 1: (1-(benzo[d][1,3]dioxolan-5-yl)azetidin-3-yl)methanol (10-1)**

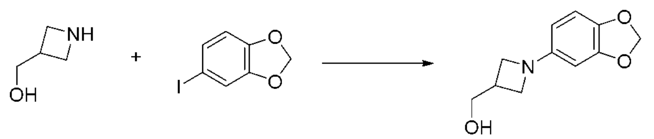

The preparation method of (1-(benzo[d][1,3]dioxolan-5-yl)azetidin-3-yl)methanol (10-1) was the same as that of compound 2-1. $^1$H NMR (400 MHz, DMSO-$d_6$) $\delta$ 6.71 (d, *J* = 8.2 Hz, 1H), 6.10 (d, *J* = 2.3 Hz, 1H), 5.86 (s, 2H), 5.79 (dd, *J* = 8.3, 2.3 Hz, 1H), 4.72 (t, *J* = 5.3 Hz, 1H), 3.71 (t, *J* = 7.4 Hz, 2H), 3.55 (dd, *J* = 6.5, 5.3 Hz, 2H), 3.43 (dd, *J* = 7.1, 5.4 Hz, 2H), 2.76-2.65 (m, 1H).

**Step 2: methyl 4-((1-((benzo[d][1,3]dioxolan-5-yl)azetidin-3-yl)methyl)-3-oxo-3,4-dihydro-2H-benzo[b][1,4]thiazin-6-carboxylate (10-2)**

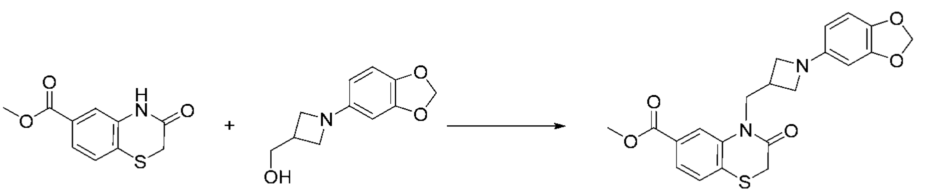

The preparation method of methyl 4-((1-((benzo[d][1,3]dioxolan-5-yl)azetidin-3-yl)methyl)-3-oxo-3,4-dihydro-2H-benzo[b][1,4]thiazin-6-carboxylate **(10-2)** was the same as that of compound **9-2.** $^1$H NMR (400 MHz, DMSO-$d_6$) $\delta$ 7.66-7.58 (m, 2H), 7.47 (d, *J* = 8.0 Hz, 1H), 6.74 (d, *J* = 8.3 Hz, 1H), 6.16 (d, *J* = 2.3 Hz, 1H), 5.90-5.81 (m, 3H), 4.54 (d, *J* = 7.0 Hz, 2H), 3.84 (d, *J* = 3.7 Hz, 5H), 3.60-3.53 (m, 4H), 3.08 (tq, *J* = 7.8, 5.6 Hz, 1H).

**Step 3: 4-((1-((benzo[d][1,3]dioxolan-5-yl)azetidin-3-yl)methyl)-3-oxo-3,4-dihydro-2H-benzo[b][1,4]thiazin-6-carboxylic acid (10-3)**

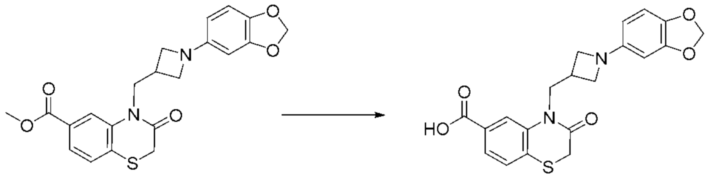

The preparation method of 4-((1-((benzo[d][1,3]dioxolan-5-yl)azetidin-3-yl)methyl)-3-oxo-3,4-dihydro-2H-benzo[b][1,4]thiazin-6-carboxylic acid **(10-3)** was the same as that of compound **9-3.** LCMS (ESI) *m/z* $[M+H]^+$: 399.1.

**Step 4: 4-((1-(3-fluoro-5-(trifluoromethyl)phenyl)azetidin-3-yl)methyl)-N-(1H-indol-3-yl)-3-oxo-3,4-dihydro-2H-benzo[b][1,4]thiazin-6-carboxamide (I-10)**

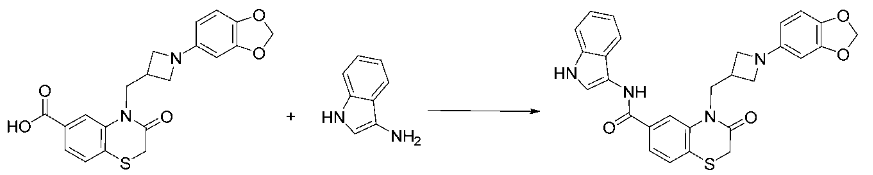

The preparation method of 4-((1-(3-fluoro-5-(trifluoromethyl)phenyl)azetidin-3-yl)methyl)-N-(1H-indol-3-yl)-3-oxo-3,4-dihydro-2H-benzo[b][1,4]thiazin-6-carboxamide **(I-10)** was the same as that of compound **I-1.** $^1H$ NMR (400 MHz, DMSO-$d_6$) $\delta$ 10.95 (s, 1H), 10.23 (s, 1H), 7.91 (d, *J* = 1.6 Hz, 1H), 7.85-7.77 (m, 2H), 7.72 (dd, *J* = 8.0, 1.6 Hz, 1H), 7.59 (d, *J* = 8.1 Hz, 1H), 7.36 (dt, *J* = 8.2, 0.9 Hz, 1H), 7.11 (ddd, *J* = 8.2, 7.0, 1.2 Hz, 1H), 7.00 (ddd, *J* = 8.0, 6.9, 1.1 Hz, 1H), 6.69 (d, *J* = 8.3 Hz, 1H), 6.10 (d, *J* = 2.2 Hz, 1H), 5.85 (s, 2H), 5.78 (dd, *J* = 8.3, 2.3 Hz, 1H), 4.44 (d, *J* = 7.3 Hz, 2H), 3.72 (t, *J* = 7.3 Hz, 2H), 3.58 (s, 2H), 3.49-3.46 (m, 2H), 2.89 (dd, *J* = 13.7, 7.1 Hz, 1H).

**Example 11 N-(1H-indol-3-yl)-3,3-dimethyl-2-oxo-1-((1-phenylazetidin-3-yl)methyl)indolin-6-carboxamide (I-11)**

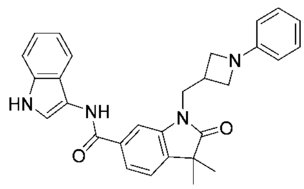

**Step 1: 6-bromo-3,3-dimethyl-1-((1-phenylazetidin-3-yl)methyl)dihydroindol-2-one (11-1)**

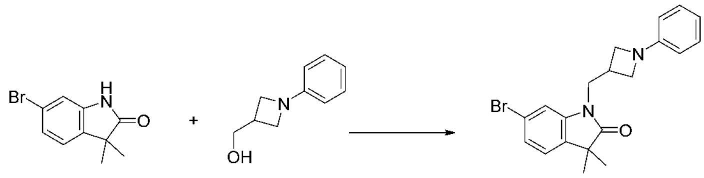

The preparation method of 6-bromo-3,3-dimethyl-1-((1-phenylazetidin-3-yl)methyldihydroindol-2-one **(11-1)** was the same as that of compound **9-2.** $^1H$ NMR (400 MHz, DMSO-$d_6$) $\delta$ 7.45 (d, *J* = 1.8 Hz, 1H), 7.31 (d, *J* = 7.9 Hz,

1H), 7.22 (dd, *J* = 7.8, 1.7 Hz, 1H), 7.16-7.11 (m, 2H), 6.65 (t, *J* = 7.3 Hz, 1H), 6.41-6.37 (m, 2H), 3.98 (d, *J* = 7.4 Hz, 2H), 3.82 (t, *J* = 7.5 Hz, 2H), 3.54 (dd, *J* = 7.3, 5.4 Hz, 2H), 3.12-3.01 (m, 1H), 1.25 (s, 6H).

**Step 2: 3,3-dimethyl-2-oxo-1-((1-phenylazetidin-3-yl)methyl)dihydroindol-6-nitrile (11-2)**

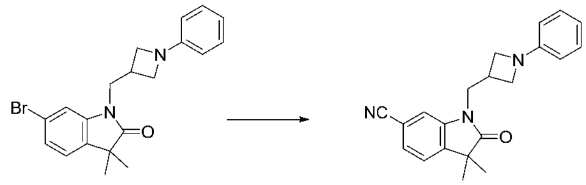

A mixture of 540 mg of 6-bromo-3,3-dimethyl-1-((1-phenylazetidin-3-yl)methyl)dihydroindol-2-one **(11-1),** 304 mg of zinc cyanide, 120 mg of tris (dibenzylideneacetone) dipalladium, 144 mg of DPPF, and 8 mL of N, N-dimethylformamide was heated and stirred at 100 °C under an argon atmosphere for 12 hours. The reaction solution was diluted with water, and extracted with ethyl acetate, and the organic layer was concentrated to dryness. The residue was subjected to column chromatography with dichloromethane: ethyl acetate = 85:15 to obtain 411.3 mg of white solid 3,3-dimethyl-2-oxo-1-((1-phenylazetidin-3-yl)methyl)dihydroindol-6-nitrile **(11-2),** with a yield of 88.5%. $^1$H NMR (400 MHz, DMSO-$d_6$) $\delta$ 7.72 (d, *J* = 1.4 Hz, 1H), 7.59-7.53 (m, 2H), 7.15-7.11 (m, 2H), 6.68-6.63 (m, 1H), 6.41-6.37 (m, 2H), 4.01 (d, *J* = 7.3 Hz, 2H), 3.83 (t, *J* = 7.5 Hz, 2H), 3.54 (dd, *J* = 7.2, 5.4 Hz, 2H), 3.12 (q, *J* = 6.6 Hz, 1H), 1.29 (s, 6H).

**Step 3: 3,3-dimethyl-2-oxo-1-((1-phenylazetidin-3-yl)methyl)dihydroindol-6-carboxylic acid (11-3)**

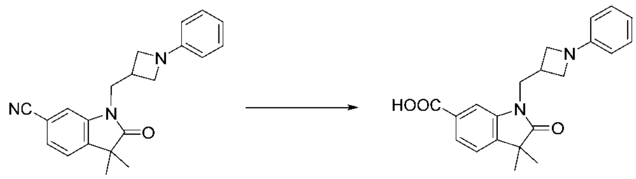

A mixture of 410 mg of 3,3-dimethyl-2-oxo-1-((1-phenylazetidin-3-yl)methyl)dihydroindol-6-nitrile, 2.1 mL of 6N NaOH, and 3 mL of ethanol was heated and stirred at 80 ° C for 3 hours. The reaction solution was concentrated to dryness to obtain solid 3,3-dimethyl-2-oxo-1-((1-phenylazetidin-3-yl)methyl)dihydroindol-6-carboxylic acid **(11-3),** which was directly used in the next step. LCMS (ESI) *m/z* [M+H]$^+$: 351.2.

**Step 4: N-(1H-indol-3-yl)-3,3-dihydro-2-oxo-1-((1-phenylazetidin-3-yl)methyl)dihydroindol-6-carboxamide (I-11)**

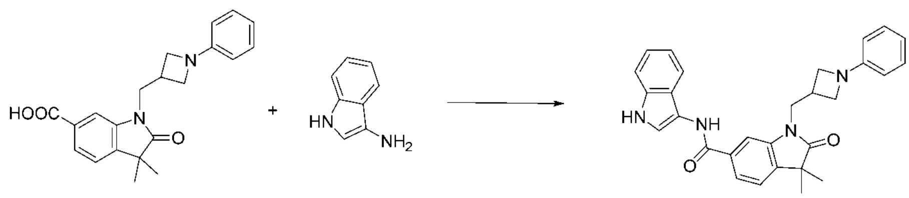

The preparation method of N-(1H-indol-3-yl)-3,3-dihydro-2-oxo-1-((1-phenylazetidin-3-yl)methyl)dihydroindol-6-carboxamide **(I-11)** was the same as that of compound **I-1.** $^1$H NMR (400 MHz, DMSO-$d_6$) $\delta$ 10.94 (s, 1H), 10.13 (s, 1H), 7.83 (d, *J* = 8.0 Hz, 1H), 7.79 (d, *J* = 2.5 Hz, 1H), 7.73-7.68 (m, 2H), 7.52 (d, *J* = 7.6 Hz, 1H), 7.38-7.35 (m, 1H), 7.17-7.11 (m, 3H), 7.01 (ddd, *J* = 8.0, 7.0, 1.0 Hz, 1H), 6.68-6.63 (m, 1H), 6.43-6.39 (m, 2H), 4.08 (d, *J* = 7.4 Hz, 2H), 3.88 (t, *J* = 7.5 Hz, 2H), 3.61 (dd, *J* = 7.3, 5.3 Hz, 2H), 3.20 (dq, *J* = 11.8, 5.8, 5.4 Hz, 1H), 1.32 (s, 6H).

**Example 12 1-(3,3-dimethyl-2-oxo-1-((1-phenylazetidin-3-yl)methyl)indolin-6-yl)-3-(1H-indol-3-yl)urea (I-12)**

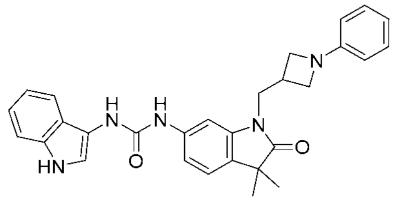

**Step 1: (3,3-dimethyl-2-oxo-1-((1-phenylazetidin-3-yl)methyl)dihydroindol-6-carbonyl azide (12-1)**

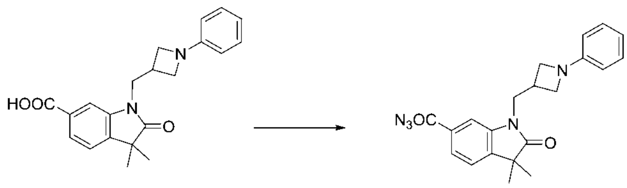

The preparation method of (3,3-dimethyl-2-oxo-1-((1-phenylazetidin-3-yl)methyl)dihydroindol-6-carbonyl azide (12-1) was the same as that of compound **3-1.** $^{1}H$ NMR (400 MHz, $CDCl_3$) $\delta$ 7.79 (dd, *J* = 7.7, 1.5 Hz, 1H), 7.51 (d, *J* = 1.5 Hz, 1H), 7.38-7.27 (m, 3H), 6.75 (tt, *J* = 7.4, 1.1 Hz, 1H), 6.47-6.42 (m, 2H), 4.06 (d, *J* = 7.3 Hz, 2H), 3.96 (d, *J* = 7.7 Hz, 2H), 3.71 (dd, *J* = 7.2, 5.2 Hz, 2H), 3.16 (ddd, *J* = 12.8, 7.6, 5.2 Hz, 1H), 1.39 (s, 6H).

**Step 2: *t*-butyl (3,3-dimethyl-2-oxo-1-((1-phenylazetidin-3-yl)methyl)dihydroindol-6-yl)carbamate (12-2)**

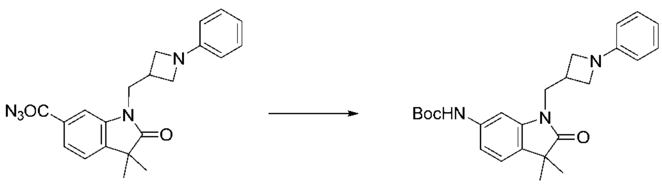

The preparation method of *t*-butyl (3,3-dimethyl-2-oxo-1-((1-phenylazetidin-3-yl)methyl)dihydroindol-6-yl)carbamate **(12-2)** was the same as that of compound **3-2.** LCMS (ESI) *m/z* $[M+H]^+$: 422.2.

**Step 3: 6-amino-3,3-dimethyl-2-oxo-1-((1-phenylazetidin-3-yl)methyl)dihydroindol-2-one (12-3)**

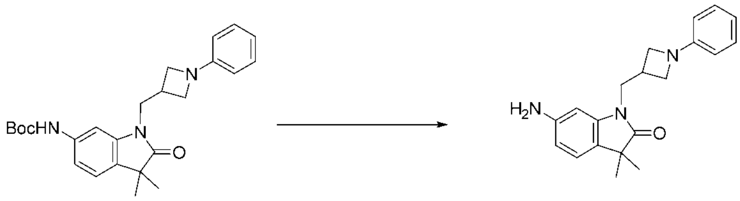

The preparation method of 6-amino-3,3-dimethyl-2-oxo-1-((1-phenylazetidin-3-yl)methyl)dihydroindol-2-one **(12-3)** was the same as that of compound **3-3.** LCMS (ESI) *m/z* $[M+H]^+$: 322.2.

**Step 4: 1-(3,3-dimethyl-2-oxo-1-((1-phenylazetidin-3-yl)methyl)dihydroindol-6-yl)-3-(1H-indol-3-yl)urea (I-12)**

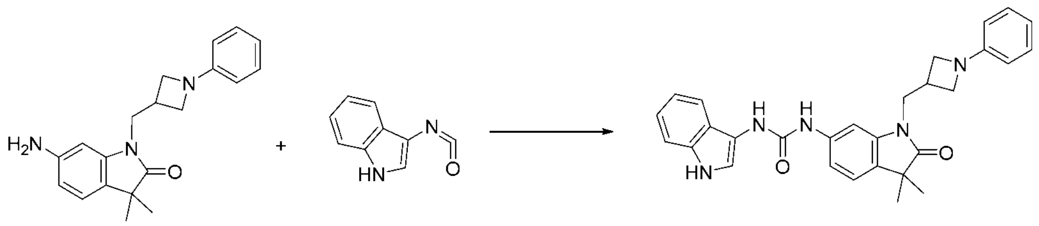

The preparation method of 1-(3,3-dimethyl-2-oxo-1-((1-phenylazetidin-3-yl)methyl)dihydroindol-6-yl)-3-(1H-indol-3-yl)urea **(I-12)** was the same as that of compound **I-3.** $^1$H NMR (400 MHz, DMSO-$d_6$) $\delta$ 10.75 (s, 1H), 8.68 (s, 1H), 8.49 (s, 1H), 7.52 (dd, *J* = 5.3, 2.8 Hz, 2H), 7.39 (d, *J* = 1.9 Hz, 1H), 7.34 (d, *J* = 8.1 Hz, 1H), 7.23 (d, *J* = 8.0 Hz, 1H), 7.15 (dd, *J* = 8.4, 7.2 Hz, 2H), 7.09 (d, *J* = 7.7 Hz, 1H), 7.04-6.99 (m, 2H), 6.67 (t, *J* = 7.3 Hz, 1H), 6.41 (d, *J* = 7.9 Hz, 2H), 3.96 (d, *J* = 7.3 Hz, 2H), 3.86 (t, *J* = 7.5 Hz, 2H), 3.60 (dd, *J* = 7.3, 5.3 Hz, 2H), 3.13-3.02 (m, 1H), 1.25 (s, 6H).

**Example 13 1-(4-((1-(benzo[d][1,3]dioxolan-5-yl)azetidin-3-yl)methyl)-3-oxo-3,4-dihydro-2H-benzo[b][1,4]thiazin-6-yl)-3-(1H-indol-3-yl)urea (I-13)**

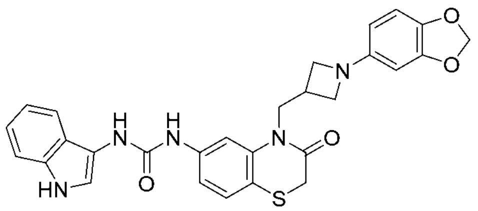

**Step 1: 4-((1-(benzo[d][1,3]dioxolan-5-yl)azetidin-3-yl)methyl)-3-oxo-3,4-dihydro-2H-benzo[b][1,4]thiazin-6-carbonyl azide (13-1)**

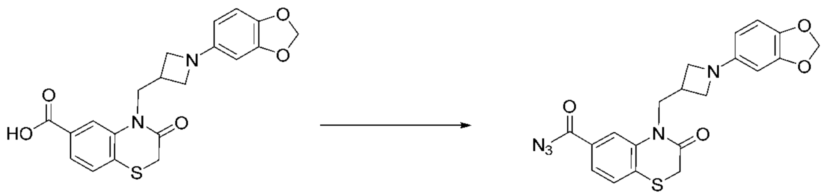

The preparation method of 4-((1-(benzo[d][1,3]dioxolan-5-yl)azetidin-3-yl)methyl)-3-oxo-3,4-dihydro-2H-benzo [b][1,4]thiazin-6-carbonyl azide **(13-1)** was the same as that of compound 3-1. $^1$H NMR (400 MHz, DMSO-$d_6$) $\delta$ 6.71 (d, *J* = 8.2 Hz, 1H), 6.10 (d, *J* = 2.3 Hz, 1H), 5.85 (s, 2H), 5.78 (dd, *J* = 8.3, 2.3 Hz, 1H), 4.72 (s, 1H), 3.71 (t, *J* = 7.4 Hz, 2H), 3.55 (d, *J* = 6.5 Hz, 2H), 3.43 (dd, *J* = 7.1, 5.5 Hz, 2H), 2.77-2.64 (m, 1H).

**Step 2: *t*-butyl (4-((1-(benzo[d][1,3]dioxolan-5-yl)azetidin-3-yl)methyl)-3-oxo-3,4-dihydro-2H-benzo[b][1,4]thiazin-6-yl)carbamate (13-2)**

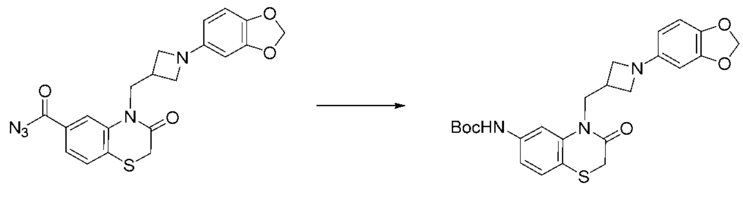

The preparation method of *t*-butyl (4-((1-(benzo[d][1,3]dioxolan-5-yl)azetidin-3-yl)methyl)-3-oxo-3,4-dihydro-2H-benzo[b][1,4]thiazin-6-yl)carbamate **(13-2)** was the same as that of compound **3-2.** LCMS (ESI) *m*/*z* $[M+H]^+$: 470.2.

**Step 3: 6-amino-4-((1-(benzo[d][1,3]dioxolan-5-yl)azetidin-3-yl)methyl)-3-oxo-3,4-dihydro-2H-benzo[b][1,4]thiazin-3(4H)-one (13-3)**

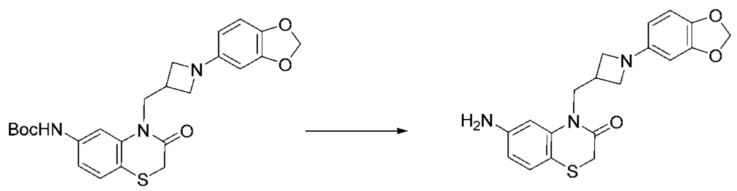

The preparation method of 6-amino-4-((1-(benzo[d][1,3]dioxolan-5-yl)azetidin-3-yl)methyl)-3-oxo-3,4-dihydro-2H-benzo[b][1,4]thiazin-3(4H)-one **(13-3)** was the same as that of compound **3-3.** LCMS (ESI) m/z $[M+H]^+$: 370.2.

**Step 4: 1-(4-((1-(benzo[d][1,3]dioxolan-5-yl)azetidin-3-yl)methyl)-3-oxo-3,4-dihydro-2H-benzo[b][1,4]thiazin-6-yl)-3-(1H-indol-3-yl)urea (I-13)**

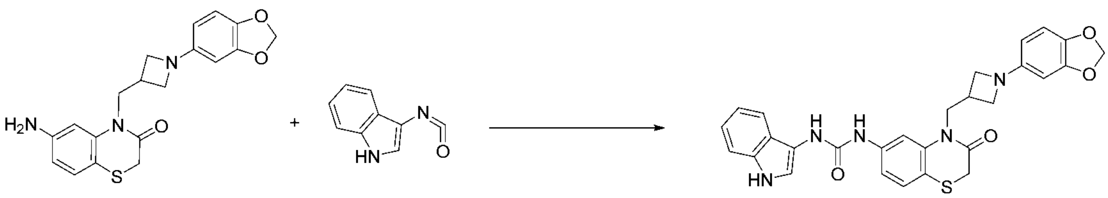

The preparation method of 1-(4-((1-(benzo[d][1,3]dioxolan-5-yl)azetidin-3-yl)methyl)-3-oxo-3,4-dihydro-2H-benzo[b][1,4]thiazin-6-yl)-3-(1H-indol-3-yl)urea **(I-13)** was the same as that of compound **I-3**. $^1$H NMR (400 MHz, DMSO-*d*$_6$) $\delta$ 10.74 (d, *J* = 2.5 Hz, 1H), 8.84 (s, 1H), 8.61 (s, 1H), 7.62 (d, *J*= 2.1 Hz, 1H), 7.53 (d, *J* = 7.9 Hz, 1H), 7.50 (d, *J* = 2.4 Hz, 1H), 7.35-7.28 (m, 2H), 7.15-7.07 (m, 2H), 7.00 (ddd, *J* = 7.9, 7.0, 1.1 Hz, 1H), 6.71 (d, *J* = 8.3 Hz, 1H), 6.12 (d, *J* = 2.3 Hz, 1H), 5.86 (s, 2H), 5.80 (dd, *J* = 8.3, 2.3 Hz, 1H), 4.26 (d, *J* = 7.1 Hz, 2H), 3.74 (t, *J* = 7.4 Hz, 2H), 3.52-3.44 (m, 4H), 2.91 (p, *J*= 6.8 Hz, 1H).

**Example 14 N-(1H-indol-3-yl)-1-((1-4-methoxyphenylazetidin-3-yl)methyl)-3,3-dimethyl-2-oxoindolin-6-carboxamide (I-14)**

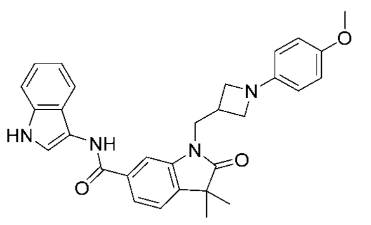

**Step 1: (1-(4-methoxyphenyl)azetidin-3-yl)methanol (14-1)**

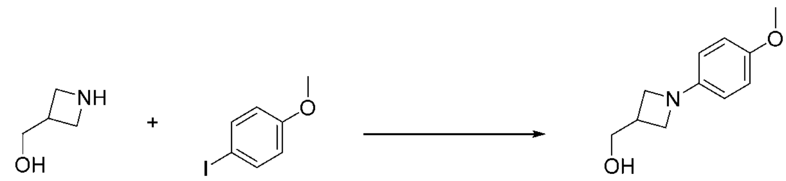

The preparation of (1-(4-methoxyphenyl)azetidin-3-yl)methanol **(14-1)** was the same as that of compound **2-1.** $^1$H NMR (400 MHz, DMSO-$d_6$) $\delta$ 6.83-6.72 (m, 2H), 6.41-6.30 (m, 2H), 4.70 (t, *J* = 5.3 Hz, 1H), 3.73 (t, *J* = 7.3 Hz, 2H), 3.65 (s, 3H), 3.56 (dd, *J* = 6.5, 5.3 Hz, 2H), 3.44 (dd, *J* = 6.9, 5.5 Hz, 2H), 2.78-2.66 (m, 1H).

**Step 2: 6-bromo-1-((1-(4-methoxyphenyl)azetidin-3-yl)methyl)-3,3-dimethylindolin-2-one (14-2)**

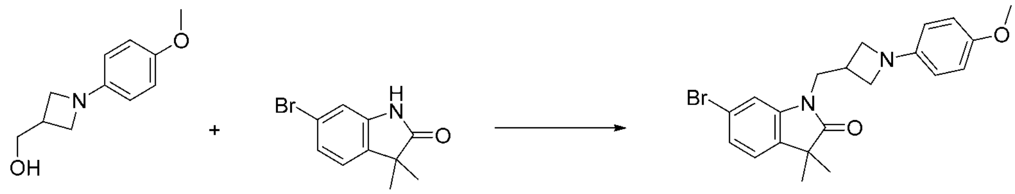

The preparation of 6-bromo-1-((1-(4-methoxyphenyl)azetidin-3-yl)methyl)-3,3-dimethylindolin-2-one **(14-2)** was the same as that of compound **11-1.** $^1$H NMR (400 MHz, DMSO-$d_6$) $\delta$ 7.43 (d, *J* = 1.8 Hz, 1H), 7.31 (d, *J* = 7.9 Hz, 1H), 7.24-7.20 (m, 1H), 6.79-6.74 (m, 2H), 6.38-6.33 (m, 2H), 3.97 (d, *J* = 7.3 Hz, 2H), 3.76 (t, *J* = 7.3 Hz, 2H), 3.65 (s, 3H), 3.47 (t, *J* = 6.2 Hz, 2H), 3.04 (p, *J* = 6.4 Hz, 1H), 1.25 (s, 6H).

**Step 3: 1-((1-(4-methoxyphenyl)azetidin-3-yl)methyl)-3,3-dimethyl-2-indolin-6-nitrile (14-3)**

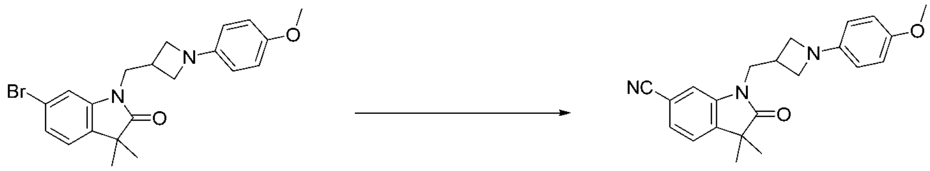

The preparation of 1-((1-(4-methoxyphenyl)azetidin-3-yl)methyl)-3,3-dimethyl-2-indolin-6-nitrile **(14-3)** was the same as that of compound **11-2.** $^1$H NMR (400 MHz, DMSO-$d_6$) $\delta$ 7.71 (d, *J* = 1.4 Hz, 1H), 7.59 (d, *J* = 7.6 Hz, 1H), 7.55-7.51 (m, 1H), 6.79-6.74 (m, 2H), 6.38-6.34 (m, 2H), 4.00 (d, *J* = 7.3 Hz, 2H), 3.76 (t, *J* = 7.4 Hz, 2H), 3.64 (s, 3H), 3.47 (dd, *J* = 7.1, 5.4 Hz, 2H), 3.07 (p, *J* = 6.5 Hz, 1H), 1.29 (s, 6H).

**Step 4: 1-((1-(4-methoxyphenyl)azetidin-3-yl)methyl)-3,3-dimethyl-2-indolin-6-carboxylic acid (14-4)**

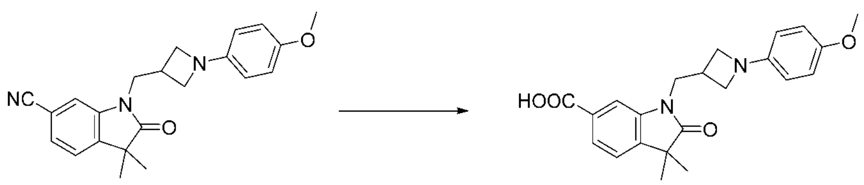

The preparation method of 1-((1-(4-methoxyphenyl)azetidin-3-yl)methyl)-3,3-dimethyl-2-indolin-6-carboxylic acid **(14-4)** was the same as that of compound **11-3.** LCMS (ESI) *m/z* $[M+H]^+$: 381.2.

**Step 5: N-(1H-indol-3-yl)-1-((1-(4-methoxyphenyl)azetidin-3-yl)methyl)-3,3-dimethyl-2-indolin-6-carboxamide (I-14)**

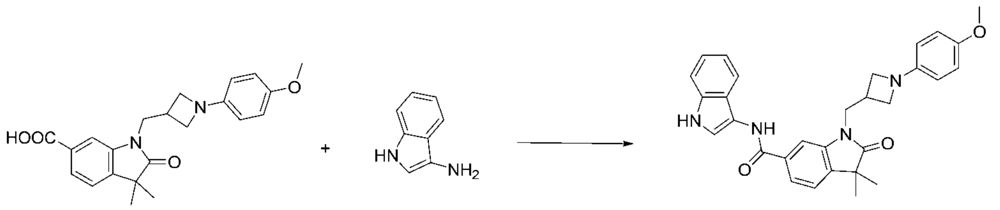

The preparation method of N-(1H-indol-3-yl)-1-((1-(4-methoxyphenyl)azetidin-3-yl)methyl)-3,3-dimethyl-2-indolin-6-carboxamide **(I-14)** was the same as that of compound **I-1.** $^1$H NMR (400 MHz, DMSO-$d_6$) $\delta$ 10.93 (s, 1H), 10.12 (s, 1H), 7.83 (d, *J* = 8.0 Hz, 1H), 7.78 (d, *J* = 2.5 Hz, 1H), 7.72 (dd, *J* = 7.7, 1.4 Hz, 1H), 7.68 (d, *J* = 1.5 Hz, 1H), 7.52 (d, *J* = 7.6 Hz, 1H), 7.37 (d, *J* = 8.1 Hz, 1H), 7.12 (ddd, *J* = 8.2, 7.0, 1.2 Hz, 1H), 7.01 (ddd, *J* = 8.0, 6.9, 1.0 Hz, 1H), 6.81-6.75 (m, 2H), 6.40-6.35 (m, 2H), 4.07 (d, *J* = 7.3 Hz, 2H), 3.81 (t, *J* = 7.3 Hz, 2H), 3.65 (s, 3H), 3.54 (dd, *J* = 7.1, 5.4 Hz, 2H), 3.17 (p, *J* = 6.8 Hz, 1H), 1.32 (s, 6H).

**Example 15 N-(1H-indol-3-yl)-1-((1-3-methoxyphenylazetidin-3-yl)methyl)-3,3-dimethyl-2-oxoindolin-6-carboxamide (I-15)**

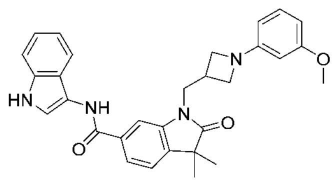

**Step 1: (1-(3-methoxyphenyl)azetidin-3-yl)methanol (15-1)**

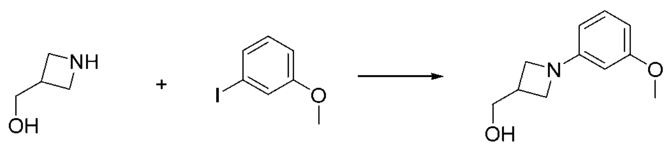

The preparation of (1-(3-methoxyphenyl)azetidin-3-yl)methanol **(15-1)** was the same as that of compound **2-1.** $^1$H NMR (400 MHz, DMSO-$d_6$) $\delta$ 7.04 (td, *J* = 8.1, 1.7 Hz, 1H), 6.22 (dd, *J* = 8.2, 2.3 Hz, 1H), 5.98 (dd, *J* = 8.0, 2.1 Hz, 1H), 5.91 (q, *J* = 2.1 Hz, 1H), 3.79 (td, *J*=7.5, 1.7 Hz, 2H), 3.69 (d, *J* = 1.8 Hz, 3H), 3.57 (dd, *J* = 6.5, 1.8 Hz, 2H), 3.51 (ddd, *J* = 7.2, 5.4, 1.7 Hz, 2H), 2.81-2.68 (m, 1H).

**Step 2: 6-bromo-1-((1-(3-methoxyphenyl)azetidin-3-yl)methyl)-3,3-dimethylindolin-2-one (15-2)**

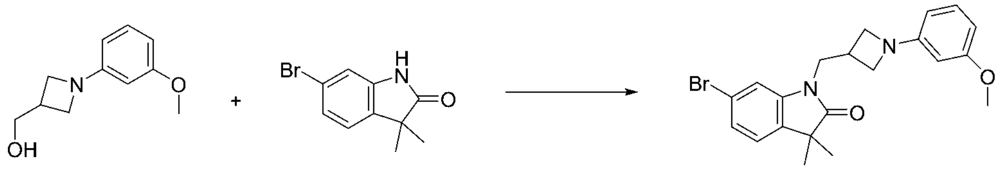

The preparation of 6-bromo-1-((1-(3-methoxyphenyl)azetidin-3-yl)methyl)-3,3-dimethylindolin-2-one (15-2) was the same as that of compound **11-1.** $^1$H NMR (400 MHz, DMSO-$d_6$) $\delta$ 7.45 (d, $J$ = 1.8 Hz, 1H), 7.31 (d, $J$ = 7.9 Hz, 1H), 7.25-7.21 (m, 1H), 7.04 (t, $J$ = 8.1 Hz, 1H), 6.24 (dd, $J$ = 8.1, 2.4 Hz, 1H), 5.98 (dd, $J$ = 7.9, 2.1 Hz, 1H), 5.91 (t, $J$ = 2.3 Hz, 1H), 3.98 (d, $J$ = 7.4 Hz, 2H), 3.82 (t, $J$ = 7.5 Hz, 2H), 3.68 (s, 3H), 3.54 (dd, $J$ = 7.3, 5.3 Hz, 2H), 3.06 (ddd, $J$ = 13.1, 6.5, 3.8 Hz, 1H), 1.26 (s, 6H).

**Step 3: 1-((1-(3-methoxyphenyl)azetidin-3-yl)methyl)-3,3-dimethyl-2-indolin-6-nitrile (15-3)**

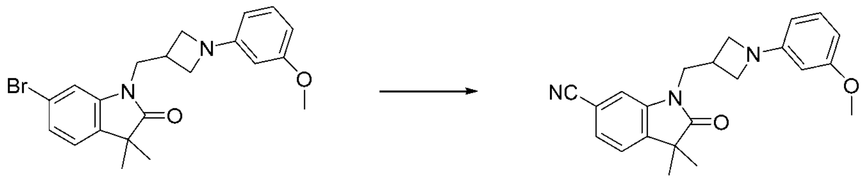

The preparation of 1-((1-(3-methoxyphenyl)azetidin-3-yl)methyl)-3,3-dimethyl-2-indolin-6-nitrile **(15-3)** was the same as that of compound **11-2.** $^1$H NMR (400 MHz, DMSO-$d_6$) $\delta$ 7.73 (d, $J$ = 1.4 Hz, 1H), 7.59 (d, $J$ = 7.6 Hz, 1H), 7.53 (dd, $J$ = 7.6, 6.3 Hz, 1H), 7.04 (t, $J$ = 8.1 Hz, 1H), 6.24 (dd, $J$ = 8.1, 2.4 Hz, 1H), 5.98 (dd, $J$ = 7.7, 2.1 Hz, 1H), 5.92 (t, $J$ = 2.3 Hz, 1H), 4.01 (d, $J$ = 7.3 Hz, 2H), 3.82 (t, $J$ = 7.5 Hz, 2H), 3.68 (s, 3H), 3.54 (dd, $J$ = 7.3, 5.3 Hz, 2H), 3.17-3.05 (m, 1H), 1.29 (s, 6H).

**Step 4: 1-((1-(3-methoxyphenyl)azetidin-3-yl)methyl)-3,3-dimethyl-2-indolin-6-carboxylic acid (15-4)**

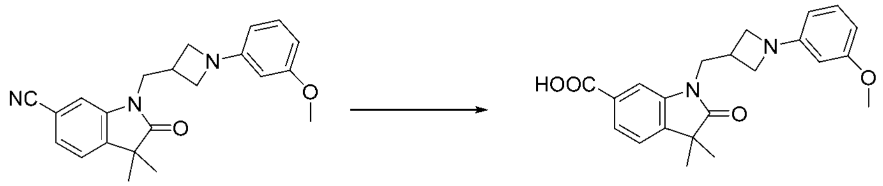

The preparation of 1-((1-(3-methoxyphenyl)azetidin-3-yl)methyl)-3,3-dimethyl-2-indolin-6-carboxylic acid **(15-4)** was the same as that of compound **11-3.** LCMS (ESI) $m/z$ $[M+H]^+$: 381.2.

**Step 5: N-(1H-indol-3-yl)-1-((1-(3-methoxyphenyl)azetidin-3-yl)methyl)-3,3-dimethyl-2-indolin-6-carboxamide (I-15)**

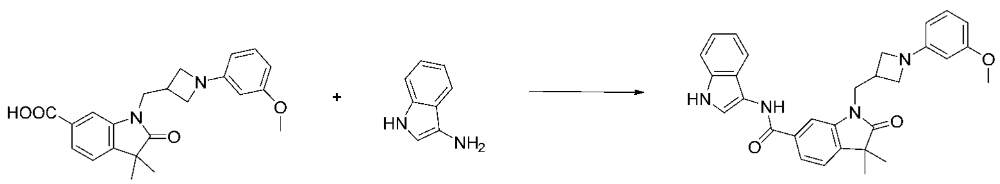

The preparation of N-(1H-indol-3-yl)-1-((1-(3-methoxyphenyl)azetidin-3-yl)methyl)-3,3-dimethyl-2-indolin-6-carboxamide **(I-15)** was the same as that of compound **I-1.** $^1$H NMR (400 MHz, DMSO-$d_6$) $\delta$ 10.93 (s, 1H), 10.12 (s, 1H), 7.83 (d, $J$ = 8.0 Hz, 1H), 7.79 (d, $J$ = 2.5 Hz, 1H), 7.74-7.68 (m, 2H), 7.52 (d, $J$ = 7.6 Hz, 1H), 7.37 (d, $J$ = 8.1 Hz, 1H), 7.12 (ddd, $J$ = 8.2, 7.0, 1.3 Hz, 1H), 7.03 (dt, $J$ = 12.9, 7.6 Hz, 2H), 6.25 (dd, $J$ = 8.0, 2.4 Hz, 1H), 6.01 (dd, $J$ = 7.9, 2.1 Hz, 1H), 5.94

(t, $J$ = 2.3 Hz, 1H), 4.08 (d, $J$ = 7.4 Hz, 2H), 3.87 (t, $J$ = 7.5 Hz, 2H), 3.69 (s, 3H), 3.62 (dd, $J$ = 7.3, 5.3 Hz, 2H), 3.25-3.15 (m, 1H), 1.33 (s, 6H).

**Example 16 N-(3,3-dimethyl-2-oxo-1-((1-phenylazetidin-3-yl)methyl)indolin-6-yl)-1H-indol-3-carboxamide (I-16)**

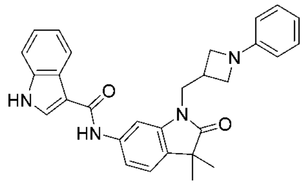

**Step 1: 3,3-dimethyl-6-nitro-1-((1-phenylazetidin-3-yl)methyl)indolin-2-one (16-1)**

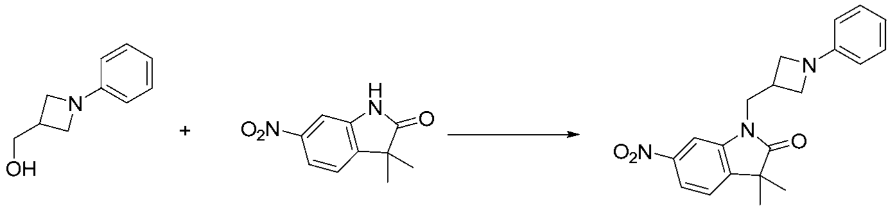

The preparation of 3,3-dimethyl-6-nitro-1-((1-phenylazetidin-3-yl)methyl)indolin-2-one **(16-1)** was the same as that of compound **11-1.** $^1$H NMR (400 MHz, $CDCl_3$) $\delta$ 8.00 (dd, $J$ = 8.1, 2.0 Hz, 1H), 7.72 (d, $J$ = 2.0 Hz, 1H), 7.35 (d, $J$ = 8.1 Hz, 1H), 7.23-7.17 (m, 2H), 6.75 (ddd, $J$ = 8.6, 6.9, 1.2 Hz, 1H), 6.47-6.43 (m, 2H), 4.10-4.07 (m, 2H), 3.96 (t, $J$ = 7.4 Hz, 2H), 3.72 (dd, $J$ = 7.2, 5.2 Hz, 2H), 3.16 (tt, $J$ = 7.6, 5.2 Hz, 1H), 1.41 (s, 6H).

**Step 2: 6-amino-3,3-dimethyl-1-((1-phenylazetidin-3-yl)methyl)indolin-2-one (16-2)**

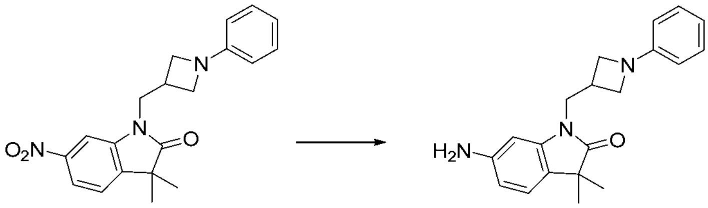

A mixture of 120 milligrams of 3,3-dimethyl-6-nitro-1-((1-phenylazetidin-3-yl)methyl)indolin-2-one **(16-1),** 100 milligrams of reduced iron powder, 370 milligrams of ammonium chloride, 3 milliliters of ethanol, and 300 microliters of water was heated and stirred at 80 ° C under argon atmosphere for 2 hours. The reaction solution was concentrated to dryness, and the residue was beaten with ethyl acetate to obtain 91.1 mg of white solid 6-amino-3,3-dimethyl-1-((1-phenylazetidin-3-yl)methyl)indolin-2-one **(16-2)** with a yield of 82.8%. $^1$H NMR (400 MHz, DMSO-$d_6$) $\delta$ 7.20-7.11 (m, 2H), 6.93 (d, J = 7.9 Hz, 1H), 6.66 (tt, $J$ = 7.3, 1.1 Hz, 1H), 6.42-6.37 (m, 2H), 6.31 (d, $J$= 1.9 Hz, 1H), 6.21 (dd, $J$= 7.9, 1.9 Hz, 1H), 5.11 (s, 2H), 3.91-3.78 (m, 4H), 3.56 (dd, $J$= 7.3, 5.3 Hz, 2H), 3.09-2.99 (m, 1H), 1.18 (s, 6H).

**Step 3: N-(3,3-dimethyl-2-oxo-1-((1-phenylazetidin-3-yl)methyl)indolin-6-yl)-1H-indol-3-carboxamide (I-16)**

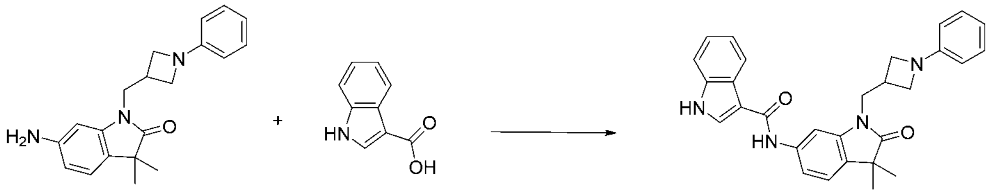

The preparation of N-(3,3-dimethyl-2-oxo-1-((1-phenylazetidin-3-yl)methyl)indolin-6-yl)-1H-indol-3-carboxamide **(I-16)** was the same as that of compound **I-1.** $^1$H NMR (600 MHz, DMSO-$d_6$) $\delta$ 11.74 (d, $J$ = 2.9 Hz, 1H), 9.75 (s, 1H), 8.30 (d, $J$ = 2.9 Hz, 1H), 8.20 (d, $J$ = 7.9 Hz, 1H), 7.61 (d, $J$ = 1.9 Hz, 1H), 7.47 (d, $J$ = 8.0 Hz, 1H), 7.41 (dd, $J$ = 8.0, 1.8 Hz, 1H), 7.29 (d, $J$ = 8.0 Hz, 1H), 7.20-7.13 (m, 4H), 6.67 (t, $J$ = 7.3 Hz, 1H), 6.42 (d, $J$ = 8.0 Hz, 2H), 3.98 (d, $J$ = 7.4 Hz, 2H), 3.88 (t, $J$ = 7.4 Hz, 2H), 3.63 (q, $J$ = 6.2, 5.3 Hz, 2H), 3.16-3.06 (m, 1H), 1.27 (s, 6H).

**Example 17 1-((1-(3-fluoro-5-trifluoromethylphenylazetidin-3-yl)methyl)-N-(1H-indol-3-yl)-3,3-dimethyl-2-oxoindolin-6-carboxamide (I-17)**

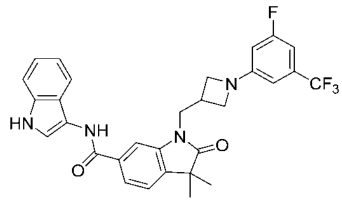

**Step 1: 6-bromo-1-((1-(3-fluoro-5-(trifluoromethyl)phenyl)azetidin-3-yl)methyl)-3,3-dimethylindolin-2-one (17-1)**

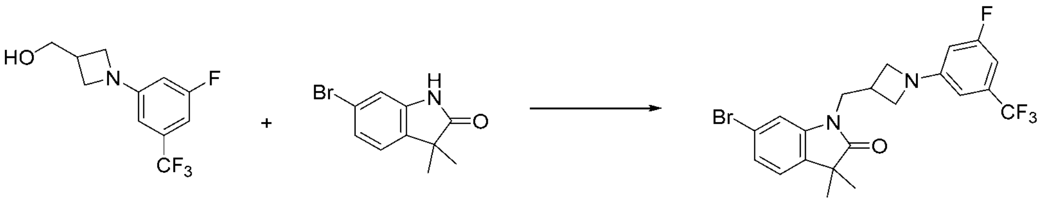

The preparation of 6-bromo-1-((1-(3-fluoro-5-(trifluoromethyl)phenyl)azetidin-3-yl)methyl)-3,3-dimethylindolin-2-one **(17-1)** was the same as that of compound **11-1.** $^1$H NMR (400 MHz, $CDCl_3$) $\delta$ 7.24-7.20 (m, 1H), 7.09 (d, $J$ = 7.8 Hz, 1H), 7.02 (d, $J$ = 1.6 Hz, 1H), 6.65 (d, $J$= 8.7 Hz, 1H), 6.38 (s, 1H), 6.22 (dt, $J$ = 10.5, 2.3 Hz, 1H), 4.03-3.93 (m, 4H), 3.75 (dd, $J$ = 7.5, 5.1 Hz, 2H), 3.23-3.12 (m, 1H), 1.36 (s, 6H).

**Step 2: 1-((1-(3-fluoro-5-(trifluoromethyl)phenyl)azetidin-3-yl)methyl)-3,3-dimethyl-2-indolin-6-nitrile (17-2)**

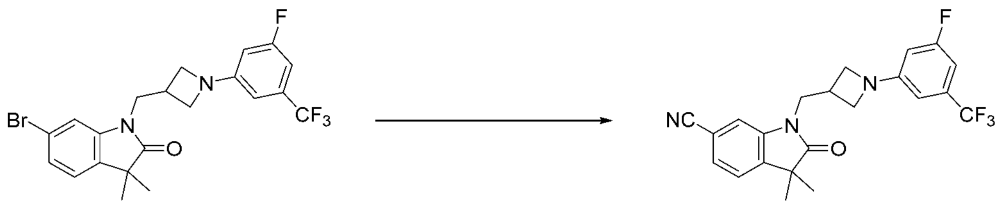

The preparation of 1-((1-(3-fluoro-5-(trifluoromethyl)phenyl)azetidin-3-yl)methyl)-3,3-dimethyl-2-indolin-6-nitrile **(17-2)** was the same as that of compound **11-2.** LCMS (ESI) $m/z$ $[M+H]^+$: 418.2.

**Step 3: 1-((1-(3-fluoro-5-(trifluoromethyl)phenyl)azetidin-3-yl)methyl)-3,3-dimethyl-2-indolin-6-carboxylic acid (17-3)**

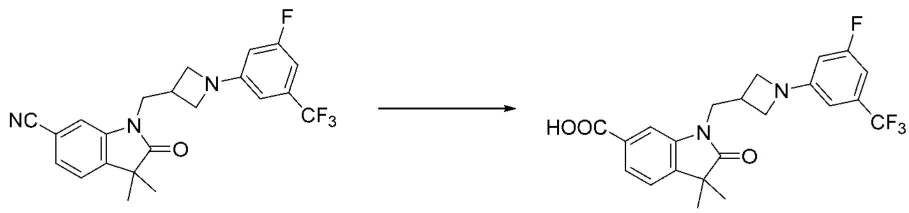

The preparation of 1-((1-(3-fluoro-5-(trifluoromethyl)phenyl)azetidin-3-yl)methyl)-3,3-dimethyl-2-indolin-6-carboxylic acid **(17-3)** was the same as that of compound **11-3.** LCMS (ESI) *m/z* $[M+H]^+$: 437.1.

**Step 4: 1-((1-(3-fluoro-5-(trifluoromethyl)phenyl)azetidin-3-yl)methyl)-N-(1H-indol-3-yl)-3,3-dimethyl-2-indolin-6-carboxamide (I-17)**

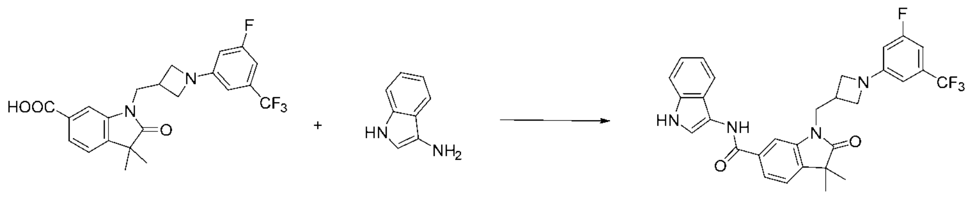

The preparation of 1-((1-(3-fluoro-5-(trifluoromethyl)phenyl)azetidin-3-yl)methyl)-N-(1H-indol-3-yl)-3,3-dimethyl-2-indolin-6-carboxamide **(I-17)** was the same as that of compound **I-1**. $^1$H NMR (400 MHz, DMSO-$d_6$) $\delta$ 10.92 (s, 1H), 10.11 (s, 1H), 7.82 (d, *J* = 7.9 Hz, 1H), 7.78 (d, *J* = 2.5 Hz, 1H), 7.71 (d, *J* = 4.4 Hz, 2H), 7.52 (d, *J* = 8.0 Hz, 1H), 7.37 (d, *J* = 8.1 Hz, 1H), 7.11 (t, *J*= 7.5 Hz, 1H), 7.00 *(t, J*= 7.5 Hz, 1H), 6.78 (d, *J* = 8.8 Hz, 1H), 6.56-6.49 (m, 2H), 4.09 (d, *J* = 7.5 Hz, 2H), 4.00 (t, *J*= 7.9 Hz, 2H), 3.74 (dd, *J* = 7.9, 5.3 Hz, 2H), 3.27 (d, *J*= 7.2 Hz, 1H), 1.33 (s, 6H).

**Example 18 1-((1-(3-chloro-5-trifluoromethylphenylazetidin-3-yl)methyl)-N-(1H-indol-3-yl)-3,3-dimethyl-2-oxoindolin-6-carboxamide (I-18)**

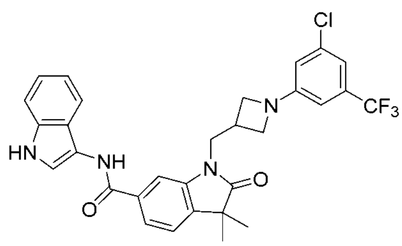

**Step 1: (1-3-chloro-5-(trifluoromethyl)phenyl)azetidin-3-yl)methanol (18-1)**

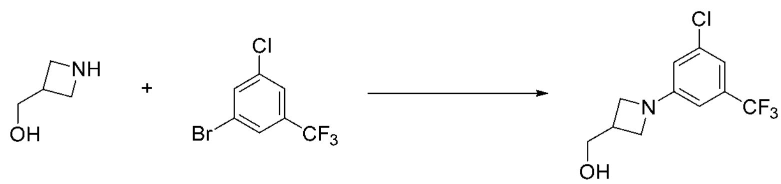

The preparation of (1-3-chloro-5-(trifluoromethyl)phenyl)azetidin-3-yl)methanol **(18-1)** was the same as that of compound **9-1.** $^1$H NMR (400 MHz, $CDCl_3$) $\delta$ 6.90 (d, $J$ = 1.7 Hz, 1H), 6.49 (dt, $J$= 14.4, 2.1 Hz, 2H), 3.98 (t, $J$ = 7.7 Hz, 2H), 3.87 (d, $J$= 6.3 Hz, 2H), 3.71 (dd, $J$ = 7.4, 5.1 Hz, 2H), 3.00-2.89 (m, 1H).

**Step 2: 6-bromo-1-((1-(3-chloro-5-(trifluoromethyl)phenyl)azetidin-3-yl)methyl)-3,3-dimethylindolin-2-one (18-2)**

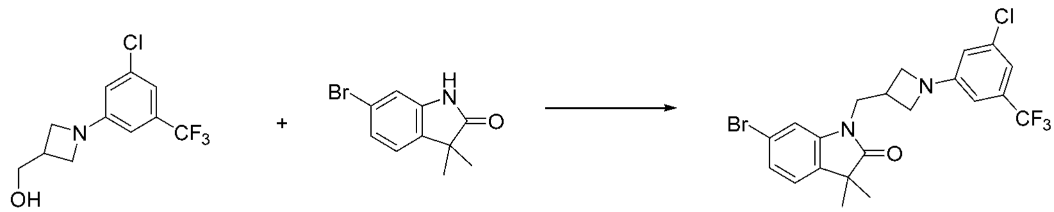

The preparation of 6-bromo-1-((1-(3-chloro-5-(trifluoromethyl)phenyl)azetidin-3-yl)methyl)-3,3-dimethylindolin-2-one **(18-2)** was the same as that of compound **11-1.** $^1$H NMR (400 MHz, $CDCl_3$) $\delta$ 7.22 (dt, $J$ = 7.9, 1.4 Hz, 1H), 7.09 (dd, $J$=7.8, 1.1 Hz, 1H), 7.02 (t, $J$ = 1.4 Hz, 1H), 6.93 (s, 1H), 6.49 (d, $J$= 15.1 Hz, 2H), 4.04-3.94 (m, 4H), 3.80-3.72 (m, 2H), 3.19 (dq, $J$= 12.9, 6.6, 5.8 Hz, 1H), 1.36 (d, $J$= 1.1 Hz, 6H).

**Step 3: 1-((1-(3-chloro-5-(trifluoromethyl)phenyl)azetidin-3-yl)methyl)-3,3-dimethyl-2-indolin-6-nitrile (18-3)**

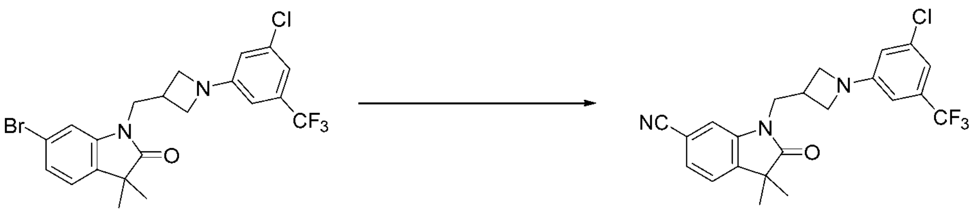

The preparation of 1-((1-(3-chloro-5-(trifluoromethyl)phenyl)azetidin-3-yl)methyl)-3,3-dimethyl-2-indolin-6-nitrile (18-3) was the same as that of compound **11-2.** LCMS (ESI) $m/z$ $[M+H]^+$: 434.1.

**Step 4: 1-((1-(3-chloro-5-(trifluoromethyl)phenyl)azetidin-3-yl)methyl)-3,3-dimethyl-2-indolin-6-carboxylic acid (18-4)**

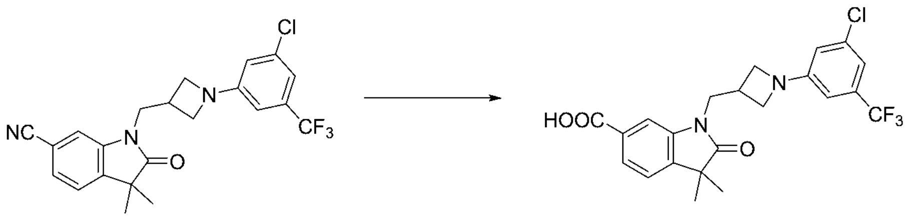

The preparation of 1-((1-(3-chloro-5-(trifluoromethyl)phenyl)azetidin-3-yl)methyl)-3,3-dimethyl-2-indolin-6-carboxylic acid **(18-4)** was the same as that of compound **11-3.** LCMS (ESI) $m/z$ $[M+H]^+$: 453.1.

**Step 5: 1-((1-(3-chloro-5-(trifluoromethyl)phenyl)azetidin-3-yl)methyl)-N-(1H-indol-3-yl)-3,3-dimethyl-2-indolin-6-carboxamide (I-18)**

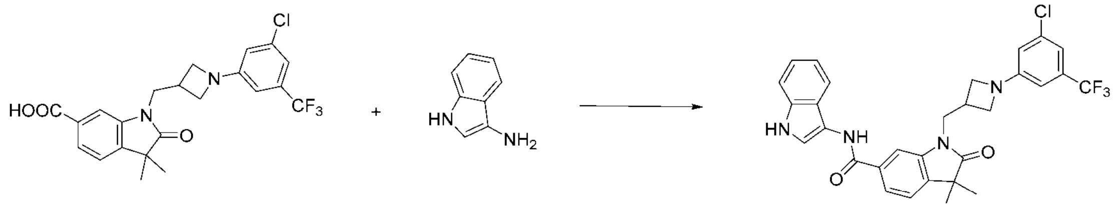

The preparation of 1-((1-(3-chloro-5-(trifluoromethyl)phenyl)azetidin-3-yl)methyl)-N-(1H-indol-3-yl)-3,3-dimethyl-2-indolin-6-carboxamide (**I-18**) was the same as that of compound **I-1**. $^1H$ NMR (400 MHz, DMSO-$d_6$) $\delta$ 10.92 (s, 1H), 10.11 (s, 1H), 7.82 (d, $J$= 8.0 Hz, 1H), 7.78 (d, $J$= 2.5 Hz, 1H), 7.71 (dt, $J$ = 4.0, 1.9 Hz, 2H), 7.52 (d, $J$ = 8.0 Hz, 1H), 7.40-7.34 (m, 1H), 7.11 (ddd, $J$ = 8.2, 7.0, 1.2 Hz, 1H), 7.03-6.95 (m, 2H), 6.73 (t, $J$= 2.1 Hz, 1H), 6.62 (s, 1H), 4.09 (d, $J$ = 7.5 Hz, 2H), 4.01 (t, $J$ = 7.9 Hz, 2H), 3.74 (dd, $J$ = 7.9, 5.2 Hz, 2H), 3.27 (dd, $J$ = 14.0, 6.3 Hz, 1H), 1.33 (s, 6H).

**Example 19 1-((1-(benzo[d][1,3]dioxolan-5-yl)azetidin-3-yl)methyl)-N-(1H-indol-3-yl)-3,3-dimethyl-2-oxoindolin-6-carboxamide (I-19)**

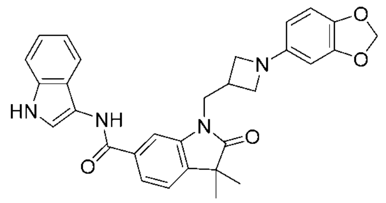

**Step 1: 1-((1-(benzo[d][1,3]dioxolan-5-yl)azetidin-3-yl)methyl)-6-bromo-3,3-dimethylindolin-2-one (19-1)**

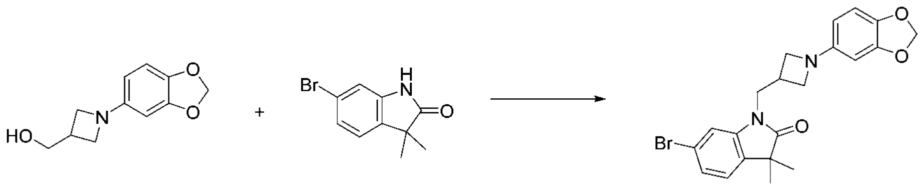

The preparation of 1-((1-(benzo[d][1,3]dioxolan-5-yl)azetidin-3-yl)methyl)-6-bromo-3,3-dimethylindolin-2-one **(19-1)** was the same as that of compound **11-1.** $^1H$ NMR (400 MHz, DMSO-$d_6$) $\delta$ 7.44 (d, $J$= 1.8 Hz, 1H), 7.31 (d, $J$= 7.8 Hz, 1H), 7.22 (dd, $J$= 7.8, 1.7 Hz, 1H), 6.71 (d, $J$= 8.3 Hz, 1H), 6.12 (d, $J$= 2.3 Hz, 1H), 5.86 (s, 2H), 5.79 (dd, $J$ = 8.3, 2.3 Hz, 1H), 3.96 (d, $J$= 7.3 Hz, 2H), 3.75 (t, $J$ = 7.4 Hz, 2H), 3.46 (dd, $J$ = 7.2, 5.4 Hz, 2H), 3.02 (p, $J$= 7.0 Hz, 1H), 1.25 (s, 6H).

**Step 2: 1-((1-(benzo[d][1,3]dioxolan-5-yl)azetidin-3-yl)methyl)-3,3-dimethyl-2-indolin-6-nitrile (19-2)**

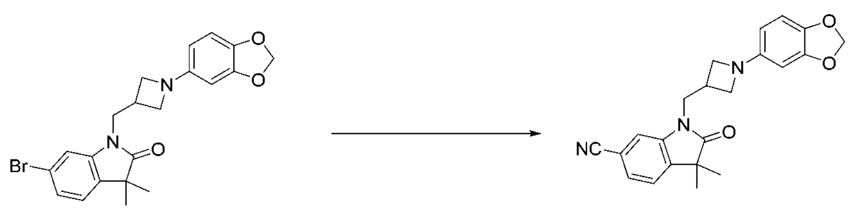

The preparation of 1-((1-(benzo[d][1,3]dioxolan-5-yl)azetidin-3-yl)methyl)-3,3-dimethyl-2-indolin-6-nitrile **(19-2)** was the same as that of compound **11-2.** $^1H$ NMR (400 MHz, DMSO-$d_6$) $\delta$ 7.71 (d, $J$= 1.4 Hz, 1H), 7.59 (d, $J$= 7.6 Hz, 1H), 7.54 (dd, $J$= 7.6, 1.4 Hz, 1H), 6.71 (d, $J$ = 8.3 Hz, 1H), 6.13 (d, $J$ = 2.2 Hz, 1H), 5.86 (s, 2H), 5.80 (dd, $J$ = 8.3, 2.3 Hz, 1H), 3.99 (d, $J$ = 7.2 Hz, 2H), 3.75 (t, $J$ = 7.4 Hz, 2H), 3.47 (dd, $J$ = 7.2, 5.4 Hz, 2H), 3.07 (q, $J$ = 7.0 Hz, 1H), 1.29 (s, 6H).

**Step 3: 1-((1-(benzo[d][1,3]dioxolan-5-yl)azetidin-3-yl)methyl)-3,3-dimethyl-2-indolin-6-carboxylic acid (19-3)**

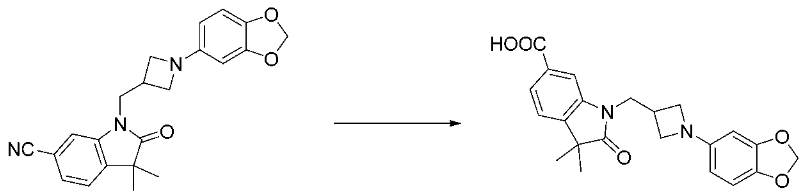

The preparation of 1-((1-(benzo[d][1,3]dioxolan-5-yl)azetidin-3-yl)methyl)-3,3-dimethyl-2-indolin-6-carboxylic acid **(19-3)** was the same as that of compound **11-3.** LCMS (ESI) *m/z* $[M+H]^+$: 395.2.

**Step 4: 1-((1-(benzo[d][1,3]dioxolan-5-yl)azetidin-3-yl)methyl)-N-(1H-indol-3-yl)-3,3-dimethyl-2-indolin-6-carboxamide** (**I-19**)

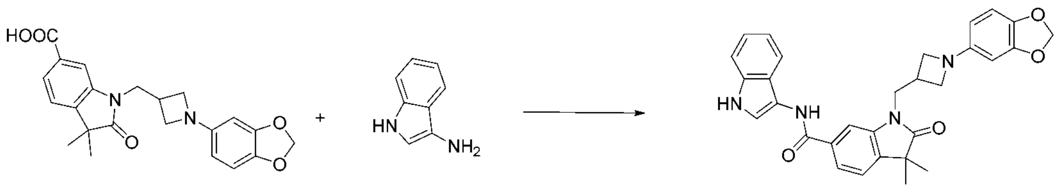

The preparation of 1-((1-(benzo[d][1,3]dioxolan-5-yl)azetidin-3-yl)methyl)-N-(1H-indol-3-yl)-3,3-dimethyl-2-indolin-6-carboxamide (**I-19**) was the same as that of compound **I-1**. $^1$H NMR (400 MHz, DMSO-$d_6$) $\delta$ 10.93 (d, *J* = 2.4 Hz, 1H), 10.12 (s, 1H), 7.83 (d, *J*= 8.0 Hz, 1H), 7.79 (d, *J*= *2.5* Hz, 1H), 7.72 (dd, *J*= 7.6, 1.5 Hz, 1H), 7.69 (d, *J* = 1.5 Hz, 1H), 7.52 (d, *J* = 7.6 Hz, 1H), 7.38 (d, *J*= 8.1 Hz, 1H), 7.12 (t, *J*= 7.5 Hz, 1H), 7.02 (t, *J*= 7.5 Hz, 1H), 6.72 (d, *J*= 8.3 Hz, 1H), 6.14 (d, *J* = 2.2 Hz, 1H), 5.87 (s, 2H), 5.82 (dd, *J* = 8.3, 2.3 Hz, 1H), 4.06 (d, *J* = 7.3 Hz, 2H), 3.80 (t, *J* = 7.4 Hz, 2H), 3.54 (dd, *J* = 7.2, 5.3 Hz, 2H), 3.20-3.10 (m, 1H), 1.33 (s, 6H).

**Example 20 N-(1H-indol-3-yl)-3,3-dimethyl-2-oxo-1-((1-(thien-3-yl)azetidin-3-yl)methyl)indolin-6-carboxamide (I-20)**

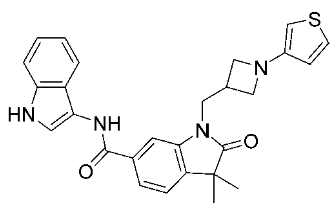

**Step 1: (1-(thien-3-yl)azetidin-3-yl)methanol (20-1)**

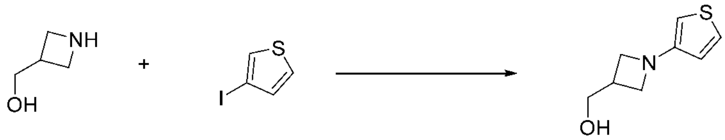

The preparation of (1-(thien-3-yl)azetidin-3-yl)methanol **(20-1)** was the same as that of compound **2-1.** $^1$H NMR (400 MHz, DMSO-$d_6$) $\delta$ 7.29 (dd, *J* = 5.1, 3.1 Hz, 1H), 6.51 (dd, *J*= 5.1, 1.5 Hz, 1H), 5.93 (dd, *J* = 3.1, 1.5 Hz, 1H), 4.67 *(t, J=*

5.3 Hz, 1H), 3.70 (t, *J*= 7.4 Hz, 2H), 3.51 (dd, *J* = 6.6, 5.3 Hz, 2H), 3.43 (dd, *J* = 7.0, 5.6 Hz, 2H), 2.73-2.62 (m, 1H).

**Step 2: 6-bromo-3,3-dimethyl-1-((1-(thien-3-yl)azetidin-3-yl)methyl)indolin-2-one (20-2)**

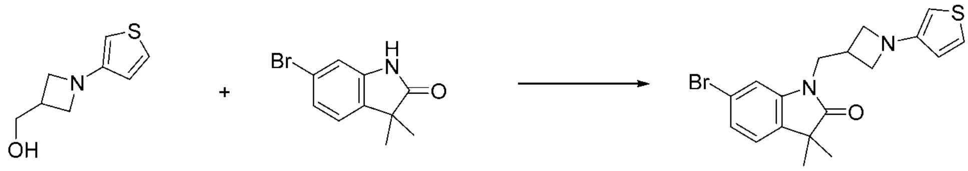

The preparation of 6-bromo-3,3-dimethyl-1-((1-(thien-3-yl)azetidin-3-yl)methyl)indolin-2-one **(20-2)** was the same as that of compound **11-1.**$^1$H NMR (400 MHz, DMSO-$d_6$) $\delta$ 7.42 (d, *J* = 1.7 Hz, 1H), 7.35-7.29 (m, 2H), 7.22 (dd, *J* = 7.9, 1.7 Hz, 1H), 6.55 (dd, *J* = 5.1, 1.5 Hz, 1H), 6.00 (dd, *J* = 3.1, 1.5 Hz, 1H), 3.96 (d, *J* = 7.3 Hz, 2H), 3.77 (t, *J* = 7.4 Hz, 2H), 3.49 (dd, *J* = 7.1, 5.5 Hz, 2H), 3.03 (tt, *J* = 7.8, 5.8 Hz, 1H), 1.24 (s, 6H).

**Step 3: 3,3-dimethyl-2-oxo-1-((1-(thien-3-yl)azetidin-3-yl)methyl)indolin-6-nitrile (20-3)**

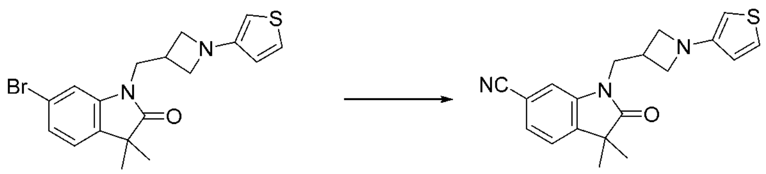

The preparation of 3,3-dimethyl-2-oxo-1-((1-(thien-3-yl)azetidin-3-yl)methyl)indolin-6-nitrile **(20-3)** was the same as that of compound **11-2.** $^1$H NMR (400 MHz, DMSO-$d_6$) $\delta$ 7.70 (d, *J*= 1.4 Hz, 1H), 7.59 (d, *J* = 7.6 Hz, 1H), 7.54 (dd, *J* = 7.7, 1.3 Hz, 1H), 7.34 (dd, *J* = 5.1, 3.1 Hz, 1H), 6.56 (dd, *J*= 5.1, 1.5 Hz, 1H), 6.02 (dd, *J* = 3.1, 1.5 Hz, 1H), 4.00 (d, *J* = 7.2 Hz, 2H), 3.78 (t, *J*= 7.4 Hz, 2H), 3.50 (dd, *J* = 7.1, 5.5 Hz, 2H), 3.07 (p, *J* = 6.6 Hz, 1H), 1.29 (s, 6H).

**Step 4: 3,3-dimethyl-2-oxo-1-((1-(thien-3-yl)azetidin-3-yl)methyl)indolin-6-carboxylic acid (20-4)**

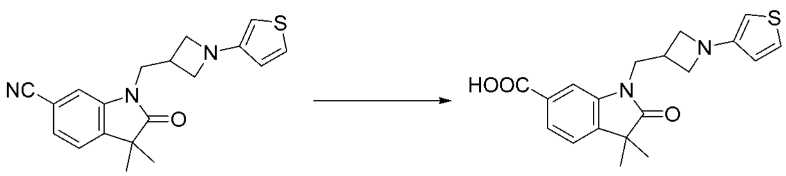

The preparation of 3,3-dimethyl-2-oxo-1-((1-(thien-3-yl)azetidin-3-yl)methyl)indolin-6-carboxylic acid **(20-4)** was the same as that of compound **11-3.** LCMS (ESI) *m*/*z* [M+H]$^+$: 357.1.

**Step 5: N-(1H-indol-3-yl)-3,3-dimethyl-2-oxo-1-((1-(thien-3-yl)azetidin-3-yl)methyl)indolin-6-carboxamide (I-20)**

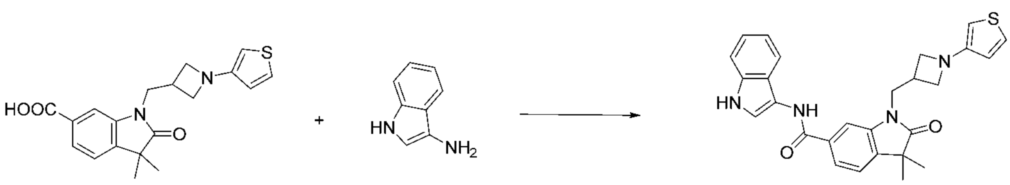

The preparation of N-(1H-indol-3-yl)-3,3-dimethyl-2-oxo-1-((1-(thien-3-yl)azetidin-3-yl)methyl)indolin-6-carboxamide (**I-20**) was the same as that of compound **I-1**. $^1$H NMR (400 MHz, DMSO-$d_6$) $\delta$ 10.92 (s, 1H), 10.12 (s, 1H), 7.83 (d, $J$= 8.0 Hz, 1H), 7.78 (d, $J$= 2.5 Hz, 1H), 7.71 (dd, $J$ = 7.6, 1.5 Hz, 1H), 7.67 (d, $J$ = 1.5 Hz, 1H), 7.51 (d, $J$= 7.7 Hz, 1H), 7.39-7.31 (m, 2H), 7.15-7.08 (m, 1H), 7.01 (t, $J$= 7.4 Hz, 1H), 6.57 (dd, $J$ = 5.0, 1.5 Hz, 1H), 6.03 (dd, $J$ = 3.1, 1.5 Hz, 1H), 4.06 (d, $J$= 7.3 Hz, 2H), 3.82 (t, $J$= 7.3 Hz, 2H), 3.56 (dd, $J$ = 7.1, 5.4 Hz, 2H), 3.16 (p, $J$ = 6.6 Hz, 1H), 1.32 (s, 6H).

**Example 21 1-((1-(2-chloro-6-fluorophenyl)azetidin-3-yl)methyl)-N-(1H-indol-3-yl)-3,3-dimethyl-2-oxoindolin-6-carboxamide (I-21)**

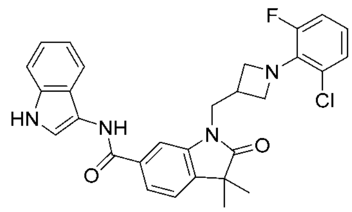

**Example 22 1-((1-(3-fluoro-5-trifluoromethylphenylazetidin-3-yl)methyl)-N-(1H-indol-3-yl)-2-oxo-2,3-dihydro-1H-thieno[2,3-b][1,4]thiazin-6-carboxamide (I-22)**

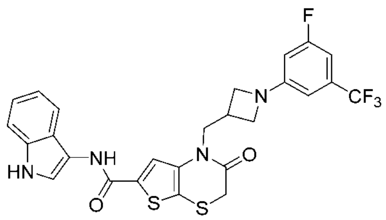

**Example 23 1-((1-(benzo[d][1,3]dioxolan-5-yl)azetidin-3-yl)methyl)-N-(1H-indol-3-yl)-2-oxo-2,3-dihydro-1H-thieno[2,3-b][1,4]thiazin-6-carboxamide (I-23)**

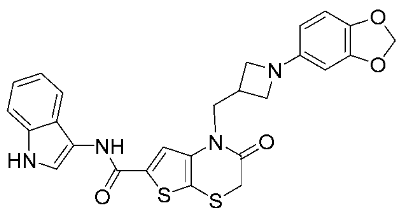

**Example 24 1-((1-(benzo[d]isoxazol-3-yl)azetidin-3-yl)methyl)-N-(1H-indol-3-yl)-3,3-dimethyl-2-oxoindolin-6-carboxamide** (**I-24**)

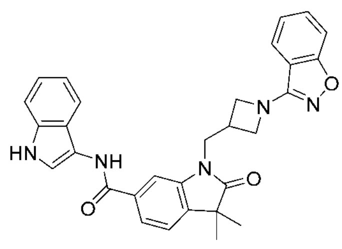

**Example 25 N-(1H-indol-3-yl)-3,3-dimethyl-2-oxo-1-((1-(pyridin-3-yl)azetidin-3-yl)methyl)indolin-6-carboxamide (I-25)**

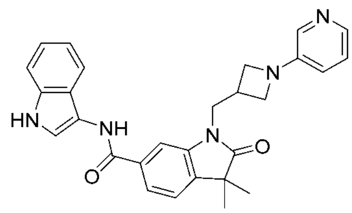

**Example 26 1-((1-(furan-3-yl)azetidin-3-yl)methyl)-N-(1H-indol-3-yl)-3,3-dimethyl-2-oxoindolin-6-carboxamide (I-26)**

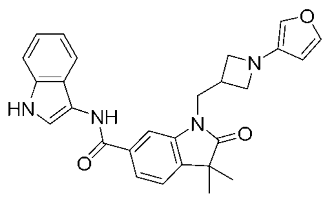

**Example 27 1-((1-(imidazo[1,2-a]pyridin-2-)azetidin-3-yl)methyl)-N-(1H-indol-3-yl)-3,3-dimethyl-2-oxoindolin-6-carboxamide (I-27)**

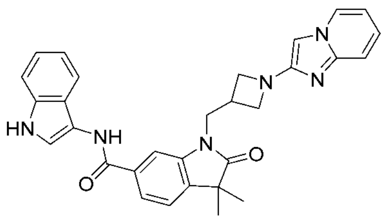

**Example 28 N-(5-fluoro-1H-indol-3-yl)-1-((1-(3-fluoro-5-trifluoromethylphenylazetidin-3-yl)methyl)-3,3-dimethyl-2-oxoindolin-6-carboxamide (I-28)**

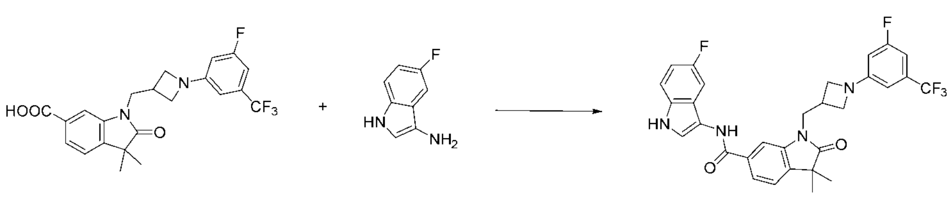

The preparation of N-(5-fluoro-1H-indol-3-yl)-1-((1-(3-fluoro-5-trifluoromethylphenylazetidin-3-yl)methyl)-3,3-dimethyl-2-oxoindolin-6-carboxamide **(I-28)** was the same as that of compound **I-1**. $^1$H NMR (400 MHz, DMSO-$d_6$) $\delta$ 11.04 (s, $J$ = 2.7 Hz, 1H), 10.10 (s, 1H), 7.87 (d, $J$= 2.6 Hz, 1H), 7.71 (dd, $J$ = 9.2, 1.6 Hz, 2H), 7.61 (dd, $J$ = 10.3, 2.6 Hz, 1H), 7.53 (d, $J$=7.6 Hz, 1H), 7.37 (dd, $J$=8.9, 4.5 Hz, 1H), 6.96 (td, $J$ = 9.1, 2.6 Hz, 1H), 6.80-6.74 (m, 1H), 6.58-6.46 (m, 2H), 4.09 (d, $J$ = 7.5 Hz, 2H), 3.99 (t, $J$ = 7.9 Hz, 2H), 3.73 (dd, $J$= 7.9, 5.2 Hz, 2H), 3.30-3.19 (m, 1H), 1.33 (s, 6H).

**Example 29 N-(5-chloro-1H-indol-3-yl)-1-((1-(3-fluoro-5-trifluoromethylphenylazetidin-3-yl)methyl)-3,3-dimethyl-2-oxoindolin-6-carboxamide (I-29)**

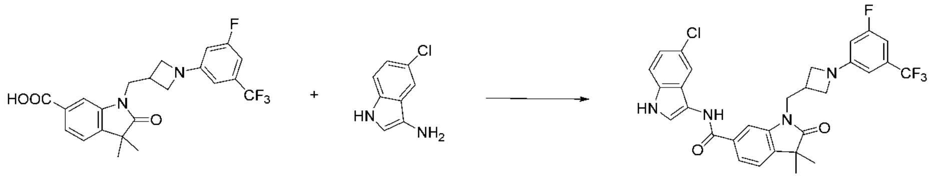

The preparation of N-(5-chloro-1H-indol-3-yl)-1-((1-(3-fluoro-5-**trifluoromethylphenylazetidin-3-yl) methyl)-3,3-dimethyl-2-oxoindolin-6-carboxamide** (**I-29**) was the same as that of compound **I-1**. $^1H$ NMR (400 MHz, DMSO-$d_6$) $\delta$ 11.14 (s, 1H), 10.15 (s, 1H), 7.94 (d, *J*= 2.1 Hz, 1H), 7.87 (d, *J* = 2.5 Hz, 1H), 7.76-7.67 (m, 2H), 7.53 (d, *J*=7.6 Hz, 1H), 7.39 (d, *J* = 8.6 Hz, 1H), 7.11 (dd, *J*= 8.7, 2.1 Hz, 1H), 6.77 (d, *J* = 8.7 Hz, 1H), 6.58-6.45 (m, 2H), 4.09 (d, *J* = 7.5 Hz, 2H), 3.99 (t, *J* = 7.9 Hz, 2H), 3.73 (dd, *J* = 7.9, 5.2 Hz, 2H), 3.26 (t, *J* = 7.5 Hz, 1H), 1.33 (s, 6H).

**Example 30 N-(5,6-difluoro-1H-indol-3-yl)-1-((1-(3-fluoro-5-trifluoromethylphenylazetidin-3-yl)methyl)-3,3-dimethyl-2-oxoindolin-6-carboxamide (I-30)**

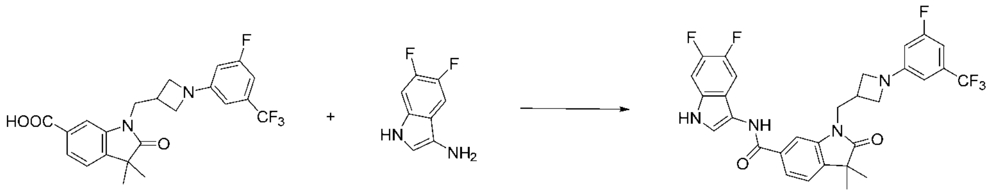

The preparation of N-(5,6-difluoro-1H-indol-3-yl)-1-((1-(3-fluoro-5-trifluoromethylphenylazetidin-3-yl) methyl)-3,3-dimethyl-2-oxoindolin-6-carboxamide (**I-30**) was the same as that of compound **I-1**. $^1H$ NMR (400 MHz, $CDCl_3$) $\delta$ 8.26 (s, 1H), 7.90 (s, 1H), 7.86 (d, *J*= 2.6 Hz, 1H), 7.58-7.52 (m, 2H), 7.36-7.29 (m, 2H), 7.17 (dd, *J* = 10.3, 6.5 Hz, 1H), 6.66-6.59 (m, 1H), 6.37 (d, *J*= 1.9 Hz, 1H), 6.20 (dt, *J* = 10.6, 2.2 Hz, 1H), 4.09 (d, *J* = 7.3 Hz, 2H), 3.96 (t, *J* = 7.7 Hz, 2H), 3.76 (dd, *J* = 7.4, 5.1 Hz, 2H), 3.29-3.17 (m, 1H), 1.42 (s, 6H).

**Example 31 1-((1-(3-chloro-5-fluorophenylazetidin-3-yl)methyl)-N-(1H-indol-3-yl)-3,3-dimethyl-2-oxoindolin-6-carboxamide (I-31)**

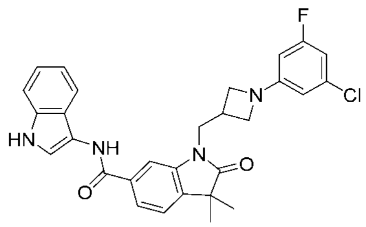

**Step 1: methyl (1-(3-chloro-5-fluorophenyl)azetidin-3-yl)methanesulfonate (31-1)**

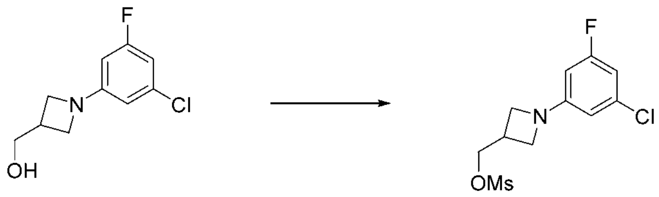

The preparation of methyl (1-(3-chloro-5-fluorophenyl)azetidin-3-yl)methanesulfonate (**31-1**) was the same as that of compound **25-2.** $^1$H NMR (400 MHz, $CDCl_3$) $\delta$ 6.45 (dt, *J* = 8.7, 2.0 Hz, 1H), 6.16 (t, *J* = 2.0 Hz, 1H), 5.99 (dt, *J* = 10.5, 2.2 Hz, 1H), 4.44 (d, *J* = 6.8 Hz, 2H), 3.98 *(t, J*= 7.8 Hz, 2H), 3.68 (dd, *J*= 7.7, 5.0 Hz, 2H), 3.14-3.07 (m, 1H), 3.05 (s, 3H).

**Step 2: methyl 1-((1-(3-chloro-5-fluorophenyl)azetidin-3-yl)methyl)-3,3-dimethyl-2-indolin-6-carboxylate (31-2)**

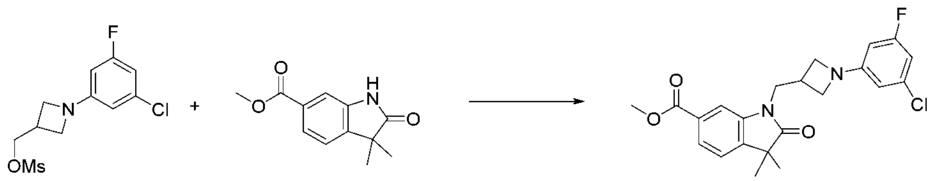

The preparation of methyl 1-((1-(3-chloro-5-fluorophenyl)azetidin-3-yl)methyl)-3,3-dimethyl-2-indolin-6-carboxylate **(31-2)** was the same as that of compound **25-3.** $^1$H NMR (400 MHz, $CDCl_3$) $\delta$ 7.81 (dd, *J* = 7.7, 1.4 Hz, 1H), 7.51 (d, *J* = 1.4 Hz, 1H), 7.28 (d, *J*= 7.7 Hz, 1H), 6.42 (dt, *J*= 8.7, 2.0 Hz, 1H), 6.15 (d, *J*= 2.1 Hz, 1H), 5.98 (dt, *J* = 10.7, 2.2 Hz, 1H), 4.05 (d, *J*= 7.3 Hz, 2H), 3.94 (d, *J*= 4.4 Hz, 5H), 3.72 (dd, *J*= 7.4, 5.2 Hz, 2H), 3.16-3.24 (m, 1H), 1.39 (s, 6H).

**Step** 3: **1-((1-(3-chloro-5-fluorophenyl)azetidin-3-yl)methyl)-3,3-dimethyl-2-indolin-6-carboxylic acid (31-3)**

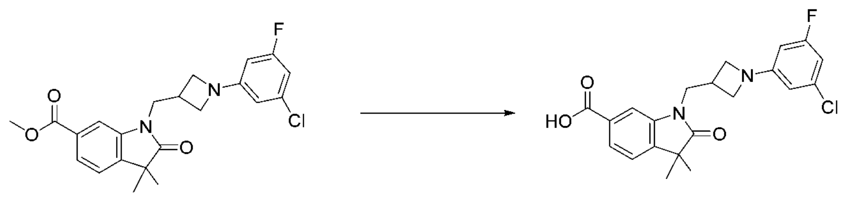

The preparation of 1-((1-(3-chloro-5-fluorophenyl)azetidin-3-yl)methyl)-3,3-dimethyl-2-indolin-6-carboxylic acid **(31-3)** was the same as that of compound **9-3.** LCMS (ESI) *m/z* [M+H]$^+$: 403.1.

**Step 4: 1-((1-(3-chloro-5-fluorophenyl)azetidin-3-yl)methyl)-N-(1H-indol-3-yl)-3,3-dimethyl-2-indolin-6-carboxamide** (**I-31**)

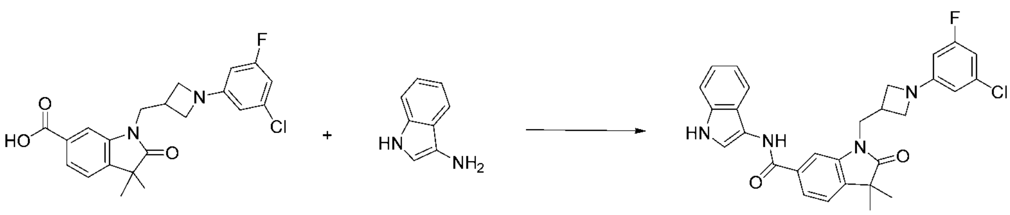

The preparation of 1-((1-(3-chloro-5-fluorophenyl)azetidin-3-yl)methyl)-N-(1H-indol-3-yl)-3,3-dimethyl-2-indolin-6-carboxamide **(I-31)** was the same as that of compound **I-1**. $^1$H NMR (400 MHz, DMSO-$d_6$) $\delta$ 10.92 (d, *J* = 2.4 Hz, 1H), 10.11 (s, 1H), 7.86-7.76 (m, 2H), 7.72 (d, *J* = 6.7 Hz, 2H), 7.52 (d, *J*= 7.9 Hz, 1H), 7.37 (d, *J* = 8.1 Hz, 1H), 7.12 (t, *J* = 7.5 Hz, 1H), 7.01 (t, *J* = 7.5 Hz, 1H), 6.56 (dt, *J* = 8.9, 2.1 Hz, 1H), 6.29 (d, *J* = 2.0 Hz, 1H), 6.23 (dt, *J* = 11.3, 2.2 Hz, 1H), 4.05 (dd, *J* =

18.5, 7.3 Hz, 3H), 3.94 (t, *J*= 7.8 Hz, 2H), 3.68 (dd, *J* = 7.8, 5.2 Hz, 2H), 3.20-3.27 (m, 1H), 1.32 (s, 6H).

**Example 32 1-((1-(benzo[b]thien-3-yl)azetidin-3-yl)methyl)-N-(1H-indol-3-yl)-3,3-dimethyl-2-oxoindolin-6-carboxamide (I-32)**

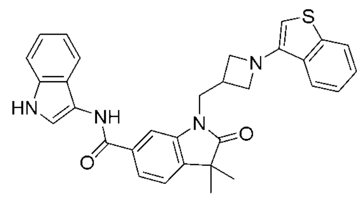

**Step 1: (1-(benzo[b]thien-3-yl)azetidin-3-yl)methanol (32-1)**

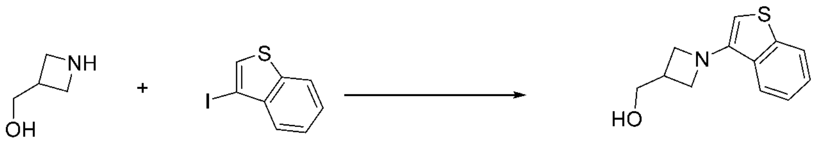

The preparation of (1-(benzo[b]thien-3-yl)azetidin-3-yl)methanol **(32-1)** was the same as that of compound **2-1.** $^1$H NMR (400 MHz, $CDCl_3$) $\delta$ 7.80-7.73 (m, 1H), 7.73-7.62 (m, 1H), 7.34-7.28 (m, 2H), 6.09 (s, 1H), 4.14 (t, *J*= 7.5 Hz, 2H), 3.93 (d, *J* = 6.3 Hz, 2H), 3.87 (dd, *J* = 7.1, 5.2 Hz, 2H), 3.00-2.88 (m, 1H).

**Step 2: methyl (1-(benzo[b]thien-3-yl)azetidin-3-yl)methanesulfonate (32-2)**

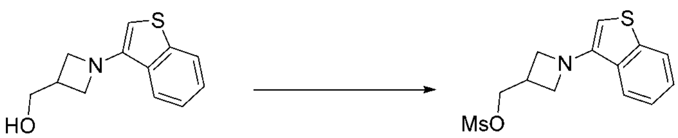

A mixture of 200 milligrams (1-(benzo[b]thien-3-yl)azetidin-3-yl)methanol **(32-1),** 200 microliters of methylsulfonyl chloride, 760 microliters of triethylamine, 20 milligrams of DMAP, and 4 milliliters of dichloromethane was stirred at room temperature for 1 hour. The reaction solution was concentrated to dryness, and the residue was subjected to column chromatography with petroleum ether: ethyl acetate = 60:40 to obtain solid methyl (1-(benzo[b]thien-3-yl)azetidin-3-yl) methanesulfonate **(32-2).** $^1$H NMR (400 MHz, $CDCl_3$) $\delta$ 7.80-7.75 (m, 1H), 7.68-7.62 (m, 1H), 7.35-7.29 (m, 2H), 6.11 (s, 1H), 4.52 (d, *J* = 7.0 Hz, 2H), 4.17 (t, *J* = 7.6 Hz, 2H), 3.87 (dd, *J* = 7.4, 5.2 Hz, 2H), 3.19-3.14 (m, 1H), 3.06 (s, 3H).

**Step 3: methyl 1-((1-(benzo[b]thien-3-yl)azetidin-3-yl)methyl)-3,3-dimethyl-2-indolin-6-carboxylate (32-3)**

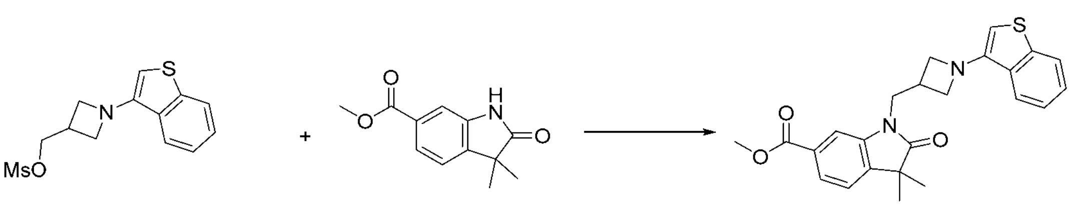

The preparation of methyl 1-((1-(benzo[b]thien-3-yl)azetidin-3-yl)methyl)-3,3-dimethyl-2-indolin-6-carboxylate **(32-3)** was the same as that of compound **1-4.** $^1$H NMR (400 MHz,$CDCl_3$) $\delta$ 7.81 (dd, *J* = 7.7, 1.4 Hz, 1H), 7.77-7.73 (m, 1H),

7.67-7.62 (m, 1H), 7.56 (d, *J*= 1.4 Hz, 1H), 7.32-7.27 (m, 3H), 6.09 (s, 1H), 4.11 (dd, *J*= 7.2, 3.2 Hz, 4H), 3.94 (s, 3H), 3.88 (dd, *J*= 7.1, 5.5 Hz, 2H), 3.22-3.25 (m, 1H), 1.40 (s, 6H).

**Step 4: 1-((1-(benzo[b]thien-3-yl)azetidin-3-yl)methyl)-3,3-dimethyl-2-indolin-6-carboxylic acid (32-4)**

The preparation method of 1-((1-(benzo[b]thien-3-yl)azetidin-3-yl)methyl)-3,3-dimethyl-2-indolin-6-carboxylic acid **(32-4)** was the same as that of compound 9-3. LCMS (ESI) *m*/*z* $[M+H]^+$: 407.1.

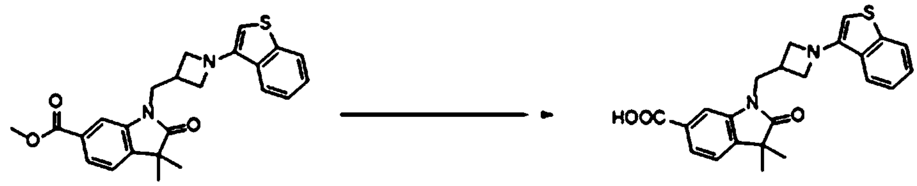

**Step 5: 1-((1-(benzo[b]thien-3-yl)azetidin-3-yl)methyl)-N-(1H-indol-3-yl)-3,3-dimethyl-2-oxoindolin-6-carboxamide (I-32)**

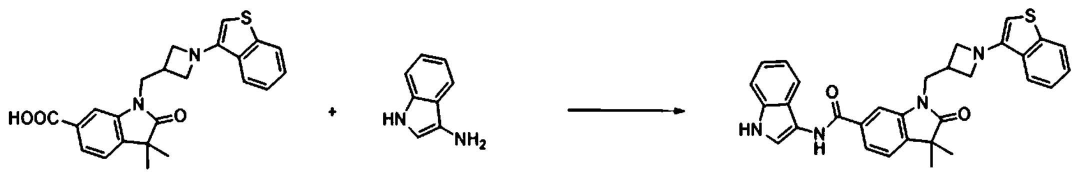

The preparation method of 1-((1-(benzo[b]thien-3-yl)azetidin-3-yl)methyl)-N-(1H-indol-3-yl)-3,3-dimethyl-2-oxoindolin-6-carboxamide (**I-32**) was the same as that of compound **I-1**. $^1$H NMR (400 MHz, $CDCl_3$) $\delta$ 8.19 (s, 1H), 8.09 (s, 1H), 7.97 (d, *J* = 2.5 Hz, 1H), 7.77-7.72 (m, 1H), 7.66-7.62 (m, 1H), 7.59-7.54 (m, 3H), 7.39 (d, *J* = 8.2 Hz, 1H), 7.32 (d, *J* = 8.0 Hz, 1H), 7.30-7.27 (m, 2H), 7.25-7.22 (m, 1H), 7.14 (t, *J*= 7.5 Hz, 1H), 6.08 (s, 1H), 4.11 (t, *J* = 7.3 Hz, 4H), 3.88 (dd, *J*= 7.1, 5.5 Hz, 2H), 3.18-3.25 (m, 1H), 1.42 (s, 6H).

**Example 33 N-(benzofuran-3-yl)-1-((1-(3-fluoro-5-trifluoromethylphenylazetidin-3-yl)methyl)-3,3-dimethyl-2-oxoindolin-6-carboxamide** (**I-33**)

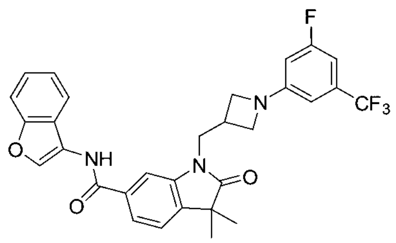

**Example 34 1-((1-(furan-3-yl)azetidin-3-yl)methyl)-N-(1H-indol-3-yl)-3,3-dimethyl-2-oxoindolin-6-carboxamide (I-34)**

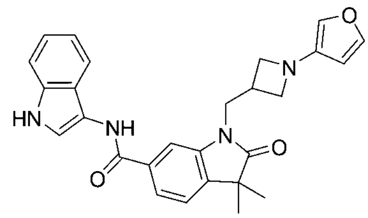

**Example 35 1-((1-(3-fluoro-5-methoxyphenylazetidin-3-yl)methyl)-N-(1H-indol-3-yl)-3,3-dimethyl-2-oxoindolin-6-carboxamide** (**I-35**)

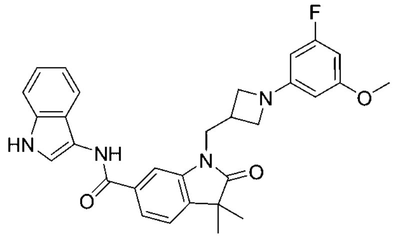

**Step 1: methyl 1-((1-(3-fluoro-5-methoxyphenyl)azetidin-3-yl)methyl))-3,3-dimethyl-2-indolin-6-carboxylate (35-1)**

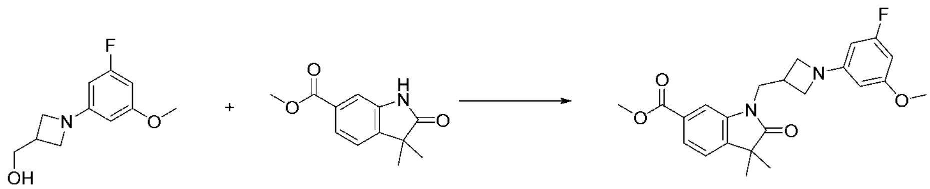

The preparation of methyl 1-((1-(3-fluoro-5-methoxyphenyl)azetidin-3-yl)methyl))-3,3-dimethyl-2-indolin-6-carboxylate **(35-1)** was the same as that of compound **11-1.** $^{1}$H NMR (400 MHz, Chloroform-d) $\delta$ 7.80 (d, $J$= 7.8 Hz, 1H), 7.51 (s, 1H), 7.28 (d, $J$ = 8.2 Hz, 1H), 6.01 (dd, $J$= 10.8, 2.5 Hz, 1H), 5.68-5.79 (m, 2H), 4.05 (d, $J$ = 7.3 Hz, 2H), 3.94 (s, 3H), 3.90 (d, $J$ = 6.9 Hz, 2H), 3.74 (s, 3H), 3.70 (dd, $J$= 7.3, 5.4 Hz, 2H), 3.11-3.26 (m, 1H), 1.38 (s, 6H).

**Step 2: 1-((1-(3-fluoro-5-methoxyphenyl)azetidin-3-yl)methyl))-3,3-dimethyl-2-indolin-6-carboxylic acid (35-2)**

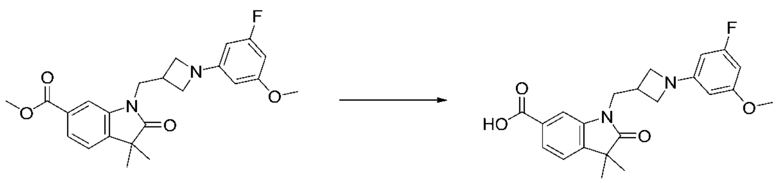

The preparation of 1-((1-(3-fluoro-5-methoxyphenyl)azetidin-3-yl)methyl))-3,3-dimethyl-2-indolin-6-carboxylic acid **(35-2)** was the same as that of compound **1-5.** LCMS (ESI) $m/z$ $[M+H]^+$: 399.2.

**Step 3: 1-((1-(3-fluoro-5-methoxyphenyl)azetidin-3-yl)methyl)-*N*-(1*H*-indol-3-yl)-3,3-dimethyl-2-indolin-6-carboxamide (I-35)**

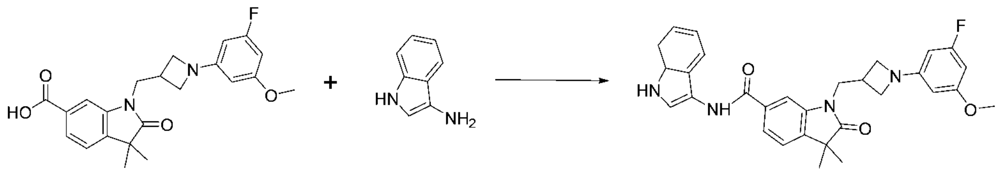

The preparation of 1-((1-(3-fluoro-5-methoxyphenyl)azetidin-3-yl)methyl)-N-(1H-indol-3-yl)-3,3-dimethyl-2-indolin-6-carboxamide (**I-35**) was the same as that of compound **I-1**. $^{1}$H NMR (400 MHz, DMSO-$d_6$) $\delta$ 10.92 (s, 1H), 10.12 (s, 1H), 7.82 (d, $J$ = 8.0 Hz, 1H), 7.78 (d, $J$ = 2.5 Hz, 1H), 7.71 (d, $J$ = 9.1 Hz, 2H), 7.52 (d, $J$= 7.5 Hz, 1H), 7.37 (d, $J$= 8.1 Hz, 1H), 7.12 (t, $J$= 7.5 Hz, 1H), 7.00 (t, $J$= 7.5 Hz, 1H), 6.07 (d, $J$ = 11.1 Hz, 1H), 5.82 (d, $J$ = 11.0 Hz, 1H), 5.75 (s, 1H), 4.07 (d, $J$ = 7.5 Hz, 2H), 3.89 (t, $J$= 7.7 Hz, 2H), 3.69 (s, 3H), 3.63 (t, $J$ = 6.4 Hz, 2H), 3.15-3.23 (m, 1H), 1.32 (s, 6H).

**Example 36 1-((1-(benzofuran-6-yl)azetidin-3-yl)methyl)-N-(1H-indol-3-yl)-3,3-dimethyl-2-oxoindolin-6-carboxamide (I-36)**

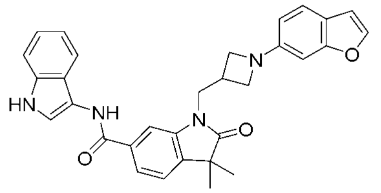

**Step 1: (1-(benzofuran-6-yl)azetidin-3-yl)methanol (36-1)**

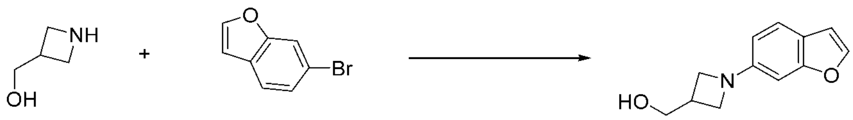

The preparation of (1-(benzofuran-6-yl)azetidin-3-yl)methanol **(36-1)** was the same as that of compound **9-1.** $^{1}$H NMR (400 MHz, $CDCl_3$) $\delta$ 7.44 (d, $J$ = 2.2 Hz, 1H), 7.40 (d, $J$ = 8.3 Hz, 1H), 6.64 (dd, $J$ = 2.2, 0.9 Hz, 1H), 6.57 (d, $J$= 2.0 Hz, 1H), 6.44 (dd, $J$= 8.4, 2.0 Hz, 1H), 3.97 (t, $J$= 7.5 Hz, 2H), 3.89 (d, $J$= 6.5 Hz, 2H), 3.69 (dd, $J$= 7.1, 5.0 Hz, 2H), 2.98-2.84 (m, 1H).

**Step 2: methyl (1-(benzofuran-6-yl)azetidin-3-yl)methanesulfonate (36-2)**

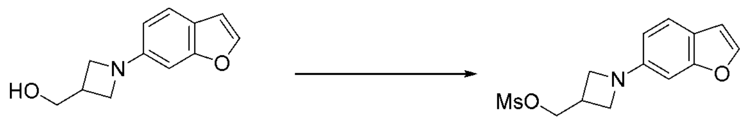

The preparation of methyl (1-(benzofuran-6-yl)azetidin-3-yl)methanesulfonate **(36-2)** was the same as that of compound **25-2.** $^{1}$H NMR (400 MHz, $CDCl_3$) $\delta$ 7.45 (d, $J$ = 2.2 Hz, 1H), 7.41 (d, $J$ = 8.3 Hz, 1H), 6.65 (t, $J$ = 1.5 Hz, 1H), 6.57 (s, 1H), 6.46-6.39 (m, 1H), 4.48 (d, $J$ = 7.0 Hz, 2H), 4.02 (t, $J$ = 7.6 Hz, 2H), 3.72 (dd, $J$ = 7.4, 5.0 Hz, 2H), 3.13 (q, $J$ = 7.2, 5.3 Hz, 1H), 3.05 (s, 3H).

**Step 3: methyl 1-((1-(benzofuran-6-yl)azetidin-3-yl)methyl)-3,3-dimethyl-2-indolin-6-carboxylate (36-3)**

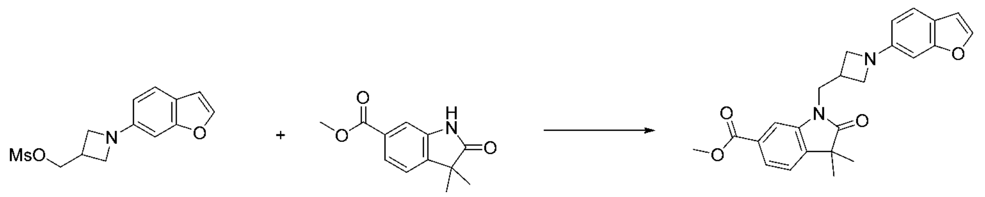

The preparation of methyl 1-((1-(benzofuran-6-yl)azetidin-3-yl)methyl)-3,3-dimethyl-2-indolin-6-carboxylate **(36-3)** was the same as that of compound **25-3.** $^1$H NMR (400 MHz, DMSO-$d_6$) $\delta$ 7.74-7.68 (m, 2H), 7.64 (s, 1H), 7.52 (d, $J$ = 7.7 Hz, 1H), 7.40 (d, $J$ = 8.3 Hz, 1H), 6.76 (d, $J$ = 2.2 Hz, 1H), 6.56 (s, 1H), 6.38 (dd, $J$ = 8.3, 1.7 Hz, 1H), 4.07 (d, $J$ = 7.4 Hz, 2H), 3.83-3.90 (m, 5H), 3.61 *(t, J*= 6.3 Hz, 2H), 3.07-3.14 (m, 1H), 1.30 (s, 6H).

**Step 4: 1-((1-(benzofuran-6-yl)azetidin-3-yl)methyl)-3,3-dimethyl-2-indolin-6-carboxylic acid (36-4)**

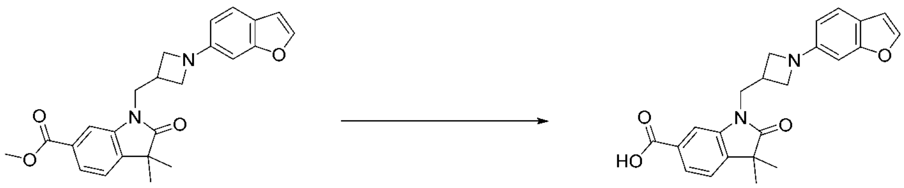

The preparation of 1-((1-(benzofuran-6-yl)azetidin-3-yl)methyl)-3,3-dimethyl-2-indolin-6-carboxylic acid **(36-4)** was the same as that of compound **9-3.** LCMS (ESI) *m*/*z* [M+H]$^+$: 391.2.

**Step 5: 1-((1-(benzofuran-6-yl)azetidin-3-yl)methyl-N-(1H-indol-3-yl)-3,3-dimethyl-2-indolin-6-carboxamide (I-36)**

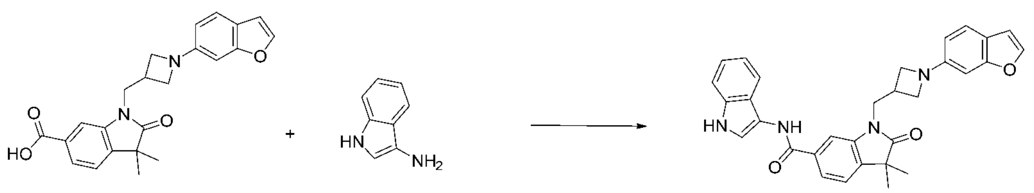

The preparation of 1-((1-(benzofuran-6-yl)azetidin-3-yl)methyl-N-(1H-indol-3-yl)-3,3-dimethyl-2-indolin-6-carboxamide **(I-36)** was the same as that of compound **I-1**. $^1$H NMR (400 MHz, DMSO-$d_6$) $\delta$ 10.92 (s, 1H), 10.12 (s, 1H), 7.83 (d, $J$ = 8.0 Hz, 1H), 7.80-7.77 (m, 1H), 7.72 (d, $J$= 8.4 Hz, 3H), 7.52 (d, $J$= 7.5 Hz, 1H), 7.39 (dd, $J$= 14.1, 8.2 Hz, 2H), 7.11 (t, $J$ = 7.5 Hz, 1H), 7.00 (t, $J$= 7.5 Hz, 1H), 6.76 (d, $J$= 2.2 Hz, 1H), 6.58 (s, 1H), 6.41 (d, $J$= 8.4 Hz, 1H), 4.09 (d, $J$= 7.4 Hz, 2H), 3.92 (t, $J$= 7.5 Hz, 2H), 3.66 (t, $J$= 6.3 Hz, 2H), 3.28-3.16 (m, 1H), 1.33 (s, 6H).

**Example 37 1-((1-cyclopropylazetidin-3-yl)methyl)-N-(1H-indol-3-yl)-3,3-dimethyl-2-oxoindolin-6-carboxamide (I-37)**

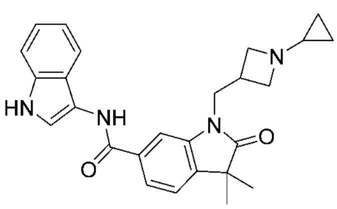

**Example 38 1-((1-(furan-2-yl)azetidin-3-yl)methyl)-N-(1H-indol-3-yl)-3,3-dimethyl-2-oxoindolin-6-carboxamide (I-38)**

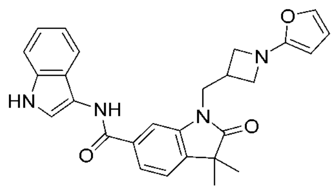

**Example 39 N-(1H-indol-3-yl)-3,3-dimethyl-2-oxo-1-((1-(thien-2-yl)azetidin-3-yl)methyl)indolin-6-carboxamide (I-39)**

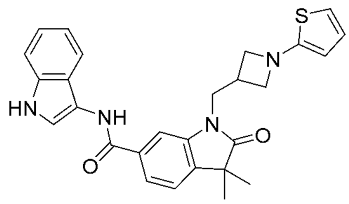

**Example 40 1-((1-(3-fluoro-5-trifluoromethylphenylazetidin-3-yl)methyl)-N-(1H-indol-3-yl)-3,3-dimethyl-2-oxoindolin-5-carboxamide (I-40)**

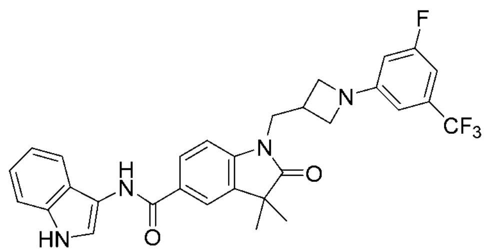

**Step 1: 5-bromo-1-((1-(3-fluoro-5-(trifluoromethyl)phenyl)azetidin-3-yl)methyl)-3,3-dimethylindolin-2-one (40-1)**

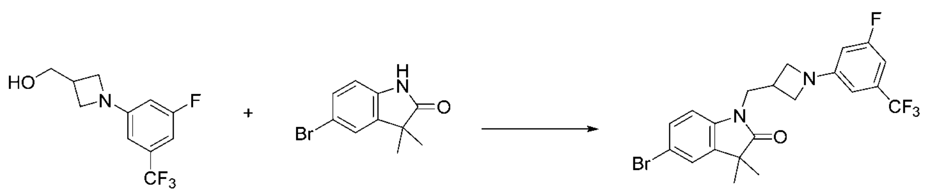

The preparation of 5-bromo-1-((1-(3-fluoro-5-(trifluoromethyl)phenyl)azetidin-3-yl)methyl)-3,3-dimethylindolin-2-one **(40-1)** was the same as that of compound **9-2.** $^1$H NMR (400 MHz, $CDCl_3$) $\delta$ 7.40 (dd, *J*= 8.3, 2.0 Hz, 1H), 7.34 (d, *J*= 2.0 Hz, 1H), 6.77 (d, *J*= 8.3 Hz, 1H), 6.64 (d, *J* = 8.7 Hz, 1H), 6.38 (d, *J* = 10.2 Hz, 1H), 6.21 (dt, *J* = 10.4, 2.2 Hz, 1H), 4.05-3.91 (m, 4H), 3.73 (dd, *J* = 7.4, 5.1 Hz, 2H), 3.23-3.13 (m, 1H), 1.37 (s, 6H).

**Step 2: 1-((1-(3-fluoro-5-(trifluoromethyl)phenyl)azetidin-3-yl)methyl)-3,3-dimethyl-2-indolin-5-nitrile (40-2)**

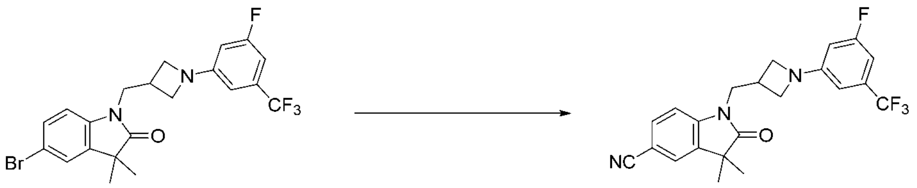

The preparation of 1-((1-(3-fluoro-5-(trifluoromethyl)phenyl)azetidin-3-yl)methyl)-3,3-dimethyl-2-indolin-5-nitrile **(40-2)** was the same as that of compound **11-2.** $^1$H NMR (400 MHz, $CDCl_3$) $\delta$ 7.61 (dd, *J*= 8.2, 1.7 Hz, 1H), 7.48 (d, *J*= 1.6 Hz, 1H), 6.97 (d, *J*= 8.2 Hz, 1H), 6.66 (d, *J* = 8.4 Hz, 1H), 6.37 (s, 1H), 6.22 (dt, *J* = 10.6, 2.3 Hz, 1H), 4.05 (d, *J* = 7.5 Hz, 2H), 3.98 *(t, J*= 7.6 Hz, 2H), 3.74 (dd, *J* = 7.5, 5.0 Hz, 2H), 3.17 (tt, *J* = 7.6, 5.0 Hz, 1H), 1.40 (s, 6H).

**Step 3: 1-((1-(3-fluoro-5-(trifluoromethyl)phenyl)azetidin-3-yl)methyl)-3,3-dimethyl-2-indolin-5-carboxylic acid (40-3)**

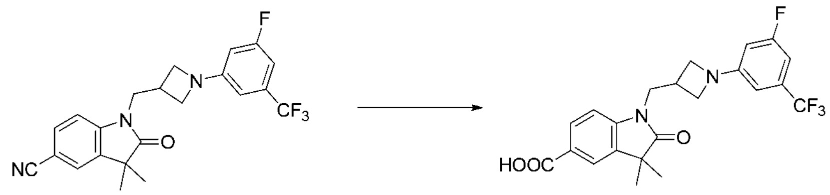

The preparation of 1-((1-(3-fluoro-5-(trifluoromethyl)phenyl)azetidin-3-yl)methyl)-3,3-dimethyl-2-indolin-5-carboxylic acid **(40-3)** was the same as that of compound **11-3.** LCMS (ESI) *m/z* $[M+H]^+$: 437.1.

**Step 4: 1-((1-(3-fluoro-5-trifluoromethylphenylazetidin-3-yl)methyl)-N-(1H-indol-3-yl)-3,3-dimethyl-2-oxoindolin-5-carboxamide** (**I-40**)

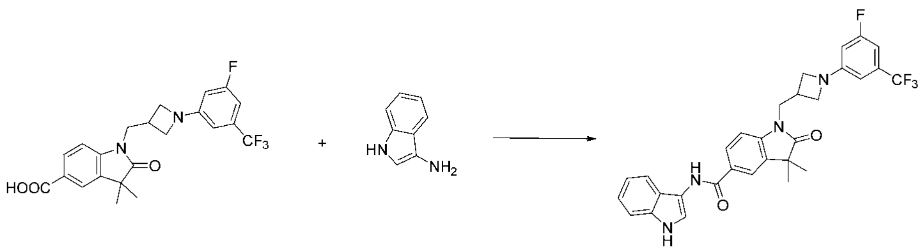

1-((1-(3-fluoro-5-trifluoromethylphenylazetidin-3-yl)methyl)-N-(1H-indol-3-yl)-3,3-dimethyl-2-oxoindolin-5-carboxamide (**I-40**). $^1$H NMR (400 MHz, $CDCl_3$) $\delta$ 8.09 (s, 1H), 7.97 (d, *J= 2.5* Hz, 1H), 7.93 (s, 1H), 7.87 (d, *J* = 6.9 Hz, 2H), 7.59 (d, *J* = 7.8 Hz, 1H), 7.42 (d, *J* = 8.1 Hz, 1H), 7.24 (s, 1H), 7.16-7.18 (m, 1H), 7.00 (d, *J*= 8.6 Hz, 1H), 6.66 (d, *J* = 8.5 Hz, 1H), 6.39 (s, 1H), 6.23 (dt, *J* = 10.5, 2.3 Hz, 1H), 4.08 (d, *J* = 7.4 Hz, 2H), 4.00 (t, *J* = 7.6 Hz, 2H), 3.77 (dd, *J* = 7.4, 5.1 Hz, 2H), 3.29-3.17 (m, 1H), 1.44 (s, 6H).

**Example 41 1-((1-(3-fluoro-5-trifluoromethylphenylazetidin-3-yl)methyl)-N-(1H-indol-3-yl)-3,3-dimethyl-2-oxoindolin-6-carboxamide (I-41)**

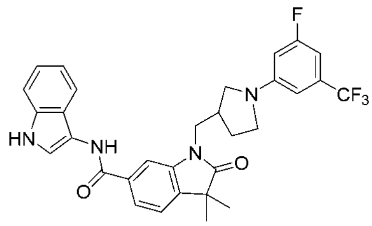

**Step 1: (1-(3-fluoro-5-(trifluoromethyl)phenyl)pyrrolidin-3-yl)methanol (41-1)**

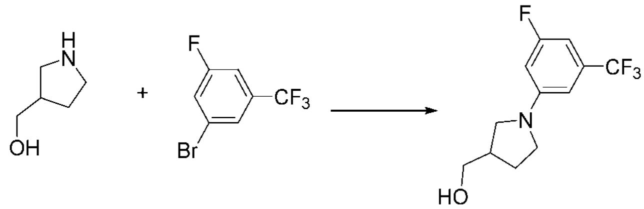

1 Gram of 3-hydroxymethyltetrahydropyrrolidine, 2.2 grams of 1-bromo-3-fluoro-5-(trifluoromethyl)benzene, 82 milligrams of tris(dibenzylidene-BASE acetone) dipalladium, 165 milligrams of (S)-(-)-1,1'-binaphthalene-2,2'-diylbis(diphenylphosphine), and 3 grams of potassium tert-butoxide were dissolved in 30 milliliters of toluene. Then 1 milliliter of triethylamine was added and the reaction mixture was stirred at 110 °C for 12 hours. The reaction solution was concentrated to dryness, and the residue was subjected to column chromatography with petroleum ether: ethyl acetate = 80:20 to obtain 725 mg of (1-(3-fluoro-5-(trifluoromethyl)phenyl)pyrrolidin-3-yl)methanol **(41-1)** as a yellow oil, with a yield of 31%. $^1$H NMR (400 MHz, DMSO-$d_6$) $\delta$ 6.67 (dt, $J$ = 8.8, 2.0 Hz, 1H), 6.59-6.53 (m, 2H), 4.72 (t, $J$= 5.3 Hz, 1H), 3.47-3.33 (m, 4H), 3.31-3.24 (m, 1H), 3.03-3.09 (m, 1H), 2.40-2.47 (m, 1H), 1.97-2.07 (m, 1H), 1.70-1.80 (m, 1H). LRMS (ESI) *m/z* [M+H]$^+$: 264.1.

**Step 2: 3-(chloromethyl)-1-(3-fluoro-5-(trifluoromethyl)phenyl)pyrrolidine (41-2)**

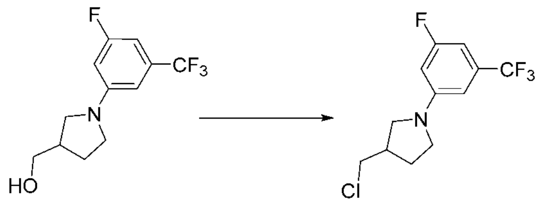

600 Milligrams of **41-1** was dissolved in 5 milliliters of dichloromethane, then 661 microliters of dichlorosulfoxide was added dropwise in an ice bath and the reaction mixture was stirred at room temperature for 12 hours. The reaction solution was concentrated to dryness, and the residue was subjected to column chromatography with ethyl acetate to obtain 138 mg of 3-(chloromethyl)-1-(3-fluoro-5-(trifluoromethyl)phenyl)pyrrolidine **(41-2)** as a yellow oil with a yield of 21%. $^1$H NMR (400 MHz, DMSO-$d_6$) $\delta$ 6.71 (d, $J$ = 8.7 Hz, 1H), 6.60 (dt, $J$ = 14.1, 2.1 Hz, 2H), 3.74 (dd, $J$ = 6.8, 1.7 Hz, 2H), 3.45-3.52 (m, 1H), 3.38-3.44 (m, ,1H), 3.26-3.31 (m, 1H), 3.08-3.15 (m, 1H), 2.67-2.77 (m, 1H), 2.20-2.12 (m, 1H), 1.77-1.87 (m, 1H).

**Step 3: methyl 1-((1-(3-fluoro-5-(trifluoromethyl)phenyl)pyrrolidin-3-yl)methyl)-3,3-dimethyl-2-oxoindolin-6-carboxylate (41-3)**

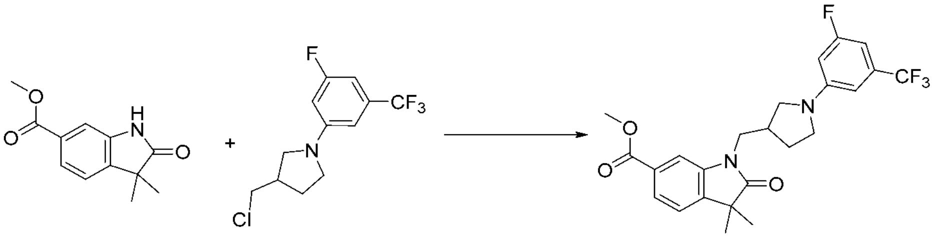

The preparation method of methyl 1-((1-(3-fluoro-5-(trifluoromethyl)phenyl)pyrrolidin-3-yl)methyl)-3,3-dimethyl-2-oxoindolin-6-carboxylate **(41-3)** was the same as that of compound **1-4.** $^1$H NMR (400 MHz, $CDCl_3$) $\delta$ 7.81 (dd, *J*= 7.7, 1.4 Hz, 1H), 7.49 (d, *J* = 1.4 Hz, 1H), 7.30 (d, *J*= 7.7 Hz, 1H), 6.59 (dt, *J*= 8.7, 1.9 Hz, 1H), 6.49 (t, *J*= 1.9 Hz, 1H), 6.32 (dt, *J* = 11.7, 2.3 Hz, 1H), 3.95-3.80 (m, 4H), 3.74-3.81 (m, 1H), 3.45-3.53 (m,1H), 3.40-3.45 (m, 1H), 3.27-3.35 (m, 1H), 3.17-3.22(m, 1H), 2.85-2.95 (m, 1H), 2.08-2.18 (m, 1H), 1.85-1.95 (m, 1H), 1.41 (s, 6H). LRMS (ESI) *m/z* [M+H]$^+$: 465.2

**Step 4: 1-((1-(3-fluoro-5-(trifluoromethyl)phenyl)pyrrolidin-3-yl)methyl)-3,3-dimethyl-2-oxoindolin-6-carboxylic acid (41-4)**

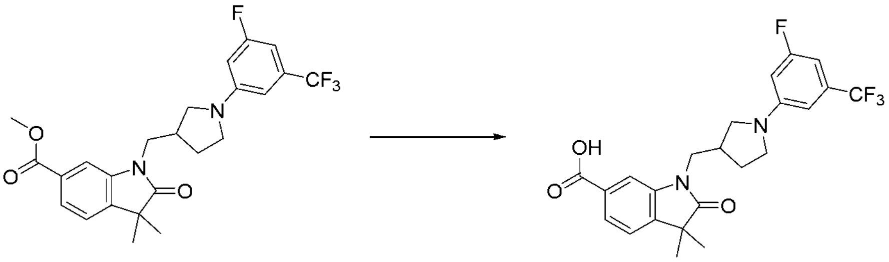

The preparation method of 1-((1-(3-fluoro-5-(trifluoromethyl)phenyl)pyrrolidin-3-yl)methyl)-3,3-dimethyl-2-oxoindolin-6-carboxylic acid **(41-4)** was the same as that of compound **1-5.** $^1$H NMR (400 MHz, DMSO-$d_6$) $\delta$ 12.99 (s, 1H), 7.69 (dd, *J* = 7.7, 1.4 Hz, 1H), 7.61 (d, *J*= 1.4 Hz, 1H), 7.50 (d, *J*= 7.7 Hz, 1H), 6.69 (d, *J*= 8.8 Hz, 1H), 6.54 (dd, *J* = 10.5, 2.2 Hz, 2H), 3.89-3.76 (m, 2H), 3.37-3.47 (m, 2H), 3.08-3.14 (m, 1H), 2.75-2.85 (m,1H), 2.00-2.10 (m, 1H), 1.70-1.80 (m, 1H), 1.32 (d, *J*= 1.7 Hz, 6H), 1.20-1.25 (m, 1H).

**Step 5: 1-((1-(3-fluoro-5-trifluoromethylphenylazetidin-3-yl)methyl)-N-(1H-indol-3-yl)-3,3-dimethyl-2-oxoindolin-6-carboxamide (I-41)**

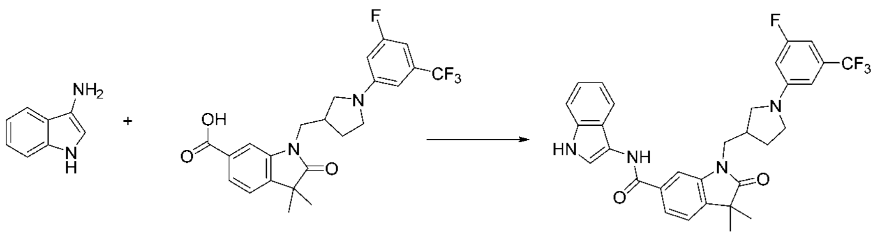

The preparation method of 1-((1-(3-fluoro-5-trifluoromethylphenylazetidin-3-yl)methyl)-N-(1H-indol-3-yl)-3,3-dimethyl-2-oxoindolin-6-carboxamide (**I-41**) was the same as that of compound **I-1**. $^1$H NMR (400 MHz, DMSO-$d_6$) $\delta$ 10.93 (s, 1H), 10.12 (s, 1H), 7.82 (d, *J* = 8.0 Hz, 1H), 7.77 (d, *J*= 2.5 Hz, 1H), 7.75-7.69 (m, 2H), 7.54 (d, *J*= 7.6 Hz, 1H), 7.37 (d, *J*= 8.1 Hz, 1H), 7.11 (t, *J* = 7.4 Hz, 1H), 7.00 (t, *J* = 7.5 Hz, 1H), 6.69 (d, *J* = 8.6 Hz, 1H), 6.57 (dd, *J* = 11.1, 2.1 Hz, 2H), 3.80-3.90 (m, 2H), 3.40-3.50 (m, 2H), 3.31-3.25 (m, 1H), 3.10-3.20 (m, 1H), 2.85-2.95 (m, 1H), 2.00-2.10 (m, 1H), 1.75-1.85 (m, 1H), 1.35 (d, *J*= 1.8 Hz, 6H). LRMS (ESI) m/z [M+H]$^+$: 565.0.

**Example 42 N-(1H-indol-3-yl)-3,3-dimethyl-2-oxo-1-((1-phenylpyrrol-3-yl)methyl)indolin-6-carboxamide (I-42)**

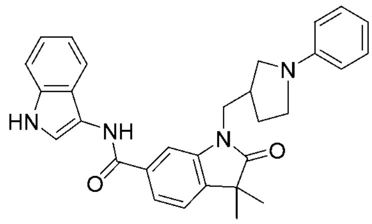

**Step 1: (1-phenylpyrrolidin-3-yl)methanol (42-1)**

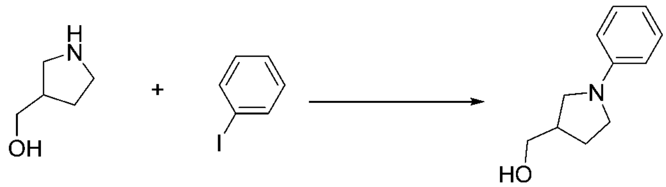

A mixture of 1 gram of 3-hydroxymethyltetrahydropyrrolidine, 2.2 grams of iodobenzene, 1.9 grams of cuprous iodide, 1.14 grams of L-proline, 4.1 grams of potassium carbonate, and 10 milliliters of dimethyl sulfoxide was stirred at 90 °C for 12 hours. Water was added to the reaction solution, the mixture was extracted with ethyl acetate, and the organic layer was concentrated to dryness. The residue was subjected to column chromatography with petroleum ether: ethyl acetate = 80:20 to obtain 795 mg of purple oily substance (1-phenylpyrrolidin-3-yl)methanol **(42-1),** with a yield of 45%. $^1$H NMR (400 MHz, DMSO-$d_6$) $\delta$ 7.14 (t, $J$ = 7.9 Hz, 2H), 6.56 (t, $J$ = 7.2 Hz, 1H), 6.50 (d, $J$ = 8.1 Hz, 2H), 4.69 (t, $J$ = 5.2 Hz, 1H), 3.47-3.34 (m, 2H), 3.31-3.15 (m, 3H), 2.95-3.05 (m, 1H), 2.35-2.45 (m, 1H), 1.95-2.05 (m,1H), 1.68-1.78 (m, 1H). LRMS (ESI) *m/z* $[M+H]^+$: 178.2.

**Step 2: 3-(chloromethyl)-1-phenylpyrrolidine (42-2)**

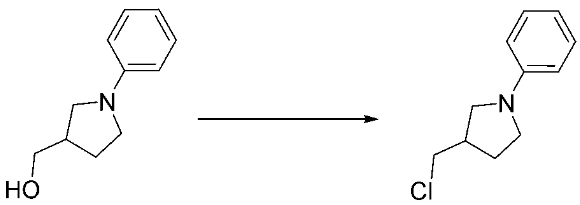

A mixture of 875 milligrams of compound **42-1**, 1.4 grams of triphenylphosphine, 952 microliters of carbon tetrachloride, and 15 milliliters of dichloromethane was stirred at room temperature for 12 hours. The reaction solution was concentrated to dryness, and the residue was subjected to column chromatography with petroleum ether: ethyl acetate = 90:10 to obtain 716 mg of 3-(chloromethyl)-1-phenylpyrrolidine **(42-2)** as a purple oily substance, with a yield of 74%. $^1$H NMR (400 MHz, DMSO-$d_6$) $\delta$ 7.19-7.12 (m, 2H), 6.59 (tt, $J$ = 7.3, 1.2 Hz, 1H), 6.55-6.49 (m, 2H), 3.71-3.76 (m, 2H), 3.40 (dd, $J$ = 9.5, 7.5 Hz, 1H), 3.36-3.32 (m, 1H), 3.20-3.28 (m, 1H), 3.05 (dd, $J$ = 9.6, 6.8 Hz, 1H), 2.65-2.75 (m, 1H), 2.10-2.20 (m, 1H), 1.75-1.88 (m, 1H). LRMS (ESI) *m/z* $[M+H]^+$: 196.1.

**Step 3: methyl 3,3-dimethyl-2-oxodihydroindol-6-carboxylate (42-3)**

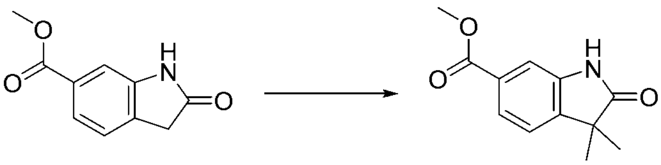

2 Grams of methyl 2-oxoindoline-6-carboxylate and 6.8 grams of cesium carbonate were dissolved in 40 milliliters of anhydrous dimethyl sulfoxide. 1.3 Milliliters of iodomethane was slowly added dropwise under argon atmosphere and the reaction mixture was stirred at room temperature for 0.5 hours. Water was added to the reaction solution, the mixture was extracted with ethyl acetate, the organic phase was concentrated to dryness, and the residue was subjected to column chromatography with petroleum ether: ethyl acetate = 90:10 to obtain 1.2 g of methyl 3,3-dimethyl-2-oxodihydroindol-6-carboxylate **(42-3)** as a white solid with a yield of 52%. $^1$H NMR (400 MHz, DMSO-$d_6$) $\delta$ 10.53 (s, 1H), 7.62 (dd, *J* = 7.7, 1.2 Hz, 1H), 7.44 (d, *J* = 7.7 Hz, 1H), 7.38-7.36 (m, 1H), 3.84 (s, 3H), 1.27 (s, 6H).

**Step 4: methyl 3,3-dimethyl-2-oxo-1-((1-phenylpyrrolidin-3-yl)methyl)dihydroindol-6-carboxylate (42-4)**

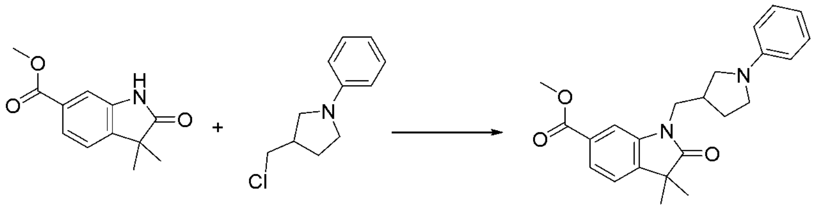

The preparation of methyl 3,3-dimethyl-2-oxo-1-((1-phenylpyrrolidin-3-yl)methyl)dihydroindol-6-carboxylate **(42-4)** was the same as that of compound **1-4.** $^1$H NMR (400 MHz, $CDCl_3$) $\delta$ 7.80 (dd, *J*= 7.7, 1.4 Hz, 1H), 7.53-7.51 (m, 2H), 7.29 (d, *J*= 7.7 Hz, 1H), 7.24-7.19 (m, 2H), 6.67 (tt, *J* = 7.3, 1.1 Hz, 1H), 6.54 (d, *J*= 7.9 Hz, 2H), 3.96-3.91 (m, 1H), 3.90 (s, 3H), 3.77 (dd, *J* = 14.1, 7.0 Hz, 1H), 3.53-3.44 (m, 1H), 3.41 (dd, *J*= 9.3, 7.1 Hz, 1H), 3.30 (dt, *J*= 9.2, 7.3 Hz, 1H), 3.18 (dd, *J* = 9.3, 6.3 Hz, 1H), 2.93-2.81 (m, 1H), 2.14-2.03 (m, 1H), 1.86 (dq, *J* = 12.4, 7.6 Hz, 1H), 1.41 (s, 6H). LRMS (ESI) *m/z* $[M+H]^+$: 379.2

**Step 5: 3,3-dimethyl-2-oxo-1-((1-phenylpyrrolidin-3-yl)methyl)dihydroindol-6-carboxylic acid (42-5)**

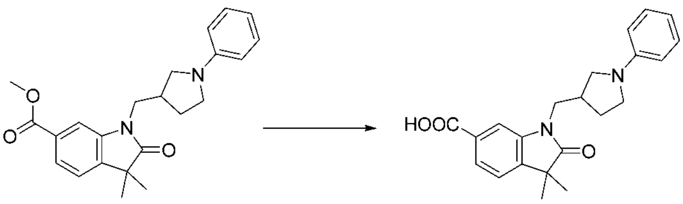

The preparation of 3,3-dimethyl-2-oxo-1-((1-phenylpyrrolidin-3-yl)methyl)dihydroindol-6-carboxylic acid **(42-5)** was the same as that of compound **1-5.** $^1$H NMR (400 MHz, DMSO-$d_6$) $\delta$ 13.00 (s, 1H), 7.70 (dd, *J* = 7.7, 1.4 Hz, 1H), 7.62 (d, *J*= 1.4 Hz, 1H), 7.53-7.48 (m, 1H), 7.19-7.09 (m, 2H), 6.57 (t, *J*= 7.3 Hz, 1H), 6.48 (d, *J*= 8.1 Hz, 2H), 3.84 (d, *J* = 7.6 Hz, 2H), 3.27-3.15 (m, 2H), 3.06 (dd, *J* = 9.5, 6.3 Hz, 1H), 2.84-2.74 (m, 1H), 2.08-1.96 (m, 1H), 1.80-1.69 (m, 1H), 1.32 (s, 7H).

**Step 6: N-(1H-indol-3-yl)-3,3-dimethyl-2-oxo-1-((1-phenylpyrrol-3-yl)methyl)indolin-6-carboxamide (I-42)**

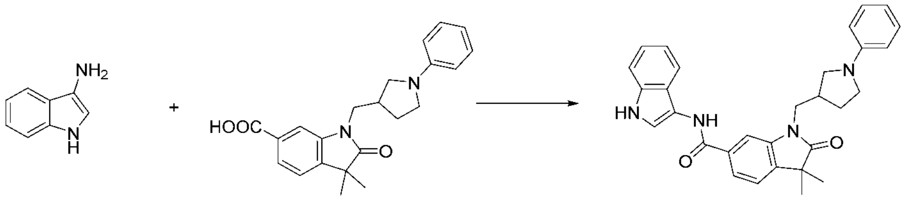

The preparation of N-(1H-indol-3-yl)-3,3-dimethyl-2-oxo-1-((1-phenylpyrrol-3-yl)methyl)indolin-6-carboxamide **(I-42)** was the same as that of compound **I-1**. $^1$H NMR (400 MHz, DMSO-$d_6$) $\delta$ 10.92 (s, 1H), 10.12 (s, 1H), 7.82 (d, *J* = 7.9 Hz, 1H), 7.77 (d, *J* = 2.5 Hz, 1H), 7.75-7.66 (m, 2H), 7.54 (d, *J* = 7.6 Hz, 1H), 7.37 (d, *J* = 8.2 Hz, 1H), 7.20-7.07 (m, 3H), 7.01 (td, *J* = 7.4, 1.0 Hz, 1H), 6.57 (t, *J*= 7.2 Hz, 1H), 6.50 (d, *J*= 8.1 Hz, 2H), 3.82-3.92 (m, 2H), 3.34-3.41 (m, 2H), 3.20-3.26 (m, 1H), 3.06-3.12 (m, 1H), 2.87-2.95 (m, 1H), 2.00-2.10 (m, 1H), 1.73-1.83 (m, 1H), 1.35 (s, 6H). LRMS (ESI) m/z [M+H]$^+$: 479.3.

**Example 43 N-(1H-indol-3-yl)-3,3-dimethyl-2-oxo-1-((1-phenylpiperidin-4-yl)methyl)indolin-6-carboxamide (I-43)**

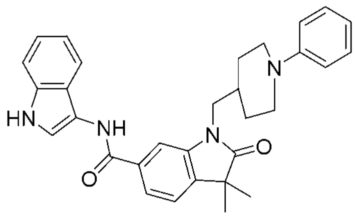

**Step 1: (1-phenylpiperidin-4-yl)methanol (43-1)**

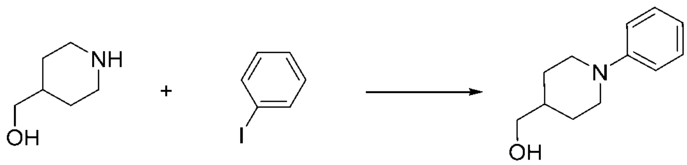

The preparation method of (1-phenylpiperidin-4-yl)methanol **(43-1)** was the same as that of compound 42-1. $^1$H NMR (400 MHz, DMSO-$d_6$) $\delta$ 7.18 (t, *J* = 7.9 Hz, 2H), 6.91 (d, *J* = 8.2 Hz, 2H), 6.72 (t, *J*= 7.2 Hz, 1H), 4.46 (t, *J*= 5.4 Hz, 1H), 3.68 (dt, *J* = 12.6, 3.5 Hz, 2H), 3.28 (t, *J* = 5.7 Hz, 2H), 2.61 (td, *J*= 12.3, 2.6 Hz, 2H), 1.73 (d, *J*= 12.5 Hz, 2H), 1.44-1.54 (m, 1H), 1.22 (qd, *J*= 12.4, 4.0 Hz, 2H). LRMS (ESI) *m/z* [M+H]$^+$: 192.1.

**Step 2: 6-bromo-3,3-dimethyl-1-((1-phenylpiperidin-4-yl)methyl)dihydroindol-2-one (43-2)**

The preparation of 6-bromo-3,3-dimethyl-1-((1-phenylpiperidin-4-yl)methyl)dihydroindol-2-one **(43-2)** was the same as that of compound **9-2.** $^1$H NMR (400 MHz, $CD_3OD$) $\delta$ 7.27-7.18

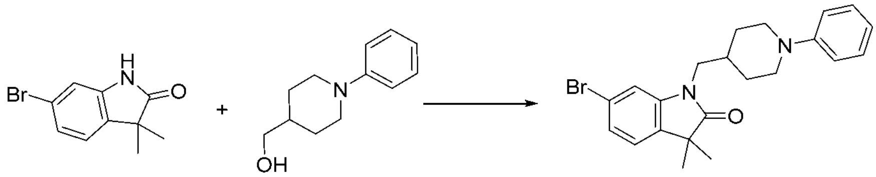

(m, 5H), 6.99-6.95 (m, 2H), 6.82 (t, *J* = 7.3 Hz, 1H), 3.70-3.62 (m, 4H), 2.65 (td, *J* = 12.2, 2.5 Hz, 2H), 1.90-2.00 (m, 1H), 1.75 (d, *J* = 12.6 Hz, 2H), 1.50 (td, *J* = 12.0, 3.4 Hz, 2H), 1.35 (s, 6H). LRMS (ESI) *m/z* [M+H]$^+$: 413.1.

**Step 3: 6-cyano-3,3-dimethyl-1-((1-phenylpiperidin-4-yl)methyl)dihydroindol-2-one** (**43-3**)

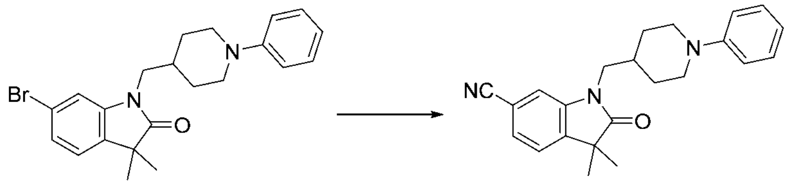

The preparation of 6-cyano-3,3-dimethyl-1-((1-phenylpiperidin-4-yl)methyl)dihydroindol-2-one (**43-3**) was the same as that of compound **11-2.** $^{1}$H NMR (400 MHz, $CD_3OD$-$d_4$) $\delta$ 7.49 (dd, *J* = 11.6, 7.7 Hz, 3H), 7.21 (t, *J* = 8.0 Hz, 2H), 6.97 (d, *J* = 8.1 Hz, 2H), 6.81 *(t, J*= 7.4 Hz, 1H), 3.72 *(d, J*= 7.3 Hz, 2H), 3.66 (d, *J* = 12.2 Hz, 2H), 2.65 (t, *J* = 12.1 Hz, 2H), 1.75 (d, *J* = 12.1 Hz, 2H), 1.50 (td, *J* = 12.2, 3.8 Hz, 2H), 1.39 (s, 6H), 1.24 (t, *J* = 7.2 Hz, 1H). LRMS (ESI) *m/z* [M+H]$^+$: 360.2.

**Step 4: 3,3-dimethyl-2-oxo-1-((1-phenylpiperidin-4-yl)methyl)dihydroindol-6-carboxylic acid (43-4)**

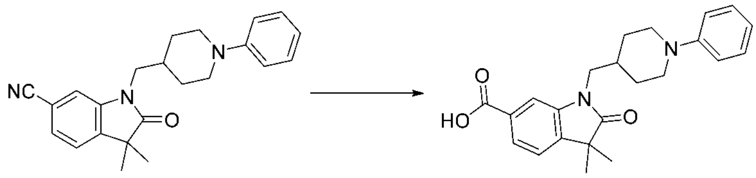

The preparation method of 3,3-dimethyl-2-oxo-1-((1-phenylpiperidin-4-yl)methyl)dihydroindol-6-carboxylic acid **(43-4)** was the same as that of compound **11-3.** $^{1}$H NMR (400 MHz, DMSO-$d_6$) $\delta$ 12.94 (s, 1H), 7.69 (dd, *J* = 7.7, 1.4 Hz, 1H), 7.58 (d, *J*= 1.4 Hz, 1H), 7.50 (d, *J*= 7.7 Hz, 1H), 7.30 (s, 5H), 3.69 (d, *J*= 7.2 Hz, 2H), 3.63 (d, *J*= 11.9 Hz, 2H), 1.99 (s, 2H), 1.74 (s, 2H), 1.31 (s, 6H), 1.26-1.21 (m, 1H), 1.17 (t, *J*= 7.1 Hz, 2H).

**Step 5: N-(1H-indol-3-yl)-3,3-dimethyl-2-oxo-1-((1-phenylpiperidin-4-yl)methyl)indolin-6-carboxamide** (**I-43**)

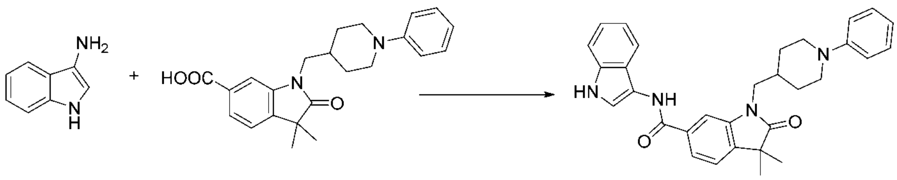

The preparation method of N-(1H-indol-3-yl)-3,3-dimethyl-2-oxo-1-((1-phenylpiperidin-4-yl)methyl)indolin-6-carboxamide (**I-43**) was the same as that of compound **I-1**. $^{1}$H NMR (400 MHz, DMSO-$d_6$) $\delta$ 10.12 (s, 1H), 7.83 (d, *J* = 7.9 Hz, 1H), 7.78 (d, *J* = 2.5 Hz, 1H), 7.72 (dd, *J* = 7.7, 1.4 Hz, 1H), 7.63 (d, *J*= 1.6 Hz, 1H), 7.52 (d, *J*= 7.7 Hz, 1H), 7.37 (d, *J*= 8.1 Hz, 1H), 7.20-7.15 (m, 2H), 7.11 (t, *J* = 7.4 Hz, 1H), 7.01 (t, *J* = 7.5 Hz, 1H), 6.91 (d, *J* = 8.2 Hz, 2H), 6.72 (t, *J*= 7.2 Hz, 1H), 3.73-3.65 (m, 4H), 2.63 (t, *J*= 12.0 Hz, 2H), 2.08-1.97 (m, 1H), 1.68 (d, *J* = 12.7 Hz, 2H), 1.43-1.35 (m, 2H), 1.34 (s, 6H). LRMS (ESI) *m/z* [M+H]$^+$: 493.3.

**Example 44 N-(1H-indol-3-yl)-3,3-dimethyl-2-oxo-1-((1-(thien-3-yl)pyrrol-3-yl)methyl)indolin-6-carboxamide** (**I-44**)

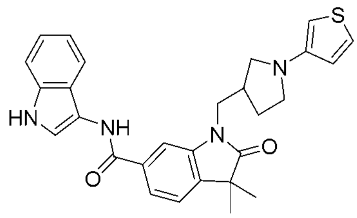

**Step 1: (1-(thien-3-yl)pyrrolidin-3-yl)methanol (44-1)**

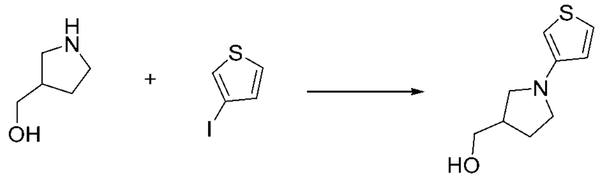

A mixture of 1.4 grams of 3-hydroxymethyltetrahydropyrrolidine, 2 grams of 3-iodothiophene, 182.3 milligrams of cuprous iodide, 6.2 grams of cesium carbonate, 641 milligrams of 2-isobutyrylcyclohexanone, and 10 milliliters of N, N-dimethylformamide was stirred at room temperature under argon atmosphere for 12 hours. Water was added to the reaction solution, the mixture was extracted with ethyl acetate, the organic phase was concentrated to dryness, and the residue was subjected to column chromatography with petroleum ether: ethyl acetate = 80:20 to obtain 1.4 grams of red oily substance (1-(thien-3-yl)pyrrolidin-3-yl)methanol **(44-1)** with a yield of 80%. $^1$H NMR (400 MHz, DMSO-$d_6$) $\delta$ 7.35 (dd, *J* = 5.2, 3.1 Hz, 1H), 6.71 (dd, *J*= 5.1, 1.5 Hz, 1H), 5.87 (dd, *J*= 3.1, 1.5 Hz, 1H), 4.67 (t, *J*= *5.2* Hz, 1H), 3.44-3.36 (m, 2H), 3.26-3.09 (m, 3H), 2.91-2.96 (m, 1H), 2.30-2.45 (m, 1H), 1.92-2.02 (m, 1H), 1.61-1.71 (m, 1H).

**Step 2: methyl (1-(thien-3-yl)pyrrolidin-3-yl)4-methylbenzenesulfonate (44-2)**

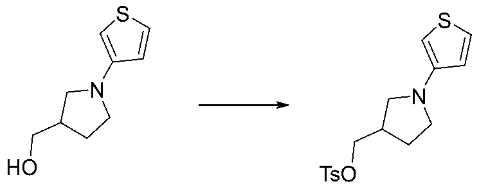

500 mg of Compound **44-1** and 33.3 mg of 4-dimethylaminopyridine were dissolved in 8 mL of dichloromethane, then 1.1 mL of triethylamine was added dropwise, followed by the addition of 624 mg of *p*-toluenesulfonyl chloride and the reaction mixture was stirred at room temperature for 2 hours. The reaction solution was concentrated to dryness, and the residue was subjected to column chromatography with petroleum ether: ethyl acetate = 80:20 to obtain 355 mg of pink solid methyl (1-(thien-3-yl)pyrrolidin-3-yl)4-methylbenzenesulfonate **(44-2)** with a yield of 38%. $^1$H NMR (400 MHz, DMSO-$d_6$) $\delta$ 7.81 (d, *J* = 8.1 Hz, 2H), 7.48 (d, *J* = 8.0 Hz, 2H), 7.35 (dd, *J* = 5.1, 3.1 Hz, 1H), 6.67 (dd, *J*= 5.2, 1.5 Hz, 1H), 3.98-4.08 (m,3H), 3.24-3.02 (m, 4H), 2.80-2.87 (m, 1H), 2.59 (p, *J* = 7.1 Hz, 1H), 2.42 (s, 3H), 1.94-2.04 (m, 1H), 1.67-1.53 (m, 1H). LRMS (ESI) *m*/*z* [M+H]$^+$: 338.1.

**Step 3: methyl 3,3-dimethyl-2-oxo-1-((1-(thien-3-yl)pyrrolidin-3-yl)methyl)dihydroindol-6-carboxylate (44-3)**

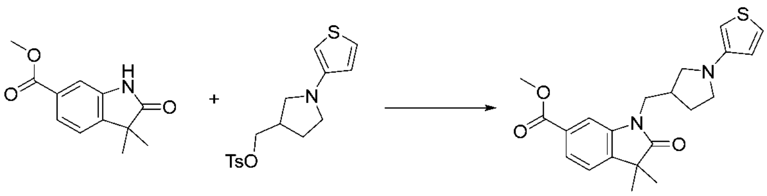

The preparation method of methyl 3,3-dimethyl-2-oxo-1-((1-(thien-3-yl)pyrrolidin-3-yl)methyl)dihydroindol-6-carboxylate **(44-3)** was the same as that of compound **1-4.** $^1$H NMR (400 MHz, DMSO-$d_6$) $\delta$ 7.71 (dd, *J*= 7.7, 1.4 Hz, 1H), 7.61 (d, *J*= 1.4 Hz, 1H), 7.53 (d, *J*= 7.7 Hz, 1H), 7.36 (dd, *J*= 5.1, 3.1 Hz, 1H), 6.68 (dd, *J*= 5.2, 1.5 Hz, 1H), 5.87 (dd, *J* = 3.1, 1.5 Hz, 1H), 3.85 (s, 3H), 3.79-3.83 (m, 2H), 3.32-3.28 (m, 1H), 3.22-3.26 (m, 1H), 3.10-3.17 (m, 1H), 2.98-3.04 (m, 1H), 2.72-2.80 (m, 1H), 1.93-2.03 (m, 1H), 1.65-1.75 (m, 1H), 1.32 (s, 6H). LRMS (ESI) *m/z* [M+H]$^+$: 385.3.

**Step 4: 3,3-dimethyl-2-oxo-1-((1-(thien-3-yl)pyrrolidin-3-yl)methyl)dihydroindol-6-carboxylic acid (44-4)**

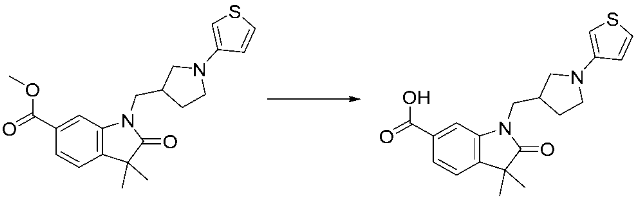

The preparation method of 3,3-dimethyl-2-oxo-1-((1-(thien-3-yl)pyrrolidin-3-yl)methyl)dihydroindol-6-carboxylic acid **(44-4)** was the same as that of compound **1-5.** $^1$H NMR (400 MHz, DMSO-$d_6$) $\delta$ 12.99 (s, 1H), 7.69 (dd, *J*= 7.7, 1.4 Hz, 1H), 7.60 (d, *J*= 1.4 Hz, 1H), 7.50 (d, *J* = 7.7 Hz, 1H), 7.35 (dd, *J*= 5.2, 3.1 Hz, 1H), 6.68 (dd, *J*= 5.1, 1.5 Hz, 1H), 5.87 (dd, *J*= 3.2, 1.5 Hz, 1H), 3.75-3.85 (m, 2H), 3.22-3.32 (m, 2H), 3.18-3.10 (m, 1H), 2.95-3.05 (m, 1H), 2.70-2.80 (m, 1H), 1.95-2.05 (m, 1H), 1.65-1.75 (m, 1H), 1.32 (s, 6H).

**Step 5: N-(1H-indol-3-yl)-3,3-dimethyl-2-oxo-1-((1-(thien-3-yl)pyrrol-3-yl)methyl)indolin-6-carboxamide (I-44)**

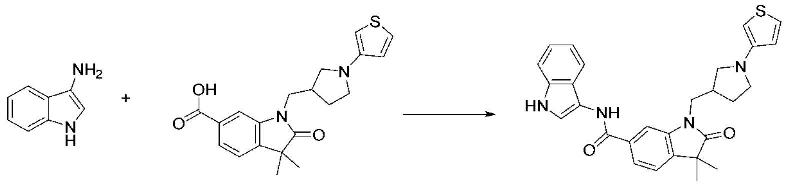

The preparation method of N-(1H-indol-3-yl)-3,3-dimethyl-2-oxo-1-((1-(thien-3-yl)pyrrol-3-yl)methyl)indolin-6-carboxamide **(I-44)** was the same as that of compound **I-1**. $^1$H NMR (400 MHz, DMSO-$d_6$) $\delta$ 10.93 (s, 1H), 10.12 (s, 1H), 7.82 (d, *J* = 8.0 Hz, 1H), 7.77 (d, *J* = 2.5 Hz, 1H), 7.72 (dd, *J* = 7.7, 1.5 Hz, 1H), 7.68 (s, 1H), 7.53 (d, *J* = 7.6 Hz, 1H), 7.40-7.33 (m, 2H), 7.11 (t, *J* = 7.5 Hz, 1H), 7.01 (t, *J* = 7.5 Hz, 1H), 6.70 (dd, *J*= 5.2, 1.5 Hz, 1H), 5.89 (dd, *J* = 3.2, 1.5 Hz, 1H), 3.81-3.88 (m, 2H), 3.27-3.30 (m, 2H), 3.15-3.20(m, 1H), 3.02-3.07 (m, 1H), 2.85-2.97 (m,1H), 1.97-2.06 (m, 1H), 1.68-1.78 (m, 1H), 1.35 (s, 6H). LRMS (ESI) *m/z* [M+H]$^+$: 485.2.

**Example 45 N-(1H-indol-3-yl)-6,6-dimethyl-5-oxo-4-((1-phenylazetidin-3-yl)methyl)-5,6-dihydro-4H-thieno[3,2-b]pyrrol-2-carboxamide** (**I-45**)

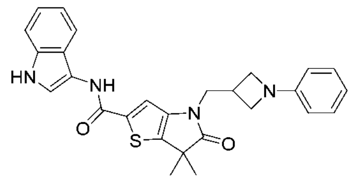

**Example 46 1-(1-benzylazetidin-3-yl)-N-(1H-indol-3-yl)-3,3-dimethyl-2-oxoindolin-6-carboxamide (I-46)**

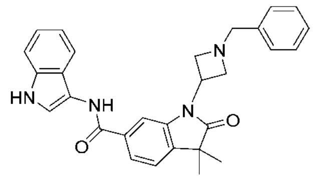

**Example 47 1-(1-benzylazetidin-3-yl)-N-(1H-indol-3-yl)-3,3-dimethyl-2-oxoindolin-5-carboxamide (I-47)**

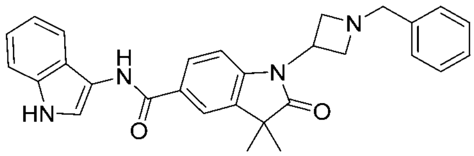

**Example 48 1'-((1-(3-fluoro-5-trifluoromethylphenylazetidin-3-yl)methyl)-N-(1H-indol-3-yl)-2'-oxospiro[cyclopropane-1,3'-indolin]-6'-carboxamide (I-48)**

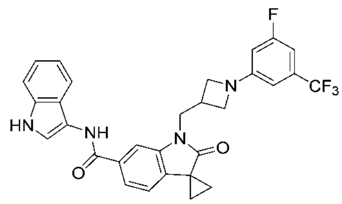

**Example 49 1-((1-benzylazetidin-3-yl)methyl)-N-(1H-indol-3-yl)-3,3-dimethyl-2-oxoindolin-6-carboxamide (I-49)**

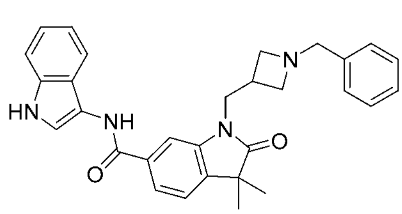

**Example 50 1-((1-benzylazetidin-3-yl)methyl)-N-(1H-indol-3-yl)-3,3-dimethyl-2-oxoindolin-5-carboxamide (I-50)**

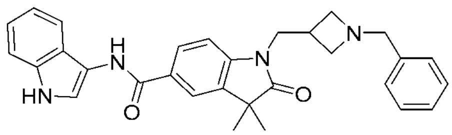

**Example 51 1-((1-(3-fluoro-5-trifluoromethylphenylazetidin-3-yl)methyl)-N-(1H-indol-3-yl)-3-methyl-2-oxoindolin-6-carboxamide (I-51)**

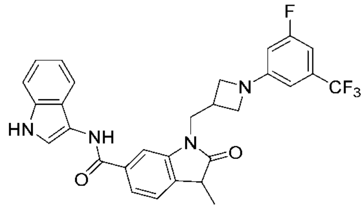

**Example 52 1-((1-(3-fluoro-5-trifluoromethylphenylazetidin-3-yl)methyl)-N-(1H-indol-3-yl)-3-methylene-2-oxoindolin-6-carboxamide (I-52)**

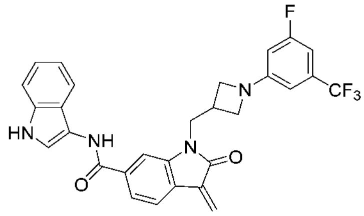

**Example 53 3,3-dichloro-1-((1-(3-fluoro-5-trifluoromethylphenylazetidin-3-yl)methyl)-N-(1H-indol-3-yl)-2-oxoindolin-6-carboxamide (I-53)**

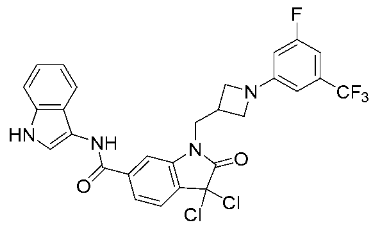

**Example 54 N-(1H-indol-3-yl)-3,3-dimethyl-2-oxo-1-((2-oxo-1-phenylazetidin-3-yl)methyl)indolin-6-carboxamide (I-54)**

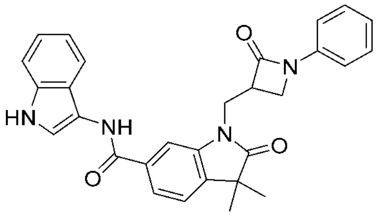

**Example 55 N-(1H-indol-3-yl)-3-oxo-4-(thien-3-ylmethyl)-3,4-dihydro-2H-benzothien[b][1,4]thiazin-6-carboxamide (I-55)**

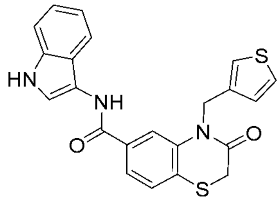

**Step 1: methyl 3-oxo-4-(thien-3-ylmethyl)-3,4-dihydro-2H-benzothien[b][1,4]thiazin-6-carboxylate (55-1)**

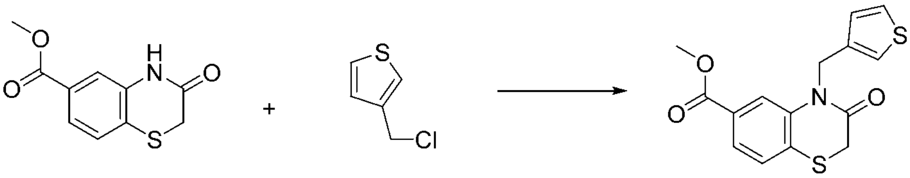

The preparation of methyl 3-oxo-4-(thien-3-ylmethyl)-3,4-dihydro-2H-benzothien[b][1,4]thiazin-6-carboxylate **(55-1)** was the same as that of compound **2-3,** with a yield of 54%. $^1$H NMR (400 MHz, DMSO-$d_6$) $\delta$ 7.71 (s, 1H), 7.59-7.54 (m, 2H), 7.54-7.46 (m, 1H), 7.29-7.24 (m, 1H), 6.99 (d, *J* = 4.9 Hz, 1H), 5.19 (s, 2H), 3.80 (s, 3H), 3.73 (s, 2H). LRMS (ESI) *m/z* [M+H] $^+$ 320.1.

**Step 2: 3-oxo-4-(thien-3-ylmethyl)-3,4-dihydro-2H-benzo[b][1,4]thiazin-6-carboxylic acid (55-2)**

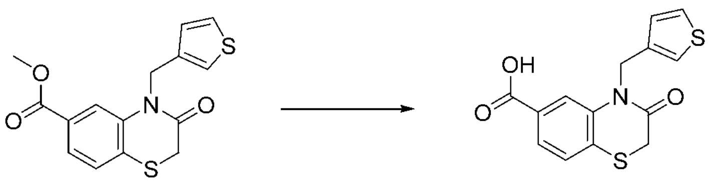

The preparation of 3-oxo-4-(thien-3-ylmethyl)-3,4-dihydro-2H-benzo[b][1,4]thiazin-6-carboxylic acid **(55-2)** was the same as that of compound **1-5,** with a yield of 91%. $^1$H NMR (400 MHz, DMSO-$d_6$) $\delta$ 13.04 (s, 1H), 7.70 (s, 1H), 7.63-7.44 (m, 3H), 7.29 (s, 1H), 7.00 (d, *J* = 5.0 Hz, 1H), 5.19 (s, 2H), 3.73 (s, 2H). LRMS (ESI) *m/z* [M-H]$^-$ 304.0.

**Step 3: N-(1H-indol-3-yl)-3-oxo-4-(thien-3-ylmethyl)-3,4-dihydro-2H-benzothien[b][1,4]thiazin-6-carboxamide (I-55)**

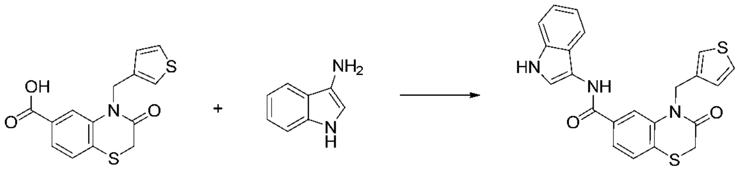

The preparation of N-(1H-indol-3-yl)-3-oxo-4-(thien-3-ylmethyl)-3,4-dihydro-2H-benzothien[b][1,4]thiazin-6-carboxamide **(I-55)** was the same as that of compound **I-1,** with a yield of : 68%. $^1$H NMR (400 MHz, DMSO-$d_6$) $\delta$ 10.94 (s, 1H), 10.18 (s, 1H), 7.83-7.75 (m, 3H), 7.70 (d, *J*= 7.4 Hz, 1H), 7.58 (d, *J*= 8.1 Hz, 1H), 7.56-7.49 (m, 1H), 7.38-7.33

(m, 2H), 7.12 (t, *J*= 7.5 Hz, 1H), 7.05-6.98 (m, 2H), 5.29 (s, 2H), 3.72 (s, 2H).

**Example 56 4-(furan-3-ylmethyl)-N-(1H-indol-3-yl)-3-oxo-3,4-dihydro-2H-benzo[b][1,4]thiazin-6-carboxamide (I-56)**

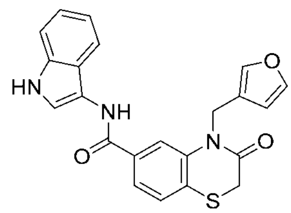

**Step 1: methyl 4-(furan-3-ylmethyl)-3-oxo-3,4-dihydro-2H-benzo[b][1,4]thiazin-6-carboxylate (56-1)**

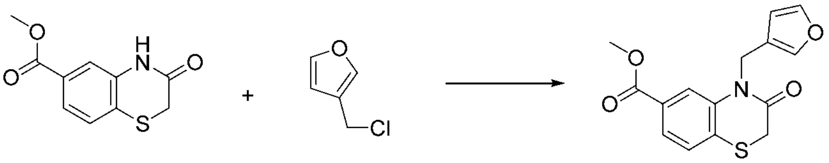

The preparation of methyl 4-(furan-3-ylmethyl)-3-oxo-3,4-dihydro-2H-benzo[b][1,4]thiazin-6-carboxylate **(56-1)** was the same as that of compound **2-3,** with a yield of 41%. $^1$H NMR (400 MHz, DMSO-$d_6$) $\delta$ 7.78 (s, 1H), 7.66-7.46 (m, 4H), 6.37 (s, 1H), 5.05 (s, 2H), 3.84 (s, 3H), 3.70 (s, 2H). LRMS (ESI) *m/z* $[M+H]^+$ 304.1.

**Step 2: 4-(furan-3-ylmethyl)-3-oxo-3,4-dihydro-2H-benzo[b][1,4]thiazin-6-carboxylic acid (56-2)**

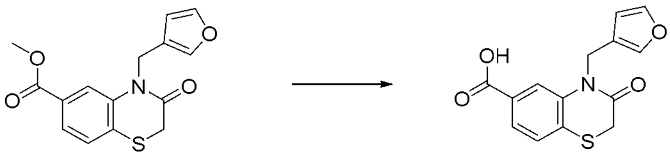

The preparation of 4-(furan-3-ylmethyl)-3-oxo-3,4-dihydro-2H-benzo[b][1,4]thiazin-6-carboxylic acid **(56-2)** was the same as that of compound **1-5,** with a yield of 76%. $^1$H NMR (400 MHz, DMSO-$d_6$) $\delta$ 13.12 (s, 1H), 7.77 (s, 1H), 7.64-7.60 (m, 1H), 7.60-7.50 (m, 3H), 6.37 (s, 1H), 5.04 (s, 2H), 3.69 (s, 2H). LRMS (ESI) *m/z* $[M-H]^-$ 288.0.

**Step 3: 4-(furan-3-ylmethyl)-N-(1H-indol-3-yl)-3-oxo-3,4-dihydro-2H-benzo[b][1,4]thiazin-6-carboxamide (I-56)**

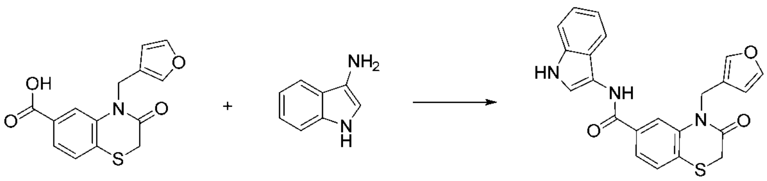

The preparation of 4-(furan-3-ylmethyl)-N-(1H-indol-3-yl)-3-oxo-3,4-dihydro-2H-benzo[b][1,4]thiazin-6-carboxamide **(I-56)** was the same as that of compound **I-1**, with a yield of :83%. $^1$H NMR (400 MHz, DMSO-$d_6$) $\delta$ 10.95 (s, 1H), 10.21 (s, 1H), 7.88-7.74 (m, 3H), 7.71 (d, *J* = 8.0 Hz, 1H), 7.63-7.53 (m, 3H), 7.37 (d, *J*= 8.2 Hz, 1H), 7.12 (t, *J*= 7.5 Hz, 1H), 7.03 (t, *J*= 7.5 Hz, 1H), 6.40 (s, 1H), 5.13 (s, 2H), 3.68 (s, 2H).

**Example 57 4-(benzofuran-5-ylmethyl)-N-(1H-indol-3-yl)-3-oxo-3,4-dihydro-2H-benzo[b][1,4]triazin-6-carboxamide (I-57)**

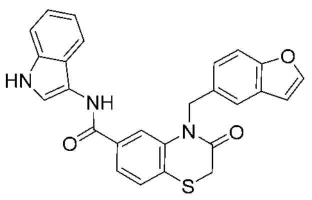

**Step 1: methyl 4-(benzofuran-5-ylmethyl)-3-oxo-3,4-dihydro-2H-benzo[b][1,4]thiazin-6-carboxylate (57-1)**

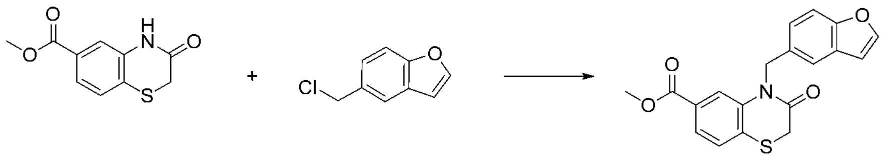

The preparation of methyl 4-(benzofuran-5-ylmethyl)-3-oxo-3,4-dihydro-2H-benzo[b][1,4]thiazin-6-carboxylate **(57-1)** was the same as that of compound **2-3,** with a yield of 57%. $^1H$ NMR (400 MHz, DMSO-$d_6$) $\delta$ 7.99-7.92 (m, 1H), 7.75-7.63 (m, 1H), 7.61-7.44 (m, 4H), 7.20 (d, *J= 8.9* Hz, 1H), 6.97-6.86 (m, 1H), 5.34 (s, 2H), 3.79 (s, 2H), 3.76 (s, 3H). LRMS (ESI) *m/z* $[M+H]^+$ 354.2.

**Step 2: 4-(benzofuran-5-ylmethyl)-3-oxo-3,4-dihydro-2H-benzo[b][1,4]thiazin-6-carboxylic acid (57-2)**

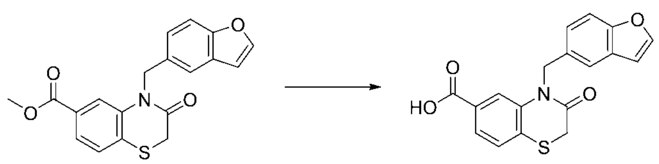

The preparation of 4-(benzofuran-5-ylmethyl)-3-oxo-3,4-dihydro-2H-benzo[b][1,4]thiazin-6-carboxylic acid **(57-2)** was the same as that of compound **1-5,** with a yield of 54%. $^1H$ NMR (400 MHz, DMSO-$d_6$) $\delta$ 12.62 (s, 1H), 8.25-7.82 (m, 1H), 7.67 (s, 1H), 7.60-7.51 (m, 3H), 7.48 (s, 1H), 7.20 (d, *J*= 8.5 Hz, 1H), 6.91 (s, 1H), 5.34 (s, 2H), 3.79 (s, 2H). LRMS (ESI) *m/z* 338.1.

**Step 3: 4-(benzofuran-5-ylmethyl)-N-(1H-indol-3-yl)-3-oxo-3,4-dihydro-2H-benzo[b][1,4]thiazin-6-carboxamide (I-57)**

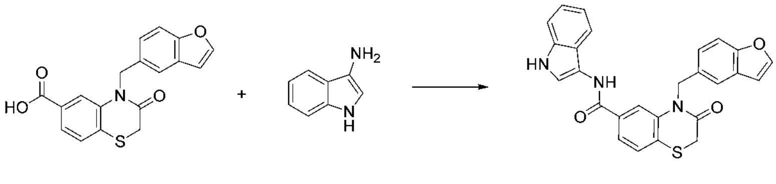

The preparation of 4-(benzofuran-5-ylmethyl)-N-(1H-indol-3-yl)-3-oxo-3,4-dihydro-2H-benzo[b][1,4]thiazin-6-carboxamide **(I-57)** was the same as that of compound **I-1,** with a yield of 40%. $^1H$ NMR (400 MHz, DMSO-$d_6$) $\delta$ 10.92 (s, 1H), 10.14 (s, 1H), 7.99-7.95 (m, 1H), 7.79-7.71 (m, 3H), 7.68 (d, *J*= 8.1 Hz, 1H), 7.63-7.53 (m, 3H), 7.35 (d, *J*= 8.1 Hz, 1H), 7.25 (d, *J* = 8.7 Hz, 1H), 7.11 (t, *J* = 7.6 Hz, 1H), 7.02 (t, *J* = 7.4 Hz, 1H), 6.91-6.88 (m, 1H), 5.43 (s, 2H), 3.79 (s, 2H).

**Example 58 4-(benzo[d][1,3]dioxolan-5-ylmethyl)-N-(1H-indol-3-yl)-3-oxo-3,4-dihydro-2H-benzo[b][1,4]thiazin-6-carboxamide (I-58)**

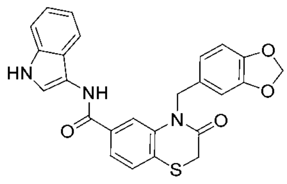

**Step 1: methyl 4-(benzo[d][1,3]dioxolan-5-ylmethyl)-3-oxo-3,4-dihydro-2H-benzo[b][1,4]thiazin-6-carboxylate (58-1)**

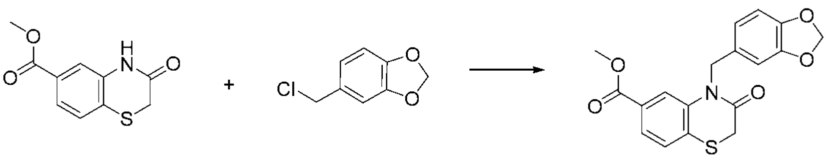

The preparation of methyl 4-(benzo[d][1,3]dioxolan-5-ylmethyl)-3-oxo-3,4-dihydro-2H-benzo[b][1,4]thiazin-6-carboxylate **(58-1)** was the same as that of compound **2-3,** with a yield of 97%. $^1$H NMR (400 MHz, DMSO-$d_6$) $\delta$ 7.67 (s, 1H), 7.56 (s, 2H), 6.89-6.83 (m, 1H), 6.77-6.75 (m, 1H), 6.70 (d, $J$ = 7.9 Hz, 1H), 5.97 (s, 2H), 5.16 (s, 2H), 3.80 (s, 3H), 3.75 (s, 2H). LRMS (ESI) *m/z* 358.1.

**Step 3: 4-(benzo[d][1,3]dioxolan-5-ylmethyl)-3-oxo-3,4-dihydro-2H-benzo[b][1,4]thiazin-6-carboxylic acid (58-2)**

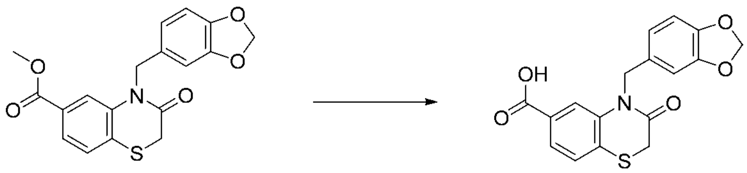

The preparation of 4-(benzo[d][1,3]dioxolan-5-ylmethyl)-3-oxo-3,4-dihydro-2H-benzo[b][1,4]thiazin-6-carboxylic acid **(58-2)** was the same as that of compound **1-5,** with a yield of 94%. $^1$H NMR (400 MHz, DMSO-$d_6$) $\delta$ 7.65 (s, 1H), 7.54 (q, $J$ = 7.9 Hz, 2H), 6.85 (d, $J$= 7.8 Hz, 1H), 6.76 (s, 1H), 6.69 (d, $J$ = 8.0 Hz, 1H), 5.98 (s, 2H), 5.14 (s, 2H), 3.74 (s, 2H). LRMS (ESI) *m/z* [M-H]$^-$ 342.0.

**Step 3: 4-(benzo[d][1,3]dioxolan-5-ylmethyl)-N-(1H-indol-3-yl)-3-oxo-3,4-dihydro-2H-benzo[b][1,4]thiazin-6-carboxamide (I-58)**

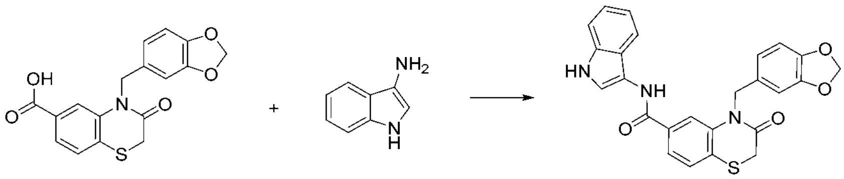

The preparation of 4-(benzo[d][1,3]dioxolan-5-ylmethyl)-N-(1H-indol-3-yl)-3-oxo-3,4-dihydro-2H-benzo[b][1,4]thiazin-6-carboxamide **(I-58)** was the same as that of compound **I-1,** with a yield of 55%. $^1$H NMR (400 MHz, DMSO-$d_6$) $\delta$ 10.94 (s, 1H), 10.16 (s, 1H), 7.80-7.74 (m, 3H), 7.70 (d, *J* = 8.1 Hz, 1H), 7.58 (d, *J* = 8.0 Hz, 1H), 7.36 *(d, J*= 8.2 Hz, 1H), 7.12 (t, *J* = 7.7 Hz, 1H), 7.02 (t, *J* = 7.4 Hz, 1H), 6.87-6.80 (m, 2H), 6.76 (d, *J* = 8.2 Hz, 1H), 5.97 (s, 2H), 5.25 (s, 2H), 3.75 (s, 2H).

**Example 59 N-(1H-indol-3-yl)-3-oxo-4-(thien-2-ylmethyl)-3,4-dihydro-2H-benzo[b][1,4]thiazin-6-carboxamide (I-59)**

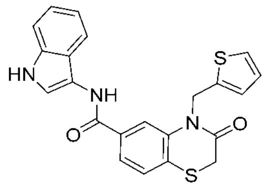

**Step 1: methyl 3-oxo-4-(thien-2-ylmethyl)-3,4-dihydro-2H-benzo[b][1,4]thiazin-6-carboxylate (59-1)**

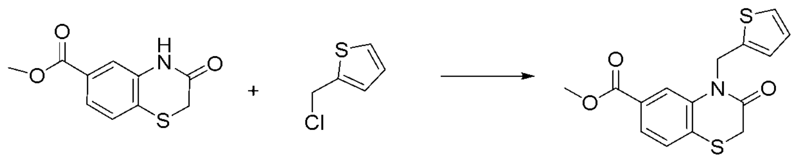

The preparation of methyl 3-oxo-4-(thien-2-ylmethyl)-3,4-dihydro-2H-benzo[b][1,4]thiazin-6-carboxylate **(59-1)** was the same as that of compound **2-3,** with a yield of 98%. $^1$H NMR (400 MHz, DMSO-$d_6$) $\delta$ 7.91 (s, 1H), 7.71-7.48 (m, 2H), 7.41 (d, *J*= 5.0 Hz, 1H), 7.12-6.99 (m, 1H), 6.95 (t, *J* = 4.4 Hz, 1H), 5.40 (s, 2H), 3.84 (s, 3H), 3.69 (s, 2H). LRMS (ESI) *m/z* $[M+H]^+$ 320.2.

**Step 2: 3-oxo-4-(thien-2-ylmethyl)-3,4-dihydro-2H-benzo[b][1,4]thiazin-6-carboxylic acid (59-2)**

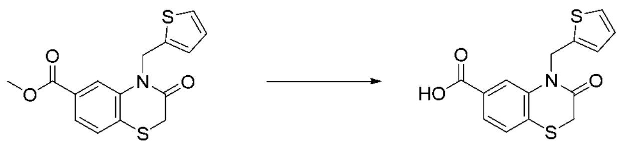

The preparation of 3-oxo-4-(thien-2-ylmethyl)-3,4-dihydro-2H-benzo[b][1,4]thiazin-6-carboxylic acid **(59-2)** was the same as that of compound **1-5,** with a yield of 73%. LRMS (ESI) *m/z* $[M-H]^-$ 304.1.

Step 3: **N-(1H-indol-3-yl)-3-oxo-4-(thien-2-ylmethyl)-3,4-dihydro-2H-benzo[b][1,4]thiazin-6-carboxamide (I-59)**

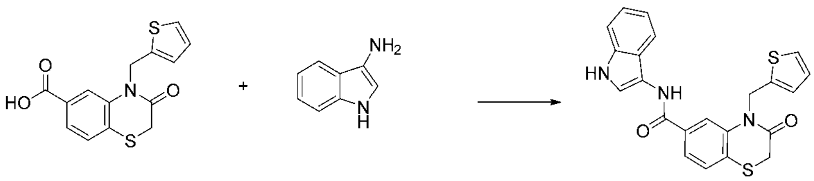

The preparation of N-(1H-indol-3-yl)-3-oxo-4-(thien-2-ylmethyl)-3,4-dihydro-2H-benzo[b][1,4]thiazin-6-carboxamide **(I-59)** was the same as that of compound **I-1**, with a yield of 44%. $^1$H NMR (600 MHz, DMSO-$d_6$) $\delta$ 10.94 (s, 1H), 10.19 (s, 1H), 7.99 (d, *J* = 1.7 Hz, 1H), 7.82 (d, *J* = 8.0 Hz, 1H), 7.79 (d, *J* = 2.5 Hz, 1H), 7.70 (dd, *J* = 8.0, 1.6 Hz, 1H), 7.57 (d,

*J* = 8.0 Hz, 1H), 7.41 (d, *J* = 5.1, 1.2 Hz, 1H), 7.37 (d, *J* = 8.1 Hz, 1H), 7.15-7.10 (m, 2H), 7.03 (t, *J* = 7.4 Hz, 1H), 6.95 (t, *J* = 5.1, 3.5 Hz, 1H), 5.49 (s, 2H), 3.69 (s, 2H).

**Example 60 4-(furan-2-ylmethyl)-N-(1H-indol-3-yl)-3-oxo-3,4-dihydro-2H-benzo[b][1,4]thiazin-6-carboxamide (I-60)**

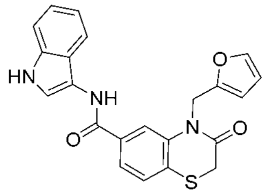

**Step 1: methyl 4-(furan-2-ylmethyl)-3-oxo-3,4-dihydro-2H-benzo[b][1,4]thiazin-6-carboxylate (60-1)**

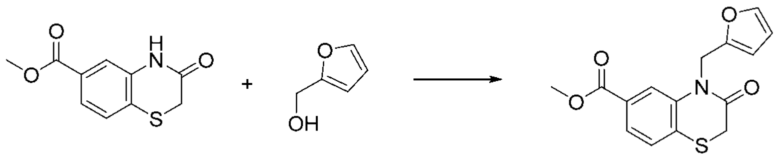

The preparation of methyl 4-(furan-2-ylmethyl)-3-oxo-3,4-dihydro-2H-benzo[b][1,4]thiazin-6-carboxylate **(60-1)** was the same as that of compound **9-2,** with a yield of 66%. $^1$H NMR (400 MHz, DMSO-$d_6$) $\delta$ 7.92 (d, *J* = 1.6 Hz, 1H), 7.64-7.53 (m, 3H), 6.43-6.35 (m, 1H), 6.29 (d, *J* = 2.7 Hz, 1H), 5.21 (s, 2H), 3.85 (s, 3H), 3.67 (s, 2H). LRMS (ESI) *m/z* $[M+H]^+$ 304.0.

**Step 2: 4-(furan-2-ylmethyl)-3-oxo-3,4-dihydro-2H-benzo[b] [1,4]thiazin-6-carboxylic acid (60-2)**

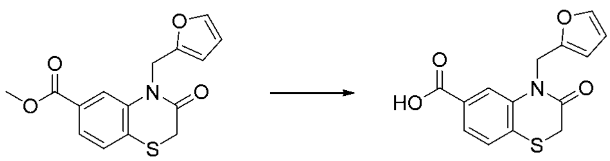

The preparation of 4-(furan-2-ylmethyl)-3-oxo-3,4-dihydro-2H-benzo[b][1,4]thiazin-6-carboxylic acid **(60-2)** was the same as that of compound **1-5,** with a yield of 79%. LRMS (ESI) *m/z* $[M-H]^-$ 288.0.

**Step 3: 4-(furan-2-ylmethyl)-N-(1H-indol-3-yl)-3-oxo-3,4-dihydro-2H-benzo[b][1,4]thiazin-6-carboxamide (I-60)**

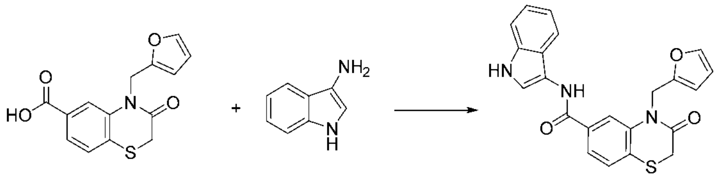

The preparation of 4-(furan-2-ylmethyl)-N-(1H-indol-3-yl)-3-oxo-3,4-dihydro-2H-benzo[b][1,4]thiazin-6-carboxamide (**I-60**) was the same as that of compound **I-1**, with a yield of 30%. $^1$H NMR (400 MHz, DMSO-$d_6$) $\delta$ 10.94 (s, 1H), 10.20 (s, 1H), 7.99 (s, 1H), 7.83-7.76 (m, 2H), 7.75-7.69 (m, 1H), 7.63-7.53 (m, 2H), 7.37 (d, *J* = 8.2 Hz, 1H), 7.12 (t, *J* = 7.6

Hz, 1H), 7.02 (t, *J* = 7.5 Hz, 1H), 6.43-6.37 (m, 1H), 6.35-6.31 (m, 1H), 5.29 (s, 2H), 3.66 (s, 2H).

**Example 61 N-(1H-indol-3-yl)-4-((1-methyl-1H-pyrazol-3-yl)methyl)-3-oxo-3,4-dihydro-2H-benzo[b] [1,4]thiazin-6-carboxamide (I-61)**

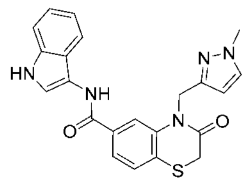

**Step 1: methyl 4-((1-methyl-1H-pyrazol-3-yl)methyl)-3-oxo-3,4-dihydro-2H-benzo[b][1,4]thiazin-6-carboxylate (61-1)**

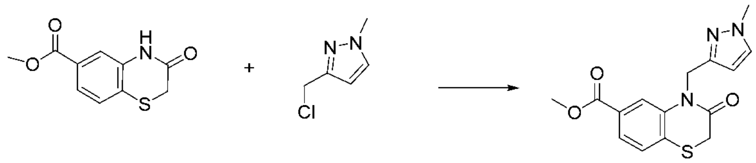

The preparation of methyl 4-((1-methyl-1H-pyrazol-3-yl)methyl)-3-oxo-3,4-dihydro-2H-benzo[b][1,4]thiazin-6-carboxylate **(61-1)** was the same as that of compound **2-3,** with a yield of 78%. $^1$H NMR (400 MHz, DMSO-$d_6$) $\delta$ 8.00 (d, *J* = 1.6 Hz, 1H), 7.79-7.37 (m, 3H), 6.04 (d, *J* = 2.2 Hz, 1H), 5.07 (s, 2H), 3.83 (s, 3H), 3.78 (s, 3H), 3.65 (s, 2H). LRMS (ESI) *m/z* [M+H]$^+$ 318.1.

**Step 2: 4-((1-methyl-1H-pyrazol-3-yl)methyl)-3-oxo-3,4-dihydro-2H-benzo[b][1,4]thiazin-6-carboxylic acid (61-2)**

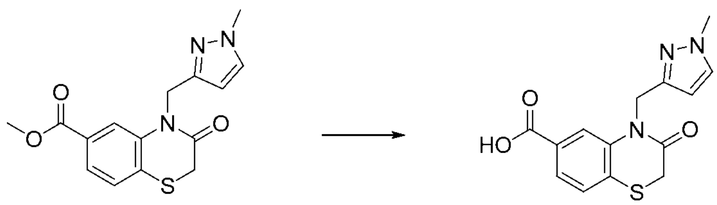

The preparation of 4-((1-methyl-1H-pyrazol-3-yl)methyl)-3-oxo-3,4-dihydro-2H-benzo[b][1,4]thiazin-6-carboxylic acid **(61-2)** was the same as that of compound **1-5,** with a yield of 70%. LRMS (ESI) *m/z* [M+H]$^+$ 304.1.

**Step 3: N-(1H-indol-3-yl)-4-((1-methyl-1H-pyrazol-3-yl)methyl)-3-oxo-3,4-dihydro-2H-benzo[b] [1,4]thiazin-6-carboxamide (I-61)**

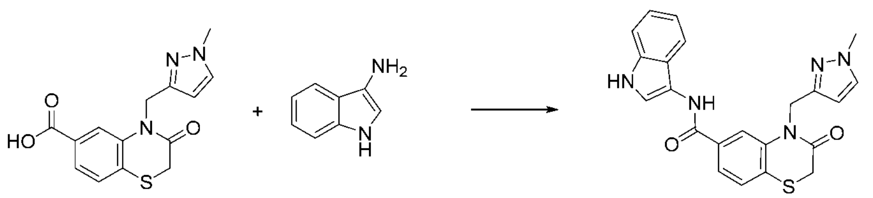

The preparation of N-(1H-indol-3-yl)-4-((1-methyl-1H-pyrazol-3-yl)methyl)-3-oxo-3,4-dihydro-2H-benzo[b][1,4]thiazin-6-carboxamide **(I-61)** was the same as that of compound **I-1,** with a yield of : 63%. $^1$H NMR (400 MHz, DMSO-$d_6$) $\delta$ 10.94 (s, 1H), 10.17 (s, 1H), 7.98 (s, 1H), 7.83 (d, *J*= 8.0 Hz, 1H), 7.79-7.74 (m, 1H), 7.66 (d, *J*= 8.1 Hz, 1H), 7.60-7.52 (m, 2H), 7.36 (d, *J* = 8.1 Hz, 1H), 7.12 (t, *J* = 7.5 Hz, 1H), 7.02 (t, *J* = 7.5 Hz, 1H), 6.10-5.99 (m, 1H), 5.19 (s, 2H), 3.75 (s, 3H), 3.65 (s, 2H).

**Example 62 4-(cyclopropylmethyl)-N-(1H-indol-3-yl)-3-oxo-3,4-dihydro-2H-benzo[b][1,4]thiazin-6-carboxamide (I-62)**

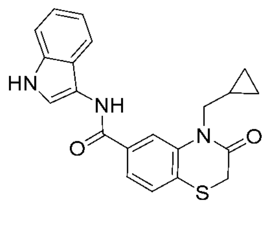

**Step 1: methyl 4-cyclopropylmethyl-3-oxo-3,4-dihydro-2H-benzo[b][1,4]thiazin-6-carboxylate (62-1)**

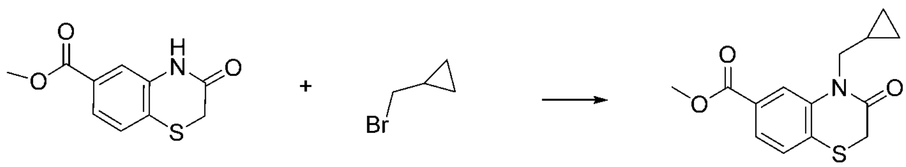

The preparation of methyl 4-cyclopropylmethyl-3-oxo-3,4-dihydro-2H-benzo[b][1,4]thiazin-6-carboxylate **(62-1)** was the same as that of compound **2-3,** with a yield of 94%. $^1$H NMR (400 MHz, DMSO-$d_6$) $\delta$ 7.88 (s, 1H), 7.65-7.44 (m, 2H), 3.94 *(d, J=* 6.7 Hz, 2H), 3.87 (s, 3H), 3.59 (s, 2H), 1.38-0.77 (m, 1H), 0.49-0.39 (m, 2H), 0.35-0.22 (m, 2H). LRMS (ESI) *m/z* [M+H]$^+$ 278.0.

**Step 2: : 4-cyclopropylmethyl-3-oxo-3,4-dihydro-2H-benzo[b][1,4]thiazin-6-carboxylic acid (62-2)**

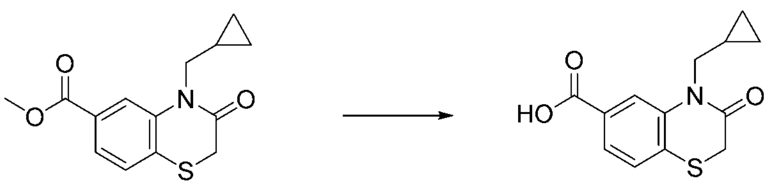

The preparation of 4-cyclopropylmethyl-3-oxo-3,4-dihydro-2H-benzo[b][1,4]thiazin-6-carboxylic acid **(62-2)** was the same as that of compound **1-5,** with a yield of 96%. LRMS (ESI) *m/z* [M+H]$^+$ 264.1.

**Step 3: 4-cyclopropylmethyl-N-(1H-indol-3-yl)-3-oxo-3,4-dihydro-2H-benzo[b][1,4]thiazin-6-carboxamide (I-62)**

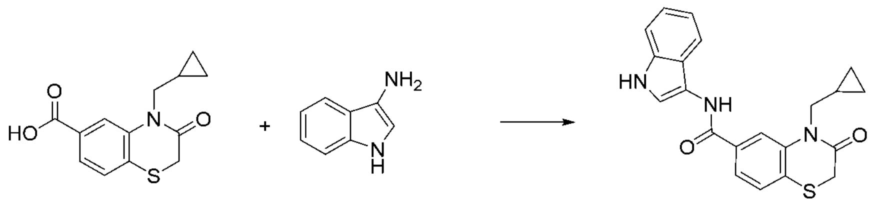

The preparation of 4-(cyclopropylmethyl)-N-(1H-indol-3-yl)-3-oxo-3,4-dihydro-2H-benzo[b][1,4]thiazin-6-carboxamide **(I-62)** was the same as that of compound **I-1,** with a yield of 42%. $^1$H NMR (600 MHz, DMSO-$d_6$) $\delta$ 10.94 (s, 1H), 10.22 (s, 1H), 7.96 (s, 1H), 7.83 (d, $J$ = 8.0 Hz, 1H), 7.80-7.78 (m, 1H), 7.73 (d, $J$= 8.0 Hz, 1H), 7.58 (d, $J$ = 8.0 Hz, 1H), 7.37 (d, $J$= 8.2 Hz, 1H), 7.12 (t, $J$ = 7.5 Hz, 1H), 7.02 (t, $J$ = 7.5 Hz, 1H), 4.02 (d, $J$ = 6.8 Hz, 2H), 3.58 (s, 2H), 1.13-1.03 (m, 1H), 0.53-0.44 (m, 2H), 0.37-0.31 (m, 2H).

**Example 63 N-(1H-indol-3-yl)-3-oxo-4-(thien-3-ylmethyl)-3,4-dihydro-2H-benzo[b][1,4]thiazin-7-carboxamide (I-63)**

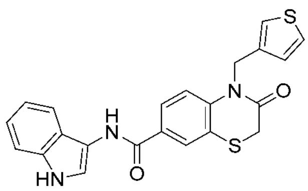

**Step 1: methyl 3-oxo-4-(thien-3-ylmethyl)-3,4-dihydro-2H-benzo[b][1,4]thiazin-7-carboxylate (63-1)**

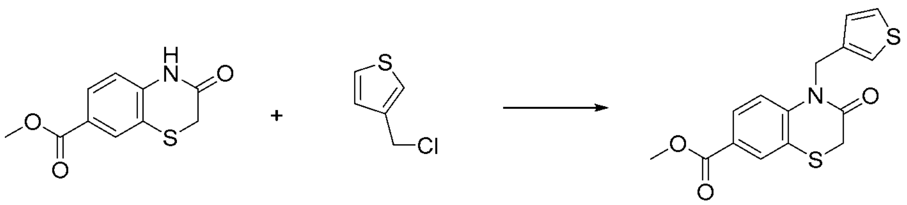

The preparation of methyl 3-oxo-4-(thien-3-ylmethyl)-3,4-dihydro-2H-benzo[b][1,4]thiazin-7-carboxylate **(63-1)** was the same as that of compound **2-3,** with a yield of 96%. $^1$H NMR (400 MHz, DMSO-$d_6$) $\delta$ 7.93 (d, $J$ = 2.0 Hz, 1H), 7.77 (dd, $J$ = 8.6, 2.1 Hz, 1H), 7.52-7.44 (m, 1H), 7.35 (d, $J$ = 8.7 Hz, 1H), 7.31-7.28 (m, 1H), 6.98 (dd, $J$= 5.0, 1.3 Hz, 1H), 5.21 (s, 2H), 3.82 (s, 3H), 3.71 (s, 2H). LRMS (ESI) $m/z$ [M+H]$^+$ 320.0.

**Step 2: 3-oxo-4-(thien-3-ylmethyl)-3,4-dihydro-2H-benzo[b][1,4]thiazin-7-carboxylic acid (63-2)**

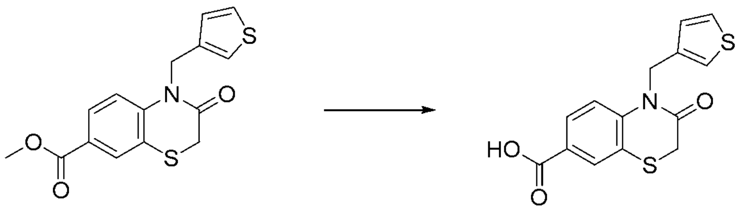

The preparation of 3-oxo-4-(thien-3-ylmethyl)-3,4-dihydro-2H-benzo[b][1,4]thiazin-7-carboxylic acid **(63-2)** was the same as that of compound **1-5,** with a yield of 94%. $^1$H NMR (400 MHz, DMSO-$d_6$) $\delta$ 7.90 (s, 1H), 7.74 (dd, $J$= 8.5, 2.0 Hz, 1H), 7.55-7.44 (m, 1H), 7.41-7.14 (m, 2H), 6.98 (d, $J$ = 5.0 Hz, 1H), 5.21 (s, 2H), 3.70 (s, 2H). LRMS (ESI) $m/z$ [M+H]$^+$ 306.0.

**Step 3: N-(1H-indol-3-yl)-3-oxo-4-(thien-3-ylmethyl)-3,4-dihydro-2H-benzo[b][1,4]thiazin-7-carboxamide** (**I-63**)

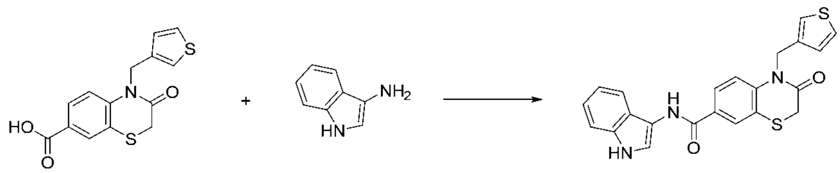

The preparation of N-(1H-indol-3-yl)-3-oxo-4-(thien-3-ylmethyl)-3,4-dihydro-2H-benzo[b][1,4]thiazin-7-carboxamide **(I-63)** was the same as that of compound **I-1**, with a yield of 33%. $^1$H NMR (400 MHz, DMSO-$d_6$) $\delta$ 10.93 (s, 1H), 10.13 (s, 1H), 8.08 (s, 1H), 7.92-7.81 (m, 2H), 7.78 (s, 1H), 7.58-7.48 (m, 1H), 7.44-7.30 (m, 3H), 7.11 (t, $J$ = 7.3 Hz, 1H), 7.05-6.95 (m, 2H), 5.26 (s, 2H), 3.73 (s, 2H).

**Example 64 4-(furan-3-ylmethyl)-N-(1H-indol-3-yl)-3-oxo-3,4-dihydro-2H-benzo[b][1,4]thiazin-7-carboxamide (I-64)**

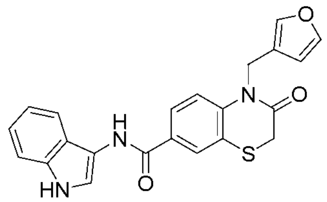

**Step 1: methyl 4-(furan-3-ylmethyl)-3-oxo-3,4-dihydro-2H-benzo[b][1,4]thiazin-7-carboxylate (64-1)**

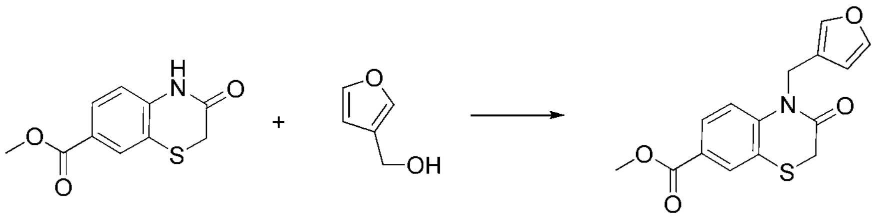

The preparation of methyl 4-(furan-3-ylmethyl)-3-oxo-3,4-dihydro-2H-benzo[b][1,4]thiazin-7-carboxylate **(64-1)** was the same as that of compound **9-2,** with a yield of 55%. $^1$H NMR (400 MHz, DMSO-$d_6$) $\delta$ 7.94 (d, $J$= 2.1 Hz, 1H), 7.81 (dd, $J$ = 8.7, 2.1 Hz, 1H), 7.64-7.53 (m, 2H), 7.42 (d, $J$= 8.7 Hz, 1H), 6.36 (d, $J$ = 1.9 Hz, 1H), 5.06 (s, 2H), 3.83 (s, 3H), 3.67 (s, 2H).LRMS (ESI) $m/z$ calcd [M+H] $^+$for $C_{15}H_{13}NO_4S$ 304.1, found 304.0.

**Step 2: 4-(furan-3-ylmethyl)-3-oxo-3,4-dihydro-2H-benzo[b] [1,4]thiazin-7-carboxylic acid (64-2)**

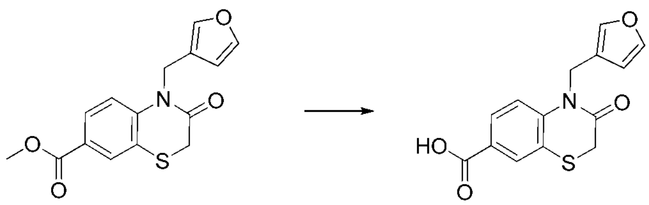

The preparation of 4-(furan-3-ylmethyl)-3-oxo-3,4-dihydro-2H-benzo[b][1,4]thiazin-7-carboxylic acid **(64-2)** was

the same as that of compound **1-5,** with a yield of 92%. $^1$H NMR (400 MHz, DMSO-$d_6$) $\delta$ 12.51 (s, 1H), 7.97-7.87 (m, 1H), 7.78 (dd, $J$= 8.6, 2.0 Hz, 1H), 7.64-7.53 (m, 2H), 7.40 (d, $J$ = 8.6 Hz, 1H), 6.36 (d, $J$ = 1.9 Hz, 1H), 5.05 (s, 2H), 3.66 (s, 2H). LRMS (ESI) $m/z$ [M+H]$^+$ 289.9.

**Step 3: 4-(furan-3-ylmethyl)-N-(1H-indol-3-yl)-3-oxo-3,4-dihydro-2H-benzo[b][1,4]thiazin-7-carboxamide** (**I-64**)

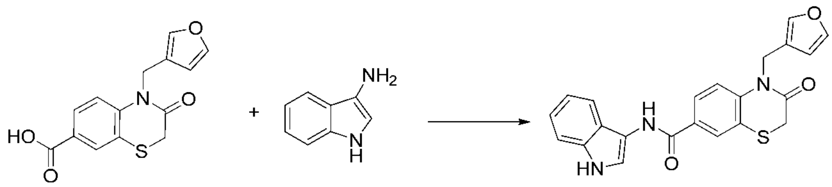

The preparation of 4-(furan-3-ylmethyl)-N-(1H-indol-3-yl)-3-oxo-3,4-dihydro-2H-benzo[b][1,4]thiazin-7-carboxamide **(I-64)** was the same as that of compound **I-1**, with a yield of 36%. $^1$H NMR (600 MHz, DMSO-$d_6$) $\delta$ 10.90 (s, 1H), 10.11 (s, 1H), 8.07 (s, 1H), 7.86 (t, $J$ = 8.3 Hz, 2H), 7.78 (s, 1H), 7.60 (d, $J$ = 17.0 Hz, 2H), 7.45 (d, $J$ = 8.6 Hz, 1H), 7.35 (d, $J$ = 8.2 Hz, 1H), 7.11 (t, $J$ = 7.5 Hz, 1H), 7.00 (t, $J$ = 7.5 Hz, 1H), 6.37 (s, 1H), 5.10 (s, 2H), 3.68 (s, 2H).

**Example 65 4-(benzofuran-5-ylmethyl)-N-(1H-indol-3-yl)-3-oxo-3,4-dihydro-2H-benzo[b][1,4]thiazin-7-carboxamide (I-65)**

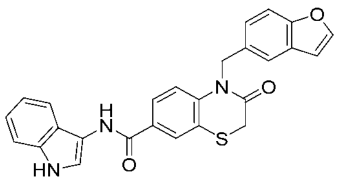

**Step 1: methyl 4-(benzofuran-5-ylmethyl)-3-oxo-3,4-dihydro-2H-benzo[b][1,4]thiazin-7-carboxylate (65-1)**

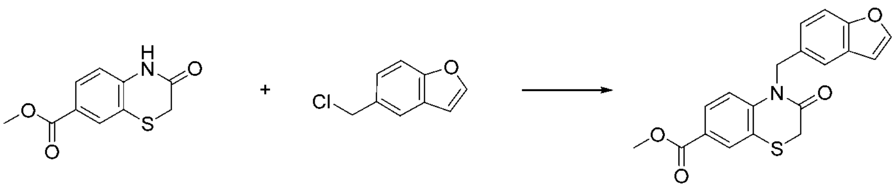

The preparation of methyl 4-(benzofuran-5-ylmethyl)-3-oxo-3,4-dihydro-2H-benzo[b][1,4]thiazin-7-carboxylate **(65-1)** was the same as that of compound 2-3, with a yield of 97%. $^1$H NMR (400 MHz, DMSO-$d_6$) $\delta$ 8.00-7.89 (m, 2H), 7.77-7.68 (m, 1H), 7.53 (d, $J$ = 8.6 Hz, 1H), 7.48 (s, 1H), 7.34 (d, $J$ = 8.6, 2.1 Hz, 1H), 7.17 (d, $J$ = 8.5 Hz, 1H), 6.89 (s, 1H), 3.80 (s, 3H), 5.36 (s, 2H), 3.76 (s, 2H). LRMS (ESI) $m/z$ [M+H]$^+$ 354.1.

**Step 2: 4-(benzofuran-5-ylmethyl)-3-oxo-3,4-dihydro-2H-benzo[b][1,4]thiazin-7-carboxylic acid (65-2)**

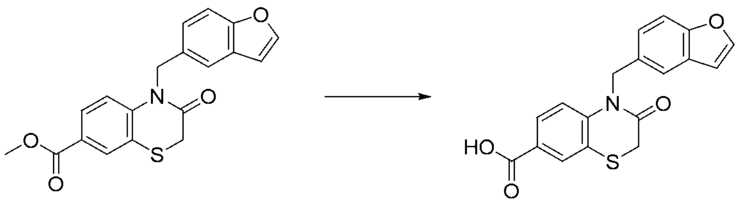

The preparation of 4-(benzofuran-5-ylmethyl)-3-oxo-3,4-dihydro-2H-benzo[b][1,4]thiazin-7-carboxylic acid **(65-2)** was the same as that of compound **1-5,** with a yield of 96%. $^1$H NMR (400 MHz, DMSO-$d_6$) $\delta$ 12.87 (s, 1H), 8.04-7.83 (m, 2H), 7.70 (dd, $J$ = 8.6, 2.0 Hz, 1H), 7.54 (d, $J$ = 8.5 Hz, 1H), 7.51-7.46 (m, 1H), 7.32 (d, $J$ = 8.7 Hz, 1H), 7.18 (dd, $J$ = 8.5, 1.9 Hz, 1H), 6.94-6.85 (m, 1H), 5.36 (s, 2H), 3.76 (s, 2H). LRMS (ESI) $m/z$ [M+H]$^+$ 340.1.

**Step 3: 4-(benzofuran-5-ylmethyl)-N-(1H-indol-3-yl)-3-oxo-3,4-dihydro-2H-benzo[b][1,4]thiazin-7-carboxamide (I-65)**

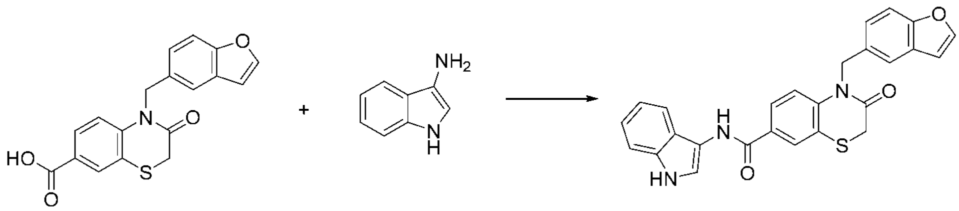

The preparation of 4-(benzofuran-5-ylmethyl)-N-(1H-indol-3-yl)-3-oxo-3,4-dihydro-2H-benzo[b][1,4]thiazin-7-carboxamide **(I-65)** was the same as that of compound **I-1**, with a yield of 30%. $^1$H NMR (600 MHz, DMSO-$d_6$) $\delta$ 10.88 (s, 1H), 10.07 (s, 1H), 8.06 (s, 1H), 8.01-7.93 (m, 1H), 7.83 (d, $J$= 8.0 Hz, 1H), 7.79 (d, $J$= 8.0 Hz, 1H), 7.76-7.74 (m, 1H), 7.55 (d, $J$ = 8.5 Hz, 1H), 7.51 (s, 1H), 7.39 (d, $J$ = 8.6 Hz, 1H), 7.34 (d, $J$ = 8.2 Hz, 1H), 7.21 (d, $J$ = 8.6 Hz, 1H), 7.09 (t, $J$= 7.5 Hz, 1H), 6.98 (t, $J$= 7.5 Hz, 1H), 6.91 (s, 1H), 5.40 (s, 2H), 3.77 (s, 2H).

**Example 66 4-(benzo[d][1,3]dioxolan-5-ylmethyl)-N-(1H-indol-3-yl)-3-oxo-3,4-dihydro-2H-benzo[b][1,4]thiazin-7-carboxamide (I-66)**

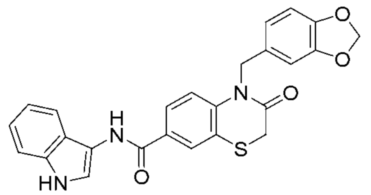

**Step 1: methyl 4-(benzo[d] [1,3]dioxolan-5-ylmethyl)-3-oxo-3,4-dihydro-2H-benzo[b][1,4]thiazin-7-carboxylate (66-1)**

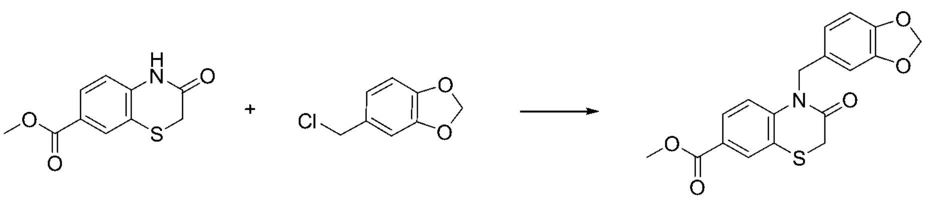

The preparation of methyl 4-(benzo[d][1,3]dioxolan-5-ylmethyl)-3-oxo-3,4-dihydro-2H-benzo[b][1,4]thiazin-7-carboxylate **(66-1)** was the same as that of compound **2-3,** with a yield of 99%. $^1$H NMR (400 MHz, DMSO-$d_6$) $\delta$ 7.94 (d, $J$=

2.0 Hz, 1H), 7.75 (dd, *J* = 8.7, 2.1 Hz, 1H), 7.32 (d, *J*= 8.6 Hz, 1H), 6.84 (d, *J*= 7.9 Hz, 1H), 6.76 (s, 1H), 6.69 (d, *J*= 8.0 Hz, 1H), 5.97 (s, 2H), 5.18 (s, 2H), 3.82 (s, 3H), 3.74 (s, 2H). LRMS (ESI) *m/z* $[M+H]^+$ 358.1.

**Step 2: 4-(benzo[d][1,3]dioxolan-5-ylmethyl)-3-oxo-3,4-dihydro-2H-benzo[b][1,4]thiazin-7-carboxylic acid (66-2)**

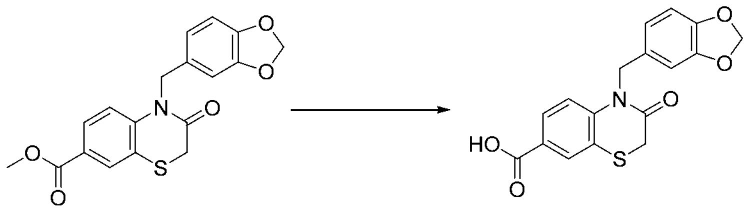

The preparation of 4-(benzo[d][1,3]dioxolan-5-ylmethyl)-3-oxo-3,4-dihydro-2H-benzo[b][1,4]thiazin-7-carboxylic acid **(66-2)** was the same as that of compound **1-5,** with a yield of 92%. $^1$H NMR (400 MHz, DMSO-$d_6$) $\delta$ 12.96 (s, 1H), 7.90 (d, *J* = 2.0 Hz, 1H), 7.72 (dd, *J* = 8.6, 2.0 Hz, 1H), 7.28 (d, *J* = 8.7 Hz, 1H), 6.83 (d, *J* = 8.0 Hz, 1H), 6.75 (d, *J* = 1.7 Hz, 1H), 6.68 (dd, *J* = 8.1, 1.8 Hz, 1H), 5.96 (s, 2H), 5.16 (s, 2H), 3.71 (s, 2H). LRMS (ESI) *m/z* $[M+H]^+$ 344.0.

**Step 3: 4-(benzo[d][1,3]dioxolan-5-ylmethyl)-N-(1H-indol-3-yl)-3-oxo-3,4-dihydro-2H-benzo[b][1,4]thiazin-7-carboxamide (I-66)**

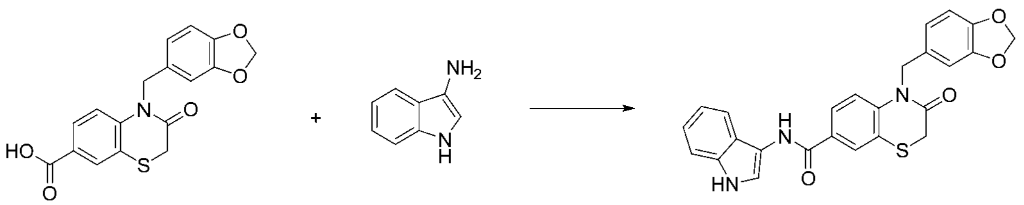

The preparation of 4-(benzo[d][1,3]dioxolan-5-ylmethyl)-N-(1H-indol-3-yl)-3-oxo-3,4-dihydro-2H-benzo[b][1,4]thiazin-7-carboxamide **(I-66)** was the same as that of compound **I-1,** with a yield of 49%. $^1$H NMR (600 MHz, DMSO-$d_6$) $\delta$ 10.89 (s, 1H), 10.10 (s, 1H), 8.11-8.01 (m, 1H), 7.85 (d, *J* = 8.0 Hz, 1H), 7.81 (d, *J* = 9.1 Hz, 1H), 7.78-7.70 (m, 1H), 7.40-7.33 (m, 2H), 7.10 (t, *J*= 7.5 Hz, 1H), 6.99 (t, *J*= 7.5 Hz, 1H), 6.85 (d, *J*= 8.0 Hz, 1H), 6.78 (s, 1H), 6.72 (d, *J*= 8.0 Hz, 1H), 5.97 (s, 2H), 5.22 (s, 2H), 3.74 (s, 2H).

**Example 67 N-(1H-indol-3-yl)-3-oxo-4-(thien-2-ylmethyl)-3,4-dihydro-2H-benzo[b][1,4]thiazin-7-carboxamide (I-67)**

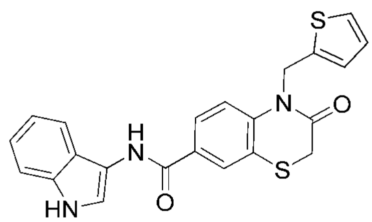

**Step 1: methyl 3-oxo-4-(thien-2-ylmethyl)-3,4-dihydro-2H-benzo[b][1,4]thiazin-7-carboxylate (67-1)**

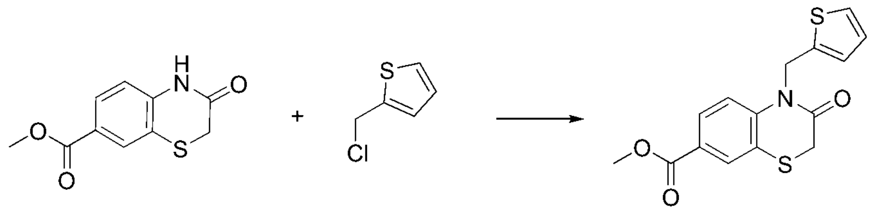

The preparation of methyl 3-oxo-4-(thien-2-ylmethyl)-3,4-dihydro-2H-benzo[b][1,4]thiazin-7-carboxylate **(67-1)** was the same as that of compound **2-3,** with a yield of 92%. $^1$H NMR (400 MHz, DMSO-$d_6$) $\delta$ 7.92 (d, $J$= 2.0 Hz, 1H), 7.80 (dd, $J$ = 8.7, 2.1 Hz, 1H), 7.55 (d, $J$ = 8.7 Hz, 1H), 7.40-7.38 (m, 1H), 7.07 (d, $J$ = 3.3 Hz, 1H), 6.96-6.93 (m, 1H), 5.41 (s, 2H), 3.82 (s, 3H), 3.67 (s, 2H). LRMS (ESI) $m/z$ [M+H]$^+$ 320.0.

**Step 2: 3-oxo-4-(thien-2-ylmethyl)-3,4-dihydro-2H-benzo[b][1,4]thiazin-7-carboxylic acid (67-2)**

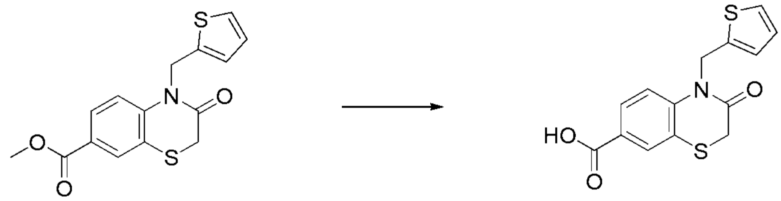

The preparation of 3-oxo-4-(thien-2-ylmethyl)-3,4-dihydro-2H-benzo[b][1,4]thiazin-7-carboxylic acid **(67-2)** was the same as that of compound **1-5,** with a yield of 96%. $^1$H NMR (400 MHz, DMSO-$d_6$) $\delta$ 7.90 (d, $J$ = 2.0 Hz, 1H), 7.78 (dd, $J$= 8.6, 2.1 Hz, 1H), 7.53 (d, $J$ = 8.7 Hz, 1H), 7.40 (d, 1H), 7.12-7.04 (m, 1H), 6.96-6.92 (m, 1H), 5.42 (s, 2H), 3.67 (s, 2H). LRMS (ESI) $m/z$ [M+H]$^+$ 305.8.

**Step 3: N-(1H-indol-3-yl)-3-oxo-4-(thien-2-ylmethyl)-3,4-dihydro-2H-benzo[b][1,4]thiazin-7-carboxamide (I-67)**

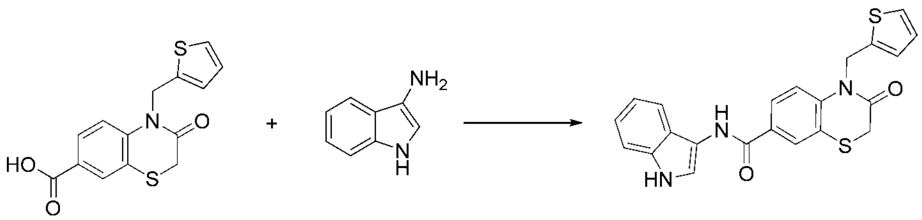

The preparation of N-(1H-indol-3-yl)-3-oxo-4-(thien-2-ylmethyl)-3,4-dihydro-2H-benzo[b][1,4]thiazin-7-carboxamide **(I-67)** was the same as that of compound **I-1,** with a yield of 36%. $^1$H NMR (400 MHz, DMSO-$d_6$) $\delta$ 10.91 (s, 1H), 10.12 (s, 1H), 8.06 (s, 1H), 7.86 (t, $J$ = 7.8 Hz, 2H), 7.79-7.75 (m, 1H), 7.57 (d, $J$ = 8.7 Hz, 1H), 7.40 (d, $J$= 5.1 Hz, 1H), 7.35 (d, $J$ = 8.1 Hz, 1H), 7.14-7.07 (m, 2H), 7.00 (t, $J$ = 7.4 Hz, 1H), 6.94 (t, $J$ = 4.4 Hz, 1H), 5.46 (s, 2H), 3.69 (s, 2H).

**Example 68 4-(furan-2-ylmethyl)-N-(1H-indol-3-yl)-3-oxo-3,4-dihydro-2H-benzo[b][1,4]thiazin-7-carboxamide (I-68)**

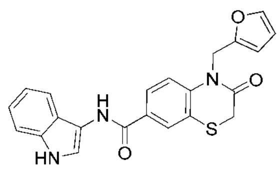

**Step 1: methyl 4-(furan-2-ylmethyl)-3-oxo-3,4-dihydro-2H-benzo[b][1,4]thiazin-7-carboxylate (68-1)**

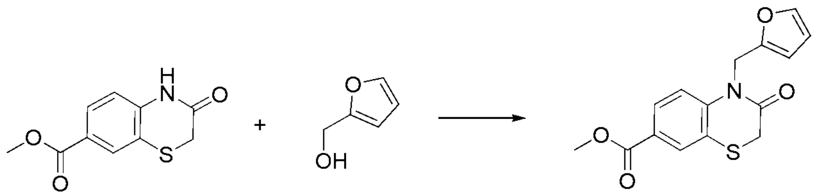

The preparation of methyl 4-(furan-2-ylmethyl)-3-oxo-3,4-dihydro-2H-benzo[b][1,4]thiazin-7-carboxylate **(68-1)** was the same as that of compound **9-2,** with a yield of 67%. $^1$H NMR (400 MHz, DMSO-$d_6$) $\delta$ 7.93 (d, $J$ = 2.0 Hz, 1H), 7.82 (dd, $J$= 8.7, 2.1 Hz, 1H), 7.56 (s, 1H), 7.52 (d, $J$= 8.7 Hz, 1H), 6.38-6.35 (m, 1H), 6.32-6.29 (m, 1H), 5.22 (s, 2H), 3.83 (s, 3H), 3.65 (s, 2H). LRMS (ESI) $m/z$ [M+H]$^+$ 304.1.

**Step 2: 4-(furan-2-ylmethyl)-3-oxo-3,4-dihydro-2H-benzo[b][1,4]thiazin-7-carboxylic acid (68-2)**

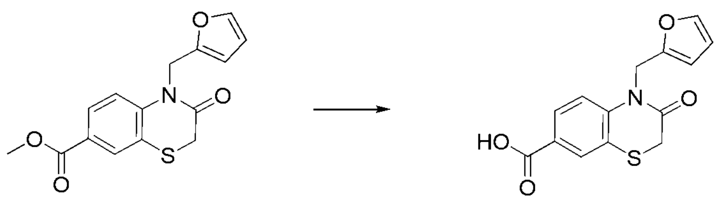

The preparation of 4-(furan-2-ylmethyl)-3-oxo-3,4-dihydro-2H-benzo[b][1,4]thiazin-7-carboxylic acid **(68-2)** was the same as that of compound **1-5,** with a yield of 89%. $^1$H NMR (400 MHz, DMSO-$d_6$) $\delta$ 12.98 (s, 1H), 7.92 (s, 1H), 7.84-7.70 (m, 1H), 7.59 (s, 1H), 7.51 (d, $J$= 8.3 Hz, 1H), 6.39 (s, 1H), 6.32 (s, 1H), 5.23 (s, 2H), 3.65 (s, 2H). LRMS (ESI) $m/z$ [M+H]$^+$ 290.1.

**Step 3: 4-(furan-2-ylmethyl)-N-(1H-indol-3-yl)-3-oxo-3,4-dihydro-2H-benzo[b][1,4]thiazin-7-carboxamide (I-68)**

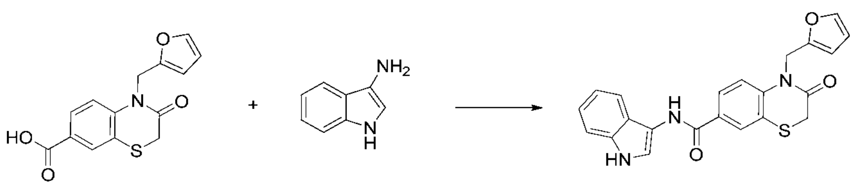

The preparation of 4-(furan-2-ylmethyl)-N-(1H-indol-3-yl)-3-oxo-3,4-dihydro-2H-benzo[b][1,4]thiazin-7-carboxamide **(I-68)** was the same as that of compound **I-1,** with a yield of 21%. $^1$H NMR (400 MHz, DMSO-$d_6$) $\delta$ 10.91 (s, 1H), 10.13 (s, 1H), 8.07 (d, $J$ = 2.1 Hz, 1H), 7.93-7.83 (m, 2H), 7.78 (d, $J$ = 2.5 Hz, 1H), 7.60-7.47 (m, 2H), 7.36 (d, $J$ = 8.1 Hz, 1H), 7.11 (t, $J$ = 7.5 Hz, 1H), 7.00 (t, $J$= 7.5 Hz, 1H), 6.38 (t, $J$= 3.3, 1.9 Hz, 1H), 6.33 (d, $J$ = 3.2 Hz, 1H), 5.27 (s, 2H), 3.67 (s, 2H).

**Example 69 4-(cyclopropylmethyl)-N-(1H-indol-3-yl)-3-oxo-3,4-dihydro-2H-benzo[b][1,4]thiazin-7-carboxamide (I-69)**

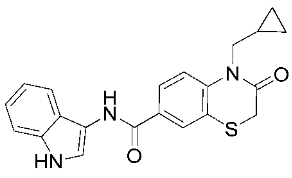

**Step 1: methyl 4-cyclopropylmethyl-3-oxo-3,4-dihydro-2H-benzo[b][1,4]thiazin-7-carboxylate (69-1)**

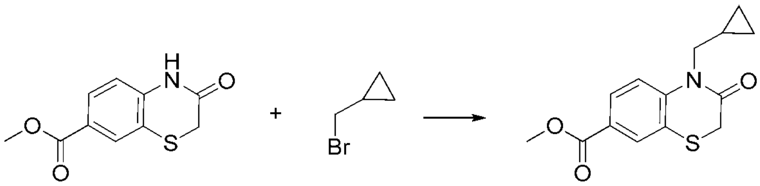

The preparation of methyl 4-cyclopropylmethyl-3-oxo-3,4-dihydro-2H-benzo[b][1,4]thiazin-7-carboxylate **(69-1)** was the same as that of compound **2-3,** with a yield of 55%. $^1$H NMR (400 MHz, DMSO-*d*$_6$) $\delta$ 7.95 (d, *J* = 2.1 Hz, 1H), 7.85 (dd, *J*= 8.6, 2.1 Hz, 1H), 7.56 (d, *J*= 8.7 Hz, 1H), 3.96 (d, *J*= *6.8* Hz, 2H), 3.85 (s, 3H), 3.57 (s, 2H), 1.10-0.98 (m, 1H), 0.53-0.37 (m, 2H), 0.34-0.24 (m, 2H). LRMS (ESI) *m/z* [M+H]$^+$ 278.0.

**Step 2: 4-cyclopropylmethyl-3-oxo-3,4-dihydro-2H-benzo[b][1,4]thiazin-7-carboxylic acid (69-2)**

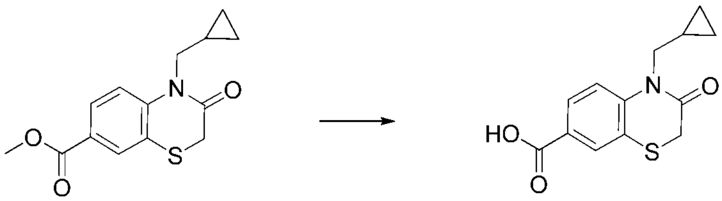

The preparation of 4-cyclopropylmethyl-3-oxo-3,4-dihydro-2H-benzo[b][1,4]thiazin-7-carboxylic acid **(69-2)** was the same as that of compound **1-5,** with a yield of 98%. $^1$H NMR (400 MHz, DMSO-*d*$_6$) $\delta$ 12.98 (s, 1H), 7.92 (d, *J* = 2.0 Hz, 1H), 7.83 (dd, *J* = 8.6, 2.1 Hz, 1H), 7.52 (d, *J* = 8.7 Hz, 1H), 3.95 (d, *J* = 6.8 Hz, 2H), 3.56 (s, 2H), 1.08-0.96 (m, 1H), 0.45-0.39 (m, 2H), 0.35-0.18 (m, 2H). LRMS (ESI) *m/z* [M+H]$^+$ 264.0.

**Step 3: 4-(cyclopropylmethyl)-N-(1H-indol-3-yl)-3-oxo-3,4-dihydro-2H-benzo[b][1,4]thiazin-7-carboxamide (I-69)**

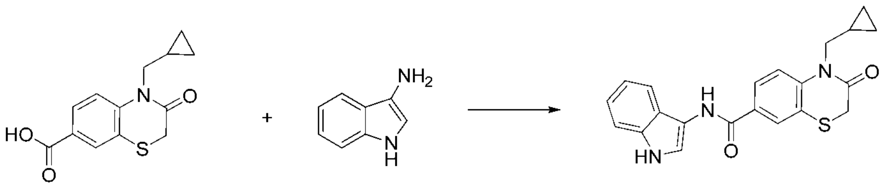

The preparation of 4-(cyclopropylmethyl)-N-(1H-indol-3-yl)-3-oxo-3,4-dihydro-2H-benzo[b][1,4]thiazin-7-carboxamide **(I-69)** was the same as that of compound **I-1**, with a yield of 46%. $^1$H NMR (400 MHz, DMSO-*d*$_6$) $\delta$ 10.92 (s, 1H), 10.14 (s, 1H), 8.10 (s, 1H), 7.93 (d, *J* = 8.6 Hz, 1H), 7.87 (d, *J*= *7.9* Hz, 1H), 7.79 (s, 1H), 7.57 (d, *J*= 8.7 Hz, 1H), 7.36 (d, *J* = 8.2 Hz, 1H), 7.11 (t, *J* = 7.6 Hz, 1H), 7.01 (t, *J* = 7.6 Hz, 1H), 3.99 (d, *J* = 6.8 Hz, 2H), 3.58 (s, 2H), 1.14-0.94 (m, 1H), 0.43 (d, *J* = 7.8 Hz, 2H), 0.32 (d, *J*= 5.0 Hz, 2H).

**Example 70 4-benzyl-N-(1H-indol-3-yl)-3-oxo-3,4-dihydro-2H-benzo[b][1,4]thiazin-6-carboxamide (I-70)**

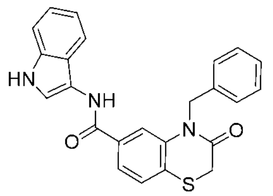

**Step 1: methyl 4-benzyl-3-oxo-3,4-dihydro-2H-benzo[b] [1,4]thiazin-6-carboxylate (70-1)**

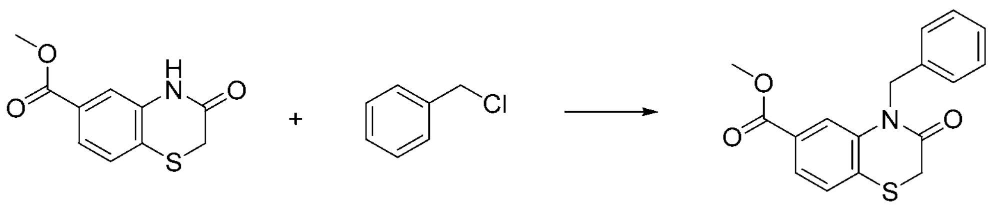

The preparation of methyl 4-benzyl-3-oxo-3,4-dihydro-2H-benzo[b][1,4]thiazin-6-carboxylate (**70-1**) was the same as that of compound **2-3,** with a yield of 58%. $^1$H NMR (400 MHz, DMSO-$d_6$) $\delta$ 7.63 (s, 1H), 7.56 (s, 2H), 7.38-7.29 (m, 2H), 7.27-7.16 (m, 3H), 5.25 (s, 2H), 3.77 (s, 3H), 3.76 (s, 2H). LRMS (ESI) *m/z* $[M+H]^+$ 314.1.

**Step 2: 4-benzyl-3-oxo-3,4-dihydro-2H-benzo[b][1,4]thiazin-6-carboxylic acid (70-2)**

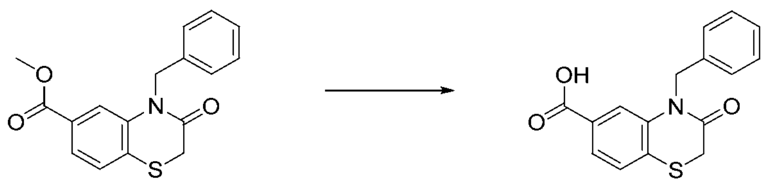

The preparation of 4-benzyl-3-oxo-3,4-dihydro-2H-benzo[b][1,4]thiazin-6-carboxylic acid **(70-2)** was the same as that of compound **1-5,** with a yield of 92%. $^1$H NMR (400 MHz, DMSO-$d_6$) $\delta$ 12.97 (s, 1H), 7.63-7.59 (m, 1H), 7.56-7.55 (m, 2H), 7.37-7.30 (m, 2H), 7.28-7.16 (m, 3H), 5.25 (s, 2H), 3.77 (s, 2H). LRMS (ESI) *m/z* $[M-H]^-$ 298.1.

**Step 3: 4-benzyl-N-(1H-indol-3-yl)-3-oxo-3,4-dihydro-2H-benzo[b][1,4]thiazin-6-carboxamide (I-70)**

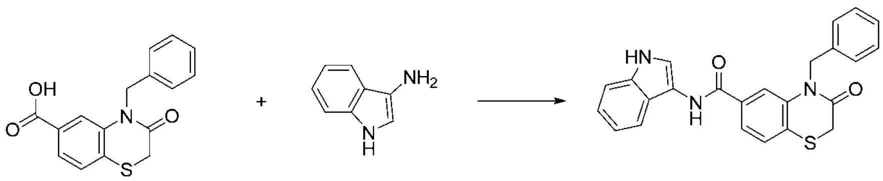

The preparation of 4-benzyl-N-(1H-indol-3-yl)-3-oxo-3,4-dihydro-2H-benzo[b][1,4]thiazin-6-carboxamide (**I-70)** was the same as that of compound **I-1**, with a yield of 62%. $^1$H NMR (400 MHz, DMSO-$d_6$) $\delta$ 10.93 (s, 1H), 10.14 (s, 1H), 7.79-7.74 (m, 2H), 7.73-7.68 (m, 2H), 7.59 (d, *J* = 8.0 Hz, 1H), 7.38-7.30 (m, 3H), 7.29-7.21 (m, 3H), 7.11 (t, *J*= 7.5 Hz, 1H), 7.02 (t, *J* = 7.5 Hz, 1H), 5.35 (s, 2H), 3.77 (s, 2H).

**Example 71 4-benzyl-N-(1H-indol-6-yl)-3-oxo-3,4-dihydro-2H-benzo[b][1,4]thiazin-6-carboxamide (I-71)**

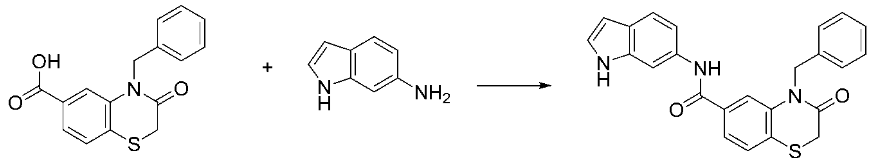

The preparation of 4-benzyl-N-(1H-indol-6-yl)-3-oxo-3,4-dihydro-2H-benzo[b][1,4]thiazin-6-carboxamide **(I-71)** was the same as that of compound **I-1**, with a yield of 87%. $^1$H NMR (400 MHz, DMSO-$d_6$) $\delta$ 11.05 (s, 1H), 10.12 (s, 1H), 7.99 (s, 1H), 7.71 (s, 1H), 7.66 (d, *J*= 8.2 Hz, 1H), 7.59 (d, *J*= 8.0 Hz, 1H), 7.47 (d, *J*= 8.4 Hz, 1H), 7.37-7.28 (m, 3H), 7.27-7.12 (m, 4H), 6.37 (s, 1H), 5.33 (s, 2H), 3.76 (s, 2H).

**Example 72 4-benzyl-N-(1H-indol-3-yl)-3-oxo-3,4-dihydro-2H-benzo[b][1,4]oxazin-6-carboxamide (I-72)**

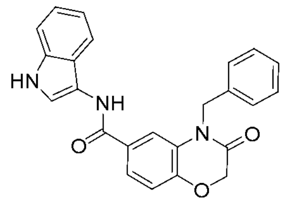

**Step 1: methyl 4-benzyl-3-oxo-3,4-dihydro-2H-benzo[b][1,4]oxazin-6-carboxylate (72-1)**

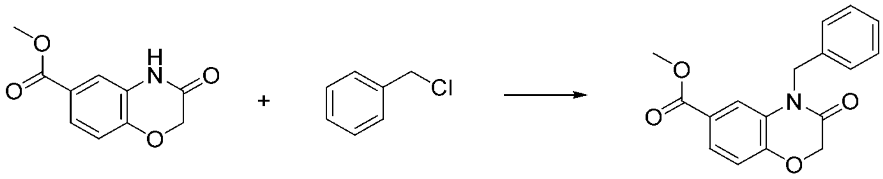

The preparation of methyl 4-benzyl-3-oxo-3,4-dihydro-2H-benzo[b][1,4]oxazin-6-carboxylate (**72-1**) was the same as that of compound **2-3,** with a yield of 91%. $^1$H NMR (400 MHz, DMSO-$d_6$) $\delta$ 7.60 (dd, *J*= 8.3, 1.9 Hz, 1H), 7.52 (d, *J*= 1.9 Hz, 1H), 7.41-7.32 (m, 2H), 7.32-7.24 (m, 3H), 7.13 (d, *J* = 8.4 Hz, 1H), 5.21 (s, 2H), 4.95 (s, 2H), 3.76 (s, 3H). LRMS (ESI) *m/z* [M+H]$^+$ 298.1.

**Step 2: 4-benzyl-3-oxo-3,4-dihydro-2H-benzo[b][1,4]oxazin-6-carboxylic acid (72-2)**

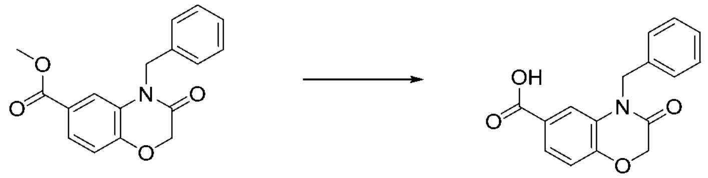

The preparation of 4-benzyl-3-oxo-3,4-dihydro-2H-benzo[b][1,4]oxazin-6-carboxylic acid **(72-2)** was the same as that of compound **1-5,** with a yield of 99%. $^1$H NMR (400 MHz, DMSO-$d_6$) $\delta$ 7.57 (dd, *J*= 8.3, 1.9 Hz, 1H), 7.51-7.46 (m, 1H), 7.38-7.32 (m, 2H), 7.30-7.23 (m, 3H), 7.08 (d, *J* = 8.3 Hz, 1H), 5.18 (s, 2H), 4.91 (s, 2H). LRMS (ESI) *m/z* [M+Na]$^+$ 306.1.

**Step 3: 4-benzyl-N-(1H-indol-3-yl)-3-oxo-3,4-dihydro-2H-benzo[b][1,4]oxazin-6-carboxamide (I-72)**

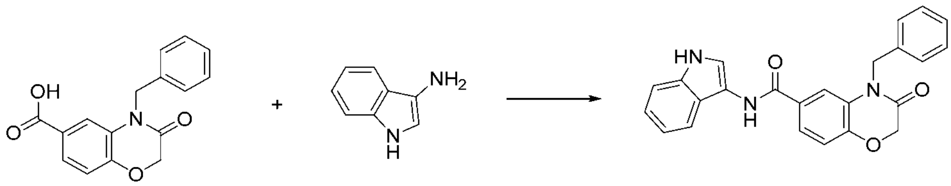

The preparation of 4-benzyl-N-(1H-indol-3-yl)-3-oxo-3,4-dihydro-2H-benzo[b][1,4]oxazin-6-carboxamide **(I-72)** was the same as that of compound **I-1**, with a yield of 37%. $^1$H NMR (400 MHz, DMSO-$d_6$) $\delta$ 10.92 (s, 1H), 10.04 (s, 1H), 7.78 (d, $J$ = 8.1 Hz, 1H), 7.76-7.66 (m, 2H), 7.62 (s, 1H), 7.41-7.30 (m, 5H), 7.30-7.22 (m, 2H), 7.18-7.08 (m, 1H), 7.03 (t, $J$= 7.8 Hz, 1H), 5.27 (s, 2H), 4.93 (s, 2H).

**Example 73 4-benzyl-N-(1H-indol-3-yl)-3-oxo-3,4-dihydro-2H-benzo[b][1,4]thiazin-7-carboxamide (I-73)**

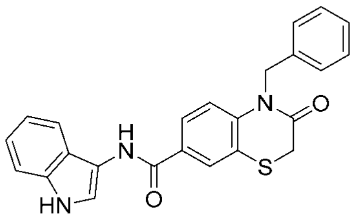

**Step 1: methyl 4-benzyl-3-oxo-3,4-dihydro-2H-benzo[b] [1,4]thiazin-7-carboxylate (73-1)**

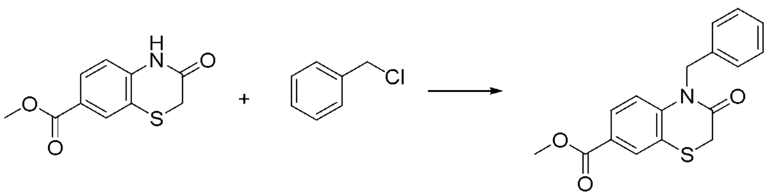

The preparation of methyl 4-benzyl-3-oxo-3,4-dihydro-2H-benzo[b][1,4]thiazin-7-carboxylate (**73-1**) was the same as that of compound **2-3,** with a yield of 88%. $^1$H NMR (400 MHz, DMSO-$d_6$) $\delta$ 7.95 (d, $J$= 2.1 Hz, 1H), 7.73 (dd, $J$ = 8.5, 2.1 Hz, 1H), 7.35-7.17 (m, 7H), 5.28 (s, 2H), 3.81 (s, 3H), 3.75 (s, 2H).

**Step 2: 4-benzyl-3-oxo-3,4-dihydro-2H-benzo[b][1,4]thiazin-7-carboxamide (73-2)**

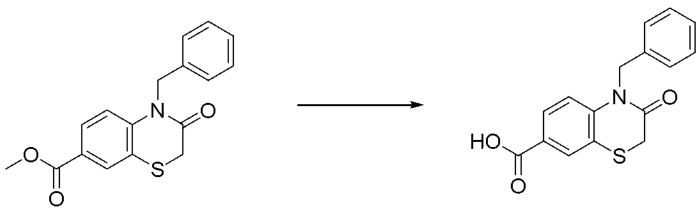

The preparation of 4-benzyl-3-oxo-3,4-dihydro-2H-benzo[b][1,4]thiazin-7-carboxamide (**73-2**) was the same as that of compound **1-5,** with a yield of 95%. LRMS (ESI) $m/z$ [M+H]$^+$ 300.1.

**Step 3: 4-benzyl-3-oxo-3,4-dihydro-2H-benzo[b][1,4]thiazin-7-carbonyl chloride (73-3)**

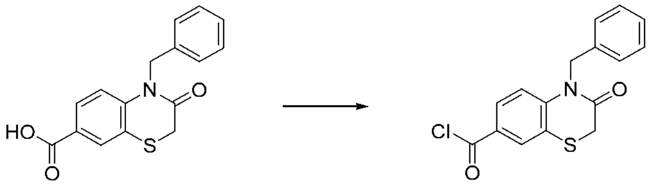

50 Milligrams of compound **73-3** was placed in a dry reaction flask, 2 milliliters of tetrahydrofuran was added, a drop of DMF solution was added thereto, the atmosphere was replaced with argon gas and the flask was placed in an ice water bath. 20 Microliters of dichlorosulfoxide was slowly added to the reaction flask. After the addition was completed, the reaction flask was moved to room temperature and stirred for 2 hours. After the reaction was completed, the mixture was concentrated to dryness to obtain a light yellow solid **73-3.**

**Step 4: 4-benzyl-N-(1H-indol-3-yl)-3-oxo-3,4-dihydro-2H-benzo[b][1,4]thiazin-7-carboxamide (I-73)**

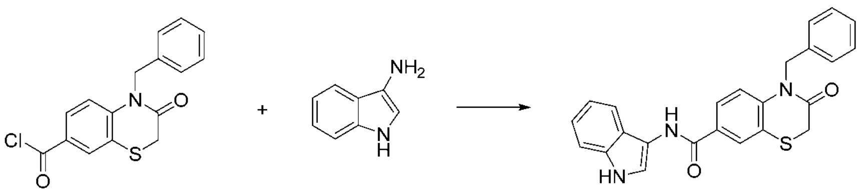

A mixed solution of 25 mg of 3-aminoindole, 87 µL of DIPEA, and 1 mL of THF was placed in an ice water bath, and a THF solution of compound **73-3** was slowly added dropwise. After dripping, the reaction mixture was moved to room temperature and stirred. After the reaction was completed, the reaction solution was concentrated to dryness, and the residue was subjected to preparative thin-layer chromatography with dichloromethane: methanol = 50:1 to obtain 41 mg of light yellow solid with a yield of 60%. $^1$H NMR (400 MHz, DMSO-$d_6$) $\delta$ 10.92 (s, 1H), 10.12 (s, 1H), 8.08 (s, 1H), 7.88-7.75 (m, 3H), 7.39-7.29 (m, 4H), 7.27-7.20 (m, 3H), 7.11 (t, *J*= 7.5 Hz, 1H), 7.00 (t, *J* = 7.5 Hz, 1H), 5.33 (s, 2H), 3.77 (s, 2H).

**Example 74 4-benzyl-N-(1H-indol-6-yl)-3-oxo-3,4-dihydro-2H-benzo[b][1,4]thiazin-7-carboxamide (I-74)**

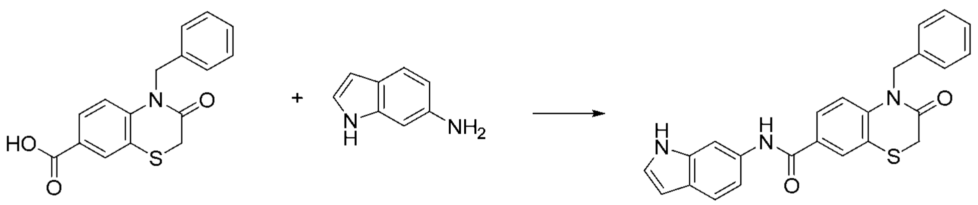

The preparation of 4-benzyl-N-(1H-indol-6-yl)-3-oxo-3,4-dihydro-2H-benzo[b][1,4]thiazin-7-carboxamide **(I-74)** was the same as that of compound **I-1,** with a yield of 30%. $^1$H NMR (400 MHz, DMSO-$d_6$) $\delta$ 11.06 (s, 1H), 10.11 (s, 1H), 8.04 (s, 2H), 7.77 (dd, *J* = 8.7, 2.1 Hz, 1H), 7.46 (d, *J* = 8.5 Hz, 1H), 7.35-7.26 (m, 3H), 7.25-7.20 (m, 5H), 6.37 (s, 1H), 5.32 (s, 2H), 3.76 (s, 2H).

**Example 75 4-benzyl-N-(1H-indol-3-yl)-3-oxo-3,4-dihydro-2H-benzo[b][1,4]oxazin-7-carboxamide (I-75)**

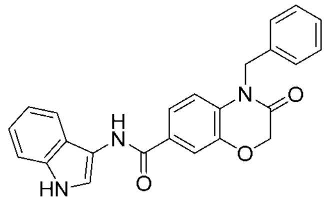

**Step 1: methyl 4-benzyl-3-oxo-3,4-dihydro-2H-benzo[b][1,4]oxazin-7-carboxylate (75-1)**

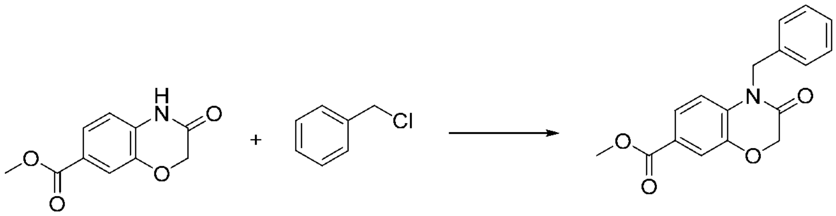

The preparation of methyl 4-benzyl-3-oxo-3,4-dihydro-2H-benzo[b][1,4]oxazin-7-carboxylate (**75-1**) was the same as that of compound **2-3,** with a yield of 84%. $^1$H NMR (400 MHz, $CD_3OD$) $\delta$ 7.66-7.48 (m, 2H), 7.37-7.20 (m, 5H), 7.07 (d, *J* = 8.9 Hz, 1H), 5.23 (s, 2H), 4.80 (s, 2H), 3.85 (s, 3H). LRMS (ESI) *m*/*z* $[M+H]^+$ 298.1.

**Step 2: 4-benzyl-3-oxo-3,4-dihydro-2H-benzo[b][1,4]oxazin-7-carboxylic acid (75-2)**

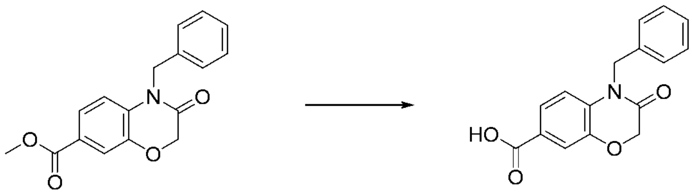

The preparation of 4-benzyl-3-oxo-3,4-dihydro-2H-benzo[b][1,4]oxazin-7-carboxylic acid **(75-2)** was the same as that of compound **1-5,** with a yield of 97%. $^1$H NMR (400 MHz, DMSO-$d_6$) $\delta$ 12.60 (s, 1H), 7.39-7.29 (m, 4H), 7.25-7.19 (m, 2H), 6.50-6.29 (m, 2H), 4.76 (s, 2H), 4.43 (d, *J*= 5.8 Hz, 2H). LRMS (ESI) *m*/*z* $[M+H]^+$ 284.0.

**Step 3: 4-benzyl-3-oxo-3,4-dihydro-2H-benzo[b][1,4]oxazin-7-formyl chloride (75-3)**

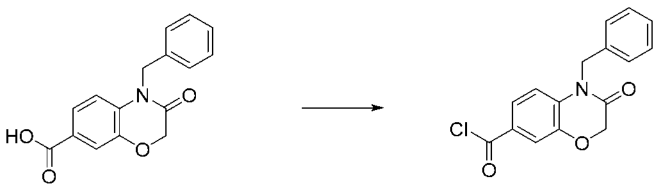

The preparation of 4-benzyl-3-oxo-3,4-dihydro-2H-benzo[b][1,4]oxazin-7-formyl chloride (**75-3**) was the same as that of compound **73-3.**

**Step 4: 4-benzyl-N-(1H-indol-3-yl)-3-oxo-3,4-dihydro-2H-benzo[b][1,4]oxazin-7-carboxamide (I-75)**

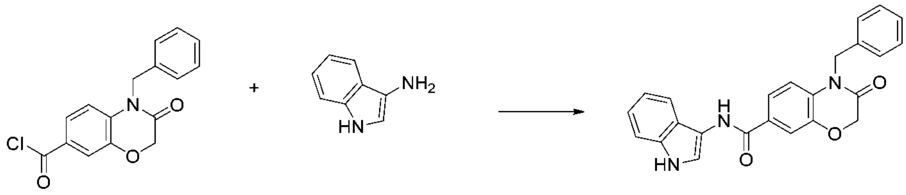

The preparation of 4-benzyl-N-(1H-indol-3-yl)-3-oxo-3,4-dihydro-2H-benzo[b][1,4]oxazin-7-carboxamide (**I-75**) was the same as that of compound **I-73**, with a yield of 23%. $^1$H NMR (400 MHz, DMSO-$d_6$) $\delta$ 10.93 (s, 1H), 10.06 (s, 1H), 7.83 (d, $J$ = 8.0 Hz, 1H), 7.76 (s, 1H), 7.69-7.61 (m, 2H), 7.40-7.24 (m, 6H), 7.16 (d, $J$= 8.3 Hz, 1H), 7.09 (t, $J$ = 7.4 Hz, 1H), 6.99 (t, $J$= 7.8 Hz, 1H), 5.24 (s, 2H), 4.90 (s, 2H).

**Example 76 1-benzyl-N-(1H-indol-3-yl)-3,3-dimethyl-2-oxoindolin-6-carboxamide (I-76)**

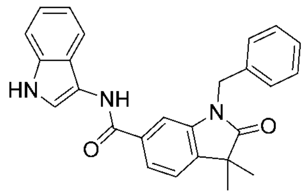

**Step 1: 1-benzyl-6-bromo-3,3-dimethylindolin-2-one (76-1)**

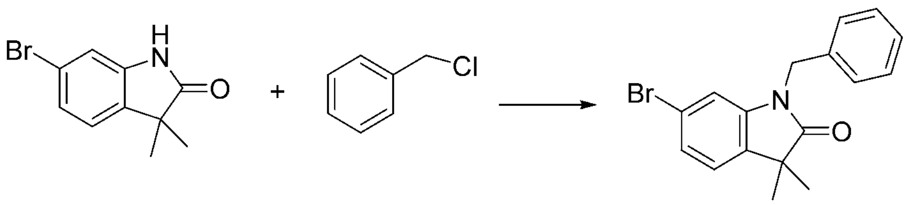

The preparation of 1-benzyl-6-bromo-3,3-dimethylindolin-2-one **(76-1)** was the same as that of compound **2-3,** with a yield of 93%. $^1$H NMR (400 MHz, DMSO-$d_6$) $\delta$ 7.38-7.32 (m, 3H), 7.31-7.24 (m, 3H), 7.22 (dd, $J$ = 7.9, 1.8 Hz, 1H), 7.14 (d, $J$ = 1.7 Hz, 1H), 4.92 (s, 1H), 1.33 (s, 6H). LRMS (ESI) $m/z$ $[M+H]^+$ 330.0 .

**Step 2: 1-benzyl-3,3-dimethyl-2-oxoindolin-6-nitrile (76-2)**

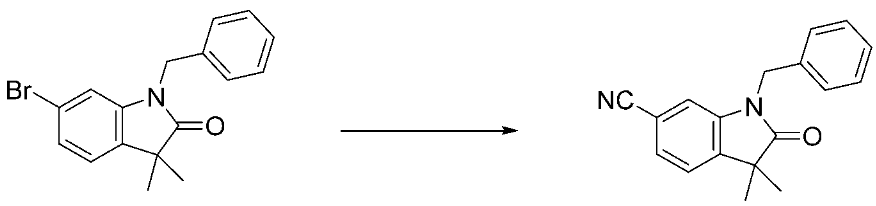

The preparation of 1-benzyl-3,3-dimethyl-2-oxoindolin-6-nitrile **(76-2)** was the same as that of compound **11-2,** with a yield of 76%. $^1$H NMR (400 MHz, DMSO-$d_6$) $\delta$ 7.62 (d, $J$ = 7.6 Hz, 1H), 7.53 (dd, $J$= 7.6, 1.5 Hz, 1H), 7.43 (s, 1H), 7.39-7.24 (m, 5H), 4.93 (s, 2H), 1.35 (s, 6H). LRMS (ESI) $m/z$ $[M+Na]^+$ 299.0.

**Step 3: 1-benzyl-3,3-dimethyl-2-oxoindolin-6-carboxylic acid (76-3)**

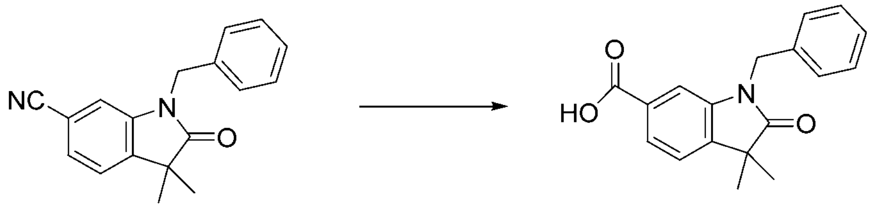

The preparation of 1-benzyl-3,3-dimethyl2-oxoindolin-6-carboxylic acid **(76-3)** was the same as that of compound **11-3,** with a yield of 80%. $^1$H NMR (400 MHz, DMSO-$d_6$) $\delta$ 12.92 (s, 1H), 7.67 (dd, $J$ = 7.7, 1.5 Hz, 1H), 7.52 (d, $J$ = 7.7 Hz, 1H), 7.38-7.33 (m, 3H), 7.30-7.24 (m, 3H), 4.97 (s, 2H), 1.38 (s, 6H). LRMS (ESI) $m/z$ [M-H]$^-$ 294.0.

**Step 4: 1-benzyl-N-(1H-indol-3-yl)-3,3-dimethyl-2-oxoindolin-6-carboxamide (I-76)**

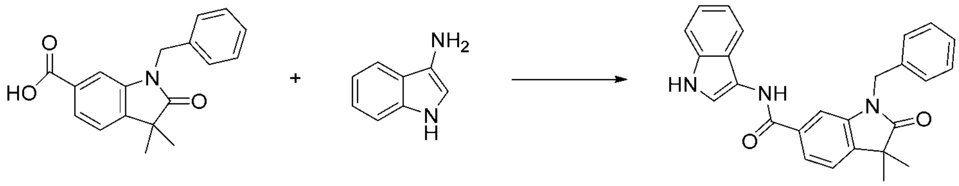

The preparation of 1-benzyl-N-(1H-indol-3-yl)-3,3-dimethyl-2-oxoindolin-6-carboxamide **(I-76)** was the same as that of compound **I-1,** with a yield of 48%. $^1$H NMR (400 MHz, DMSO-$d_6$) $\delta$ 10.90 (s, 1H), 10.06 (s, 1H), 7.79 (d, $J$ = 8.0 Hz, 1H), 7.76-7.69 (m, 2H), 7.55 (d, $J$ = 7.7 Hz, 1H), 7.44 (s, 1H), 7.39-7.23 (m, 6H), 7.10 (t, $J$ = 7.5 Hz, 1H), 7.00 (t, $J$ = 7.5 Hz, 1H), 4.99 (s, 2H), 1.39 (s, 6H).

**Example 77 N-(1H-indol-3-yl)-3,3-dimethyl-2-oxo-1-(thien-3-ylmethyl)indolin-6-carboxamide (I-77)**

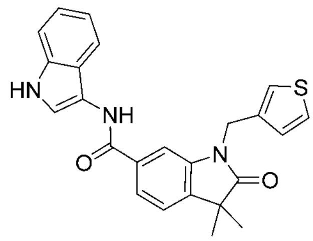

**Step 1: methyl 3,3-dimethyl-2-oxo-1-(thien-3-ylmethyl)indolin-6-carboxylate (77-1)**

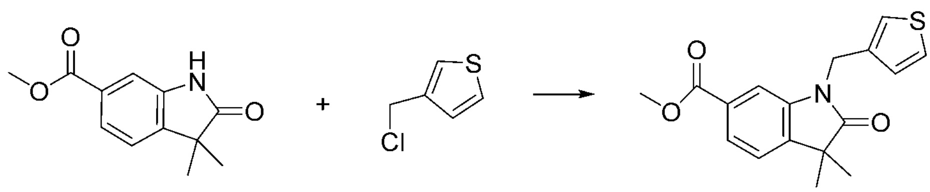

The preparation of methyl 3,3-dimethyl-2-oxo-1-(thien-3-ylmethyl)indolin-6-carboxylate **(77-1)** was the same as that of compound **2-3,** with a yield of 93%. $^1$H NMR (400 MHz, DMSO-$d_6$) $\delta$ 7.69 (dd, $J$ = 7.7, 1.5 Hz, 1H), 7.57-7.48 (m, 2H), 7.45 (s, 1H), 7.37-7.32 (m, 1H), 6.98 (dd, $J$ = 5.0, 1.3 Hz, 1H), 4.95 (s, 2H), 3.82 (s, 3H), 1.34 (s, 6H). LRMS (ESI) $m/z$ [M+H]$^+$ 316.0.

**Step 3: 3,3-dimethyl-2-oxo-1-(thien-3-ylmethyl)indolin-6-carboxylic acid (77-2)**

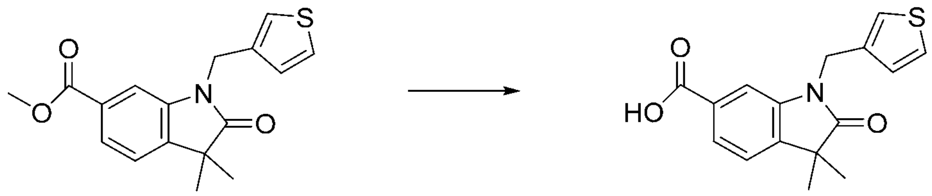

The preparation of 3,3-dimethyl-2-oxo-1-(thien-3-ylmethyl)indolin-6-carboxylic acid **(77-2)** was the same as that of compound **1-5,** with a yield of 99 %. $^1$H NMR (400 MHz, DMSO-*d*$_6$) $\delta$ 7.73-7.62 (m, 1H), 7.55-7.45 (m, 2H), 7.44 (s, 1H), 7.36 (d, *J* = 2.9 Hz, 1H), 6.98 (d, *J*= 5.0 Hz, 1H), 4.95 (s, 2H), 1.35 (s, 6H). LRMS (ESI) *m*/*z* [M+H]$^+$ 302.0.

**Step 4: N-(1H-indol-3-yl)-3,3-dimethyl-2-oxo-1-(thien-3-ylmethyl)indolin-6-carboxamide** (**I-77**)

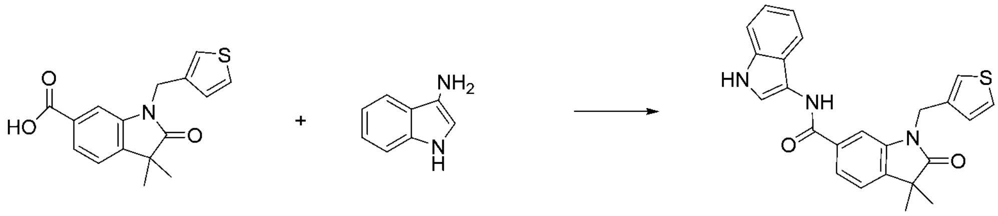

The preparation of N-(1H-indol-3-yl)-3,3-dimethyl-2-oxo-1-(thien-3-ylmethyl)indolin-6-carboxamide (**I-77**) was the same as that of compound **I-**1, with a yield of 53%. $^1$H NMR (400 MHz, DMSO-*d*$_6$) $\delta$ 10.91 (s, 1H), 10.07 (s, 1H), 7.81 (d, *J* = 8.0 Hz, 1H), 7.76 (d, *J*= *2.5* Hz, 1H), 7.71 (d, *J* = 7.5, 1.6 Hz, 1H), 7.55-7.49 (m, 3H), 7.41 (s, 1H), 7.36 (d, *J*= 8.1 Hz, 1H), 7.11 (t, *J*= 7.5 Hz, 1H), 7.04-6.96 (m, 2H), 4.96 (s, 2H), 1.36 (s, 6H).

**Example 78 1-cyclopropylmethyl-N-(1H-indol-3-yl)-3,3-dimethyl-2-oxoindolin-6-carboxamide (I-78)**

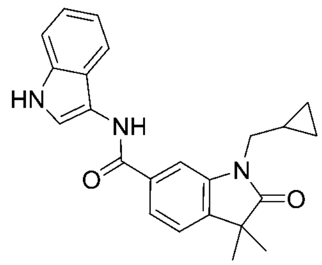

**Step 1: 6-bromo-1-cyclopropylmethyl-3,3-dimethylindolin-2-one (78-1)**

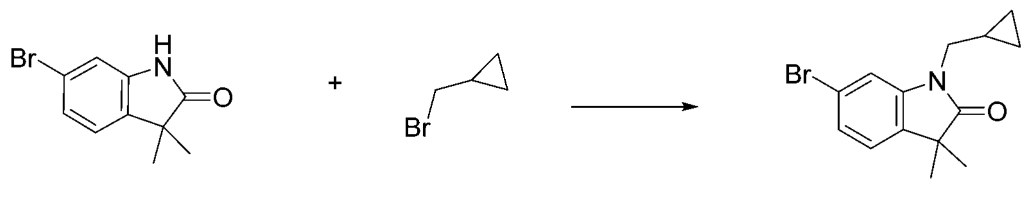

The preparation of 6-bromo-1-cyclopropylmethyl-3,3-dimethylindolin-2-one **(78-1)** was the same as that of

compound **2-3** to obtain a white solid, with a yield of 91 %. $^1$H NMR (400 MHz, DMSO-$d_6$) δ 7.38 (d, *J* = 1.8 Hz, 1H), 7.32 (d, *J*= 7.8 Hz, 1H), 7.21 (dd, *J* = 7.8, 1.7 Hz, 1H), 3.58 (d, *J= 6.9* Hz, 2H), 1.25 (s, 6H), 1.17-0.95 (m, 1H), 0.51-0.39 (m, 2H), 0.37-0.23 (m, 2H). LRMS (ESI) *m/z* [M+H]$^+$ 294.0.

**Step 2: 1-cyclopropylmethyl-3,3-dimethyl-2-oxoindolin-6-carbonitrile (78-2)**

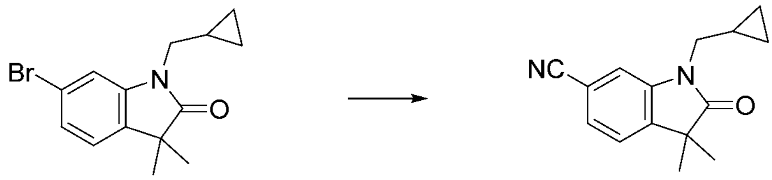

The preparation of 1-cyclopropylmethyl-3,3-dimethyl-2-oxoindolin-6-carbonitrile **(78-2)** was the same as that of compound **11-2** to obtain a white solid, with a yield of 78%. $^1$H NMR (400 MHz, DMSO-$d_6$) δ 7.65 (s, 1H), 7.59 (d, *J*= 7.6 Hz, 1H), 7.54-7.46 (m, 1H), 3.60 (d, *J*= 7.0 Hz, 2H), 1.29 (s, 6H), 1.26-1.08 (m, 1H), 0.48-0.38 (m, 2H), 0.34-0.27 (m, 2H). LRMS (ESI) *m/z* [M+H]$^+$ 241.1.

**Step 3: 1-cyclopropylmethyl-3,3-dimethyl-2-oxoindolin-6-carboxylic acid (78-3)**

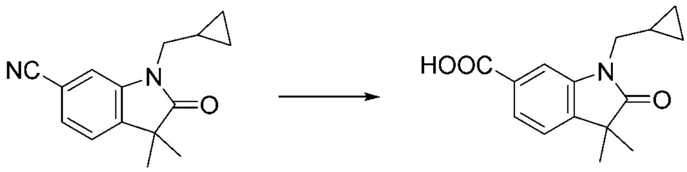

The preparation of **1-cyclopropylmethyl-3,3-dimethyl-2-oxoindolin-6-carboxylic acid (78-3)** was the same as that of compound 11-3, with a yield of 93%. $^1$H NMR (400 MHz, DMSO-$d_6$) δ 12.97 (s, 1H), 7.68 (dd, *J*= 7.6, 1.4 Hz, 1H), 7.57 (d, *J*= 1.4 Hz, 1H), 7.49 (d, *J*= 7.6 Hz, 1H), 3.63 (d, *J* = 6.8 Hz, 2H), 1.29 (s, 6H), 1.19-1.07 (m, 1H), 0.49-0.42 (m, 2H), 0.35-0.27 (m, 2H). LRMS (ESI) *m/z* [M+H]$^+$ 260.1.

**Step 4: 1-cyclopropylmethyl-N-(1H-indol-3-yl)-3,3-dimethyl-2-oxoindolin-6-carboxamide** (**I-78**)

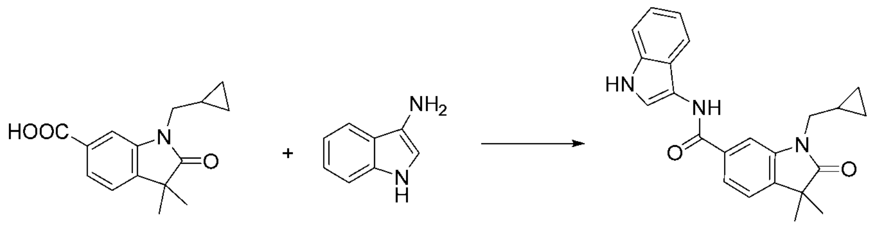

The preparation of 1-cyclopropylmethyl-N-(1H-indol-3-yl)-3,3-dimethyl-2-oxoindolin-6-carboxamide (**I-78**) was the same as that of compound **I-1,** with a yield of 36%. $^1$H NMR (400 MHz, DMSO-$d_6$) δ 10.93 (s, 1H), 10.13 (s, 1H), 7.83 (d, *J*= 8.0 Hz, 1H), 7.78 (d, *J* = 2.5 Hz, 1H), 7.75-7.66 (m, 2H), 7.52 *(d, J*= 7.6 Hz, 1H), 7.37 (d, *J* = 8.1 Hz, 1H), 7.12 (t, *J* = 7.1 Hz, 1H), 7.02 (t, *J* = 7.5 Hz, 1H), 3.66 (d, *J* = 6.9 Hz, 2H), 1.33 (s, 6H), 1.29-1.22 (m, 1H), 0.52-0.44 (m, 2H), 0.40-0.32 (m, 2H).

**Example 79 N-(1H-indol-3-yl)-3,3-dimethyl-2-oxo-1-(thien-2-ylmethyl)indolin-6-carboxamide (I-79)**

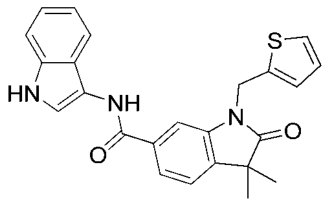

**Step 1: 6-bromo-3,3-dimethyl-1-(thien-2-ylmethyl)indolin-2-one (79-1)**

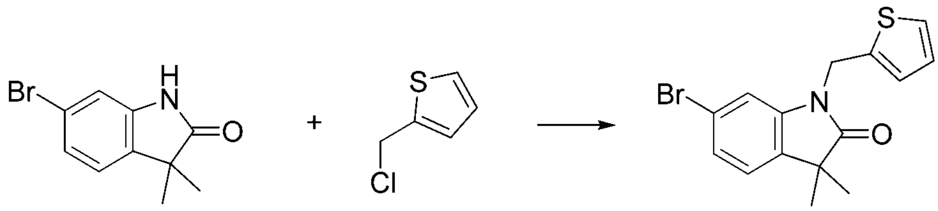

The preparation of 6-bromo-3,3-dimethyl-1-(thien-2-ylmethyl)indolin-2-one **(79-1)** was the same as that of compound **2-3** to obtain a transparent oily substance, with a yield of 92%. $^1$H NMR (400 MHz, DMSO-$d_6$) $\delta$ 7.42 (dd, *J*= 5.1, 1.3 Hz, 1H), 7.37-7.33 (m, 1H), 7.32 (s, 1H), 7.22 (dd, *J*= 7.9, 1.8 Hz, 1H), 7.19-7.17 (m, 1H), 7.03-6.83 (m, 1H), 5.10 (s, 2H), 1.28 (s, 6H). LRMS (ESI) *m/z* [M+H]$^+$ 335.9.

**Step 2: 3,3-dimethyl-2-oxo-1-(thien-2-ylmethyl)indolin-6-carbonitrile (79-2)**

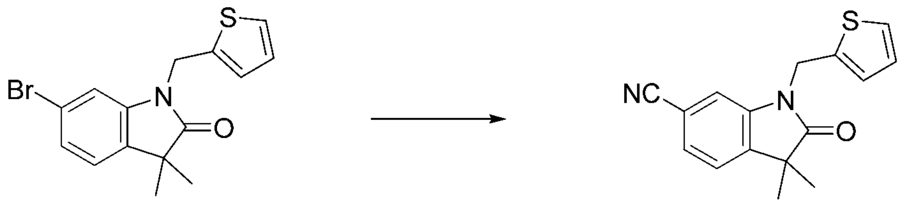

The preparation of 3,3-dimethyl-2-oxo-1-(thien-2-ylmethyl)indolin-6-carbonitrile **(79-2)** was the same as that of compound **11-2** to obtain a white solid, with a yield of 98%. $^1$H NMR (400 MHz, DMSO-$d_6$) $\delta$ 7.62-7.60 (m, 1H), 7.59 (s, 1H), 7.53 (dd, *J* = 7.6, 1.4 Hz, 1H), 7.42 (dd, *J*= 5.1, 1.3 Hz, 1H), 7.28-7.21 (m, 1H), 7.00-6.94 (m, 1H), 5.11 (s, 2H), 1.31 (s, 6H).LRMS (ESI) *m/z* [M+H]$^+$: 283.1.

**Step 3: 3,3-dimethyl-2-oxo-1-(thien-2-ylmethyl)indolin-6-carboxylic acid (79-3)**

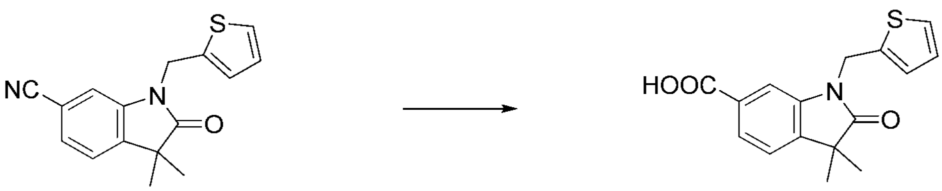

The preparation of 3,3-dimethyl-2-oxo-1-(thien-2-ylmethyl)indolin-6-carboxylic acid **(79-3)** was the same as that of compound **11-3** to obtain a white solid, with a yield of 80%. $^1$H NMR (400 MHz, DMSO-$d_6$) $\delta$ 12.94 (s, 1H), 7.67 (dd, *J*= 7.6, 1.4 Hz, 1H), 7.55 (d, *J*= 1.5 Hz, 1H), 7.50 (d, *J*= 7.7 Hz, 1H), 7.41 (dd, *J* = 5.1, 1.3 Hz, 1H), 7.19-7.08 (m, 1H), 7.06-6.89 (m, 1H), 5.15 (s, 2H), 1.32 (s, 6H). LRMS (ESI) *m/z* [M+H]$^+$ 302.1.

**Step 4: N-(1H-indol-3-yl)-3,3-dimethyl-2-oxo-1-(thien-2-ylmethyl)indolin-6-carboxamide** (**I-79**)

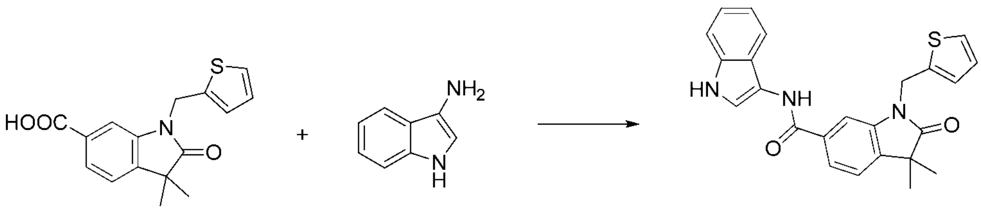

The preparation of N-(1H-indol-3-yl)-3,3-dimethyl-2-oxo-1-(thien-2-ylmethyl)indolin-6-carboxamide **(I-79)** was the same as that of compound **I-1,** with a yield of 58%. $^1$H NMR (400 MHz, DMSO-$d_6$) $\delta$ 10.92 (s, 1H), 10.09 (s, 1H), 7.82 (d, $J$ = 8.0 Hz, 1H), 7.77 (s, 1H), 7.72 (d, $J$ = 7.4 Hz, 1H), 7.63 (s, 1H), 7.53 (d, $J$= 7.6 Hz, 1H), 7.44-7.32 (m, 2H), 7.19 (s, 1H), 7.11 (t, $J$= 7.5 Hz, 1H), 7.07-6.92 (m, 2H), 5.17 (s, 2H), 1.35 (s, 6H).

**Example 80 1-(furan-2-ylmethyl)-N-(1H-indol-3-yl)-3,3-dimethyl-2-oxoindolin-6-carboxamide (I-80)**

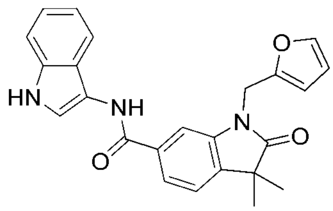

**Step 1: methyl 1-(furan-2-ylmethyl)-3,3-dimethyl-2-oxoindolin-6-carboxylate (80-1)**

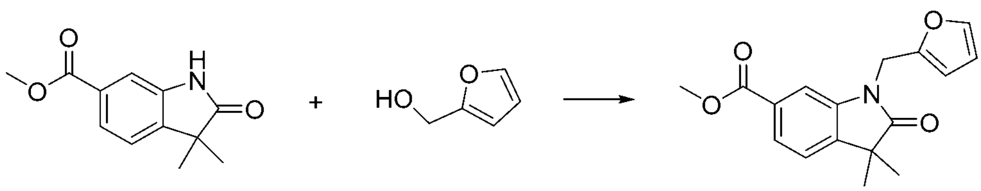

The preparation of methyl 1-(furan-2-ylmethyl)-3,3-dimethyl-2-oxoindolin-6-carboxylate **(80-1)** was the same as that of compound **9-2,** with a yield of 35%. $^1$H NMR (400 MHz, DMSO-$d_6$) $\delta$ 7.70 (dd, $J$= 7.7, 1.5 Hz, 1H), 7.60-7.57 (m, 2H), 7.53 (d, $J$ = 7.7 Hz, 1H), 6.41-6.38 (m, 2H), 4.98 (s, 2H), 3.85 (s, 3H), 1.32 (s, 6H). LRMS (ESI) $m/z$ $[M+H]^+$ 300.1.

**Step 2: 1-(furan-2-ylmethyl)-3,3-dimethyl-2-oxoindolin-6-carboxylic acid (80-2)**

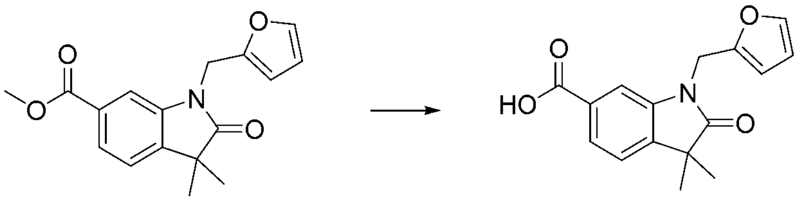

The preparation of 1-(furan-2-ylmethyl)-3,3-dimethyl-2-oxoindolin-6-carboxylic acid **(80-2)** was the same as compound **1-5,** with a yield of 85%. $^1$H NMR (400 MHz, DMSO-$d_6$) $\delta$ 12.93 (s, 1H), 7.68 (d, $J$= 7.7, 1.4 Hz, 1H), 7.58 (d, 2H), 7.49 (d, $J$ = 7.7 Hz, 1H), 6.48-6.36 (m, 2H), 4.96 (s, 2H), 1.31 (s, 6H). LRMS (ESI) $m/z$ $[M+H]^+$ 286.2.

**Step 3: 1-(furan-2-ylmethyl)-N-(1H-indol-3-yl)-3,3-dimethyl-2-oxoindolin-6-carboxamide (I-80)**

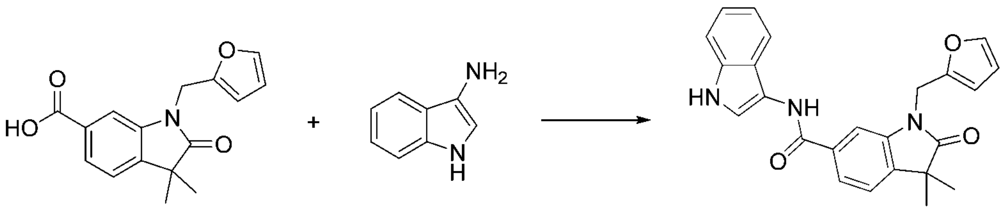

The preparation of 1-(furan-2-ylmethyl)-N-(1H-indol-3-yl)-3,3-dimethyl-2-oxoindolin-6-carboxamide (I-80) was the same as that of compound **I-1,** with a yield of 49%. $^1$H NMR (400 MHz, DMSO-$d_6$) $\delta$ 10.92 (s, 1H), 10.10 (s, 1H), 7.83 (d, $J$= 8.0 Hz, 1H), 7.79 (d, $J$ = 2.5 Hz, 1H), 7.75-7.69 (m, 1H), 7.66-7.57 (m, 2H), 7.53 (d, $J$ = 7.7 Hz, 1H), 7.37 (d, $J$ = 8.1 Hz, 1H), 7.16-7.07 (m, 1H), 7.06-6.95 (m, 1H), 6.45-6.37 (m, 2H), 4.99 (s, 2H), 1.35 (s, 6H).

**Example 81 N-(1H-indol-3-yl)-3,3-dimethyl-2-oxo-1-phenethylindolin-6-carboxamide (I-81)**

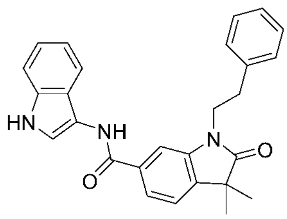

**Step 1: 6-bromo-3,3-dimethyl-1-phenethylindolin-2-one (81-1)**

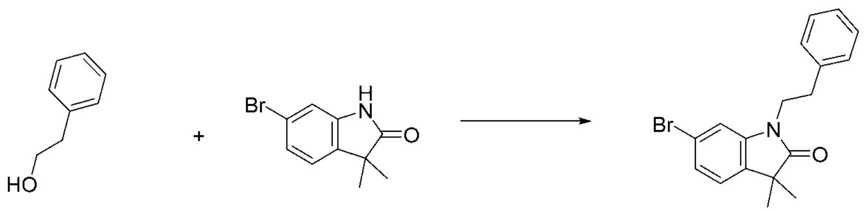

The preparation of 6-bromo-3,3-dimethyl-1-phenethylindolin-2-one **(81-1)** was the same as that of compound **9-2.** $^1$H NMR (400 MHz, $CDCl_3$) $\delta$ 7.31-7.27 (m, 2H), 7.22 (tt, $J$= 7.7, 1.5 Hz, 3H), 7.17 (dd, $J$= 7.8, 1.7 Hz, 1H), 7.05 (d, $J$= 7.9 Hz, 1H), 6.90 (d, $J$= 1.7 Hz, 1H), 3.94 (t, $J$ = 7.4 Hz, 2H), 2.99 (t, $J$ = 7.4 Hz, 2H), 1.30 (s, 6H).

**Step 2: 3,3-dimethyl-2-oxo-1-phenethylindolin-6-nitrile (81-2)**

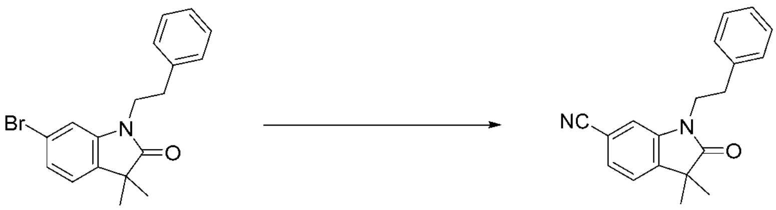

The preparation of 3,3-dimethyl-2-oxo-1-phenethylindolin-6-nitrile **(81-2)** was the same as that of compound **9-3.**

$^1H$ NMR (400 MHz, $CDCl_3$) δ 7.32 (dd, *J* = 7.6, 1.3 Hz, 1H), 7.27 (d, *J* = 2.0 Hz, 1H), 7.25-7.20 (m, 3H), 7.18-7.13 (m, 2H), 6.86 (d, *J*= 1.4 Hz, 1H), 3.95 (t, *J*= 7.2 Hz, 2H), 2.97 (t, *J*= 7.2 Hz, 2H), 1.31 (s, 6H).

**Step 3: 3,3-dimethyl-2-oxo-1-phenethylindolin-6-carboxylic acid (81-3)**

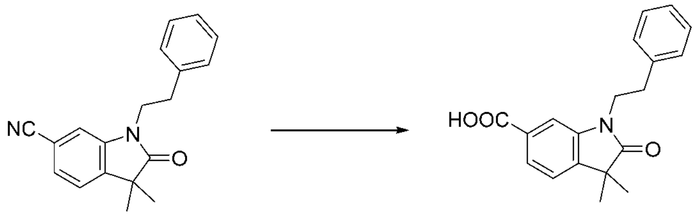

The preparation of 3,3-dimethyl-2-oxo-1-phenethylindolin-6-carboxylic acid **(81-3)** was the same as that of compound 11-3. LCMS (ESI) *m/z* $[M+H]^+$: 310.0.

**Step 4: N-(1H-indol-3-yl)-3,3-dimethyl-2-oxo-1-phenethylindolin-6-carboxamide (I-81)**

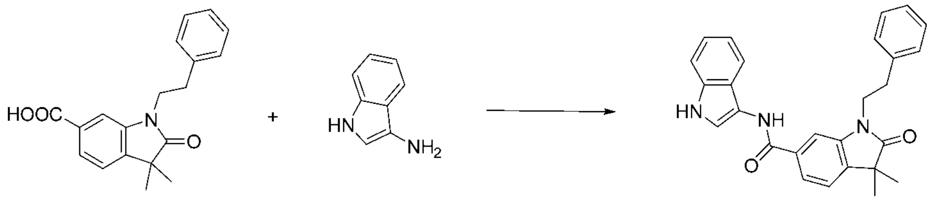

The preparation of N-(1H-indol-3-yl)-3,3-dimethyl-2-oxo-1-phenethylindolin-6-carboxamide **(I-26)** was the same as that of compound **I-1.** $^1H$ NMR (600 MHz, DMSO-$d_6$) δ 10.93 (s, 1H), 10.12 (s, 1H), 7.85 (d, *J*= 8.0 Hz, 1H), 7.80 (d, *J*=2.0 Hz, 1H), 7.69 (d, *J*=7.7 Hz, 1H), 7.62 (s, 1H), 7.49-7.46 (m, 1H), 7.38 (d, *J* = 8.1 Hz, 1H), 7.23 (dt, *J* = 14.0, 7.4 Hz, 4H), 7.16 (t, *J*=7.0 Hz, 1H), 7.13 (t, *J*=7.5 Hz, 1H), 7.03 (t, *J*=7.5 Hz, 1H), 4.00 (t, *J* = 7.3 Hz, 2H), 3.00 (t, *J* = 7.3 Hz, 2H), 1.22 (s, 6H).

**Example 82 N-(1H-indol-3-yl)-2'-oxo-1'-(thien-3-ylmethyl)spiro[cyclopropane-1,3'-dihydroindol]-6'-carboxamide (I-82)**

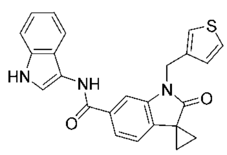

**Step 1: methyl 2'-oxospiro[cyclopropane-1,3'-dihydroindol]-6'-carboxylate (82-1)**

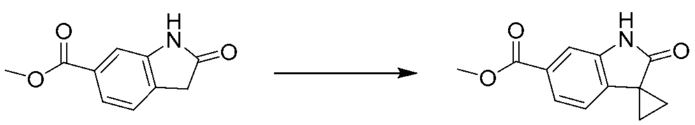

A mixture of 500 mg of methyl 2-oxoindole-6-carboxylate, 1.8 g of (2-bromoethyl) diphenylsulfonium trifluoromethanesulfonate, 1.1 ml of triethylamine, and 10 ml of N, N-dimethylformamide was stirred at room temperature under

argon protection for 7 hours. The reaction solution was concentrated to dryness, and the residue was subjected to column chromatography with ethyl acetate: petroleum ether = 1:5 to obtain 400 mg of methyl 2'-oxospiro[cyclopropane-1,3'-dihydroindol]-6'-carboxylate **(82-1)** with a yield of 71%. $^1$H NMR (400 MHz, DMSO-$d_6$) $\delta$ 10.75 (s, 1H), 7.58 (dd, $J$ = 7.8, 1.5 Hz, 1H), 7.42 (d, $J$ = 1.4 Hz, 1H), 7.11 (d, $J$ = 7.8 Hz, 1H), 3.84 (s, 3H), 1.67 (q, $J$ = 3.8, 3.2 Hz, 2H), 1.55 (q, $J$ = 3.8 Hz, 2H). LCMS (ESI) $m/z$ [M+H]$^+$: 218.1.

**Step 2: methyl 2'-oxo-1'-(thien-3-ylmethyl)spiro[cyclopropane-1,3'-dihydroindol]-6'-carboxylate (82-2)**

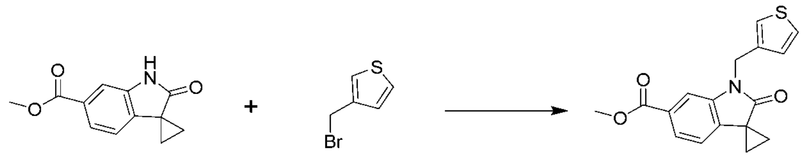

A mixture of 100 mg of methyl 2'-oxospiro[cyclopropane-1,3'-dihydroindol]-6'-carboxylate, 106 mg of 3-bromomethylthiophene, 450 mg of cesium carbonate, and 2 mL of N, N-dimethylformamide was stirred at room temperature for 5 hours. The reaction solution was quenched with water, and extracted with ethyl acetate. The organic phase was concentrated to dryness, and subjected to preparative thin-layer chromatography with ethyl acetate: petroleum ether = 1:2 to obtain 95 mg of methyl 2'-oxo-1'-(thien-3-ylmethyl)spiro[cyclopropane-1,3'-dihydroindol]-6'-carboxylate **(82-2),** with a yield of 66%. $^1$H NMR (400 MHz, DMSO-$d_6$) $\delta$ 7.65 (dd, $J$ = 7.8, 1.3 Hz, 1H), 7.55 (s, 1H), 7.51 (dd, $J$= 5.0, 3.0 Hz, 1H), 7.40 (d, $J$= 2.8 Hz, 1H), 7.19 (d, $J$= 7.8 Hz, 1H), 7.01 (d, $J$= 5.0 Hz, 1H), 5.03 (s, 2H), 3.83 (s, 3H), 1.78 (q, $J$= 3.9, 3.3 Hz, 2H), 1.68 (q, $J$ = 4.2, 3.8 Hz, 2H). LCMS (ESI) $m/z$ [M+H]$^+$: 314.1.

**Step 3: 2'-oxo-1'-(thien-3-ylmethyl)spiro[cyclopropane-1,3'-dihydroindol]-6'-carboxylic acid (82-3)**

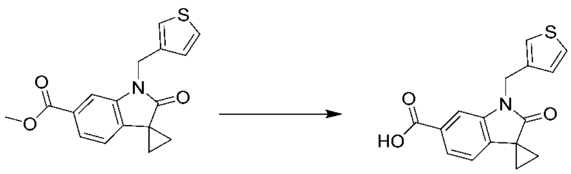

The preparation of 2'-oxo-1'-(thien-3-ylmethyl)spiro[cyclopropane-1,3'-dihydroindol]-6'-carboxylic acid (82-3) was the same as that of compound **1-5.** $^1$H NMR (400 MHz, DMSO-$d_6$) $\delta$ 7.62 (d, $J$ = 7.7 Hz, 1H), 7.60-7.45 (m, 2H), 7.40 (d, $J$ = 2.8 Hz, 1H), 7.14 (d, $J$ = 7.7 Hz, 1H), 7.00 (d, $J$ = 5.0 Hz, 1H), 5.01 (s, 2H), 1.75 (q, $J$ = 3.8, 3.2 Hz, 2H), 1.66 (t, $J$ = 3.8 Hz, 2H). LCMS (ESI) m/z [M+H]$^+$: 300.1.

**Step 4: N-(1H-indol-3-yl)-2'-oxo-1'-(thien-3-ylmethyl)spiro[cyclopropane-1,3'-dihydroindol]-6'-carboxamide (I-82)**

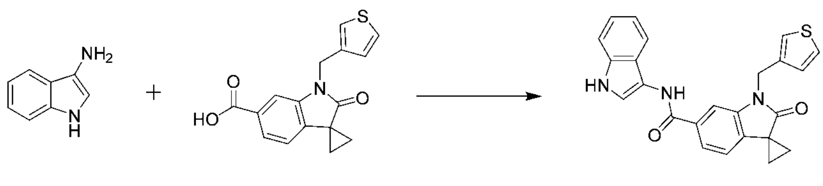

The preparation of N-(1H-indol-3-yl)-2'-oxo-1'-(thien-3-ylmethyl)spiro[cyclopropane-1,3'-dihydroindol]-6'-carboxamide **(I-82)** was the same as that of compound **I-1.** $^1$H NMR (400 MHz, DMSO-$d_6$) $\delta$ 10.92 (s, 1H), 10.06 (s, 1H), 7.82 (d, $J$= 8.0 Hz, 1H), 7.77 (d, $J$= 2.4 Hz, 1H), 7.73 (d, $J$= 7.8 Hz, 1H), 7.68 (s, 1H), 7.51 (dd, $J$ = 5.0, 2.8 Hz, 1H), 7.46 (d, $J$ = 2.8 Hz, 1H), 7.36 (d, $J$ = 8.1 Hz, 1H), 7.20 (d, $J$= 7.7 Hz, 1H), 7.11 (t, $J$= 7.5 Hz, 1H), 7.06 (d, $J$= 5.0 Hz, 1H), 7.01 (t, $J$= 7.5 Hz, 1H), 5.04 (s, 2H), 1.76 (q, $J$ = 3.9, 3.3 Hz, 2H), 1.66 (q, $J$ = 4.2, 3.7 Hz, 2H). LCMS (ESI) m/z [M+H]$^+$: 414.2.

**Example 83 1-((2-bromothien-3-yl)methyl)-N-(1H-indol-3-yl)-3,3-dimethyl-2-oxoindolin-6-carboxamide (I-83)**

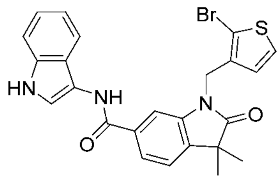

**Step 1: methyl 1-((2-bromothien-3-yl)methyl)-3,3-dimethyl-2-oxoindolin-6-carboxylate (83-1)**

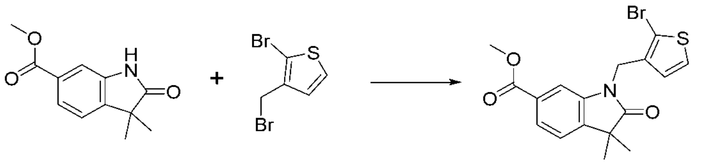

The preparation of methyl 1-((2-bromothien-3-yl)methyl)-3,3-dimethyl-2-oxoindolin-6-carboxylate **(83-1)** was the same as that of compound **82-2.** $^1$H NMR (400 MHz, DMSO-$d_6$) $\delta$ 7.69 (dd, *J*= 7.7, 1.5 Hz, 1H), 7.60-7.52 (m, 2H), 7.43 (d, *J*= 1.5 Hz, 1H), 6.76 (d, *J*= 5.7 Hz, 1H), 4.85 (s, 2H), 3.82 (s, 3H), 1.34 (s, 6H). LC-MS (ESI) *m*/*z* [M+H]$^+$: 394.0.

**Step 2: 1-((2-bromothien-3-yl)methyl)-3,3-dimethyl-2-oxoindolin-6-carboxylic acid (83-2)**

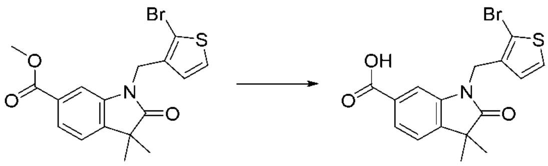

The preparation of 1-((2-bromothien-3-yl)methyl)-3,3-dimethyl-2-oxoindolin-6-carboxylic acid **(83-2)** was the same as that of compound **1-5.** $^1$H NMR (400 MHz, DMSO-$d_6$) $\delta$ 12.91 (s, 1H), 7.68 (dd, *J* = 7.7, 1.5 Hz, 1H), 7.57 (d, *J* = 5.6 Hz, 1H), 7.51 (d, *J* = 7.7 Hz, 1H), 7.39 (d, *J* = 1.4 Hz, 1H), 6.75 (d, *J* = 5.7 Hz, 1H), 4.84 (s, 2H), 1.34 (s, 6H). LC-MS (ESI) *m*/*z* [M-H]$^+$: 377.8.

**Step 3: 1-((2-bromothien-3-yl)methyl)-N-(1H-indol-3-yl)-3,3-dimethyl-2-oxoindolin-6-carboxamide (I-83)**

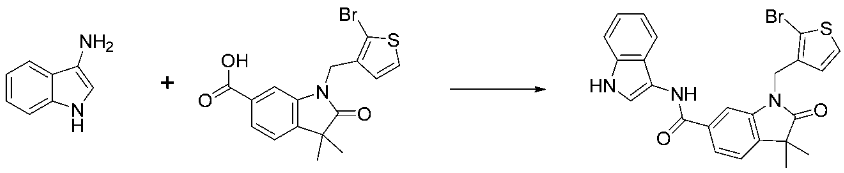

The preparation of 1-((2-bromothien-3-yl)methyl)-N-(1H-indol-3-yl)-3,3-dimethyl-2-oxoindolin-6-carboxamide **(I-83)** was the same as that of compound **I-1.** $^1$H NMR (400 MHz, $CDCl_3$) $\delta$ 8.11 (s, 1H), 7.98 (d, *J* = 2.5 Hz, 1H), 7.92 (s, 1H), 7.64 (dd, *J* = 7.7, 1.6 Hz, 1H), 7.56 (dd, *J* = 7.9, 1.1 Hz, 1H), 7.44 (d, *J*= 1.5 Hz, 1H), 7.45-7.35 (m, 1H), 7.33 (d, *J*= 7.7 Hz, 1H), 7.25-7.15 (m, 3H), 6.85 (d, *J*= 5.7 Hz, 1H), 4.93 (s, 2H), 1.45 (s, 6H). LC-MS (ESI) m/z [M+H]$^+$: 494.0.

**Example 84 1-((5-chlorothien-3-yl)methyl)-N-(1H-indol-3-yl)-3,3-dimethyl-2-oxoindolin-6-carboxamide (I-84)**

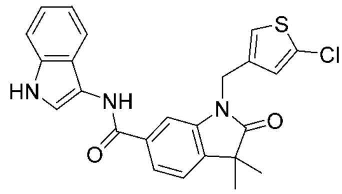

**Step 1: methyl 1-((5-chlorothien-3-yl)methyl)-3,3-dimethyl-2-oxoindolin-6-carboxylate (84-1)**

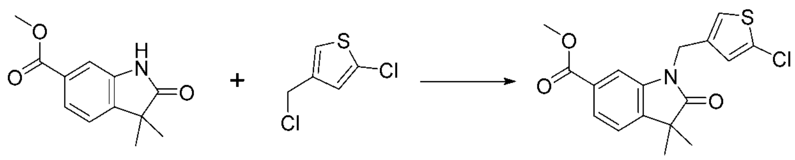

The preparation of methyl 1-((5-chlorothien-3-yl)methyl)-3,3-dimethyl-2-oxoindolin-6-carboxylate **(84-1)** was the same as that of compound **2-3.** $^1$H NMR (400 MHz, $CD_3OD$) $\delta$ 7.79 (dd, *J* = 7.7, 1.5 Hz, 1H), 7.55 (d, *J*= 1.5 Hz, 1H), 7.43 (dd, *J* = 7.7, 0.5 Hz, 1H), 7.16 (dd, *J* = 1.8, 1.0 Hz, 1H), 6.89 (d, *J*= 1.7 Hz, 1H), 4.89 (d, *J*= 1.0 Hz, 2H), 3.89 (s, 3H), 1.40 (s, 6H). LC-MS (ESI) *m/z* $[M+H]^+$ : 350.1.

**Step 2: 1-((5-chlorothien-3-yl)methyl)-3,3-dimethyl-2-oxoindolin-6-carboxylic acid (84-2)**

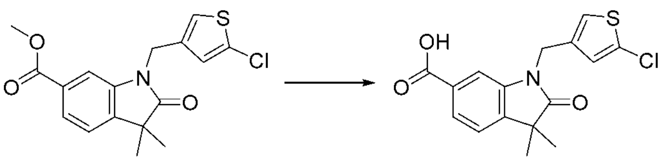

The preparation of 1-((5-chlorothien-3-yl)methyl)-3,3-dimethyl-2-oxoindolin-6-carboxylic acid **(84-2)** was the same as that of compound **1-5.** $^1$H NMR (400 MHz, DMSO-$d_6$) $\delta$ 12.91 (s, 1H), 7.68 (dd, *J*= 7.7, 2.9 Hz, 1H), 7.51 (dd, *J*= 7.9, 3.4 Hz, 1H), 7.48-7.42 (m, 1H), 7.26-7.22 (m, 1H), 6.99-6.95 (m, 1H), 4.88 (d, *J* = 3.2 Hz, 2H), 1.35 (d, *J* = 3.5 Hz, 6H).

**Step 3: 1-((5-chlorothien-3-yl)methyl)-N-(1H-indol-3-yl)-3,3-dimethyl-2-oxoindolin-6-carboxamide (I-84)**

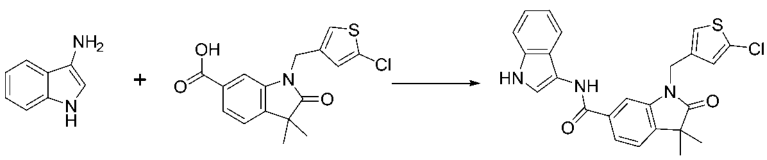

The preparation of 1-((5-chlorothien-3-yl)methyl)-N-(1H-indol-3-yl)-3,3-dimethyl-2-oxoindolin-6-carboxamide **(I-84)** was the same as that of compound **I-1.** $^1$H NMR (400 MHz, DMSO-$d_6$) $\delta$ 10.93 (s, 1H), 10.10 (s, 1H), 7.82 (d, *J*= *8.0* Hz, 1H), 7.76 (d, *J*= 2.5 Hz, 1H), 7.73 (dd, *J* = 7.7, 1.4 Hz, 1H), 7.59-7.51 (m, 2H), 7.36 (d, *J*= 8.2 Hz, 1H), 7.29 (d, *J*= 1.7 Hz, 1H), 7.15-7.06 (m, 1H), 7.04-6.98 (m, 2H), 4.90 (s, 2H), 1.37 (s, 6H). LC-MS (ESI) *m/z* $[M+H]^+$: 450.2.

**Example 85 N-(1H-indol-3-yl)-6,6-dimethyl-5-oxo-4-(thien-3-ylmethyl)-5,6-dihydro-4H-thieno[3,2-b]pyrrol-2-carboxamide (I-85)**

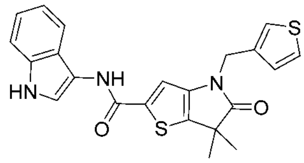

**Step 1: dimethyl 2-(3-nitrothien-2-yl)malonate (85-1)**

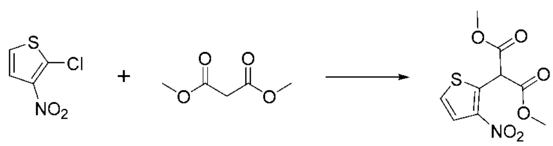

12.7 Grams of potassium tert-butoxide and 30 milliliters of ultra-dry dimethyl sulfoxide were added into a dry two-necked bottle, the mixture was stirred at room temperature for 30 minutes under argon protection, 15 grams of dimethyl malonate was added, the mixture was stirred at room temperature for 1 hour, 5.3 grams of 2-chloro-3-nitrothiophene was added, and the mixture was stirred at 100 °C for 1 hour. The reaction solution was acidified with 100 mL of 1 M hydrochloric acid, and extracted with ethyl acetate, and the organic phase was concentrated to dryness. The residue was purified by silica gel column chromatography (ethyl acetate/petroleum ether = 10/90) to obtain 8.3 g of dimethyl 2-(3-nitrothien-2-yl) malonate **(85-1)** as a yellow oil with a yield of 99%. $^1$H NMR (400 MHz, DMSO-$d_6$) $\delta$ 7.76 (d, $J$ = 5.7 Hz, 1H), 7.69 (d, $J$ = 5.7 Hz, 1H), 5.91 (s, 1H), 3.74 (s, 6H). LC-MS (ESI) $m/z$ [M+H]$^+$: 260.0.

**Step 2: 2-(3-nitrothien-2-yl)acetic acid (85-2)**

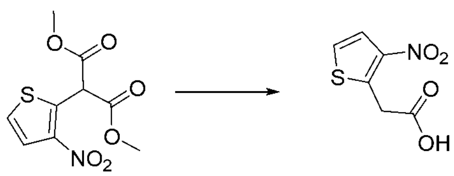

8.3 Grams of compound **85-1** and 5 M hydrochloric acid aqueous solution were added into a round bottom flask and the reaction mixture was stirred overnight at 110 °C. The reaction solution was added to 50 mL of water, and extracted with ethyl acetate, and the organic phase was concentrated to dryness to obtain 5.8 g of brown solid 2-(3-nitrothien-2-yl) acetic acid **(85-2)** with a yield of 96%. LC-MS (ESI) m/z [M-H]-: 186.0. LC-MS (ESI) $m/z$ [M-H]$^-$: 186.0.

**Step 3: methyl 2-(3-nitrothien-2-yl)acetate (85-3)**

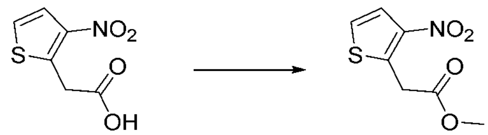

5.8 Grams of compound **85-2,** 1 milliliter of sulfuric acid, and 100 milliliters of methanol were added into a round bottom flask and the reaction mixture was stirred overnight at 65 °C. The reaction solution was concentrated to dryness, and the residue was purified by silica gel column chromatography (ethyl acetate/petroleum ether = 15/85) to obtain 6.1 g of

methyl 2-(3-nitrothien-2-yl)acetate **(85-3)** as a yellow oily substance with a yield of 99%. $^1$H NMR (400 MHz, $CD_3OD$) $\delta$ 7.63 (d, *J* = 5.7 Hz, 1H), 7.43 (d, *J* = 5.7 Hz, 1H), 4.25 (s, 2H), 3.72 (s, 3H). LC-MS (ESI) *m/z* $[M+H]^+$: 202.1.

**Step 4: methyl 2-(3-aminothien-2-yl)acetate (85-4)**

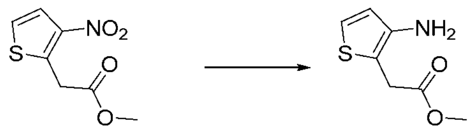

6.1 Grams of compound **85-3,** 5.1 grams of reduced iron powder, 40 milliliters of ethanol, and 40 milliliters of water were added into a round bottom flask, and the reaction mixture was stirred at room temperature under argon protection for 18 hours. The pH of the reaction solution was adjusted to neutral by adding saturated sodium bicarbonate aqueous solution, the mixture was filtered through diatomaceous earth, and the filtrate was extracted with ethyl acetate. The organic phase was concentrated to dryness to obtain 4.9 g of methyl 2-(3-aminothien-2-yl)acetate **(85-4)** as a brown oily substance with a yield of 93%. LC-MS (ESI) *m/z* $[M+H]^+$: 172.1.

**Step 5: 4,6-dihydro-5H-thieno[3,2-b]pyrrol-5-one (85-5)**

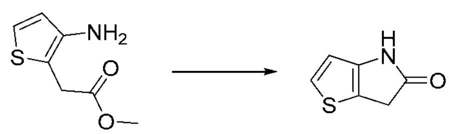

4.9 Grams of compound **85-4** and 50 milliliters of ultra-dry toluene were added into a dry two-necked bottle, and 35 milliliters of 2 M trimethylaluminum solution in toluene was added dropwise under argon protection at 0 °C. The reaction mixture was stirred at 0 °C for 2 hours. The reaction solution was quenched with saturated ammonium chloride aqueous solution, filtered through diatomaceous earth, and extracted with ethyl acetate, and the organic phase was concentrated to dryness. The residue was purified by silica gel column chromatography (ethyl acetate/petroleum ether = 25/75) to obtain 2.4 g of yellow solid 4,6-dihydro-5H-thieno[3,2-b]pyrrol-5-one **(85-5)** with a yield of 61%. $^1$H NMR (600 MHz, DMSO-$d_6$) $\delta$ 10.30 (s, 1H), 7.42 (d, *J* = 5.1 Hz, 1H), 6.77 (d, *J* = 5.0 Hz, 1H), 3.52 (s, 2H). LC-MS (ESI) *m/z* $[M+H]^+$: 140.0.

**Step 6: *t*-butyl 5-oxo-5,6-dihydro-4H-thieno[3,2-b]pyrrol-4-carboxylate (86-6)**

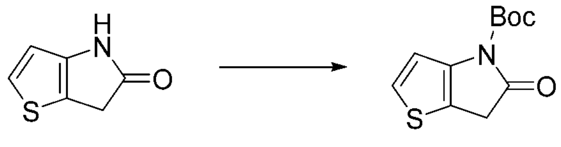

2.4 Grams of compound **85-5,** 9.4 grams of di-*tert*-butyl dicarbonate, 5.1 grams of sodium bicarbonate, and 40 milliliters of tetrahydrofuran were added into a round bottom flask, protected by argon, and the reaction mixture was stirred overnight at 60 °C. The reaction solution was concentrated to dryness, and the residue was purified by silica gel column chromatography (ethyl acetate/petroleum ether = 5/95) to obtain 3.4 g of t-butyl 5-oxo-5,6-dihydro-4H-thieno[3,2-b] pyrrol-4-carboxylate **(86-6)** as a red oily substance with a yield of 82%. $^1$H NMR (400 MHz, $CD_3OD$) $\delta$ 7.39 (dd, *J* = 5.2, 1.0 Hz, 1H), 7.20 (d, *J* = 5.2 Hz, 1H), 3.82-3.72 (m, 2H), 1.63 (s, 9H). LC-MS (ESI) *m/z* $[M+H]^+$: 262.0.

**Step 7: *t*-butyl 6,6-dimethyl-5-oxo-5,6-dihydro-4H-thieno[3,2-b]pyrrol-4-carboxylate (85-7)**

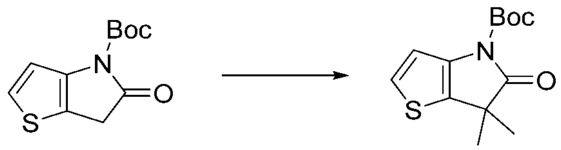

3.4 Grams of compound **85-6,** 4.0 grams of iodomethane, 9.3 grams of cesium carbonate, and 40 milliliters of ultra-dry N, N-dimethylformamide were added to a round bottom flask, and the reaction mixture was stirred at room temperature under argon protection for 1 hour. The reaction solution was added to 50 mL of water, and extracted with ethyl acetate, and the organic phase was concentrated to dryness. The residue was purified by silica gel column chromatography (ethyl acetate/dichloromethane = 5/95) to obtain 2.2 g of t-butyl 6,6-dimethyl-5-oxo-5,6-dihydro-4H-thieno[3,2-b]pyrrol-4-carboxylate **(85-7)** as a yellow oily substance, with a yield of 58%. $^1$H NMR (400 MHz, $CD_3OD$) $\delta$ 7.41 (d, *J* = 5.2 Hz, 1H), 7.22 (d, *J* = 5.2 Hz, 1H), 1.63 (s, 9H), 1.44 (s, 6H). LC-MS (ESI) *m*/*z* $[M+Na]^+$: 290.2.

**Step 8: *t*-butyl 2-bromo-6,6-dimethyl-5-oxo-5,6-dihydro-4H-thieno[3,2-b]pyrrol-4-carboxylate (85-8)**

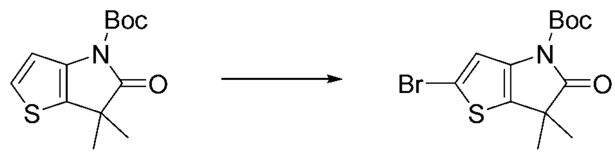

2.2 Grams of compound **85-7** and 30 milliliters of ultra-dry tetrahydrofuran were added into a dry two necked flask. 1.5 Grams of N-bromosuccinimide was added at 0 °C and the mixture was stirred in the dark for 2 hours at 0 °C. The reaction solution was concentrated to dryness, and the residue was purified by silica gel column chromatography (ethyl acetate/petroleum ether = 5/95) to obtain 2.3 g of t-butyl 2-bromo-6,6-dimethyl-5-oxo-5,6-dihydro-4H-thieno[3,2-b]pyrrol-4-carboxylate **(85-8)** as a purple oily substance with a yield of 80%. $^1$H NMR (400 MHz, $CD_3OD$) $\delta$ 7.27 (s, 1H), 1.62 (s, 9H), 1.42 (s, 6H).

**Step 9: 2-bromo-6,6-dimethyl-4,6-dihydro-5H-thieno[3,2-b]pyrrol-5-one (85-9)**

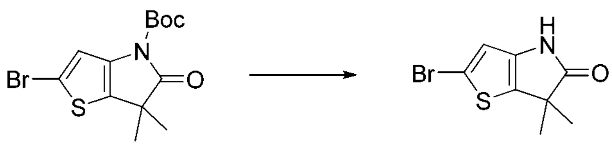

1 Gram of compound **85-8,** 2 milliliters of trifluoroacetic acid, and 10 milliliters of dichloromethane were added to a round bottom flask and the mixture was stirred at room temperature for 2 hours. Saturated sodium bicarbonate was added to the reaction solution to adjust the pH to neutral, the mixture was extracted with dichloromethane, the organic phase was concentrated to dryness, and the residue was purified by silica gel column chromatography (ethyl acetate/petroleum ether = 5/95) to obtain 698.0 mg of 2-bromo-6,6-dimethyl-4,6-dihydro-5H-thieno[3,2-b]pyrrol-5-one **(85-9)** as a purple solid, with a yield of 97%. $^1$H NMR (400 MHz, DMSO-*d*$_6$) $\delta$ 10.37 (s, 1H), 6.97 (s, 1H), 1.26 (s, 6H).

**Step 10: 2-cyano-6,6-dimethyl-4,6-dihydro-5H-thieno[3,2-b]pyrrol-5-one (85-10)**

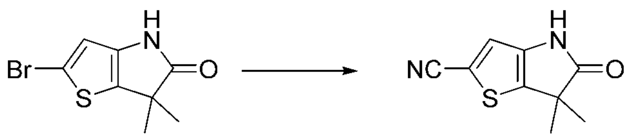

698 Milligrams of compound **85-9,** 666 milligrams of zinc cyanide, 328 milligrams of tetrakis(triphenylphosphine)

palladium, and 10 milliliters of N, N-dimethylformamide were added into a sealed tube, the mixture was stirred overnight at 180 °C under argon protection. When TLC detected that the raw materials was reacted completely, the reaction stopped. 20 mL of water was added to the reaction solution, the mixture was extracted with ethyl acetate, the organic phase was concentrated to dryness, and the residue was purified by silica gel column chromatography (ethyl acetate/petroleum ether = 10/90) to obtain 25.4 mg of 2-cyano-6,6-dimethyl-4,6-dihydro-5H-thieno[3,2-b]pyrrol-5-one **(85-10)** as a yellow solid, with a yield of 5%. $^1$H NMR (400 MHz, $CD_3OD$) $\delta$ 7.40 (s, 1H), 1.42 (s, 6H).

**Step 11: 6,6-dimethyl-5-oxo-4-(thien-3-ylmethyl)-5,6-dihydro-4H-thieno[3,2-b]pyrrol-2-carbonitrile (85-11)**

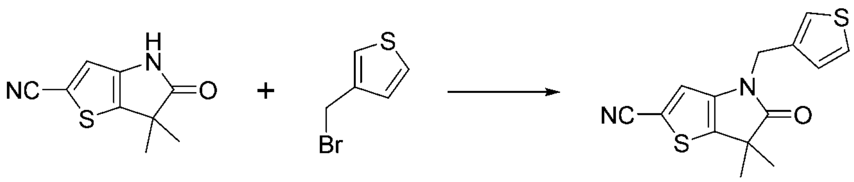

The preparation method of 6,6-dimethyl-5-oxo-4-(thien-3-ylmethyl)-5,6-dihydro-4H-thieno[3,2-b]pyrrol-2-carbonitrile (85-11) was the same as that of compound 2-3. $^1$H NMR (400 MHz, $CD_3OD$) $\delta$ 7.41 (s, 1H), 7.41-7.36 (m, 2H), 7.00 (dd, $J$ = 4.8, 1.5 Hz, 1H), 4.89 (s, 2H), 1.43 (s, 6H).

**Step 12: 6,6-dimethyl-5-oxo-4-(thien-3-ylmethyl)-5,6-dihydro-4H-thieno[3,2-b]pyrrol-2-carboxylic acid (85-12)**

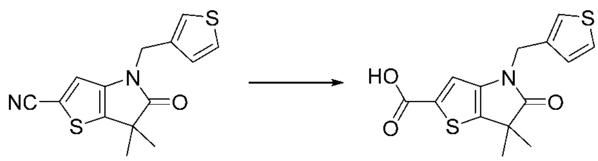

The preparation method of 6,6-dimethyl-5-oxo-4-(thien-3-ylmethyl)-5,6-dihydro-4H-thieno[3,2-b]pyrrol-2-carboxylic acid **(85-12)** was the same as that of compound **11-3.** $^1$H NMR (400 MHz, DMSO-$d_6$) $\delta$ 7.57-7.42 (m, 3H), 7.05-6.95 (m, 1H), 4.85 (s, 1H), 1.35 (d, $J$ = 6.6 Hz, 6H).

**Step 13: N-(1H-indol-3-yl)-6,6-dimethyl-5-oxo-4-(thien-3-ylmethyl)-5,6-dihydro-4H-thieno[3,2-b]pyrrol-2-carboxamide (I-85)**

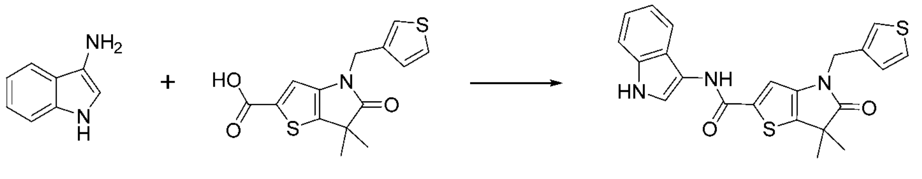

The preparation method of N-(1H-indol-3-yl)-6,6-dimethyl-5-oxo-4-(thien-3-ylmethyl)-5,6-dihydro-4H-thieno [3,2-b]pyrrol-2-carboxamide **(I-85)** was the same as that of compound **I-1.** $^1$H NMR (400 MHz, DMSO-$d_6$) $\delta$ 10.95 (s, 1H), 10.09 (s, 1H), 7.88 (s, 1H), 7.76 (d, $J$= 8.0 Hz, 1H), 7.67 (d, $J$= 2.5 Hz, 1H), 7.56 (dd, $J$= 5.0, 2.9 Hz, 1H), 7.41 (d, $J$= 2.9 Hz, 1H), 7.36 (d, $J$ = 8.1 Hz, 1H), 7.15-7.08 (m, 1H), 7.07-6.99 (m, 2H), 4.86 (s, 2H), 1.38 (s, 6H). LC-MS (ESI) $m/z$ [M+H]$^+$: 422.0.

**Example 86 4-(furan-3-ylmethyl)-N-(1H-indol-3-yl)-6,6-dimethyl-5-oxo-5,6-dihydro-4H-thieno[3,2-b]pyrrol-2-carboxamide (I-86)**

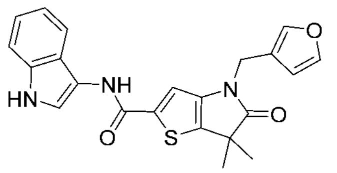

**Step 1: 4-(furan-3-ylmethyl)-6,6-dimethyl-5-oxo-5,6-dihydro-4H-thieno[3,2-b]pyrrol-2-carbonitrile (86-1)**

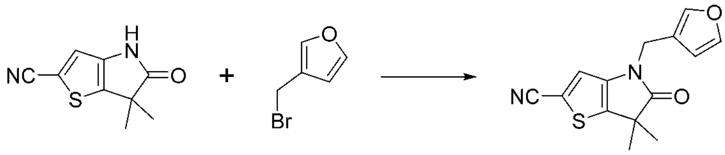

The preparation method of 4-(furan-3-ylmethyl)-6,6-dimethyl-5-oxo-5,6-dihydro-4H-thieno[3,2-b]pyrrol-2-carbonitrile **(86-1)** was the same as that of compound **2-3.** $^1$H NMR (400 MHz, $CD_3OD$) $\delta$ 7.63-7.56 (m, 1H), 7.52 (s, 1H), 7.49-7.43 (m, 1H), 6.35 (dd, *J*= 1.9, 0.9 Hz, 1H), 4.75 (s, 1H), 1.43 (s, 6H).

**Step 2: 4-(furan-3-ylmethyl)-6,6-dimethyl-5-oxo-5,6-dihydro-4H-thieno[3,2-b]pyrrol-2-carboxylic acid (86-2)**

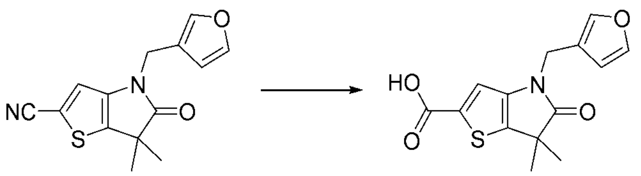

The preparation method of 4-(furan-3-ylmethyl)-6,6-dimethyl-5-oxo-5,6-dihydro-4H-thieno[3,2-b]pyrrol-2-carboxylic acid **(86-2)** was the same as that of compound **11-3.** LC-MS (ESI) *m/z* [M-H]$^+$: 290.1.

**Step 3: 4-(furan-3-ylmethyl)-N-(1H-indol-3-yl)-6,6-dimethyl-5-oxo-5,6-dihydro-4H-thieno[3,2-b]pyrrol-2-carboxamide (I-86)**

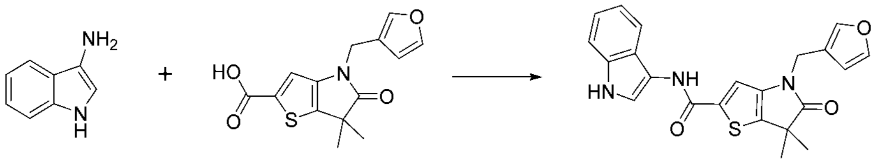

The preparation method of 4-(furan-3-ylmethyl)-N-(1H-indol-3-yl)-6,6-dimethyl-5-oxo-5,6-dihydro-4H-thieno [3,2-b]pyrrol-2-carboxamide **(I-86)** was the same as that of compound **I-1.** $^1$H NMR (400 MHz, DMSO-$d_6$) $\delta$ 10.98 (s, 1H), 10.14 (s, 1H), 7.96 (s, 1H), 7.79 (d, *J* = 8.0 Hz, 1H), 7.75 (s, 1H), 7.72-7.61 (m, 2H), 7.37 (d, *J*= 8.1 Hz, 1H), 7.16-7.08 (m, 1H), 7.06-6.98 (m, 1H), 6.48-6.40 (m, 1H), 4.71 (s, 2H), 1.36 (s, 6H). LC-MS (ESI) *m/z* [M+H]$^+$: 406.2.

**Example 87 4-benzyl-N-(1H-indol-3-yl)-6,6-dimethyl-5-oxo-5,6-dihydro-4H-thieno[3,2-b]pyrrol-2-carboxamide (I-87)**

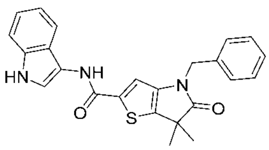

**Step 1: 4-benzyl-6,6-dimethyl-5-oxo-5,6-dihydro-4H-thieno[3,2-b]pyrrol-2-carbonitrile (87-1)**

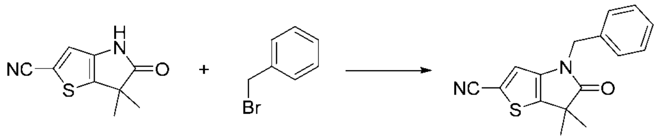

The preparation method of 4-benzyl-6,6-dimethyl-5-oxo-5,6-dihydro-4H-thieno[3,2-b]pyrrol-2-carbonitrile **(87-1)** was the same as that of compound **2-3.** $^1$H NMR (400 MHz, $CD_3OD$) $\delta$ 7.37-7.26 (m, 6H), 4.89 (s, 2H), 1.45 (s, 6H). LC-MS (ESI) *m/z* $[M+H]^+$: 283.1.

**Step 2: 4-benzyl-6,6-dimethyl-5-oxo-5,6-dihydro-4H-thieno[3,2-b]pyrrol-2-carboxylic acid (87-2)**

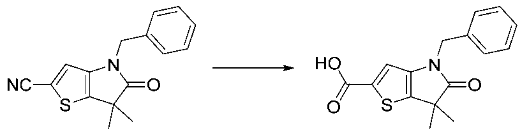

The preparation method of 4-benzyl-6,6-dimethyl-5-oxo-5,6-dihydro-4H-thieno[3,2-b]pyrrol-2-carbonitrile **(87-2)** was the same as that of compound **11-3.** LC-MS (ESI) m/z $[M-H]^-$: 299.8.

**Step 3: 4-benzyl-N-(1H-indol-3-yl)-6,6-dimethyl-5-oxo-5,6-dihydro-4H-thieno[3,2-b]pyrrol-2-carboxamide (I-87)**

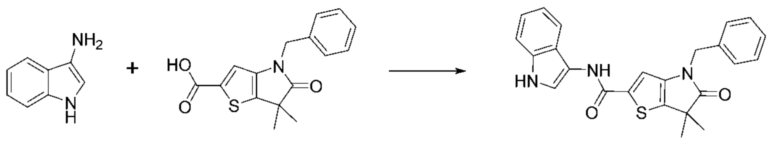

The preparation method of 4-benzyl-N-(1H-indol-3-yl)-6,6-dimethyl-5-oxo-5,6-dihydro-4H-thieno[3,2-b]pyrrol-2-carboxamide **(I-87)** was the same as that of compound **I-1.** $^1$H NMR (400 MHz, DMSO-$d_6$) $\delta$ 10.95 (s, 1H), 10.09 (s, 1H), 7.83 (s, 1H), 7.73 (d, *J*= 8.0 Hz, 1H), 7.65 (d, *J* = 2.5 Hz, 1H), 7.42-7.27 (m, 6H), 7.14-7.08 (m, 1H), 7.05-6.95 (m, 1H), 4.88 (s, 2H), 1.41 (s, 6H). LC-MS (ESI) *m/z* $[M+H]^+$: 416.2.

**Example 88 4-(3-fluoro-5-(trifluoromethyl)benzyl)-N-(1H-indol-3-yl)-6,6-dimethyl-5-oxo-5,6-dihydro-4H-thieno [3,2-b]pyrrol-2-carboxamide (I-88)**

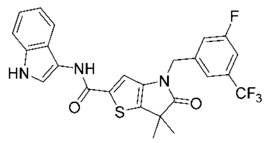

**Step 1: dimethyl 2-(5-(methoxycarbonyl)-3-nitrothien-2-yl)malonate (88-1)**

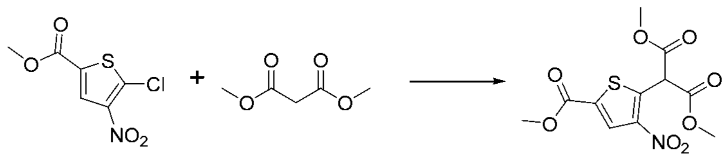

The preparation method of dimethyl 2-(5-(methoxycarbonyl)-3-nitrothien-2-yl)malonate **(88-1)** was the same as that of compound **85-1.** $^1$H NMR (400 MHz, DMSO-$d_6$) $\delta$ 8.20 (s, 1H), 6.01 (s, 1H), 3.88 (s, 3H), 3.76 (s, 6H).

**Step 2: methyl 5-(2-methoxy-2-oxoethyl)-4-nitrothien-2-carboxylate (88-2)**

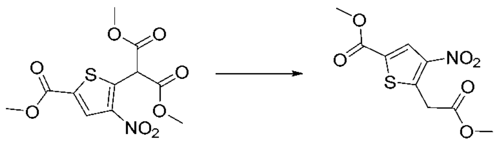

5.3 Grams of compound **88-1** and glacial acetic acid were added into a round bottom flask and the mixture was stirred at 110 °C for 22 hours. The reaction solution was concentrated to dryness, and added with ethyl acetate, the pH was adjusted to neutral with saturated sodium bicarbonate aqueous solution, and the mixture was extracted with ethyl acetate. The organic phase was concentrated to dryness. The residue was purified by silica gel column chromatography (ethyl acetate/petroleum ether = 5/95) to obtain 1.8 g of methyl 5-(2-methoxy-2-oxoethyl)-4-nitrothien-2-carboxylate **(88-2)** as a yellow solid, with a yield of 42%. $^1$H NMR (400 MHz, DMSO-$d_6$) $\delta$ 8.18 (s, 1H), 4.44 (s, 2H), 3.88 (s, 3H), 3.67 (s, 3H).

**Step 3: methyl 5-(2-methoxy-2-oxoethyl)-4-aminothien-2-carboxylate (88-3)**

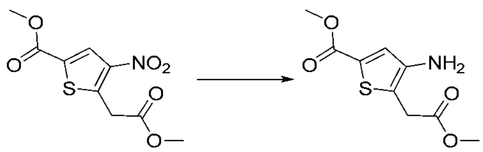

The preparation method of methyl 5-(2-methoxy-2-oxoethyl)-4-aminothien-2-carboxylate **(88-3)** was the same as that of compound **85-4.** $^1$H NMR (400 MHz, DMSO-$d_6$) $\delta$ 7.19 (s, 1H), 4.97 (s, 2H), 3.75 (s, 3H), 3.74 (s, 2H), 3.63 (s, 3H).

**Step 4: methyl 5-oxo-5,6-dihydro-4H-thieno[3,2-b]pyrrol-2-carboxylate (88-4)**

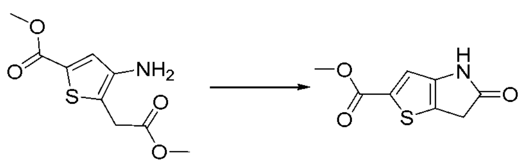

The preparation method of methyl 6-dihydro-4H-thieno[3,2-b]pyrrol-2-carboxylate **(88-4)** was the same as that of compound **85-5.** $^1$H NMR (400 MHz, DMSO-$d_6$) $\delta$ 10.45 (s, 1H), 7.33 (s, 1H), 3.81 (s, 3H), 3.65 (s, 2H). LC-MS (ESI) *m/z* [M+H]$^+$: 198.1.

**Step 5: methyl 4-Boc-5-oxo-5,6-dihydro-4H-thieno[3,2-b]pyrrol-2-carboxylate (88-5)**

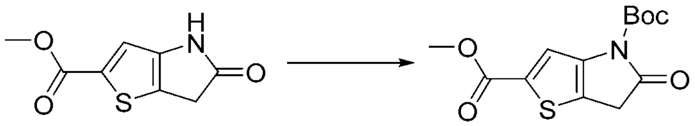

The preparation method of methyl 4-Boc-5-oxo-5,6-dihydro-4H-thieno[3,2-b]pyrrol-2-carboxylate **(88-5)** was the same as that of compound **85-6.** $^1$H NMR (400 MHz, DMSO-$d_6$) $\delta$ 7.73 (s, 1H), 3.90 (s, 2H), 3.83 (s, 3H), 1.55 (s, 9H).

**Step 6: methyl 4-Boc-6,6-dimethyl-5-oxo-5,6-dihydro-4H-thieno[3,2-b]pyrrol-2-carboxylate (88-6)**

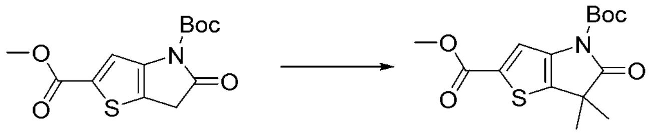

The preparation method of methyl 4-Boc-6,6-dimethyl-5-oxo-5,6-dihydro-4H-thieno[3,2-b]pyrrol-2-carboxylate **(88-6)** was the same as that of compound **85-7.** $^1$H NMR (400 MHz, $CD_3OD$) $\delta$ 7.86 (s, 1H), 3.89 (s, 3H), 1.64 (s, 9H), 1.47 (s, 6H).

**Step 7: methyl 6,6-dimethyl-5-oxo-5,6-dihydro-4H-thieno[3,2-b]pyrrol-2-carboxylate (88-7)**

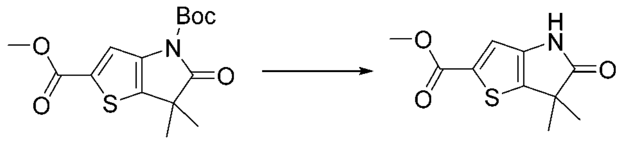

The preparation method of methyl 6,6-dimethyl-5-oxo-5,6-dihydro-4H-thieno[3,2-b]pyrrol-2-carboxylate **(88-7)** was the same as that of compound **85-8.** $^1$H NMR (400 MHz, $CDCl_3$) $\delta$ 8.51 (s, 1H), 7.43 (s, 1H), 3.88 (s, 3H), 1.45 (s, 6H). LC-MS (ESI) *m/z* $[M+H]^+$: 226.1.

**Step 8: methyl 4-(3-fluoro-5-(trifluoromethyl)benzyl)-6,6-dimethyl-5-oxo-5,6-dihydro-4H-thieno[3,2-b]pyrrol-2-carboxylate (88-8)**

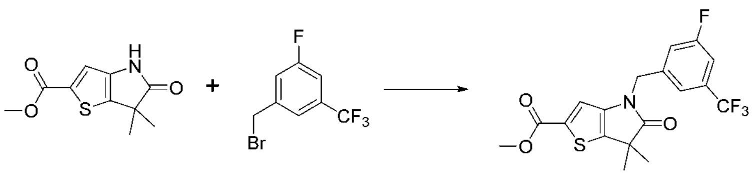

The preparation method of methyl 4-(3-fluoro-5-(trifluoromethyl)benzyl)-6,6-dimethyl-5-oxo-5,6-dihydro-4H-thieno[3,2-b]pyrrol-2-carboxylate **(88-8)** was the same as that of compound 2-3. $^1$H NMR (400 MHz, $CD_3OD$) $\delta$ 7.48-7.44 (m, 2H), 7.44-7.34 (m, 2H), 3.84 (s, 3H), 1.46 (s, 6H).

**Step 9: 4-(3-fluoro-5-(trifluoromethyl)benzyl)-6,6-dimethyl-5-oxo-5,6-dihydro-4H-thieno[3,2-b]pyrrol-2-carboxylic acid (88-9)**

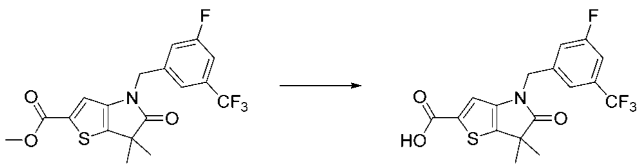

The preparation method of 4-(3-fluoro-5-(trifluoromethyl)benzyl)-6,6-dimethyl-5-oxo-5,6-dihydro-4H-thieno[3,2-b]pyrrol-2-carboxylic acid **(88-9)** was the same as that of compound **1-5.** LC-MS (ESI) *m/z* $[M-H]^+$: 386.1.

**Step 10: 4-(3-fluoro-5-(trifluoromethyl)benzyl)-N-(1H-indol-3-yl)-6,6-dimethyl-5-oxo-5,6-dihydro-4H-thieno[3,2-b]pyrrol-2-carboxamide (I-88)**

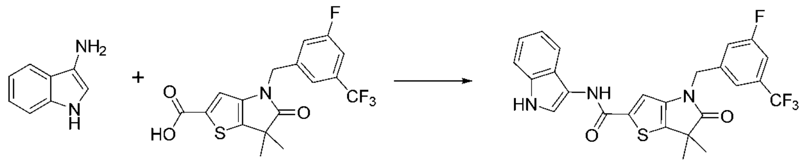

The preparation method of 4-(3-fluoro-5-(trifluoromethyl)benzyl)-N-(1H-indol-3-yl)-6,6-dimethyl-5-oxo-5,6-dihydro-4H-thieno[3,2-b]pyrrol-2-carboxamide **(I-88)** was the same as that of compound **I-1.** $^1H$ NMR (400 MHz, DMSO-$d_6$) $\delta$ 10.95 (s, 1H), 10.07 (s, 1H), 7.84 (s, 1H), 7.73 (d, *J*= 8.0 Hz, 1H), 7.69-7.64 (m, 2H), 7.52 (s, 1H), 7.45 (d, *J* = 9.1 Hz, 1H), 7.36 (dt, *J* = 8.2, 0.9 Hz, 1H), 7.14-7.04 (m 1H), 7.03-6.99 (m, 1H), 5.02 (s, 2H), 1.42 (s, 6H). LC-MS (ESI) *m/z* $[M+H]^+$: 502.2.

**Example 89 1-((4-chlorothien-3-yl)methyl)-N-(1H-indol-3-yl)-3,3-dimethyl-2-oxoindolin-6-carboxamide (I-89)**

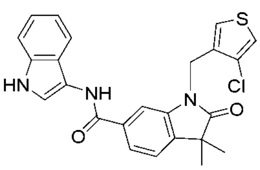

**Step 1: methyl 1-((4-chlorothien-3-yl)methyl)-3,3-dimethyl-2-oxoindolin-6-carboxylate (89-1)**

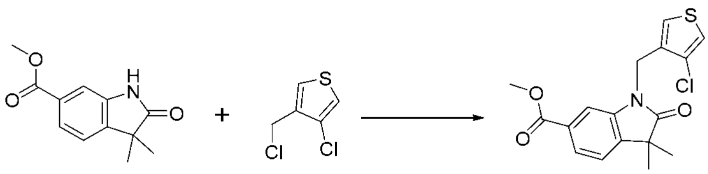

The preparation method of methyl 1-((4-chlorothien-3-yl)methyl)-3,3-dimethyl-2-oxoindolin-6-carboxylate **(89-1)** was the same as that of compound **2-3.** $^1H$ NMR (400 MHz, $CD_3OD$) $\delta$ 7.42 (s, 1H), 7.18-7.13 (m, 1H), 6.83 (d, *J* = 1.8 Hz, 1H), 4.75 (s, 2H), 3.80 (s, 3H), 1.37 (s, 6H). LC-MS (ESI) *m/z* $[M+H]^+$: 356.2.

**Step 2: 1-((4-chlorothien-3-yl)methyl)-3,3-dimethyl-2-oxoindolin-6-carboxylic acid (89-2)**

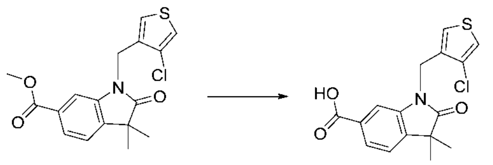

The preparation method of 1-((4-chlorothien-3-yl)methyl)-3,3-dimethyl-2-oxoindolin-6-carboxylic acid **(89-2)** was the same as that of compound **1-5.** LC-MS (ESI) *m/z* $[M-H]^+$: 340.1.

**Step 3: 1-((4-chlorothien-3-yl)methyl)-N-(1H-indol-3-yl)-3,3-dimethyl-2-oxoindolin-6-carboxamide (I-89)**

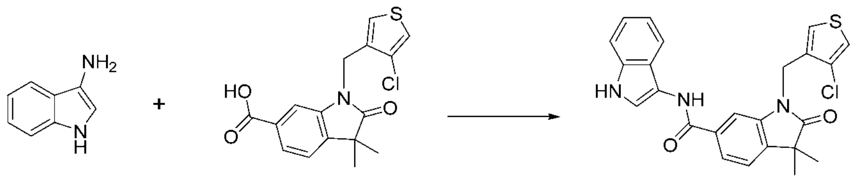

The preparation method of 1-((4-chlorothien-3-yl)methyl)-N-(1H-indol-3-yl)-3,3-dimethyl-2-oxoindolin-6-carboxamide **(I-89)** was the same as that of compound **I-1.** $^1$H NMR (400 MHz, DMSO-$d_6$) $\delta$ 10.96 (s, 1H), 10.09 (s, 1H), 7.87 (s, 1H), 7.76 (d, *J* = 8.0 Hz, 1H), 7.67 (d, *J*= 2.5 Hz, 1H), 7.36 (d, *J*= 8.1 Hz, 1H), 7.27 (d, *J*= 1.8 Hz, 1H), 7.12 (ddd, *J*= 8.2, 6.9, 1.2 Hz, 1H), 7.06 (d, *J*= 1.8 Hz, 1H), 7.02 (ddd, *J*= 7.9, 6.9, 1.0 Hz, 1H), 4.79 (s, 2H), 1.39 (s, 6H). LC-MS (ESI) *m/z* $[M-H]^+$: 456.0.

**Example 90 N-(1H-indol-3-yl)-6,6-dimethyl-5-oxo-4-((5-(trifluoromethyl)thien-3-yl)methyl)-5,6-dihydro-4H-thieno[3,2-b]pyrrol-2-carboxamide (I-90)**

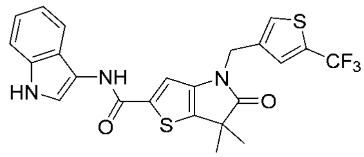

**Step 1: methyl 4-((5-bromothien-3-yl)methyl)-6,6-dimethyl-5-oxo-5,6-dihydro-4H-thieno[3,2-b]pyrrol-2-carboxylate (90-1)**

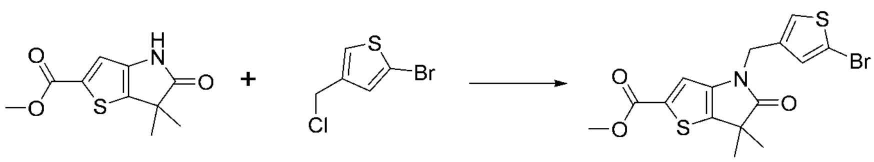

The preparation method of methyl 4-((5-bromothien-3-yl)methyl)-6,6-dimethyl-5-oxo-5,6-dihydro-4H-thieno[3,2-b]pyrrol-2-carboxylate **(90-1)** was the same as that of compound **2-3.** $^1$H NMR (400 MHz, $CD_3OD$) $\delta$ 7.45 (s, 1H), 7.31 (dd, *J*= 1.7, 0.9 Hz, 1H), 6.99 (d, *J*= *1.7* Hz, 1H), 4.81 (s, 2H), 3.83 (s, 3H), 1.41 (s, 6H). LC-MS (ESI) *m/z* $[M+H]^+$: 400.0.

**Step 2: methyl 4-((5-trifluoromethylthien-3-yl)methyl)-6,6-dimethyl-5-oxo-5,6-dihydro-4H-thieno[3,2-b]pyrrol-2-carboxylate (90-2)**

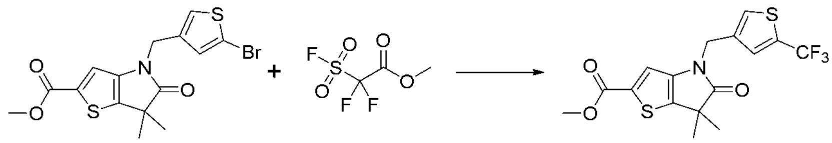

74 mg of Compound **90-1,** 355 mg of methyl fluorosulfonyldifluoroacetate, 352.1 mg of cuprous iodide, and 1 mL of DMF were added into a round bottom flask, and the mixture was stirred overnight at 130 °C under argon protection. The reaction solution was extracted with ethyl acetate, and the organic phase was concentrated to dryness. The residue was purified by preparative thin layer chromatography using silica gel (ethyl acetate/petroleum ether = 1/5) to obtain 20.0 mg of methyl 4-((5-trifluoromethylthien-3-yl)methyl)-6,6-dimethyl-5-oxo-5,6-dihydro-4H-thieno[3,2-b]pyrrol-2-carboxylate **(90-2)** as a yellow solid with a yield of 28%. $^1$H NMR (400 MHz, $CD_3OD$) $\delta$ 7.66 (d, *J* = 1.5 Hz, 1H), 7.51 (s, 1H), 7.48-7.45 (m, 1H), 4.91 (s, 2H), 3.84 (s, 3H), 1.43 (s, 6H).

**Step 3: 4-((5-trifluoromethylthien-3-yl)methyl)-6,6-dimethyl-5-oxo-5,6-dihydro-4H-thieno[3,2-b]pyrrol-2-carboxylic acid (90-3)**

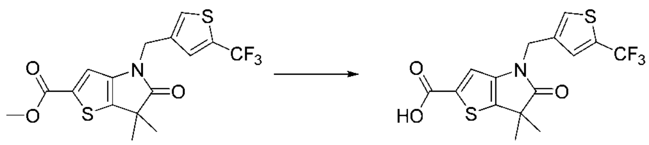

The preparation method of 4-((5-trifluoromethylthien-3-yl)methyl)-6,6-dimethyl-5-oxo-5,6-dihydro-4H-thieno [3,2-b]pyrrol-2-carboxylic acid **(90-3)** was the same as that of compound **1-5.** LC-MS (ESI) m/z [M-H]$^+$: 374.0.

**Step 4: N-(1H-indol-3-yl)-6,6-dimethyl-5-oxo-4-((5-(trifluoromethyl)thien-3-yl)methyl)-5,6-dihydro-4H-thieno [3,2-b]pyrrol-2-carboxamide (I-90)**

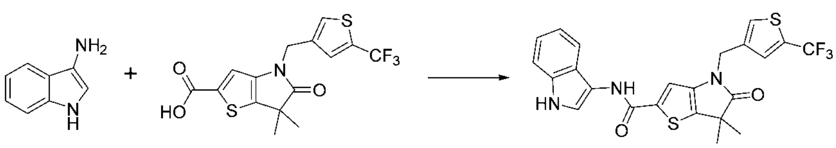

The preparation method of N-(1H-indol-3-yl)-6,6-dimethyl-5-oxo-4-((5-(trifluoromethyl)thien-3-yl)methyl)-5,6-dihydro-4H-thieno[3,2-b]pyrrol-2-carboxamide **(I-90)** was the same as that of compound **I-1.** $^1$H NMR (400 MHz, DMSO-$d_6$) $\delta$ 10.96 (s, 1H), 10.08 (s, 1H), 7.89 (s, 1H), 7.79-7.70 (m, 2H), 7.68-7.64 (m, 2H), 7.39-7.33 (m, 1H), 7.15-7.06 (m, 1H), 7.04-6.96 (m, 1H), 4.90 (s, 2H), 1.40 (s, 6H). LC-MS (ESI) *m/z* [M+H]$^+$: 490.2.

**Example 91 4-((5-chlorofuran-3-yl)methyl)-N-(1H-indol-3-yl)-6,6-dimethyl-5-oxo-5,6-dihydro-4H-thieno[3,2-b] pyrrol-2-carboxamide (I-91)**

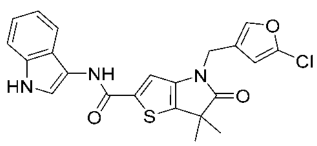

**Step 1: methyl 4-((5-chlorofuran-3-yl)methyl)-6,6-dimethyl-5-oxo-5,6-dihydro-4H-thieno[3,2-b]pyrrol-2-carboxylate (91-1)**

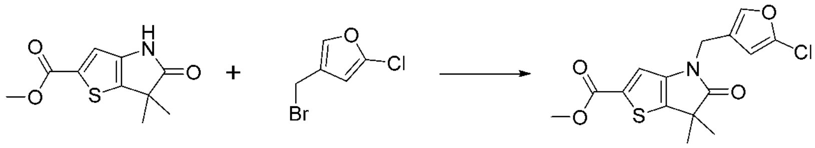

The preparation method of methyl 4-((5-chlorofuran-3-yl)methyl)-6,6-dimethyl-5-oxo-5,6-dihydro-4H-thieno [3,2-b]pyrrol-2-carboxylate **(91-1)** was the same as that of compound **2-3.** $^1$H NMR (400 MHz, $CD_3OD$) $\delta$ 7.55-7.50 (m, 2H), 6.20 (d, *J* = 1.1 Hz, 1H), 4.70 (d, *J* = 1.0 Hz, 2H), 3.85 (s, 3H), 1.40 (s, 6H). LC-MS (ESI) *m/z* $[M+H]^+$: 340.0.

**Step 2: 4-((5-chlorofuran-3-yl)methyl)-6,6-dimethyl-5-oxo-5,6-dihydro-4H-thieno[3,2-b]pyrrol-2-carboxylic acid (91-2)**

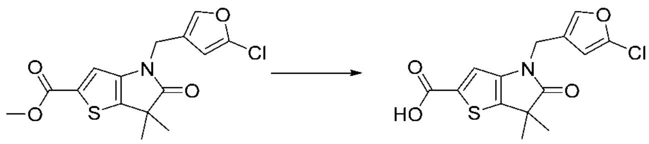

The preparation method of 4-((5-chlorofuran-3-yl)methyl)-6,6-dimethyl-5-oxo-5,6-dihydro-4H-thieno[3,2-b]pyrrol-2-carboxylic acid **(91-2)** was the same as that of compound **1-5.** $^1$H NMR (400 MHz, $CDCl_3$) $\delta$ 9.92 (s, 1H), 7.39 (s, 1H), 7.33 (s, 1H), 6.11 (s, 1H), 4.66 (s, 2H), 1.46 (s, 6H). LC-MS (ESI) *m/z* $[M-H]^-$: 324.0.

**Step 3: 4-((5-chlorofuran-3-yl)methyl)-N-(1H-indol-3-yl)-6,6-dimethyl-5-oxo-5,6-dihydro-4H-thieno[3,2-b]pyrrol-2-carboxamide (I-91)**

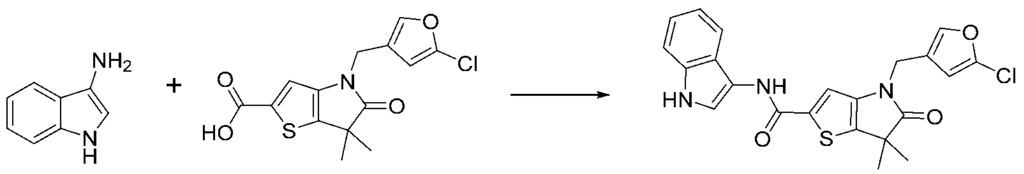

The preparation method of 4-((5-chlorofuran-3-yl)methyl)-N-(1H-indol-3-yl)-6,6-dimethyl-5-oxo-5,6-dihydro-4H-thieno[3,2-b]pyrrol-2-carboxamide **(I-91)** was the same as that of compound **I-1.** $^1$H NMR (400 MHz, DMSO-$d_6$) $\delta$ 10.96 (s, 1H), 10.10 (s, 1H), 7.88 (s, 1H), 7.77 (d, *J* = 8.0 Hz, 1H), 7.73 (s, 1H), 7.68 (d, *J* = 2.5 Hz, 1H), 7.37 (d, *J* = 8.1 Hz, 1H), 7.15-7.09 (m, 1H), 7.06-7.00 (m, 1H), 6.46 (d, *J* = 1.2 Hz, 1H), 4.69 (s, 2H), 1.37 (s, 6H). LC-MS (ESI) *m/z* $[M+H]^+$: 440.1.

**Example 92 1-(benzo[d][1,3]dihydroxyl-5-ylmethyl)-N-(1H-indol-3-yl)-3,3-dimethyl-2-oxoindol-6-carboxamide (I-92)**

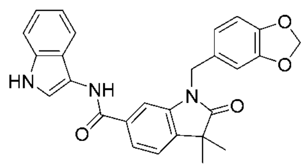

**Step 1: methyl 1-(benzo[d][1,3]dihydroxyl-5-ylmethyl)-3,3-dimethyl-2-oxoindolin-6-carboxylate (92-1)**

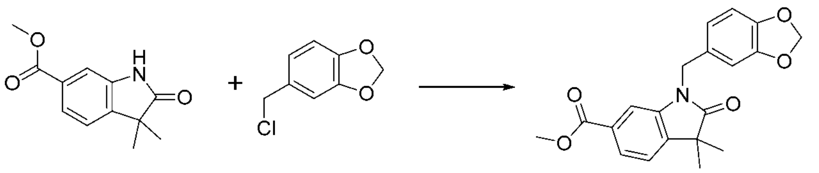

The preparation method of methyl 1-(benzo[d][1,3]dihydroxyl-5-ylmethyl)-3,3-dimethyl-2-oxoindolin-6-carboxylate **(92-1)** was the same as that of compound **2-3.** $^1$H NMR (400 MHz, $CD_3OD$) $\delta$ 7.70 (dd, *J*= 7.7, 1.5 Hz, 1H), 7.45 (d, *J*= 1.4 Hz, 1H), 7.34 (d, *J*= 7.7 Hz, 1H), 6.76 (dd, *J* = 7.9, 1.7 Hz, 1H), 6.72-6.66 (m, 2H), 5.82 (s, 2H), 4.81 (s, 2H), 3.81 (s, 3H), 1.37 (s, 6H). LC-MS (ESI) *m/z* [M+H]$^+$: 354.1.

**Step 2: 1-(benzo[d][1,3]dihydroxyl-5-ylmethyl)-3,3-dimethyl-2-oxoindolin-6-carboxylic acid (92-2)**

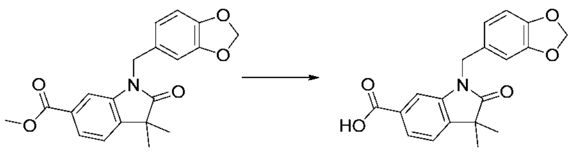

The preparation method of 1-(benzo[d][1,3]dihydroxyl-5-ylmethyl)-3,3-dimethyl-2-oxoindolin-6-carboxylic acid **(92-2)** was the same as that of compound **1-5.** $^1$H NMR (400 MHz, DMSO-$d_6$) $\delta$ 12.95 (s, 1H), 7.66 (dd, *J* = 7.7, 1.4 Hz, 1H), 7.50 (d, *J*= 7.7 Hz, 1H), 7.39 (d, *J* = 1.4 Hz, 1H), 6.87 (d, *J* = 8.0 Hz, 1H), 6.83 (d, *J*= 1.7 Hz, 1H), 6.75 (dd, *J* = 8.0, 1.8 Hz, 1H), 5.98 (s, 2H), 4.86 (s, 2H), 1.35 (s, 6H). LC-MS (ESI) m/z [M-H]$^+$: 338.1.

**Step 3: 1-(benzo[d][1,3]dihydroxyl-5-ylmethyl)-N-(1H-indol-3-yl)-3,3-dimethyl-2-oxoindol-6-carboxamide (I-92)**

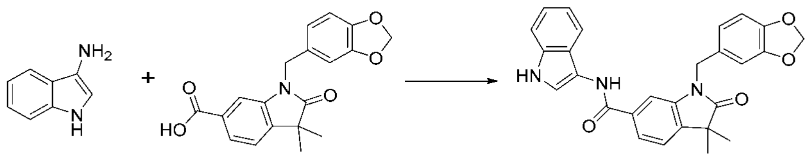

The preparation method of 1-(benzo[d][1,3]dihydroxyl-5-ylmethyl)-N-(1H-indol-3-yl)-3,3-dimethyl-2-oxoindol-6-carboxamide **(I-92)** was the same as that of compound **I-1.** $^1$H NMR (400 MHz, DMSO-$d_6$) $\delta$ 10.91 (s, 1H), 10.07 (s, 1H), 7.81 (d, *J* = 8.0 Hz, 1H), 7.76 (d, *J* = 2.5 Hz, 1H), 7.71 (dd, *J*= 7.7, 1.5 Hz, 1H), 7.53 (d, *J*= 7.6 Hz, 1H), 7.47 (d, *J* = 1.5 Hz, 1H), 7.36 (d, *J* = 8.1 Hz, 1H), 7.15-7.06 (m, 1H), 7.03-6.96 (m, 1H), 6.90-6.85 (m, 2H), 6.80 (dd, *J* = 8.0, 1.7 Hz, 1H), 5.98 (s, 2H), 4.88 (s, 2H), 1.37 (s, 6H). LC-MS (ESI) *m/z* [M+H]$^+$: 454.3.

**Example 93 1-(benzofuran-5-ylmethyl)-N-(1H-indol-3-yl)-3,3-dimethyl-2-oxoindolin-6-carboxamide (I-93)**

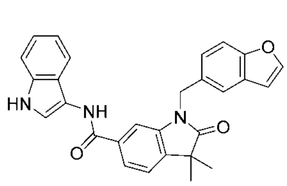

**Step 1: methyl 1-(benzofuran-5-ylmethyl)-3,3-dimethyl-2-oxoindolin-6-carboxylate (93-1)**

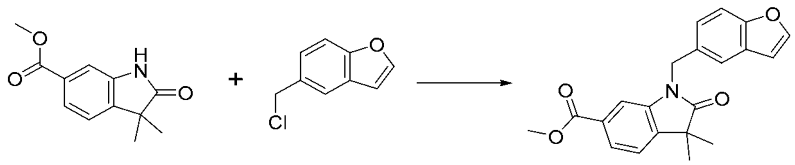

The preparation method of methyl 1-(benzofuran-5-ylmethyl)-3,3-dimethyl-2-oxoindolin-6-carboxylate **(93-1)** was the same as that of compound **2-3.** $^1$H NMR (400 MHz, DMSO-$d_6$) $\delta$ 7.98 (d, $J$ = 2.2 Hz, 1H), 7.68 (dd, $J$ = 7.8, 1.5 Hz, 1H), 7.59-7.53 (m, 3H), 7.40 (d, $J$ = 1.4 Hz, 1H), 7.24 (dd, $J$ = 8.6, 1.8 Hz, 1H), 6.94 (dd, $J$ = 2.2, 1.0 Hz, 1H), 5.06 (s, 2H), 3.79 (s, 3H), 1.38 (s, 6H). LC-MS (ESI) $m/z$ [M+H]$^+$: 350.3.

**Step 2: 1-(benzofuran-5-ylmethyl)-3,3-dimethyl-2-oxoindolin-6-carboxylic acid (93-2)**

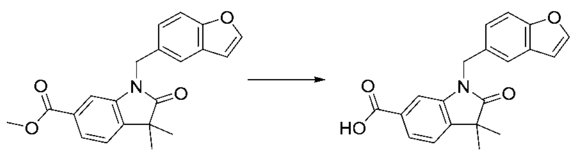

The preparation method of 1-(benzofuran-5-ylmethyl)-3,3-dimethyl-2-oxoindolin-6-carboxylic acid **(93-2)** was the same as that of compound **1-5.** $^1$H NMR (400 MHz, DMSO-$d_6$) $\delta$ 12.97 (s, 1H), 7.98 (d, $J$ = 2.2 Hz, 1H), 7.65 (dd, $J$ = 7.7, 1.4 Hz, 1H), 7.57 (d, $J$ = 8.5 Hz, 1H), 7.54 (d, $J$= 1.8 Hz, 1H), 7.50 (d, $J$= 7.7 Hz, 1H), 7.38 (d, $J$ = 1.4 Hz, 1H), 7.24 (dd, $J$ = 8.5, 1.8 Hz, 1H), 6.94 (d, $J$ = 2.3 Hz, 1H), 5.05 (s, 2H), 1.38 (s, 6H). LC-MS (ESI) $m/z$ [M-H]$^+$: 334.2.

**Step 3: 1-(benzofuran-5-ylmethyl)-N-(1H-indol-3-yl)-3,3-dimethyl-2-oxoindolin-6-carboxamide (I-93)**

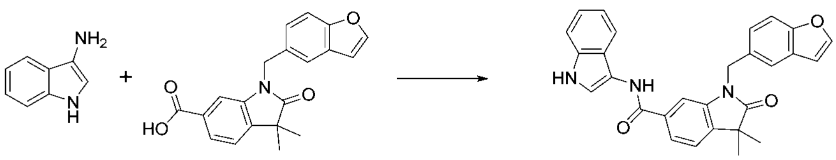

The preparation method of 1-(benzofuran-5-ylmethyl)-N-(1H-indol-3-yl)-3,3-dimethyl-2-oxoindolin-6-carboxamide **(I-93)** was the same as that of compound **I-1.** $^1$H NMR (400 MHz, DMSO-$d_6$) $\delta$ 10.89 (s, 1H), 10.06 (s, 1H), 7.97 (d, $J$= 2.2 Hz, 1H), 7.79 (d, $J$ = 8.0 Hz, 1H), 7.73 (d, $J$ = 2.4 Hz, 1H), 7.71 (dd, $J$= 7.7, 1.5 Hz, 1H), 7.61 (d, $J$= 1.8 Hz, 1H), 7.58 (d, $J$= 8.5 Hz, 1H), 7.54 (d, $J$= 7.7 Hz, 1H), 7.49 (d, $J$= 1.5 Hz, 1H), 7.35 (d, $J$= 8.1 Hz, 1H), 7.29 (dd, $J$ = 8.5, 1.8 Hz, 1H), 7.14-7.06 (m, 1.2 Hz, 1H), 7.05-6.95 (m, 1H), 6.94 (dd, $J$ = 2.2, 0.9 Hz, 1H), 5.07 (s, 2H), 1.40 (s, 6H). LC-MS (ESI) m/z [M+H]$^+$: 450.0.

**Example 94 1-(indol-5-ylmethyl)-N-(1H-indol-3-yl)-3,3-dimethyl-2-oxoindolin-6-carboxamide (I-94)**

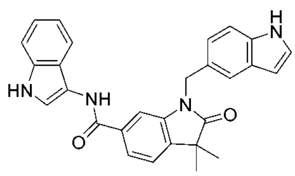

**Step 1: *t*-butyl 5-((6-(methoxycarbonyl)-3,3-dimethyl-2-oxoindol-1-yl)methyl)-1H-indol-1-carboxylate (94-1)**

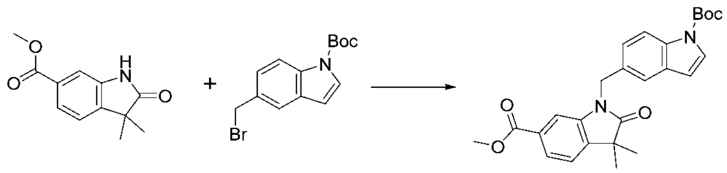

The preparation method of *t*-butyl 5-((6-(methoxycarbonyl)-3,3-dimethyl-2-oxoindol-1-yl)methyl)-1H-indol-1-carboxylate **(94-1)** was the same as that of compound **2-3.** $^1H$ NMR (400 MHz, $CD_3OD$) $\delta$ 8.06 (d, *J* = 8.6 Hz, 1H), 7.74 (dd, *J* = 7.7, 1.5 Hz, 1H), 7.58 (d, *J* = 3.8 Hz, 1H), 7.48 (d, *J*= 1.5 Hz, 2H), 7.40 (d, *J*= *7.7* Hz, 1H), 7.23 (dd, *J*= 8.6, 1.8 Hz, 1H), 6.58-6.52 (m, 1H), 5.04 (s, 2H), 3.82 (s, 3H), 1.63 (s, 9H), 1.43 (s, 6H). LC-MS (ESI) m/z $[M+Na]^+$: 449.1.

**Step 2: 5-((6-(methoxycarbonyl)-3,3-dimethyl-2-oxoindol-1-yl)methyl)-1H-indol-1-carboxylic acid (94-2)**

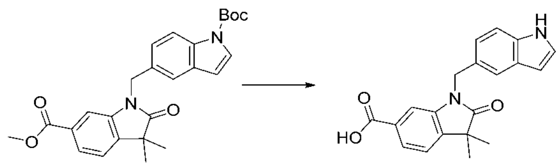

The preparation method of 5-((6-(methoxycarbonyl)-3,3-dimethyl-2-oxoindol-1-yl)methyl)-1H-indol-1-carboxylic acid **(94-2)** was the same as that of compound **1-5.** $^1H$ NMR (400 MHz, DMSO-$d_6$) $\delta$ 12.80 (s, 1H), 11.10 (s, 1H), 7.63 (dd, *J* = 7.7, 1.4 Hz, 1H), 7.50 (d, *J*= 7.7 Hz, 1H), 7.45 (d, *J*= 1.5 Hz, 1H), 7.38 (d, *J* = 1.4 Hz, 1H), 7.35 (d, *J* = 8.4 Hz, 1H), 7.36-7.29 (m, 1H), 7.00 (dd, *J* = 8.4, 1.7 Hz, 1H), 6.40-6.36 (m, 1H), 5.00 (s, 2H), 1.37 (s, 6H). LC-MS (ESI) *m/z* $[M-H]^-$: 333.1.

**Step 3: 1-(indol-5-ylmethyl)-N-(1H-indol-3-yl)-3,3-dimethyl-2-oxoindolin-6-carboxamide (I-94)**

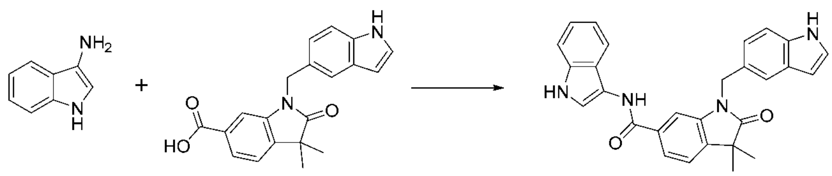

The preparation method of 1-(indol-5-ylmethyl)-N-(1H-indol-3-yl)-3,3-dimethyl-2-oxoindolin-6-carboxamide **(I-94)** was the same as that of compound **I-1.** $^1H$ NMR (400 MHz, DMSO-$d_6$) $\delta$ 11.08 (s, 1H), 10.89 (s, 1H), 10.05 (s, 1H), 7.80 (d, *J* = 7.9 Hz, 1H), 7.73 (d, *J* = 2.5 Hz, 1H), 7.69 (dd, *J* = 7.7, 1.5 Hz, 1H), 7.54-7.49 (m, 3H), 7.35 (dd, *J* = 8.3, 3.2 Hz, 2H), 7.34-7.28 (m, 1H), 7.14-7.06 (m, 1H), 7.06 (dd, *J* = 8.4, 1.7 Hz, 1H), 7.05-6.96 (m, 1H), 6.40-6.37 (m, 1H), 5.02 (s, 2H), 1.39 (s, 6H). LC-MS (ESI) *m/z* $[M+H]^+$: 449.0.

**Example 95 1-([1,1'-biphenyl]-2-ylmethyl)-N-(1H-indol-3-yl)-3,3-dimethyl-2-oxoindolin-6-carboxamide (I-95)**

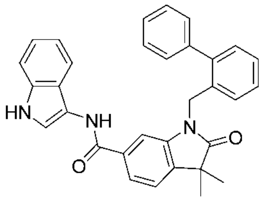

**Step 1: methyl 1-([1,1'-biphenyl]-2-ylmethyl)-3,3-dimethyl-2-oxoindolin-6-carboxylate (95-1)**

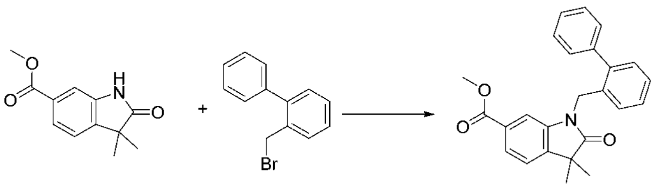

The preparation method of methyl 1-([1,1'-biphenyl]-2-ylmethyl)-3,3-dimethyl-2-oxoindolin-6-carboxylate **(95-1)** was the same as that of compound **2-3.** $^1$H NMR (400 MHz, DMSO-$d_6$) $\delta$ 7.63 (dd, *J* = 7.7, 1.5 Hz, 1H), 7.56-7.48 (m, 5H), 7.45 (td, *J* = 6.6, 2.2 Hz, 1H), 7.38-7.28 (m, 3H), 7.08-7.04 (m, 1H), 6.97 (d, *J*= 1.5 Hz, 1H), 4.87 (s, 2H), 3.81 (s, 3H), 1.31 (s, 6H). LC-MS (ESI) m/z [M+H]$^+$: 386.2.

**Step 2: 1-([1,1'-biphenyl]-2-ylmethyl)-3,3-dimethyl-2-oxoindolin-6-carboxylic acid (95-2)**

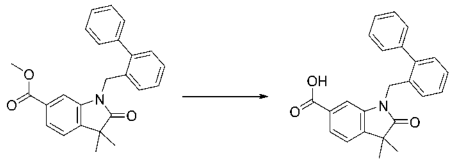

The preparation method of 1-([1,1'-biphenyl]-2-ylmethyl)-3,3-dimethyl-2-oxoindolin-6-carboxylic acid **(95-2)** was the same as that of compound **1-5.** $^1$H NMR (400 MHz, DMSO-$d_6$) $\delta$ 12.87 (s, 1H), 7.62 (dd, *J*= 7.7, 1.5 Hz, 1H), 7.51-7.41 (m, 6H), 7.36-7.28 (m, 3H), 7.03 (dt, *J* = 4.1, 2.3 Hz, 2H), 4.86 (s, 2H), 1.31 (s, 6H). LC-MS (ESI) *m*/*z* [M-H]$^+$: 370.1.

**Step 3: 1-([1,1'-biphenyl]-2-ylmethyl)-N-(1H-indol-3-yl)-3,3-dimethyl-2-oxoindolin-6-carboxamide (I-95)**

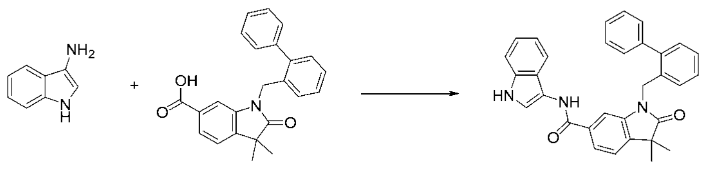

The preparation method of 1-([1,1'-biphenyl]-2-ylmethyl)-N-(1H-indol-3-yl)-3,3-dimethyl-2-oxoindolin-6-carboxamide **(I-95)** was the same as that of compound **I-1.** $^1$H NMR (400 MHz, DMSO-$d_6$) $\delta$ 10.89 (s, 1H), 10.05 (s, 1H), 7.80 (d, *J*= 8.0 Hz, 1H), 7.75 (d, *J* = 2.5 Hz, 1H), 7.66 (dd, *J*= *7.7,* 1.5 Hz, 1H), 7.54-7.46 (m, 5H), 7.44-7.39 (m, 1H), 7.37-7.28 (m, 4H), 7.14-6.99 (m, 4H), 4.90 (s, 2H), 1.35 (s, 6H). LC-MS (ESI) *m*/*z* [M+H]$^+$: 486.4.

**Example 96 N-(1H-indol-3-yl)-3,3-dimethyl-1-((1-methylpiperidin-4-yl)methyl)-2-oxoindolin-6-carboxamide (I-96)**

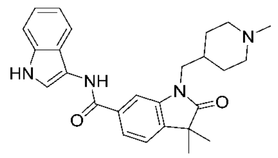

**Step 1: methyl 1-((1-(*tert*-butoxycarbonyl)piperidin-4-yl)methyl)-3,3-dimethyl-2-oxoindolin-6-carboxylate (96-1)**

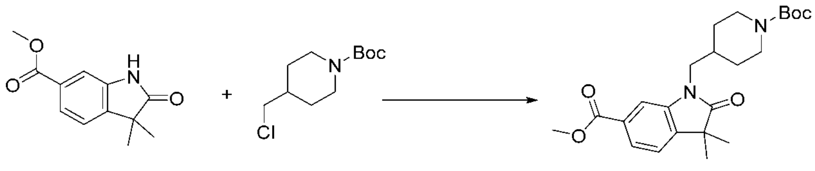

The preparation method of methyl 1-((1-(*tert*-butoxycarbonyl)piperidin-4-yl)methyl)-3,3-dimethyl-2-oxoindolin-6-carboxylate **(96-1)** was the same as that of compound **2-3.** $^1H$ NMR (400 MHz, DMSO-$d_6$) $\delta$ 7.69 (dd, *J*= 7.7, 1.4 Hz, 1H), 7.56 (d, *J*= 1.5 Hz, 1H), 7.51 (d, *J*= 7.7 Hz, 1H), 3.95-3.85(m, 5H), 3.63 (d, *J*= 7.4 Hz, 2H), 2.75-2.55 (m, 2H), 1.98-1.87 (m, 1H), 1.59-1.49 (m, 2H), 1.38 (s, 9H), 1.30 (s, 6H), 1.14-1.03 (m, 2H).

**Step 2: methyl 3,3-dimethyl-2-oxo-1-(piperidin-4-ylmethyl)indolin-6-carboxylate (96-2)**

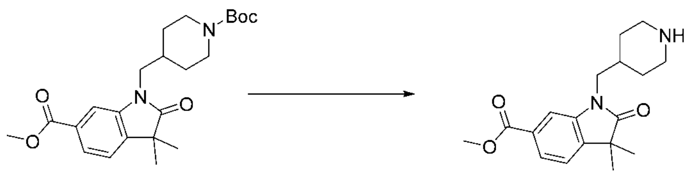

The preparation method of methyl 3,3-dimethyl-2-oxo-1-(piperidin-4-ylmethyl)indolin-6-carboxylate **(96-2)** was the same as that of compound **85-9.** $^1H$ NMR (400 MHz, DMSO-$d_6$) $\delta$ 8.38 (s, 1H), 7.71 (dd, *J* = 7.6, 1.4 Hz, 1H), 7.57 (d, *J*= 1.4 Hz, 1H), 7.53 (d, *J* = 7.7 Hz, 1H), 3.87 (s, 3H), 3.67 (d, *J* = 7.3 Hz, 2H), 3.29-3.22 (m, 2H), 2.88-2.79 (m, 2H), 2.10-2.02 (m, 1H), 1.78-1.70 (m, 2H), 1.43-1.34 (m, 2H), 1.31 (s, 6H).

**Step 3: methyl 3,3-dimethyl-1-((1-methylpiperidin-4-yl)methyl)-2-oxoindolin-6-carboxylate (96-3)**

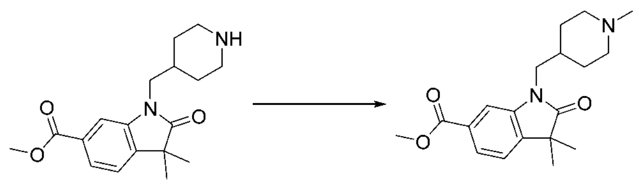

100 mg of Compound **96-2,** 95 mg of acetic acid, 77 mg of formaldehyde, and 1 mL of methanol were stirred at room temperature for 1 hour, then sodium cyanoborohydride was added and the mixture was stirred at room temperature for 1 hour, and finally saturated sodium bicarbonate aqueous solution was added and the mixture was stirred at room temperature for 2 hours. The reaction solution was extracted with ethyl acetate, and the organic phase was concentrated to dryness. The residue was subjected to preparative thin-layer chromatography with dichloromethane: methanol = 10:1 to obtain 88.3 mg of methyl 3,3-dimethyl-1-((1-methylpiperidin-4-yl)methyl)-2-oxoindolin-6-carboxylate **(96-3)** as a yellow oily substance, with a yield of 84.6%. $^1H$ NMR (400 MHz, $CD_3OD$) $\delta$ 7.81 (dd, *J* = 7.7, 1.4 Hz, 1H), 7.64 (d, *J* = 1.4 Hz, 1H), 7.44 (d, *J* = 7.7 Hz, 1H), 3.92 (s, 3H), 3.78 (d, *J* = 7.3 Hz, 2H), 3.53-3.46 (m, 2H), 3.05-2.94 (m, 2H), 2.84 (s, 3H), 2.25-2.14 (m,, 1H), 2.01-1.92 (m, 2H), 1.70-1.58 (m, 2H), 1.39 (s, 6H).

**Step 4: 3,3-dimethyl-1-((1-methylpiperidin-4-yl)methyl)-2-oxoindolin-6-carboxylic acid (96-4)**

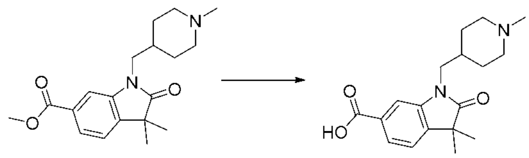

The preparation method of 3,3-dimethyl-1-((1-methylpiperidin-4-yl)methyl)-2-oxoindolin-6-carboxylic acid (96-4) was the same as that of compound **1-5.** LC-MS (ESI) *m/z* $[M-H]^-$: 315.1.

**Step 5: N-(1H-indol-3-yl)-3,3-dimethyl-1-((1-methylpiperidin-4-yl)methyl)-2-oxoindolin-6-carboxamide (I-96)**

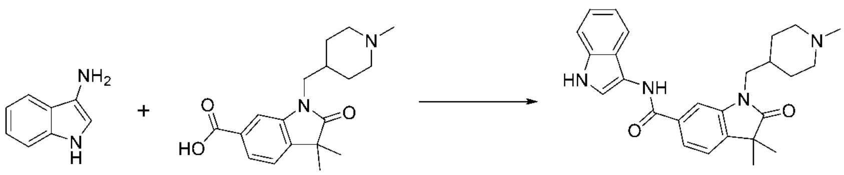

The preparation method of N-(1H-indol-3-yl)-3,3-dimethyl-1-((1-methylpiperidin-4-yl)methyl)-2-oxoindolin-6-carboxamide **(I-96)** was the same as that of compound **I-1.** $^1$H NMR (400 MHz, $CD_3OD$) $\delta$ 7.78 (d, *J*= 7.7 Hz, 1H), 7.70 (d, *J*= 8.0 Hz, 1H), 7.67-7.64 (m, 2H), 7.48 (d, *J* = 7.6 Hz, 1H), 7.39 (d, *J*= 8.2 Hz, 1H), 7.16 (t, *J*= 7.5 Hz, 1H), 7.06 (t, *J*= 7.5 Hz, 1H), 3.79 (d, *J* = 7.3 Hz, 2H), 3.47-3.40 (m,2H), 2.95-2.85 (m, 2H), 2.24 (s, 1H), 1.98-1.90 (m, 2H), 1.68-1.54 (m, 2H), 1.42 (s, 6H), 1.29 (s, 3H). LC-MS (ESI) *m/z* $[M+H]^+$: 431.2.

**Example 97 1-(benzofuran-7-ylmethyl)-N-(1H-indol-3-yl)-3,3-dimethyl-2-oxoindolin-6-carboxamide (I-97)**

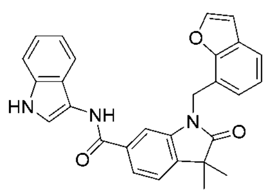

**Step 1: methyl 1-(benzofuran-7-ylmethyl)-3,3-dimethyl-2-oxoindolin-6-carboxylate (97-1)**

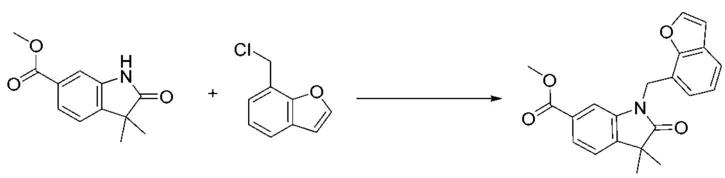

The preparation method of methyl 1-(benzofuran-7-ylmethyl)-3,3-dimethyl-2-oxoindolin-6-carboxylate **(97-1)** was the same as that of compound **2-3.** $^1$H NMR (400 MHz, $CD_3OD$) $\delta$ 7.80 (d, *J* = 1.7 Hz, 1H), 7.74 (d, *J*= 7.7 Hz, 1H), 7.58-7.54 (m, 1H), 7.51 (s, 1H), 7.41 (d, *J*= 7.8 Hz, 1H), 7.19 (d, *J* = 4.4 Hz, 2H), 6.86 (t, *J*= 1.4 Hz, 1H), 5.29 (s, 2H), 3.83 (s, 3H), 1.44 (s, 6H).

**Step 2: 1-(benzofuran-7-ylmethyl)-3,3-dimethyl-2-oxoindolin-6-carboxylic acid (97-2)**

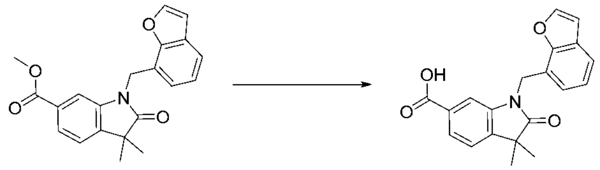

The preparation method of 1-(benzofuran-7-ylmethyl)-3,3-dimethyl-2-oxoindolin-6-carboxylic acid **(97-2)** was the same as that of compound **1-5.** $^1$H NMR (400 MHz, DMSO-$d_6$) $\delta$ 12.88 (s, 1H), 8.02 (d, *J* = 2.2 Hz, 1H), 7.64 (d, *J*= 7.7 Hz, 1H), 7.58 (d, *J*= 7.8 Hz, 1H), 7.51 (d, *J* = 7.7 Hz, 1H), 7.34 (s, 1H), 7.21 (t, *J*= 7.6 Hz, 1H), 7.09 (d, *J* = 7.4 Hz, 1H), 6.98 (d, *J*= 2.2 Hz, 1H), 5.25 (s, 2H), 1.39 (s, 6H).

**Step 3: 4-((5-chlorofuran-3-yl)methyl)-N-(1H-indol-3-yl)-6,6-dimethyl-5-oxo-5,6-dihydro-4H-thieno[3,2-b]pyrrol-2-carboxamide (I-97)**

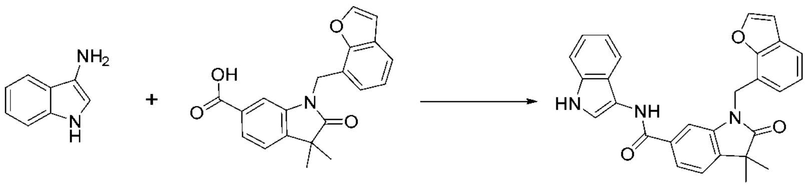

The preparation method of 1-(benzofuran-7-ylmethyl)-N-(1H-indol-3-yl)-3,3-dimethyl-2-oxoindolin-6-carboxamide **(I-97)** was the same as that of compound **I-1.** $^1$H NMR (400 MHz, DMSO-$d_6$) $\delta$ 10.88 (s, 1H), 10.03 (s, 1H), 8.02 (d, *J* = 2.2 Hz, 1H), 7.76 (d, *J* = 7.9 Hz, 1H), 7.73-7.65 (m, 2H), 7.60-7.53 (m, 2H), 7.41 (s, 1H), 7.34 (d, *J*= 8.1 Hz, 1H), 7.21 *(t, J*= 7.6 Hz, 1H), 7.09 (t, *J* = 8.1 Hz, 2H), 7.04-6.93 (m, 2H), 5.28 (s, 2H), 1.42 (s, 6H). LC-MS (ESI) *m/z* [M+H]$^+$: 450.2.

**Example 98 *N*-(benzo[b]thien-3-yl)-1-((1-(3-fluoro-5-(trifluoromethyl)phenyl) azetidin-3-yl)methyl)-3,3-dimethyl-2-indolin-6-carboxamide (I-98)**

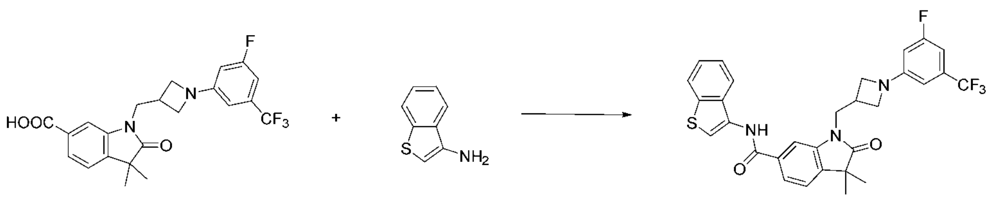

The preparation method of *N*-(benzo[b]thien-3-yl)-1-((1-(3-fluoro-5-(trifluoromethyl)phenyl) azetidin-3-yl) methyl)-3,3-dimethyl-2-indolin-6-carboxamide **(I-98)** was the same as that of compound **I-1.** $^1$H NMR (400 MHz, DMSO-$d_6$) $\delta$ 10.39 (s, 1H), 8.08 (dd, *J* = 6.1, 3.3 Hz, 1H), 7.99 (d, *J* = 7.9 Hz, 2H), 7.73 (d, *J* = 7.1 Hz, 2H), 7.55 (d, *J*= 7.7 Hz, 1H), 7.42-7.44 (m, 2H), 6.78 (d, *J*= 8.8 Hz, 1H), 6.48-6.57 (m, 2H), 4.10 (d, *J* = 7.5 Hz, 2H), 3.99 (t, *J* = 8.0 Hz, 2H), 3.74 (dd, *J* = 7.6, 5.5 Hz, 2H), 3.26-3.28 (m, 1H), 1.33 (s, 6H).

**Example 99 *N*-(1H-indol-3-yl)-3,3,7-trimethyl-2-oxo-1-(thien-3-ylmethyl)indolin-6-carboxamide (I-99)**

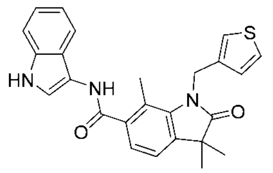

**Step 1: *N'*-(3-bromo-2-methylphenyl)isobutyrylhydrazine (99-1)**

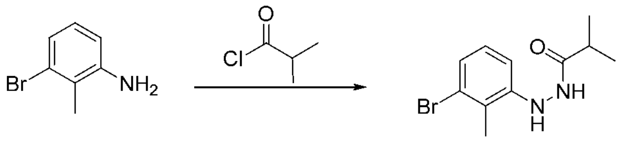

After a mixture of 330 microliters of 3-bromo-2-methylaniline, 3 milliliters of 3N hydrochloric acid, and 205 milligrams of sodium nitrite in water was reacted at -10 °C for 30 minutes, 1.5 grams of tin chloride in concentrated hydrochloric acid was slowly added dropwise to the reaction solution to react for 1 hour. The reaction solution was filtered, and the filter cake was washed with ice water, 2N hydrochloric acid, and ethyl acetate. After drying, it was dissolved in 5 milliliters of methanol. A mixture of 370 microliters of triethylamine and 135 microliters of isobutyrylchloride was added and reacted at 0 °C for 1 hour. The reaction solution was concentrated to dryness, and the residue was subjected to column chromatography with petroleum ether: ethyl acetate = 2:1 to obtain 291 mg of *N'*-(3-bromo-2-methylphenyl)isobutyrylhydrazine **(99-1)** with a yield of 40%. $^1$H NMR (400 MHz, DMSO-$d_6$) $\delta$ 9.69 (d, *J*= 2.4 Hz, 1H), 7.35 (d, *J* = 2.5 Hz, 1H), 6.92-6.97 (m, 2H), 6.60 (dd, *J*= 6.4, 2.9 Hz, 1H), 2.49-2.54 (m, 1H), 2.23 (s, 3H), 1.08 (d, *J*= 6.8 Hz, 6H).

**Step 2: 6-bromo-3,3,7-trimethylindolin-2-one (99-2)**

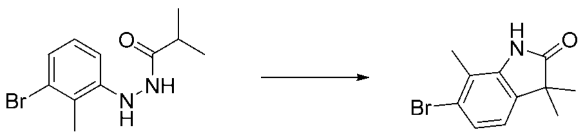

A mixture of 200 milligrams of *N'*-(3-bromo-2-methylphenyl)isobutyrylhydrazine **(99-1),** 60 milligrams of calcium hydride, and 3 milliliters of tetrahydronaphthalene was reacted at 200 °C for 17 hours. After the reaction solution was cooled to below 0 °C, 100 microliters each of methanol and water was added, and concentrated hydrochloric acid was added to adjust the pH to 1. The mixture was heated and refluxed for 1 hour. The reaction solution was cooled to room temperature, 3N sodium hydroxide solution was added to adjust the pH to 5, the reaction mixture was extracted with ethyl acetate, the organic phase was concentrated to dryness, and the residue was subjected to column chromatography with petroleum ether: ethyl acetate = 3:1 to obtain 110 mg of 6-bromo-3,3,7-trimethylindolin-2-one **(99-2)** with a yield of 41%. $^1$H NMR (400 MHz, DMSO-$d_6$) $\delta$ 10.57 (s, 1H), 7.20 (d, *J* = 7.9 Hz, 1H), 7.07 (d, *J* = 7.9 Hz, 1H), 2.26 (s, 3H), 1.23 (s, 6H).

**Step 3: methyl 3,3,7-trimethyl-2-oxoindolin-6-carboxylate (99-3)**

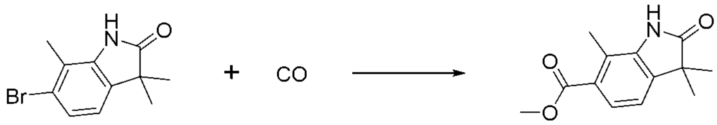

A mixture of 500 mg of 6-bromo-3,3,7-trimethylindolin-2-one **(99-2),** 150 mg of 1,1'-di(diphenylphosphine) ferrocene palladium chloride, 850 mL of triethylamine, and 4 mL of methanol was heated and stirred at 100 °C under a carbon monoxide atmosphere for 12 hours. The reaction solution was concentrated to dryness, and the residue was subjected to column chromatography with petroleum ether: ethyl acetate = 3:1 to obtain 164 mg of methyl 3,3,7-trimethyl-2-oxoindolin-6-carboxylate **(99-3)** with a yield of 36%. $^1$H NMR (400 MHz, Chloroform-*d*) $\delta$ 8.81 (s, 1H), 7.63 (d, *J* = 7.8 Hz, 1H), 7.08 (d, *J* = 7.8 Hz, 1H), 3.90 (s, 3H), 2.51 (s, 3H), 1.41 (s, 6H).

**Step 4: methyl 3,3,7-trimethyl-2-oxo-1-(thien-3-ylmethyl)indolin-6-carboxylate (99-4)**

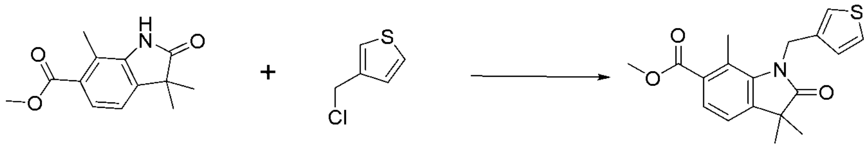

The preparation method of methyl 3,3,7-trimethyl-2-oxo-1-(thien-3-ylmethyl)indolin-6-carboxylate **(99-4)** was the same as that of **2-3.** $^1$H NMR (400 MHz, Chloroform-*d*) $\delta$ 7.45 (d, *J*= 7.7 Hz, 1H), 7.29 (dd, *J* = 4.9, 3.1 Hz, 1H), 7.11 (d, *J* = 7.8 Hz, 1H), 6.90-6.97 (m, 2H), 5.22 (s, 2H), 3.86 (s, 3H), 2.50 (s, 3H), 1.42 (s, 6H).

**Step 5: 3,3,7-trimethyl-2-oxo-1-(thien-3-ylmethyl)indolin-6-carboxylic acid (99-5)**

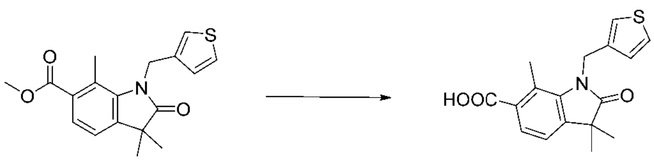

The preparation method of 3,3,7-trimethyl-2-oxo-1-(thien-3-ylmethyl)indolin-6-carboxylic acid **(99-5)** was the same as that **of 1-5.** LCMS (ESI) *m/z* [M+H]$^+$: 316.1.

**Step 6: *N*-(1H-indol-3-yl)-3,3,7-trimethyl-2-oxo-1-(thien-3-ylmethyl)indolin-6-carboxamide (I-99)**

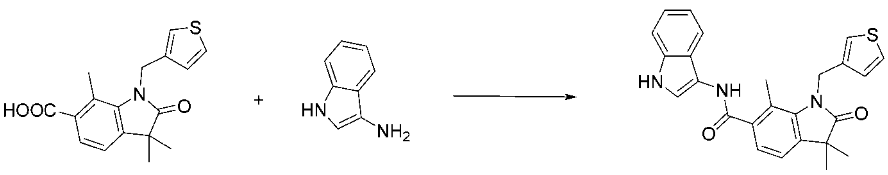

The preparation method of *N*-(1H-indol-3-yl)-3,3,7-trimethyl-2-oxo-1-(thien-3-ylmethyl)indolin -6-carboxamide **(I-99)** was the same as that of **I-1**. $^1$H NMR (400 MHz, Chloroform-*d*) $\delta$ 8.13 (s, 1H), 7.94 (d, *J*= 2.5 Hz, 1H), 7.57 (s, 1H), 7.46 (d, *J* = 7.9 Hz, 1H), 7.38 (d, *J* = 8.3 Hz, 1H), 7.30 (dd, *J* = 5.0, 3.0 Hz, 1H), 7.23 (td, *J* = 7.8, 1.6 Hz, 2H), 7.11-7.18 (m, 2H), 6.96 (ddd, *J* = 6.4, 4.0, 1.4 Hz, 2H), 5.20-5.25 (m, 2H), 2.50 (s, 3H), 1.46 (s, 6H).

**Example 100 *N*-(1*H*-indol-3-yl)-3,3-dimethyl-2-oxo-1-(1-phenethyl)indolin-6-carboxamide (I-100)**

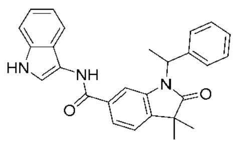

**Step 1: methyl 3,3-dimethyl-2-carbonyl-1-(1-phenethyl)indolin-6-carboxylate (100-1)**

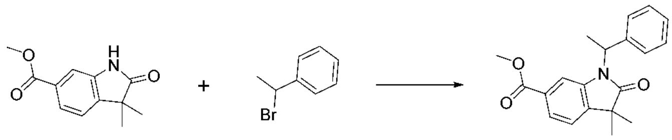

The preparation of methyl 3,3-dimethyl-2-carbonyl-1-(1-phenethyl)indolin-6-carboxylate **(100-1)** was the same as that of compound **2-3.** $^1$H NMR (400 MHz, $CDCl_3$) $\delta$ 7.70 (dd, *J* = 7.7, 1.5 Hz, 1H), 7.33-7.36 (m, 4H), 7.26-7.29 (m, 1H), 7.24 (d, *J* = 7.7 Hz, 1H), 7.21 (d, *J* = 1.4 Hz, 1H), 5.84 (q, *J* = 7.2 Hz, 1H), 3.83 (s, 3H), 1.86 (d, *J* = 7.2 Hz, 3H), 1.43 (d, *J* = 6.5 Hz, 6H).

**Step 2: 3,3-dimethyl-2-carbonyl-1-(1-phenethyl)indolin-6-carboxylic acid (100-2)**

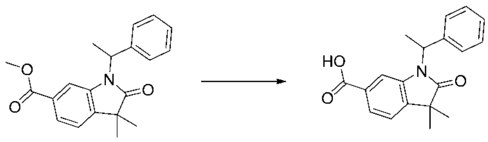

The preparation of 3,3-dimethyl-2-carbonyl-1-(1-phenethyl)indolin-6-carboxylic acid **(100-2)** was the same as that of compound **1-5.** LCMS (ESI) *m*/*z* $[M+H]^+$: 310.1.

**Step 3: *N*-(1*H*-indol-3-yl)-3,3-dimethyl-2-oxo-1-(1-phenethyl)indolin-6-carboxamide (I-100)**

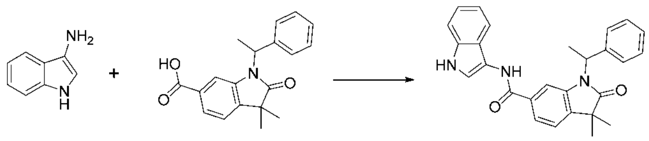

The preparation of N-(1H-indol-3-yl)-3,3-dimethyl-2-oxo-1-(1-phenethyl)indolin-6-carboxamide **(I-100)** was the same as that of compound **I-1.** $^1$H NMR (400 MHz, DMSO-$d_6$) $\delta$ 10.89 (d, *J*= 2.6 Hz, 1H), 10.04 (s, 1H), 7.77 (d, *J*= 8.0 Hz, 1H), 7.72 (d, *J*= 2.5 Hz, 1H), 7.67 (dd, *J* = 7.7, 1.4 Hz, 1H), 7.52 (d, *J*= 7.7 Hz, 1H), 7.32-7.38 (m, 5H), 7.24-7.29 (m, 1H), 7.17 (d, *J* = 1.6 Hz, 1H), 7.10 (ddd, *J* = 8.2, 6.9, 1.2 Hz, 1H), 7.00 (ddd, *J* = 8.0, 6.9, 1.1 Hz, 1H), 5.71 (t, *J*= 7.2 Hz, 1H), 1.85 (d, *J*= 7.1 Hz, 3H), 1.38 (d, *J* = 11.0 Hz, 6H).

**Example 102 1-phenyl-3-ethyl-*N*-(1*H*-indol-3-yl)-3-methyl-2-indolin-6-carboxamide (I-101)**

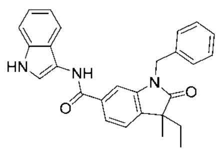

**Step 1: 6-bromo-3-ethyl-3-methylindolin-2-one (101-1)**

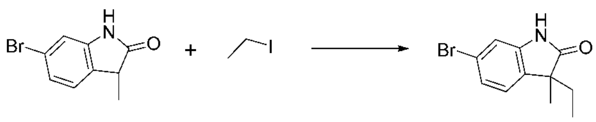

A mixture of 500 milligrams of 6-bromo-3-methylindolin-2-one, 1.9 milliliters of n-butyl lithium solution in *n*-hexane, 1 milliliter of N,N,N',N'-tetramethylethylenediamine, and 3 milliliters of ultra-dry tetrahydrofuran was stirred under argon atmosphere at -78 °C for 0.5 hours. Then, 270 microliters of iodoethane dissolved in tetrahydrofuran were added dropwise to the reaction solution, and the reaction was continued for 2 hours at 0 °C. After the reaction solution was quenched, it was concentrated to dryness. The residue was subjected to column chromatography with petroleum ether: ethyl acetate = 3:1 to obtain 246 mg of 6-bromo-3-ethyl-3-methylindolin-2-one **(101-1)** with a yield of 44%. $^1$H NMR (400 MHz, $CDCl_3$) $\delta$ 7.73 (s, 1H), 7.18 (dd, *J*= 7.9, 1.7 Hz, 1H), 7.05 (d, *J*= 1.7 Hz, 1H), 7.01 *(d, J*= 7.9 Hz, 1H), 1.87-1.98 (m, 1H), 1.70-1.80 (m, 1H), 1.36 (s, 3H), 0.65 *(t, J*= 7.4 Hz, 3H).

**Step 2: 1-phenyl-6-bromo-3-ethyl-3-methylindolin-2-one (101-2)**

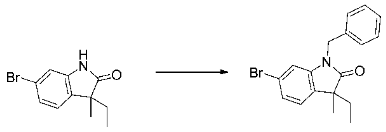

The preparation method of 1-phenyl-6-bromo-3-ethyl-3-methylindolin-2-one **(101-2)** was the same as that of compound **2-3.** $^1$H NMR (400 MHz, $CDCl_3$) $\delta$ 7.30-7.35 (m, 2H), 7.28 (dt, *J* = 3.5, 1.7 Hz, 3H), 7.16 (dd, *J* = 7.8, 1.7 Hz, 1H), 7.02 (d, *J* = 7.8 Hz, 1H), 6.86 (d, *J* = 1.7 Hz, 1H), 4.77-5.01 (m, 2H), 1.93-2.06 (m, 1H), 1.80 (dq, *J* = 13.5, 7.4 Hz, 1H), 1.39 (s, 3H), 0.63 (t, *J* = 7.4 Hz, 3H).

**Step 3: 1-phenyl-3-ethyl-3-methyl-2-indolin-6-carbonitrile (101-3)**

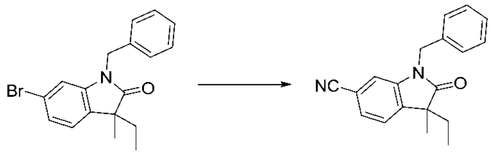

The preparation of 1-phenyl-3-ethyl-3-methyl-2-indolin-6-nitrile **(101-3)** was the same as that of compound **11-2.** $^1$H NMR (400 MHz, $CDCl_3$) $\delta$ 7.29-7.37 (m, 4H), 7.26-7.28 (m, 2H), 7.264-7.26 (m, 1H), 6.91 *(d, J*= 1.4 Hz, 1H), 4.81-5.02 (m, 2H), 2.00-2.10 (m, 1H), 1.84 (dq, *J* = 13.6, 7.4 Hz, 1H), 1.42 (s, 3H), 0.63 (t, *J*= 7.4 Hz, 3H).

**Step 4: 1-phenyl-3-ethyl-3-methyl-2-indolin-6-carboxylic acid (101-4)**

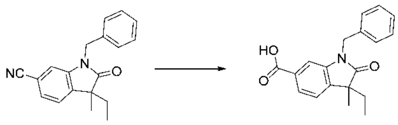

The preparation of 1-phenyl-3-ethyl-3-methyl-2-indolin-6-carboxylic acid **(101-4)** was the same as that of compound **11-3.** LCMS (ESI) *m*/*z* [M+H]$^+$: 310.1.

**Step 5: 1-phenyl-3-ethyl-*N*-(1*H*-indol-3-yl)-3-methyl-2-indolin-6-carboxamide (I-101)**

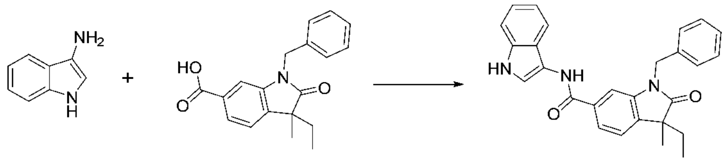

The preparation of 1-phenyl-3-ethyl-N-(1H-indol-3-yl)-3-methyl-2-indolin-6-carboxamide (I-**101)** was the same as that of compound **I-1.** $^1$H NMR (400 MHz, DMSO-$d_6$) $\delta$ 10.90 (s, 1H), 10.09 (s, 1H), 7.81 (d, *J*= 7.9 Hz, 1H), 7.71-7.76 (m, 2H), 7.49 (d, *J*= 7.7 Hz, 1H), 7.46 (d, *J*= 1.6 Hz, 1H), 7.31-7.38 (m, 5H), 7.27 (td, *J* = 5.9, 2.6 Hz, 1H), 7.10 (ddd, *J* = 8.2, 7.0, 1.2 Hz, 1H), 7.00 (ddd, *J* = 8.0, 7.0, 1.1 Hz, 1H), 4.99 (q, *J* = 15.9 Hz, 2H), 1.83-1.95 (m, 2H), 1.36 (s, 3H), 0.56 (t, *J* = 7.4 Hz, 3H).

**Example 102 *N*-(1*H*-indol-3-yl-3,3-dimethyl-2-carbonyl-1-((5-(trifluoromethyl) thien-3-yl)methyl)indolin-6-carboxamide (I-102)**

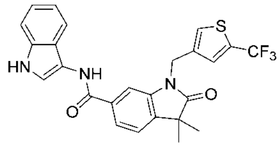

**Step 1: methyl 1-((5-bromothien-3-yl)methyl)-3,3-dimethyl-2-indolin-6-carboxylate (102-1)**

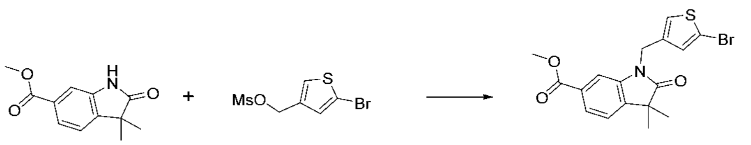

The preparation method of methyl 1-((5-bromothien-3-yl)methyl)-3,3-dimethyl-2-indolin-6-carboxylate **(102-1)** was the same as that of compound **2-3.** $^1$H NMR (400 MHz, $CDCl_3$) $\delta$ 7.77 (dd, *J* = 7.7, 1.4 Hz, 1H), 7.44 (d, *J* = 1.5 Hz, 1H), 7.25-7.29 (m, 1H), 7.07 (dd, *J* = 1.8, 0.9 Hz, 1H), 6.96 (d, *J* = 1.7 Hz, 1H), 4.84 (s, 2H), 3.90 (s, 3H), 1.41 (s, 6H).

**Step 2: methyl 3,3-dimethyl-2-carbonyl-1-((5-(trifluoromethyl)thien-3-yl)methyl)indolin-6-carboxylate (102-2)**

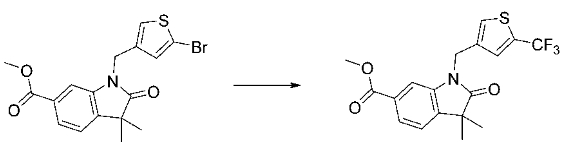

The preparation method of methyl 3,3-dimethyl-2-carbonyl-1-((5-(trifluoromethyl)thien-3-yl)methyl)indolin-6-carboxylate **(102-2)** was the same as that of compound **90-2.** $^1$H NMR (400 MHz, $CDCl_3$) $\delta$ 7.80 (dd, *J* = 7.7, 1.5 Hz, 1H), 7.44 (d, *J* = 1.5 Hz, 1H), 7.35 (dd, *J* = 6.6, 1.4 Hz, 2H), 7.29 (d, *J*= 7.7 Hz, 1H), 4.90 (d, *J*= 1.0 Hz, 2H), 3.91 (s, 3H), 1.42 (s, 6H).

**Step 5: 3,3-dimethyl-2-carbonyl-1-((5-(trifluoromethyl)thien-3-yl)methyl)indolin-6-carboxylic acid (102-3)**

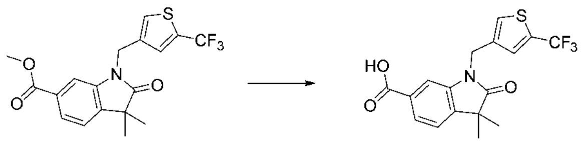

The preparation method of 3,3-dimethyl-2-carbonyl-1-((5-(trifluoromethyl)thien-3-yl)methyl)indolin-6-carboxylic acid **(102-3)** was the same as that of compound **1-5.** LCMS (ESI) *m/z* $[M+H]^+$: 370.1.

**Step 6: *N*-(1*H*-indol-3-yl-3,3-dimethyl-2-carbonyl-1-((5-(trifluoromethyl)thien-3-yl)methyl)indolin-6-carboxamide (I-102)**

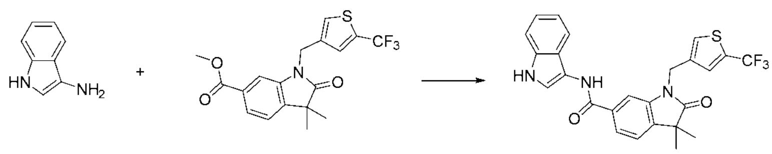

The preparation method of *N*-(1*H*-indol-3-yl-3,3-dimethyl-2-carbonyl-1-((5-(trifluoromethyl) thien-3-yl)methyl) indolin-6-carboxamide **(I-102)** was the same as that of compound **I-1.** $^1$H NMR (400 MHz, DMSO-$d_6$) $\delta$ 10.91 (s, 1H), 10.08 (s, 1H), 7.81 (d, *J*= 8.0 Hz, 1H), 7.76 *(t, J*= 2.1 Hz, 2H), 7.73 (dd, *J* = *7.7,* 1.5 Hz, 1H), 7.64 (q, *J* = 1.4 Hz, 1H), 7.60 (d, *J* = 1.5 Hz, 1H), 7.55 (d, *J* = 7.7 Hz, 1H), 7.35-7.38 (m, 1H), 7.09-7.14 (m, 1H), 6.98-7.02 (m, 1H), 5.00 (s, 2H), 1.38 (s, 6H).

**Example 103 *N*-(5-fluoro-1*H*-indol-3-yl)-3,3-dimethyl-2-carbonyl-1-((5-(trifluoromethyl)thien-3-yl)methyl)indolin-6-carboxamide** (I-103)

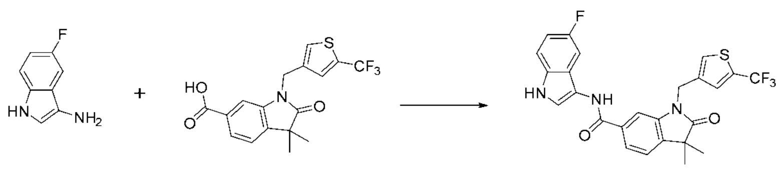

The preparation method of *N*-(5-fluoro-1H-indol-3-yl)-3,3-dimethyl-2-carbonyl-1-((5-(trifluoromethyl)thien-3-yl) methyl)indolin-6-carboxamide **(I-103)** was the same as that of compound **I-1.** $^1$H NMR (400 MHz, DMSO-$d_6$) $\delta$ 11.02 (s, 1H), 10.07 (s, 1H), 7.85 (d, *J* = 2.6 Hz, 1H), 7.68-7.78 (m, 2H), 7.51-7.65 (m, 4H), 7.36 (dd, *J* = 8.8, 4.5 Hz, 1H), 6.93-6.98 (m, 1H), 5.00 (s, 2H), 1.37 (s, 6H).

**Example 104 1-(3-fluoro-5-(trifluoromethyl)phenyl)-N-(1H-indol-3-yl)-3,3-dimethyl -2-indolin-6-carboxamide (I-104)**

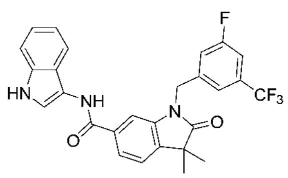

**Step 1: methyl 1-(3-fluoro-5-(trifluoromethyl)phenyl)-3,3-dimethyl-2-indolin-6-carboxylate (104-1)**

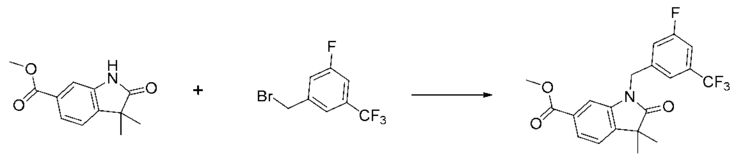

The preparation method of methyl 1-(3-fluoro-5-(trifluoromethyl)phenyl)-3,3-dimethyl-2-indolin-6-carboxylate **(104-1)** was the same as that of compound **2-3.** $^1$H NMR (400 MHz, $CDCl_3$) $\delta$ 7.81 *(d, J* = 7.7 Hz, 1H), 7.33 (dd, *J* = 14.1, 6.9 Hz, 3H), 7.24 (d, *J* = 8.4 Hz, 1H), 7.12 (d, *J* = 8.9 Hz, 1H), 4.98 (s, 2H), 3.89 (s, 3H), 1.46 (s, 6H).

**Step 2: 1-(3-fluoro-5-(trifluoromethyl)phenyl)-3,3-dimethyl-2-indolin-6-carboxylic acid (104-2)**

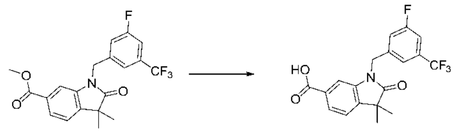

The preparation method of 1-(3-fluoro-5-(trifluoromethyl)phenyl)-3,3-dimethyl-2-indolin-6-carboxylic acid **(104-2)** was the same as that of compound **1-5.** LCMS (ESI) *m/z* $[M+H]^+$: 382.1.

**Step 3: 1-(3-fluoro-5-(trifluoromethyl)phenyl)-*N*-(1H-indol-3-yl)-3,3-dimethyl-2-indolin-6-carboxamide (I-104)**

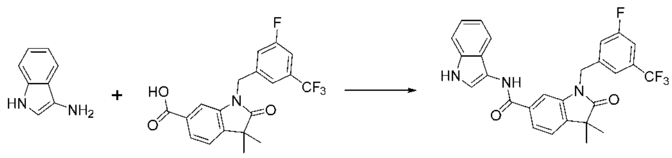

The preparation method of 1-(3-fluoro-5-(trifluoromethyl)phenyl)-*N*-(1H-indol-3-yl)-3,3-dimethyl-2-indolin-6-carboxamide **(I-104)** was the same as that of compound **I-1.** $^1$H NMR (400 MHz, DMSO-$d_6$) $\delta$ 10.90 (d, *J* = 2.5 Hz, 1H), 10.08 (s, 1H), 7.80 (d, *J* = 8.0 Hz, 1H), 7.72-7.77 (m, 2H), 7.64 (d, *J* = 8.7 Hz, 1H), 7.54-7.59 (m, 3H), 7.46 (d, *J* = 9.2 Hz, 1H), 7.35 (dt, *J* = 8.2, 0.9 Hz, 1H), 7.09-7.13 (m, 1H), 6.97-7.01 (m, 1H), 5.12 (s, 2H), 1.40 (s, 6H).

**Example 106 1-phenyl-N-(5-fluoro-1H-indol-3-yl)-3,3-dimethyl-2-indolin-6-carboxamide (I-105)**

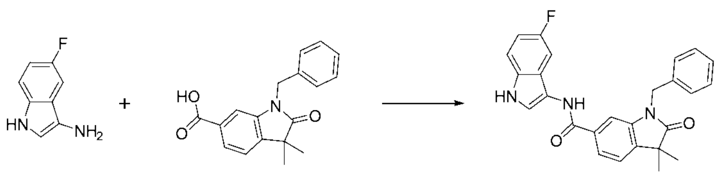

The preparation method of 1-phenyl-*N*-(5-fluoro-1H-indol-3-yl)-3,3-dimethyl-2-indolin-6-carboxamide **(I-105)** was the same as that of compound **I-1.** $^1$H NMR (400 MHz, DMSO-$d_6$) $\delta$ 11.01 (s, 1H), 10.05 (s, 1H), 7.82 (d, *J* = 2.6 Hz, 1H), 7.71 (d, *J* = 7.7 Hz, 1H), 7.50-7.63 (m, 2H), 7.42 (s, 1H), 7.35 (dd, *J* = 8.9, 5.0 Hz, 3H), 7.24-7.32 (m, 3H), 6.90-7.00 (m, 1H), 4.99 (s, 2H), 1.39 (s, 6H).

**Example 106 *N*-(5-fluoro-1*H*-indol-3-yl)-3,3-dimethyl-2-carbonyl-1-(thien-3-ylmethyl)indolin-6-carboxamide (I-106)**

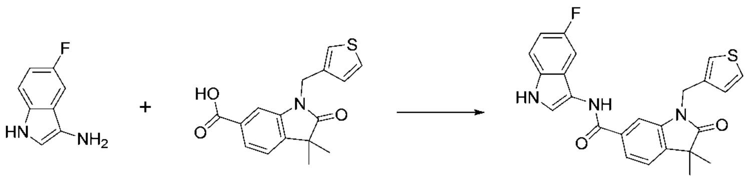

The preparation method of *N*-(5-fluoro-1H-indol-3-yl)-3,3-dimethyl-2-carbonyl-1-(thien-3-ylmethyl)indolin-6-carboxamide **(I-106)** was the same as that of compound **I-1.** $^1$H NMR (400 MHz, DMSO-$d_6$) $\delta$ 11.02 (s, 1H), 10.07 (s, 1H), 7.85 (d, *J* = 2.6 Hz, 1H), 7.71 (dd, *J* = 7.7, 1.6 Hz, 1H), 7.60 (dd, *J*= 10.3, 2.5 Hz, 1H), 7.48-7.55 (m, 3H), 7.41 (d, *J* = 2.9 Hz, 1H), 7.36 (dd, *J* = 8.9, 4.5 Hz, 1H), 7.02 (d, *J* = 5.0 Hz, 1H), 6.91-6.99 (m, 1H), 4.96 (s, 2H), 1.36 (s, 6H).

**Example 107 *N*-(5-fluoro-1*H*-indol-3-yl)-3,3-dimethyl-2-carbonyl-1-phenethylindolin-6-carboxamide (I-107)**

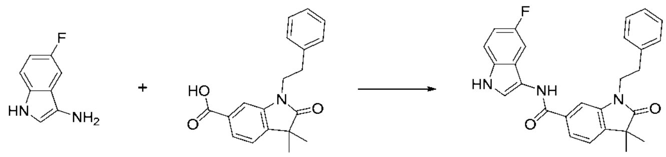

The preparation method of *N*-(5-fluoro-1H-indol-3-yl)-3,3-dimethyl-2-carbonyl-1-phenethylindolin-6-carboxamide **(I-107)** was the same as that of compound **I-1.** $^1$H NMR (400 MHz, DMSO-$d_6$) $\delta$ 11.04 (s, 1H), 10.11 (s, 1H), 7.88 (d, *J* = 2.6 Hz, 1H), 7.58-7.72 (m, 3H), 7.48 (d, *J* = 7.6 Hz, 1H), 7.38 (dd, *J* = 8.9, 4.5 Hz, 1H), 7.14-7.26 (m, 5H), 6.93-7.01 (m, 1H), 4.00 (t, *J* = 7.1 Hz, 2H), 2.99 (t, *J* = 7.2 Hz, 2H), 1.22 (s, 6H).

**Example 108 *N*-(1*H*-indol-3-yl)-3,3-dimethyl-2-carbonyl-1-(2-(thien-3-yl)ethyl) indolin-6-carboxamide (I-108)**

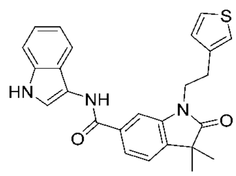

**Step 1: methyl 3,3-dimethyl-2-carbonyl-1-(2-(thien-3-yl)ethyl)indolin-6-carboxylate (108-1)**

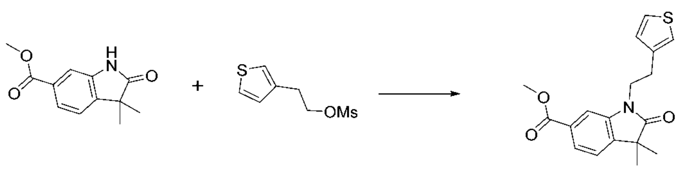

The preparation method of methyl 3,3-dimethyl-2-carbonyl-1-(2-(thien-3-yl)ethyl)indolin-6-carboxylate **(108-1)** was the same as that of compound **2-3.** $^1$H NMR (400 MHz, $CDCl_3$) $\delta$ 7.76 (dd, *J* = 7.7, 1.3 Hz, 1H), 7.43 *(d, J*= 1.4 Hz, 1H), 7.24 (d, *J* = 3.4 Hz, 1H), 7.23 (d, *J*= 2.6 Hz, 1H), 6.97 (t, *J*= 4.4 Hz, 2H), 4.01 (t, *J* = 7.3 Hz, 2H), 3.94 (s, 3H), 3.05 (t, *J* = 7.3 Hz, 2H), 1.31 (s, 6H).

**Step 2: 3,3-dimethyl-2-carbonyl-1-(2-(thien-3-yl)ethyl)indolin-6-carboxylic acid (108-2)**

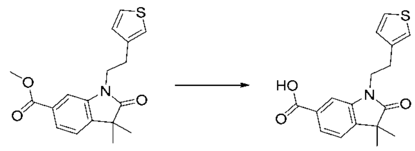

The preparation method of 3,3-dimethyl-2-carbonyl-1-(2-(thien-3-yl)ethyl)indolin-6-carboxylic acid **(108-2)** was the same as that of compound **1-5.** LCMS (ESI) m/z $[M+H]^+$: 316.1.

**Step 3: *N*-(1*H*-indol-3-yl)-3,3-dimethyl-2-carbonyl-1-(2-(thien-3-yl)ethyl)indolin-6-carboxamide (I-108)**

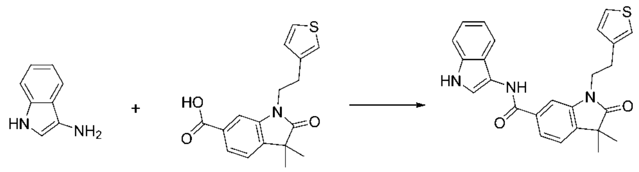

The preparation method of *N*-(1H-indol-3-yl)-3,3-dimethyl-2-carbonyl-1-(2-(thien-3-yl)ethyl)indolin-6-carboxamide **(I-108)** was the same as that of compound **I-1.** $^1$H NMR (400 MHz, DMSO-$d_6$) $\delta$ 10.93 (s, 1H), 10.11 (s, 1H), 7.84 (d, *J* = 8.0 Hz, 1H), 7.79 (d, *J* = 2.5 Hz, 1H), 7.69 (d, *J* = 7.7 Hz, 1H), 7.61 (s, 1H), 7.48 (d, *J*= 7.6 Hz, 1H), 7.42 (dd, *J* = 4.9, 2.9 Hz, 1H), 7.37 (d, *J*= 8.1 Hz, 1H), 7.20 (d, *J*= 2.9 Hz, 1H), 7.12 (t, *J*= 7.5 Hz, 1H), 7.03 (dd, *J*= 10.1, 5.8 Hz, 2H), 4.01 (t, *J* = 5.6 Hz, 2H), 3.02 (t, *J* = 7.3 Hz, 2H), 1.25 (s, 6H).

**Example 109 1-(benzo[b]thien-3-ylmethyl)-*N*-(1*H*-indol-3-yl)-3,3-dimethyl-2-indolin-6-carboxamide (I-109)**

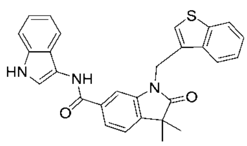

**Step 1: methyl 1-(benzo[b]thien-3-ylmethyl)-3,3-dimethyl-2-indolin-6-carboxylate (109-1)**

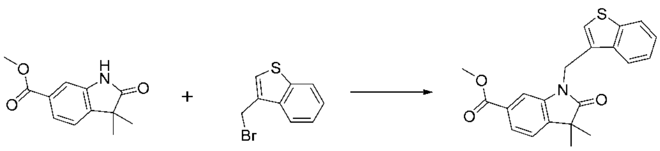

The preparation method of methyl 1-(benzo[b]thien-3-ylmethyl)-3,3-dimethyl-2-indolin-6-carboxylate **(109-1)** was the same as that of compound **2-3.** $^1$H NMR (400 MHz, $CDCl_3$) $\delta$ 7.94 (d, *J* = 7.8 Hz, 1H), 7.84 (d, *J* = 7.8 Hz, 1H), 7.75 (d, *J*= 7.7 Hz, 1H), 7.52 (s, 1H), 7.38 (dt, *J*= 18.1, 7.2 Hz, 2H), 7.32 (s, 1H), 7.25 (s, 2H), 5.19 (s, 2H), 3.87 (s, 3H), 1.44 (s, 6H).

**Step 2: 1-(benzo[b]thien-3-ylmethyl)-3,3-dimethyl-2-indolin-6-carboxylic acid (109-2)**

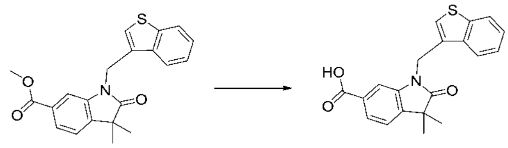

The preparation method of 1-(benzo[b]thien-3-ylmethyl)-3,3-dimethyl-2-indolin-6-carboxylic acid **(109-2)** was the same as that of compound **1-5.** LCMS (ESI) *m*/*z* [M+H]$^+$: 352.1.

**Step 3: 1-(benzo[b]thien-3-ylmethyl)-*N*-(1*H*-indol-3-yl)-3,3-dimethyl-2-indolin-6-carboxamide (I-109)**

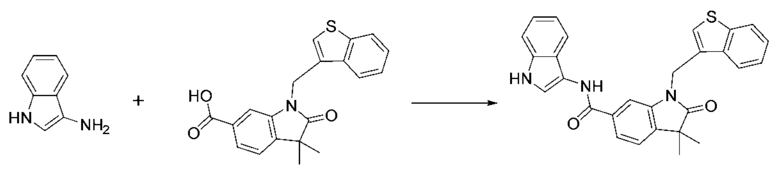

The preparation method of 1-(benzo[b]thien-3-ylmethyl)-*N*-(1H-indol-3-yl)-3,3-dimethyl-2-indolin-6-carboxamide **(I-109)** was the same as that of compound **I-1.** $^1$H NMR (400 MHz, DMSO-$d_6$) $\delta$ 10.84-10.96 (m, 1H), 10.05 (s, 1H), 7.99 (td, *J* = 7.4, 1.8 Hz, 2H), 7.78 (d, *J*= 8.0 Hz, 1H), 7.68-7.76 (m, 3H), 7.57 (d, *J*= 1.5 Hz, 1H), 7.53 (d, *J* = 7.7 Hz, 1H), 7.41 (td, *J* = 7.6, 1.6 Hz, 2H), 7.35 (d, *J*= *8.1* Hz, 1H), 7.07-7.13 (m, 1H), 7.00 (t, *J*= 7.5 Hz, 1H), 5.24 (s, 2H), 1.39 (s, 6H)

**Example 110 N-(5-fluoro-1H-indol-3-yl)-1-(3-fluoro-5-(trifluoromethyl)phenyl)-3,3-dimethyl-2-indolin-6-carboxamide (I-110)**

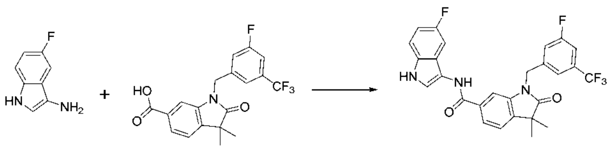

The preparation method of *N*-(5-fluoro-1H-indol-3-yl)-1-(3-fluoro-5-(trifluoromethyl)phenyl)-3,3-dimethyl-2-indolin-6-carboxamide **(I-110)** was the same as that of compound **I-1.** $^1$H NMR (400 MHz, DMSO-$d_6$) $\delta$ 11.01 (s, 1H), 10.08 (s, 1H), 7.84 (d, *J*= 2.6 Hz, 1H), 7.73 (dd, *J* = 7.7, 1.5 Hz, 1H), 7.63 (d, J= 8.7 Hz, 1H), 7.57 (ddd, *J* = 15.4, 7.8, 2.0 Hz, 4H), 7.47 (d, *J* = 9.0 Hz, 1H), 7.35 (dd, *J*= 8.9, 4.5 Hz, 1H), 6.95 (td, *J* = 9.1*,* 2.6 Hz, 1H), 5.12 (s, 2H), 1.39 (s, 6H).

**Example 111 *N*-(5,6-difluoro-1*H*-indol-3-yl)-1-(3-fluoro-5-(trifluoromethyl)phenyl)-3,3-dimethyl-2-indolin-6-carboxamide (I-111)**

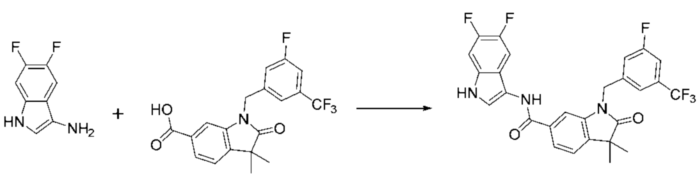

The preparation method of *N*-(5, 6-difluoro-1*H*-indol-3-yl)-1-(3-fluoro-5-(trifluoromethyl)phenyl)-3,3-dimethyl-2-indolin-6-carboxamide **(I-111)** was the same as that of compound **I-1.** $^1$H NMR (400 MHz, DMSO-$d_6$) $\delta$ 11.07 (d, *J* = 2.6 Hz, 1H), 10.12 (s, 1H), 7.78-7.86 (m, 2H), 7.71 (dd, *J* = 7.7, 1.5 Hz, 1H), 7.64 (d, *J* = 8.7 Hz, 1H), 7.55-7.59 (m, 2H), 7.53 (d, *J* = 1.5 Hz, 1H), 7.46 (d, *J*= 9.3 Hz, 1H), 7.37 (dd, *J*= 11.2, 7.0 Hz, 1H), 5.11 (s, 2H), 1.39 (s, 6H).

**Example 112 N-(1H-indol-3-yl)-2-oxo-1-(thien-3-ylmethyl)-2,3-dihydro-1H-thieno[2,3-*b*][1,4]thiazin-6-carboxamide (1-112)**

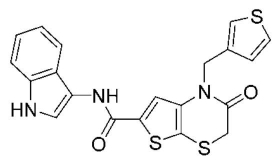

**Step 1: methyl 2-oxo-1-(thien-3-ylmethyl)-2,3-dihydro-1*H*-thieno[2,3-*b*][1,4]thiazin-6-carboxylate (112-1)**

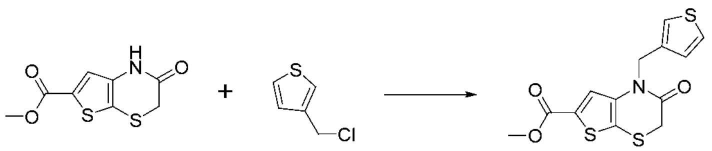

The preparation of methyl 2-oxo-1-(thien-3-ylmethyl)-2,3-dihydro-1H-thieno[2,3-b][1,4]thiazin-6-carboxylate **(112-1)** was the same as that of compound **2-3.** $^1$H NMR (400 MHz, DMSO-$d_6$) $\delta$ 7.72 (s, 1H), 7.48-7.54(m, 1H), 7.30(s, 1H), 6.95(d, *J*= 5.0 Hz, 1H), 5.13 (s, 2H), 3.77 (s, 2H), 3.80 (s, 3H). LCMS (ESI) *m*/*z* [M+H]$^+$: 326.0.

**Step 2: 2-oxo-1-(thien-3-ylmethyl)-2,3-dihydro-1*H*-thieno[2,3-*b*][1,4]thiazin-6-carboxylic acid (112-2)**

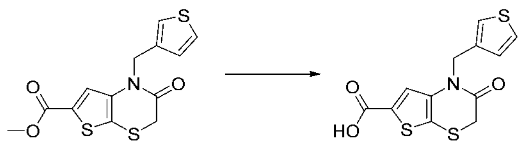

The preparation of 2-oxo-1-(thien-3-ylmethyl)-2,3-dihydro-1*H*-thieno[2,3-*b*][1,4]thiazin-6-carboxylic acid **(112-2)** was the same as that of compound **1-5.** $^1$H NMR (400 MHz, DMSO-$d_6$) $\delta$ 7.61 (s, 1H), 7.50 (t, *J*= 4.0 Hz, 1H), 7.30 (s, 1H), 6.96 (d, *J*= 5.0 Hz, 1H), 5.11 (s, 2H), 3.80 (s, 2H). LCMS (ESI) *m*/*z* [M+H]$^+$: 312.0.

**Step 3: *N*-(1*H*-indol-3-yl)-2-oxo-1-(thien-3-ylmethyl)-2,3-dihydro-1*H*-thieno[2,3-b][1,4]thiazin-6 -carboxamide (I-112)**

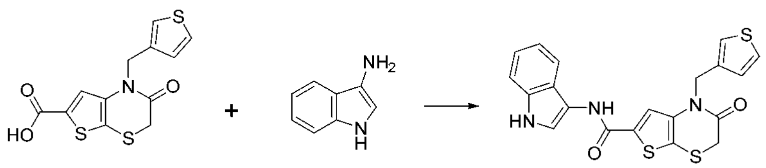

The preparation of *N*-(1*H*-indol-3-yl)-2-oxo-1-(thien-3-ylmethyl)-2,3-dihydro-1*H*-thieno[2,3-b][1,4]thiazin-6-carboxamide **(I-112)** was the same as that of compound **I-1.** $^1$H NMR (400 MHz, $CD_3OD$) $\delta$ 7.69 (d, *J* = 7.2 Hz, 1H), 7.38-7.50 (m, 2H), 6.33 (dd, *J* = 1.9, 0.9 Hz, 1H), 4.98 (d, *J* = 0.9 Hz, 2H), 3.84 (s, 3H), 3.67 (s, 2H). LCMS (ESI) *m*/*z* [M+H]$^+$ : 426.2.

**Example 113 1-(benzofuran-5-ylmethyl)-*N*-(1*H*-indol-3-yl)-2-oxo-2,3-dihydro-1*H*-thieno[2,3-*b*][1,4]thiazin-6-carboxamide (I-113)**

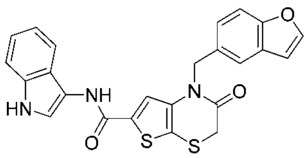

**Step 1: methyl 1-(benzofuran-5-ylmethyl)-2-oxo-2,3-dihydro-1*H*-thieno[2,3-*b*][1,4]thiazin-6-carboxylate (113-1)**

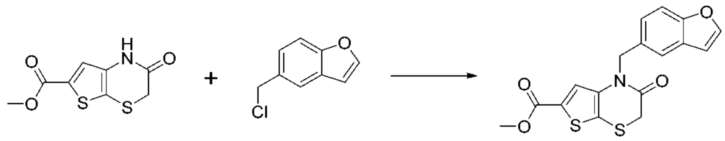

The preparation of methyl 1-(benzofuran-5-ylmethyl)-2-oxo-2,3-dihydro-1*H*-thieno[2,3-*b*][1,4]thiazin-6-carboxylate **(113-1)** was the same as that of compound **2-3.** $^{1}$H NMR (400 MHz, DMSO-$d_6$) $\delta$ 7.97 (d, *J*= 1.9 Hz, 1H), 7.71 (s, 1H), 7.55 (d, *J*= 8.5 Hz, 1H), 7.50 (s, 1H), 7.19 (d, *J*= 8.5 Hz, 1H), 6.93 (s, 1H), 5.27 (s, 2H), 3.87 (s, 2H). LCMS (ESI) *m*/*z* [M+H]$^+$: 360.0.

**Step 2: 1-(benzofuran-5-ylmethyl)-2-oxo-2,3-dihydro-1*H*-thieno[2,3-*b*][1,4]thiazin-6-carboxylic acid (113-2)**

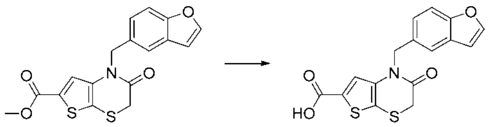

The preparation of 1-(benzofuran-5-ylmethyl)-2-oxo-2,3-dihydro-1*H*-thieno[2,3-*b*][1,4]thiazin-6-carboxylic acid **(113-2)** was the same as that of compound **1-5.** $^{1}$H NMR (400 MHz, DMSO-$d_6$) $\delta$ 7.98 (s, 1H), 7.54-7.60 (m, 2H), 7.50 (s, 1H), 7.19 (d, *J*= 8.5 Hz, 1H), 6.93 (s, 1H), 5.24 (s, 2H), 3.85 (s, 2H). LCMS (ESI) *m*/*z* [M+H]$^+$: 346.0.

**Step 3: 1-(benzofuran-5-ylmethyl)-*N*-(1*H*-indol-3-yl)-2-oxo-2,3-dihydro-1*H*-thieno[2,3-*b*][1,4]thiazin-6-carboxamide (I-113)**

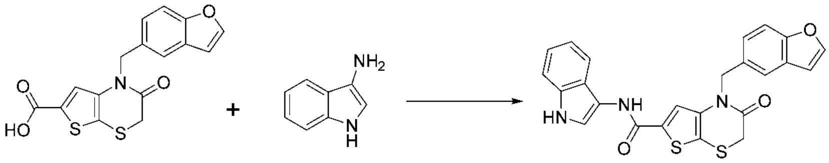

The preparation of 1-(benzofuran-5-ylmethyl)-*N*-(1*H*-indol-3-yl)-2-oxo-2,3-dihydro-1*H*-thieno[2,3-*b*][1,4]thiazin-6-carboxamide **(I-113)** was the same as that of compound **I-100.** $^{1}$H NMR (400 MHz, $CD_3OD$) $\delta$ 7.88 (s, 1H), 7.72 (d, *J*= 2.3 Hz, 1H), 7.61 (d, *J*= 7.5 Hz, 1H), 7.56 (d, *J* = 6.9 Hz, 2H), 7.47 (d, *J*= 8.4 Hz, 1H), 7.34 (d, *J*= 8.1 Hz, 1H), 7.27 (d, *J*= 9.1 Hz, 1H), 7.12 (d, *J*= 8.0 Hz, 1H), 7.04 (t, *J*= 7.5 Hz, 1H), 6.80 (s, 1H), 5.30 (s, 2H), 3.76 (s, 2H). LCMS (ESI) *m*/*z* [M+H]$^+$: 460.1.

**Example 114 *N*-(1*H*-indol-3-yl)-2-oxo-1-(thien-2-ylmethyl)-2,3-dihydro-1*H*-thieno[2,3-*b*][1,4]thiazin-6-carboxamide (I-114)**

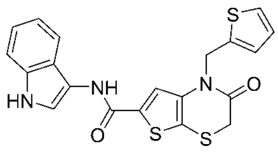

**Step 1: methyl 2-oxo-1-(thien-2-ylmethyl)-2,3-dihydro-1*H*-thieno[2,3-*b*][1,4]thiazin-6-carboxylate (114-1)**

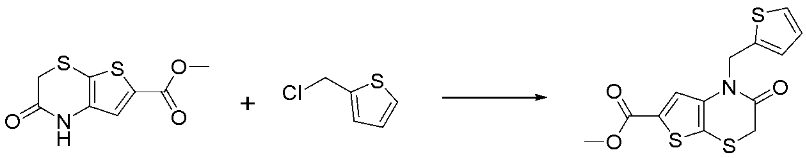

The preparation of methyl 2-oxo-1-(thien-2-ylmethyl)-2,3-dihydro-1*H*-thieno[2,3-*b*][1,4]thiazin-6-carboxylate **(114-1)** was the same as that of compound **2-3.** $^1$H NMR (400 MHz, DMSO-$d_6$) $\delta$ 7.93 (s, 1H), 7.40 (d, *J* = 5.1 Hz, 1H), 7.04 (d, *J*= 3.4 Hz, 1H), 6.94 (t, *J*= 4.3 Hz, 1H), 5.33 (s, 2H), 3.80 (d, *J* = 6.8 Hz, 5H). LCMS (ESI) *m*/*z* [M+H]$^+$: 326.0.

**Step 2: 2-oxo-1-(thien-2-ylmethyl)-2,3-dihydro-1*H*-thieno[2,3-*b*][1,4]thiazin-6-carboxylic acid (114-2)**

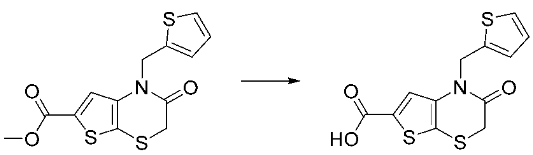

The preparation of 2-oxo-1-(thien-2-ylmethyl)-2,3-dihydro-1*H*-thieno[2,3-b][1,4]thiazin-6-carboxylic acid **(114-2)** was the same as that of compound **1-5.** LCMS (ESI) *m*/*z* [M+H]$^+$: 312.0.

**Step 3: *N*-(1*H*-indol-3-yl)-2-oxo-1-(thien-2-ylmethyl)-2,3-dihydro-1*H*-thieno[2,3-*b*][1,4]thiazin-6-carboxamide (I-114)**

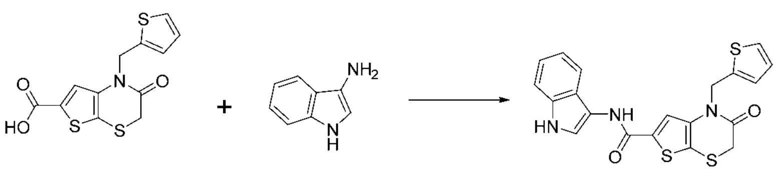

The preparation of *N*-(1*H*-indol-3-yl)-2-oxo-1-(thien-2-ylmethyl)-2,3-dihydro-1*H*-thieno[2,3-b][1,4]thiazin-6 -carboxamide **(I-114)** was the same as that of compound **I-1.** $^1$H NMR (400 MHz, $CD_3OD$) $\delta$ 8.00 (s, 1H), 7.67 (d, *J*= 8.0 Hz, 1H), 7.59 (s, 1H), 7.36 (d, *J*= 8.1 Hz, 1H), 7.31 (dd, *J*= 5.1, 1.3 Hz, 1H), 7.11-7.17 (m, 2H), 7.06 (t, *J*= 7.5 Hz, 1H), 6.94 (dd, *J* = 5.1, 3.5 Hz, 1H), 5.33 (s, 2H), 3.69 (s, 2H). LCMS (ESI) *m*/*z* [M+H]$^+$: 426.0.

**Example 115 1-(3-fluoro-5-(trifluoromethyl)benzyl)-*N*-(1*H*-indol-3-yl)-2-oxo-2,3-dihydro-1*H*-thieno[2,3-*b*][1,4] thiazin-6-carboxamide (I-115)**

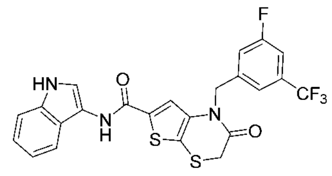

**Step 1: methyl 1-(3-fluoro-5-(trifluoromethyl)benzyl)-2-oxo-2,3-dihydro-1*H*-thieno[2,3-*b*][1,4]thiazin-6-carboxylate (115-1)**

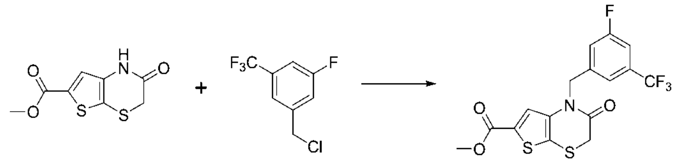

The preparation of methyl 1-(3-fluoro-5-(trifluoromethyl)benzyl)-2-oxo-2,3-dihydro-1*H*-thieno[2,3-b][1,4]thiazin-6-carboxylate **(115-1)** was the same as that of compound **2-3.** $^1H$ NMR (400 MHz, DMSO-$d_6$) $\delta$ 7.75 (s, 1H), 7.59 (d, *J* = 8.7 Hz, 1H), 7.47 (s, 1H), 7.34 (d, *J* = 9.4 Hz, 1H), 5.30 (s, 2H), 4.04 (s, 3H), 3.89 (s, 2H). LCMS (ESI) *m/z* $[M+H]^+$: 406.0.

**Step 2: 1-(3-fluoro-5-(trifluoromethyl)benzyl)-2-oxo-2,3-dihydro-1*H*-thieno[2,3-*b*][1,4]thiazin-6-carboxylic acid (115-2)**

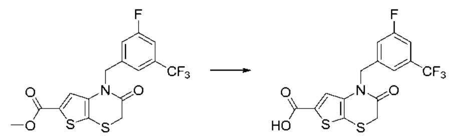

The preparation of 1-(3-fluoro-5-(trifluoromethyl)benzyl)-2-oxo-2,3-dihydro-1*H*-thieno[2,3-b][1,4]thiazin-6-carboxylic acid **(115-2)** was the same as that of compound **1-5.** $^1H$ NMR (400 MHz, DMSO-$d_6$) $\delta$ 7.59 (d, *J*= 7.0 Hz, 2H), 7.47 (s, 1H), 7.35 (d, *J*= 9.5 Hz, 1H), 5.28 (s, 2H), 3.88 (s, 2H). LCMS (ESI) *m/z* $[M+H]^+$: 392.0.

**Step 3: 1-(3-fluoro-5-(trifluoromethyl)benzyl)-*N*-(1*H*-indol-3-yl)-2-oxo-2,3-dihydro-1*H*-thieno[2,3-*b*][1, 4]thiazin-6-carboxamide (I-115)**

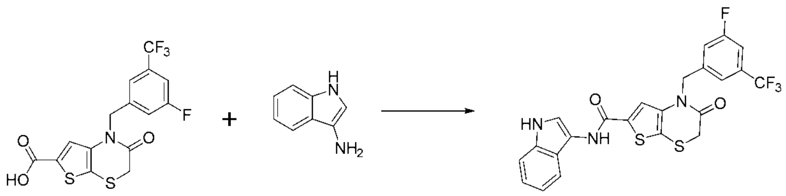

The preparation of 1-(3-fluoro-5-(trifluoromethyl)benzyl)-*N*-(1*H*-indol-3-yl)-2-oxo-2,3-dihydro-1H-thieno[2,3-b][1,4]thiazin-6-carboxamide **(I-115)** was the same as that of compound **I-1.** $^1H$ NMR (400 MHz, $CD_3OD$) $\delta$ 7.84 (s, 1H), 7.63 (d, *J* = 7.9 Hz, 1H), 7.57 (s, 1H), 7.47 (s, 1H), 7.30-7.40 (m, 4H), 7.12-7.17 (m, 1H), 7.05 (t, *J*= 7.5 Hz, 1H), 5.28 (s, 2H), 3.79 (s, 2H). LCMS (ESI) *m/z* $[M+H]^+$: 506.0.

**Example 116 1-(cyclopropylmethyl)-*N*-(1*H*-indol-3-yl)-2-oxo-2,3-dihydro-1*H*-thieno[2,3-*b*][1,4]thiazin-6-carboxamide (I-116)**

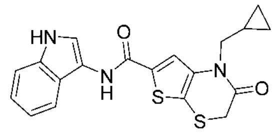

**Step 1: methyl 1-(cyclopropylmethyl)-2-oxo-2,3-dihydro-1*H*-thieno[2,3-*b*][1,4]thiazin-6-carboxylate (116-1)**

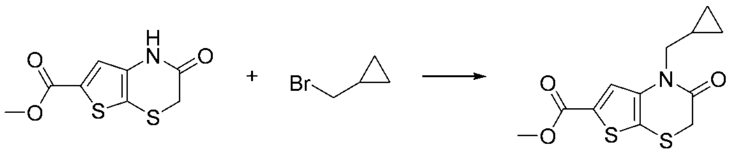

The preparation of methyl 1-(cyclopropylmethyl)-2-oxo-2,3-dihydro-1*H*-thieno[2,3-*b*][1,4]thiazin-6-carboxylate **(116-1)** was the same as that of compound **2-3.** $^1$H NMR (400 MHz, DMSO-$d_6$) $\delta$ 7.86 (s, 1H), 3.82-3.88 (m, 5H), 3.71 (s, 2H), 1.25 (d, *J*= 5.1 Hz, 1H), 0.38-0.47 (m, 2H), 0.24-0.31 (m, 2H). LCMS (ESI) *m/z* [M+H]$^+$: 284.0.

**Step 2: 1-(cyclopropylmethyl)-2-oxo-2,3-dihydro-1*H*-thieno[2,3-*b*][1,4]thiazin-6-carboxylic acid (116-2)**

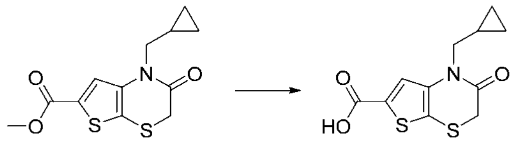

The preparation of 1-(cyclopropylmethyl)-2-oxo-2,3-dihydro-1*H*-thieno[2,3-*b*][1,4]thiazin-6-carboxylic acid **(116-2)** was the same as that of compound **1-5.** $^1$H NMR (400 MHz, DMSO-$d_6$) $\delta$ 7.75 (s, 1H), 3.83 (d, *J*= 6.9 Hz, 2H), 3.69 (s, 2H), 1.00 (m, 1H), 0.39-0.45 (m, 2H), 0.24-0.30 (m, 2H).

**Step 3: 1-(cyclopropylmethyl)-*N*-(1*H*-indol-3-yl)-2-oxo-2,3-dihydro-1*H*-thieno[2,3-*b*][1,4]thiazin-6-carboxamide (I-116)**

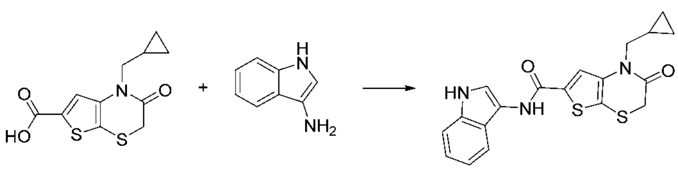

The preparation of 1-(cyclopropylmethyl)-*N*-(1*H*-indol-3-yl)-2-oxo-2,3-dihydro-1*H*-thieno[2,3-b][1,4]thiazin-6-carboxamide **(I-116)** was the same as that of compound **I-1.** $^1$H NMR (400 MHz, $CD_3OD$) $\delta$ 8.02 (s, 1H), 7.71 (d, *J* = 8.0 Hz, 1H), 7.62 (s, 1H), 7.37 (d, *J* = 8.2Hz, 1H), 7.12-7.18 (m, 1H), 7.03-7.10 (m, 1H), 3.90 (d, *J* = 6.9 Hz, 2H), 3.63 (s, 2H), 0.50-0.57 (m, 2H), 0.38-0.45(m, 2H). LCMS (ESI) *m/z* [M+H]$^+$: 384.0.

**Example 117 1-(furan-3-ylmethyl)-N-(1H-indol-3-yl)-2-oxo-2,3-dihydro-1H-thieno[2,3-*b*][1,4]thiazin-6-carboxamide (1-117)**

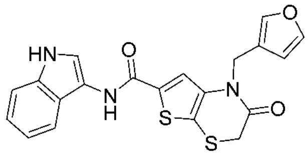

**Step 1: methyl 1-(furan-3-ylmethyl)-2-oxo-2,3-dihydro-1*H*-thieno[2,3-*b*][1,4]thiazin-6-carboxylate (117-1)**

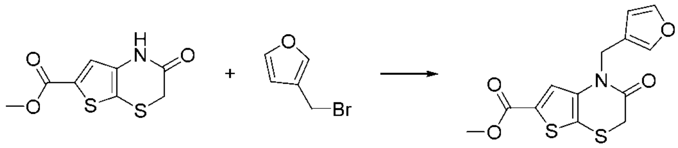

The preparation of methyl 1-(furan-3-ylmethyl)-2-oxo-2,3-dihydro-1*H*-thieno[2,3-*b*][1,4]thiazin-6-carboxylate **(117-1)** was the same as that of compound **2-3.** $^{1}$H NMR (400 MHz, DMSO-$d_6$) $\delta$ 7.77 (s, 1H), 7.57-7.59 (m, 2H), 6.31 (dd, *J*= 1.8, 0.9 Hz, 1H), 4.98 (s, 2H), 3.80 (s, 3H), 3.78(s, 2H). LCMS (ESI) *m/z* [M+H]$^+$: 310.0.

**Step 2: 1-(furan-3-ylmethyl)-2-oxo-2,3-dihydro-1*H*-thieno[2,3-*b*][1,4]thiazin-6-carboxylic acid (117-2)**

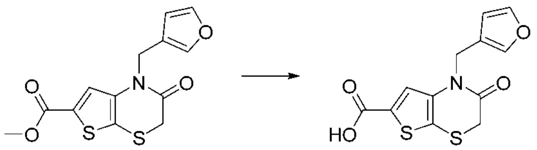

The preparation of 1-(furan-3-ylmethyl)-2-oxo-2,3-dihydro-1*H*-thieno[2,3-b][1,4]thiazin-6-carboxylic acid **(117-2)** was the same as that of compound **1-5.** $^{1}$H NMR (400 MHz, DMSO-$d_6$) $\delta$ 13.28 (br s, 1H), 7.67 (s, 1H), 7.55-7.63 (m, 2H), 6.32 (dd, *J*= 1.8 Hz,0.9 Hz, 1H), 4.96 (s, 2H), 3.77 (s, 2H).

**Step 3: 1-(furan-3-ylmethyl)-*N*-(1*H*-indol-3-yl)-2-oxo-2,3-dihydro-1*H*-thieno[2,3-*b*][1,4]thiazin-6-carboxamide (I-117)**

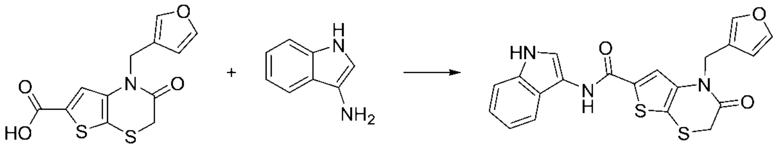

The preparation of *1-(furan-3-ylmethyl)-N-(1H-indol-3-yl)-2-oxo-2,3-dihydro-1H*-thieno[2,3-b][1,4]thiazin-6-carboxamide **(I-117)** was the same as that of compound **I-1.** 1H NMR (400 MHz, DMSO-$d_6$) $\delta$ 10.99 (s, 1H), 8.09(s, 1H), 7.75 (d, *J* = 8.0 Hz, 1H), 7.70 (s, 1H), 7.67 (d, *J*= *2.5* Hz, 1H), 7.63 (d, *J* = 1.8 Hz, 1H), 7.37(d, *J* = 8.1Hz, 1H), 7.13 (t, *J* = 7.5 Hz, 1H), 7.04 (t, *J* = 7.4 Hz, 1H), 6.44 (d, *J* = 1.9Hz, 1H), 4.92 (s, 2H), 3.76(s, 2H). LCMS (ESI) *m/z* [M+H]$^+$: 410.0.

**Example 118 1-(furan-2-ylmethyl)-*N*-(1*H*-indol-3-yl)-2-oxo-2,3-dihydro-1*H*-thieno[2,3-*b*] [1,4]thiazin-6-carboxamide (I-118)**

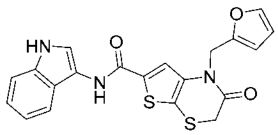

**Step 1: methyl 1-(furan-2-ylmethyl)-2-oxo-2,3-dihydro-1*H*-thieno[2,3-*b*][1,4]thiazin-6-carboxylate (118-1)**

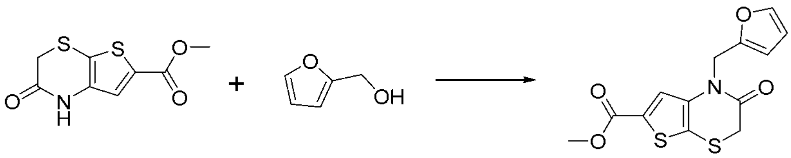

The preparation of methyl 1-(furan-2-ylmethyl)-2-oxo-2,3-dihydro-1*H*-thieno[2,3-b][1,4]thiazin-6-carboxylate **(118-1)** was the same as that of compound **9-2.** $^1$H NMR (400 MHz, DMSO-$d_6$) $\delta$ 7.87 (s, 1H), 7.57-7.59(m, 2H), 6.37-6.39 (m, 1H), 5.15 (s, 2H), 3.81 (s, 3H), 3.77(s, 2H). LCMS (ESI) *m*/*z* [M+H]$^+$: 310.0.

**Step 2: 1-(furan-2-ylmethyl)-2-oxo-2,3-dihydro-1*H*-thieno[2,3-*b*][1,4]thiazin-6-carboxylic acid (118-2)**

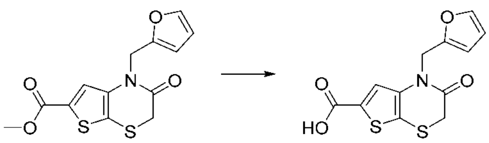

The preparation of 1-(furan-2-ylmethyl)-2-oxo-2,3-dihydro-1*H*-thieno[2,3-*b*][1,4]thiazin-6-carboxylic acid **(118-2)** was the same as that of compound **1-5.** $^1$H NMR (400 MHz, $CD_3OD$) $\delta$ 7.75 (s, 1H), 7.42 (d, *J*= 1.9Hz, 1H), 6.29-6.37 (m, 2H), 5.11 (s, 2H), 3.65 (s, 2H).

**Step 3: 1-(furan-2-ylmethyl)-*N*-(1*H*-indol-3-yl)-2-oxo-2,3-dihydro-1*H*-thieno[2,3-*b*][1,4]thiazin-6-carboxamide (I-118)**

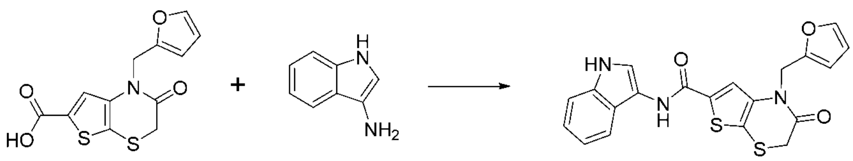

The preparation of 1-(furan-2-ylmethyl)-*N*-(1*H*-indol-3-yl)-2-oxo-2,3-dihydro-1*H*-thieno[2,3-*b*][1,4]thiazin-6-carboxamide **(I-118)** was the same as that of compound **I-1.** $^1$H NMR (400 MHz, $CD_3OD$) $\delta$ 8.05(s, 1H), 7.70 (d, *J*= 8.0 Hz, 1H), 7.62 (s, 1H), 7.47 (s, 1H), 7.39 (d, *J*= 8.1 Hz, 1H), 7.17(t, *J* = 7.6Hz, 1H), 7.08 *(t, J*= 7.5 Hz, 1H), 6.34-6.41 (m, 2H), 5.17 (s, 2H), 3.69 (s, 2H). LCMS (ESI) *m*/*z* [M+H]$^+$: 410.0.

**Example 119 *N*-(1*H*-indol-3-yl)-2-oxo-1-((tetrahydro-2*H*-pyran-4-yl)methyl)-2,3-dihydro-1*H*-thieno[2,3-*b*][1,4] thiazin-6-carboxamide (I-119)**

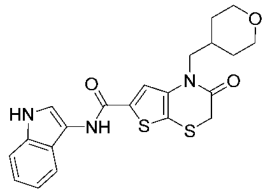

**Step 1: methyl 2-oxo-1-((tetrahydro-2*H*-pyran-4-yl)methyl)-2,3-dihydro-1*H*-thieno[2,3-*b*][1,4]thiazin-6-carboxylate (119-1)**

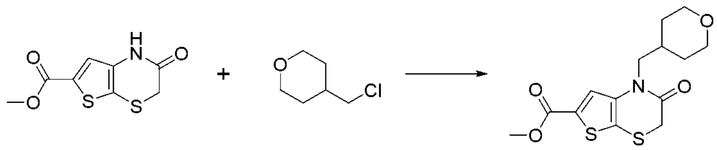

The preparation of methyl 2-oxo-1-((tetrahydro-2*H*-pyran-4-yl)methyl)-2,3-dihydro-1*H*-thieno[2,3-b][1,4]thiazin-6-carboxylate **(119-1)** was the same as that of compound **2-3.** LCMS (ESI) *m*/*z* $[M+H]^+$: 328.0.

**Step 2: 2-oxo-1-((tetrahydro-2*H*-pyran-4-yl)methyl)-2,3-dihydro-1*H*-thieno[2,3-*b*][1,4]thiazin-6-carboxylic acid (119-2)**

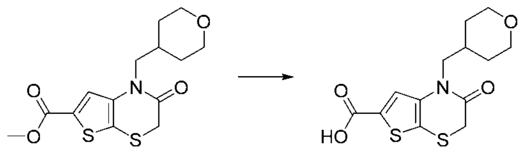

The preparation of 2-oxo-1-((tetrahydro-2*H*-pyran-4-yl)methyl)-2,3-dihydro-1*H*-thieno[2,3-b][1,4]thiazin-6-carboxylic acid **(119-2)** was the same as that of compound **1-5.** $^1$H NMR (400 MHz, DMSO-$d_6$) $\delta$ 7.77 (s, 1H), 3.85 (d, *J* = 7.5 Hz, 2H), 3.79 (dt, *J* = 10.0, 3.6 Hz, 2H), 3.68 (s, 2H), 3.15-3.22 (m, 2H), 1.72-1.84 (m, 1H), 1.42- 1.50 (m, 2H), 1.17-1.27 (m, 2H).

**Step 3: *N*-(1*H*-indol-3-yl)-2-oxo-1-((tetrahydro-2*H*-pyran-4-yl)methyl)-2,3-dihydro-1*H*-thieno[2,3-*b*][1,4]thiazin-6-carboxamide (I-119)**

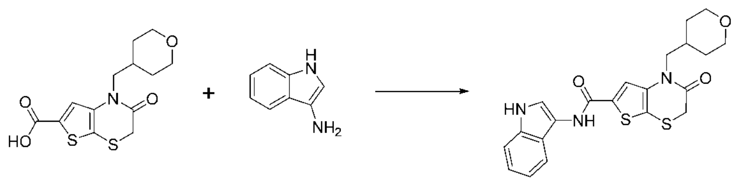

The preparation of *N*-(1*H*-indol-3-yl)-2-oxo-1-((tetrahydro-2H-pyran-4-yl)methyl)-2,3-dihydro-1H-thieno[2,3-*b*][1,4]thiazin-6-carboxamide **(I-119)** was the same as that of compound **I-1.** $^1$H NMR (400 MHz, DMSO-$d_6$) $\delta$ 11.00 (s, 1H), 10.10 (s, 1H), 8.10 (s, 1H), 7.75 (d, *J* = 8.0 Hz, 1H), 7.67 (d, *J*= 2.5 Hz, 1H), 7.38 (d, *J*= 8.1 Hz, 1H), 7.13 (t, *J*= 7.4 Hz, 1H), 7.04 (t, *J*= 7.5 Hz, 1H), 3.80-3.88 (m, 4H), 3.70 (s, 2H), 3.24 (t, *J* = 11.3 Hz, 2H), 1.97 (d, *J* = 14.1 Hz, 1H), 1.52 (d, *J*= 12.9 Hz, 2H), 1.20-1.36 (m, 2H).

**Example 120 N-(5-fluoro-1*H*-indol-3-yl)-1-(furan-2-ylmethyl)-2-oxo-2,3-dihydro-1*H*-thieno[2,3-*b*][1,4]thiazin-6-carboxamide (1-120)**

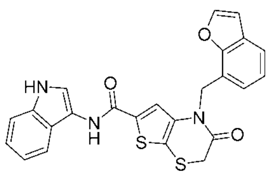

**Step 1: methyl 1-(benzofuran-7-ylmethyl)-2-oxo-2,3-dihydro-1*H*-thieno[2,3-b][1,4]thiazin-6-carboxylate (120-1)**

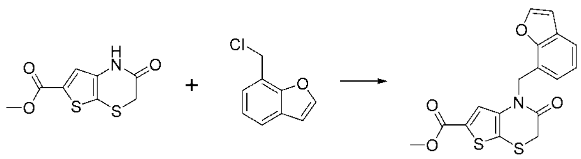

The preparation of methyl 1-(benzofuran-7-ylmethyl)-2-oxo-2,3-dihydro-1*H*-thieno[2,3-*b*][1,4]thiazin-6-carboxylate **(120-1)** was the same as that of compound **2-3.** $^1$H NMR (400 MHz, DMSO-$d_6$) $\delta$ 8.05 (d, *J* = 2.3 Hz, 1H), 7.64 (s, 1H), 7.56 (d, *J*= 7.7 Hz, 1H), 7.18 (t, *J*= 7.6 Hz, 1H), 6.98-7.03 (m, 2H), 5.45 (s, 2H), 3.88 (s, 2H), 3.75 (s, 3H). LCMS (ESI) *m/z* [M+H]$^+$: 360.0.

**Step 2: 1-(benzofuran-7-ylmethyl)-2-oxo-2,3-dihydro-1*H*-thieno[2,3-*b*][1,4]thiazin-6-carboxylic acid (120-2)**

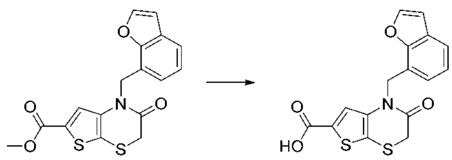

The preparation of 1-(benzofuran-7-ylmethyl)-2-oxo-2,3-dihydro-1*H*-thieno[2,3-*b*][1,4]thiazin-6-carboxylic acid **(120-2)** was the same as that of compound **1-5.** LCMS (ESI) *m/z* [M+H]$^+$: 346.0.

**Step 3: 1-(benzofuran-5-ylmethyl)-*N*-(1*H*-indol-3-yl)-2-oxo-2,3-dihydro-1H-thieno[2,3-*b*] [1,4]thiazin-6-carboxamide (I-120)**

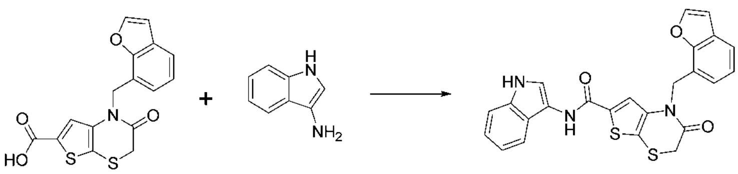

The preparation of 1-(benzofuran-7-ylmethyl)-*N*-(1*H*-indol-3-yl)-2-oxo-2,3-dihydro-1H-thieno[2,3-*b*][1,4]thiazin-6-carboxamide **(I-120)** was the same as that of compound **I-1.** $^1$H NMR (400 MHz, DMSO-$d_6$) $\delta$ 10.95 (s, 1H), 10.05 (s, 1H), 8.08 (d, *J* = 2.2 Hz, 1H), 8.03 (s, 1H), 7.56-7.67 (m, 3H), 7.34 (d, *J* = 8.2 Hz, 1H), 7.21 (t, *J* = 7.6 Hz, 1H), 7.09 (t, *J* = 7.6 Hz, 1H), 6.96-7.05 (m, 3H), 5.42 (s, 2H), 3.87 (s, 2H). LCMS (ESI) *m/z* [M+H]$^+$: 460.1.

**Example 121 *N*-(5-fluoro-1*H*-indol-3-yl)-1-(furan-2-ylmethyl)-2-oxo-2,3-dihydro-1H-thieno[2,3-*b*][1,4]thiazin-6-carboxamide (I-121)**

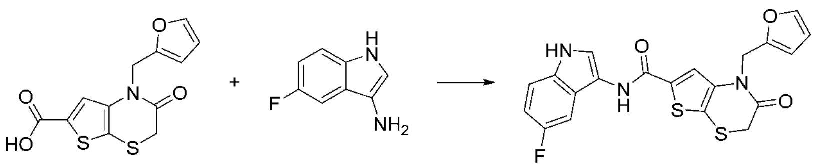

The preparation of *N*-(5-fluoro-1*H*-indol-3-yl)-1-(furan-2-ylmethyl)-2-oxo-2,3-dihydro-1*H*-thieno[2,3-*b*][1,4]thiazin-6-carboxamide **(I-121)** was the same as that of compound **I-1.** $^1$H NMR (400 MHz, $CD_3OD$) $\delta$ 8.01 (s, 1H), 7.65 (s, 1H), 7.45 (d, *J* = 1.5 Hz, 1H), 7.30-7.39 (m, 2H), 6.92 (td, *J* = 9.2, 2.6 Hz, 1H), 6.32-6.38 (m, 2H), 5.15 (s, 2H), 3.67 (s, 2H). LCMS (ESI) *m/z* $[M+H]^+$: 428.0.

**Example 122 *N*-(5-fluoro-1*H*-indol-3-yl)-1-(furan-3-ylmethyl)-2-oxo-2,3-dihydro-1*H*-thieno[2,3-*b*][1,4]thiazin-6-carboxamide (I-122)**

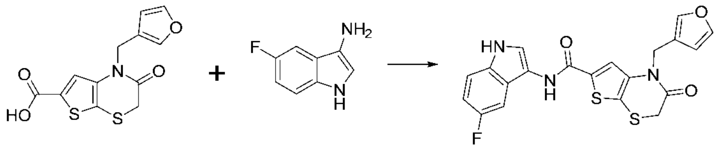

The preparation of *N*-(5-fluoro-1*H*-indol-3-yl)-1-(furan-3-ylmethyl)-2-oxo-2,3-dihydro-1*H*-thieno[2,3-b][1,4]thiazin-6-carboxamide **(I-122)** was the same as that of compound **I-1.** $^1$H NMR (400 MHz, DMSO-$d_6$) $\delta$ 11.10 (s, 1H), 10.10 (s, 1H), 8.06 (s, 1H), 7.75 (d, *J*= 2.6 Hz, 1H), 7.70 (s, 1H), 7.62 (s, 1H), 7.51 (m, 1H), 7.38 (dd, *J*= 8.8, 4.5 Hz, 1H), 6.97 (td, *J* = 9.1, 2.6 Hz, 1H), 6.43 (s, 1H), 4.92 (s, 2H), 3.76 (s, 2H). LCMS (ESI) *m/z* $[M+H]^+$: 428.0.

**Example 123 *N*-(5-chloro-1*H*-indol-3-yl)-1-(3-fluoro-5-(trifluoromethyl)benzyl)-2-oxo-2,3-dihydro-1*H*-thieno[2,3-*b*][1,4]thiazin-6-carboxamide (I-123)**

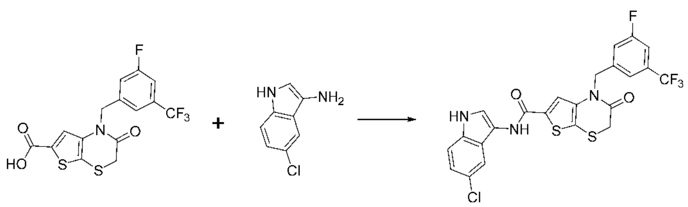

The preparation method of *N*-(5-chloro-1*H*-indol-3-yl)-1-(3-fluoro-5-(trifluoromethyl) benzyl)-2-oxo-2,3-dihydro-1*H*-thieno[2,3-b][1,4]thiazin-6-carboxamide **(I-123)** was the same as that of compound **I-1.** $^1$H NMR (400 MHz, DMSO-$d_6$) $\delta$ 11.20 (s, 1H), 10.10 (s, 1H), 7.98 (s, 1H), 7.79 (d, *J* = 2.1 Hz, 1H), 7.75 (d, *J* = 2.6 Hz, 1H), 7.56-7.68 (m, 2H), 7.35-7.47 (m, 2H), 7.12 (dd, *J* = 8.6, 2.1 Hz, 1H), 5.22 (s, 2H), 3.90 (s, 2H). LCMS (ESI) *m/z* $[M+H]^+$: 540.1.

**Example 124 *N*-(5-fluoro-1*H*-indol-3-yl)-1-(3-fluoro-5-(trifluoromethyl)benzyl)-2-oxo-2,3-dihydro-1*H*-thieno[2,3-*b*][1,4]thiazin-6-carboxamide (I-124)**

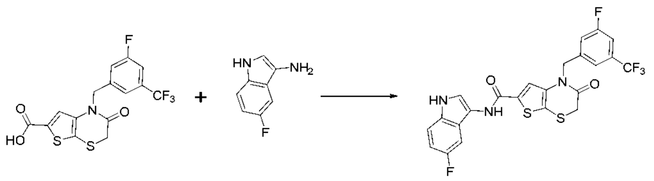

The preparation method of *N*-(5-fluoro-1*H*-indol-3-yl)-1-(3-fluoro-5-(trifluoromethyl) benzyl)-2-oxo-2,3-dihydro-1H-thieno[2,3-*b*][1,4]thiazin-6-carboxamide **(I-124)** was the same as that of compound **I-1.** $^1$H NMR (400 MHz, DMSO-$d_6$) $\delta$ 11.10 (s, 1H), 10.05 (s, 1H), 7.96 (s, 1H), 7.72 (d, *J* = 2.6 Hz, 1H), 7.55-7.68 (m, 2H), 7.33-7.49 (m, 3H), 6.97 (td, *J*= 9.1, 2.6 Hz, 1H), 5.22 (s, 2H), 3.89 (s, 2H). LCMS (ESI) *m*/*z* [M+H]$^+$: 524.2.

**Example 125 N-(5,6-difluoro-1*H*-indol-3-yl)-1-(3-fluoro-5-(trifluoromethyl)benzyl)-2-oxo-2,3-dihydro-1*H*-thieno [2,3-*b*][1,4]thiazin-6-carboxamide (I-125)**

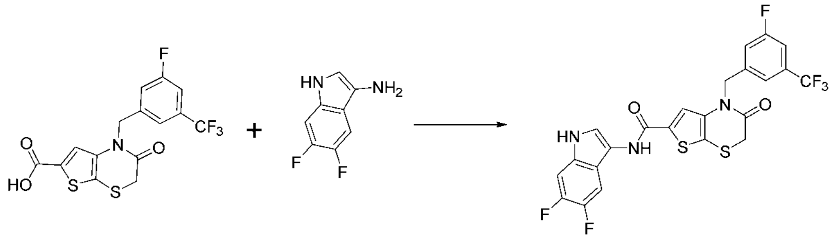

The preparation method of *N*-(5,6-difluoro-1*H*-indol-3-yl)-1-(3-fluoro-5-(trifluoromethyl)benzyl)-2-oxo-2,3-dihydro-1*H*-thieno[2,3-b][1,4]thiazin-6-carboxamide **(I-125)** was the same as that of compound **I-1.** $^1$H NMR (400 MHz, DMSO-$d_6$) $\delta$ 11.16 (s, 1H), 10.09 (s, 1H), 7.95 (s, 1H), 7.56-7.74 (m, 4H), 7.34-7.45 (m, 2H), 5.22 (s, 2H), 3.89 (s, 2H). LCMS (ESI) *m*/*z* [M+H]$^+$: 541.2.

**Example 126 1-(benzofuran-7-ylmethyl)-*N*-(5-fluoro-1*H*-indol-3-yl)-3,3-dimethyl-2-oxoindolin-6-carboxamide (I-126)**

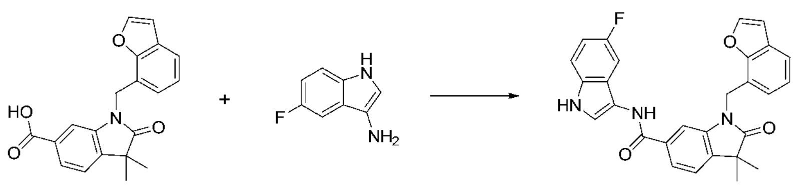

The preparation of 1-(benzofuran-7-ylmethyl)-*N*-(5-fluoro-1*H*-indol-3-yl)-3,3-dimethyl-2-oxoindolin-6-carboxamide **(I-126)** was the same as that of compound **I-1.** $^1$H NMR (400 MHz, $CD_3OD$) $\delta$ 7.76 (d, *J* = 2.2 Hz, 1H), 7.68 (dd, *J*= 7.7, 1.6 Hz, 1H), 7.66 (s, 1H), 7.53-7.57 (m, 1H), 7.49 (d, *J*= 1.5 Hz, 1H), 7.45 (d, *J*= 7.7 Hz, 1H), 7.28-7.34 (m, 2H), 7.17-7.23 (m, 2H), 6.91 (td, *J* = 9.1, 2.5 Hz, 1H), 6.84 (d, *J* = 2.3 Hz, 1H), 5.33 (s, 2H), 1.47 (s, 6H). LCMS (ESI) *m*/*z* [M+H]$^+$: 468.2.

**Example 127 1-(benzofuran-7-ylmethyl)-*N*-(5,6-difluoro-1*H*-indol-3-yl)-3,3-dimethyl-2-oxoindolin-6-carboxamide (I-127)**

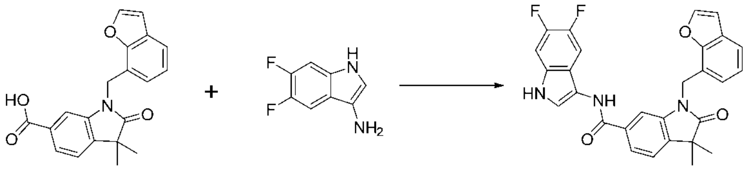

The preparation of 1-(benzofuran-7-ylmethyl)-*N*-(5,6-difluoro-1*H*-indol-3-yl)-3,3-dimethyl-2-oxoindolin-6-carboxamide **(I-127)** was the same as that of compound **I-1.** $^1$H NMR (400 MHz, $CD_3OD$) $\delta$ 7.76 (d, *J* = 2.3 Hz, 1H), 7.68 (dd, *J* = 7.7, 1.6 Hz, 1H), 7.64 (s, 1H), 7.53-7.57 (m, 1H), 7.48 (d, *J* = 1.7 Hz, 1H), 7.47-7.43 (m, 2H), 7.17-7.23 (m, 3H), 6.84 (d, *J* = 2.2 Hz, 1H), 5.33 (s, 2H), 1.47 (s, 6H). LCMS (ESI) *m/z* [M+H]$^+$: 486.2.

**Example 128 1-(benzofuran-7-ylmethyl)-*N*-(5-chloro-1*H*-indol-3-yl)-3,3-dimethyl-2-oxoindolin-6-carboxamide (1-128)**

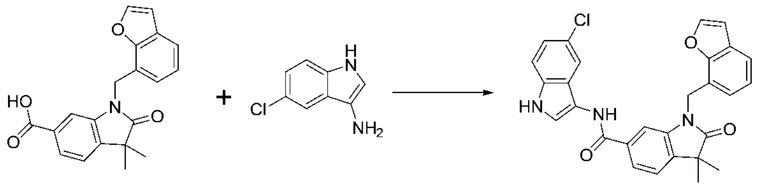

The preparation of 1-(benzofuran-7-ylmethyl)-*N*-(5-chloro-1*H*-indol-3-yl)-3,3-dimethyl-2-oxoindolin-6-carboxamide **(I-129)** was the same as that of compound **I-1.** $^1$H NMR (400 MHz, $CD_3OD$) $\delta$ 7.77 (t, *J*= 1.8 Hz, 1H), 7.66-7.70 (m, 2H), 7.65 (d, *J*= 2.0 Hz, 1H), 7.53-7.58 (m, 1H), 7.49 (d, *J*= 1.5 Hz, 1H), 7.45 (dd, *J*= 7.7, 1.7 Hz, 1H), 7.32 (d, *J*= 8.7 Hz, 1H), 7.18-7.24 (m, 2H), 7.10 (dd, *J*= 8.7, 2.1 Hz, 1H), 6.85 (t, *J*= 1.8 Hz, 1H), 5.33 (d, *J*= 1.7 Hz, 2H), 1.47 (s, 6H). LCMS (ESI) *m/z* [M+H]$^+$: 484.1.

**Example 129 *N*-(1*H*-indol-3-yl)-3,3-dimethyl-2-oxo-1-(thien-3-ylmethyl)-2,3-dihydro-1*H*-pyrrolo[3,2-*b*]pyrid-6-carboxamide (I-129)**

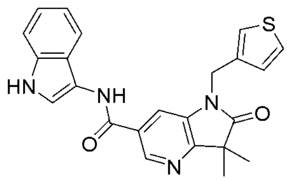

**Step 1: diethyl 2-(5-(methoxycarbonyl)-3-nitropyrid-2-yl)malonate (129-1)**

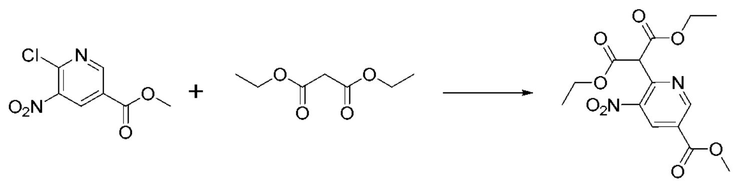

83 Milligrams of sodium hydride was mixed with 10 milliliters of dry tetrahydrofuran in a double-necked round bottom flask equipped with a condenser, then 444 milligrams of diethyl malonate was added to the flask, and the mixture was stirred at 0 °C for 15 minutes under argon atmosphere. Then the reaction system returned to room temperature and a tetrahydrofuran solution containing 500 milligrams of methyl 6-chloro-5-nitronicotinate was added to the reaction solution. The reaction mixture was stirred at 0 °C for 2 hours and then heated at 80 °C for 3 hours. Saturated ammonium chloride solution was added to the reaction solution to quench the reaction, the mixture was extracted with ethyl acetate, and the organic phase was concentrated to dryness. 601 mg of diethyl 2-(5-(methoxycarbonyl)-3-nitropyrid-2-yl)malonate **(129-1)** was obtained as a pale yellow solid with a yield of 76%. $^1$H NMR (400 MHz, DMSO-$d_6$) $\delta$ 9.32 (d, *J* = 1.9 Hz, 1H), 8.88 (d, *J* = 2.0 Hz, 1H), 5.71 (s, 1H), 4.13-4.28 (m, 4H), 3.95 (s, 3H), 1.19 (t, *J*= 7.1 Hz, 6H).

**Step 2: methyl 6-(2-ethoxy-2-oxoethyl)-5-nitronicotinate (129-2)**

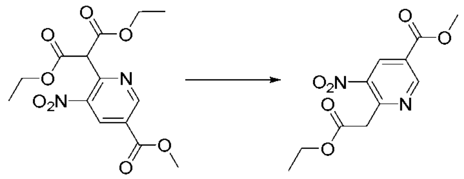

A mixture of 500 mg of **129-1** and 10 mL of acetic acid was stirred overnight at 120 °C. The reaction mixture was added with water and extracted with ethyl acetate. The organic phase was concentrated to dryness, and the residue was subjected to column chromatography with ethyl acetate: petroleum ether = 5:95 to obtain 474 mg of methyl 6-(2-ethoxy-2-oxoethyl)-5-nitronicotinate **(129-2)** as a red oily substance with a yield of 60%. $^1$H NMR (400 MHz, DMSO-$d_6$) $\delta$ 9.29 (d, *J* = 1.9 Hz, 1H), 8.83 (d, *J*= 1.9 Hz, 1H), 4.34 (s, 2H), 4.11 (q, *J*= 7.1 Hz, 2H), 3.95 (s, 3H), 1.17 (t, *J* = 7.1 Hz, 3H).

**Step 3: methyl 6-(1-ethoxy-2-methyl-1-oxoprop-2-yl)-5-nitronicotinate (129-3)**

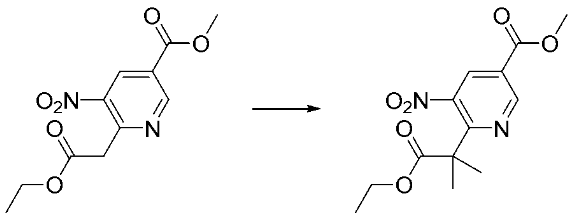

The preparation of methyl 6-(1-ethoxy-2-methyl-1-oxoprop-2-yl)-5-nitronicotinate **(129-3)** was the same as that of compound 85-7. $^1$H NMR (400 MHz, DMSO-$d_6$) $\delta$ 9.32 (d, *J* = 2.0 Hz, 1H), 8.75 (d, *J* = 2.0 Hz, 1H), 4.04 (q, *J* = 7.1 Hz, 2H), 3.95 (s, 3H), 1.65 (s, 6H), 1.11 (t,*J* = 7.1 Hz, 3H).

**Step 4: methyl 3,3-dimethyl-2-oxo-2,3-dihydro-1*H*-pyrrolo[3,2-*b*]pyrid-6-carboxylate (129-4)**

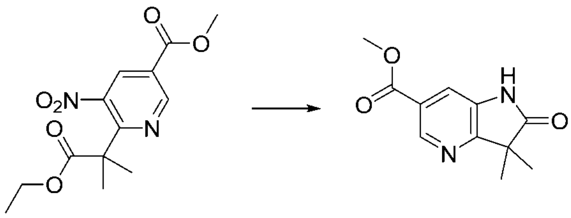

The preparation of methyl 3,3-dimethyl-2-oxo-2,3-dihydro-1*H*-pyrrolo[3,2-*b*]pyrid-6-carboxylate **(129-4)** was the same as that of compound **1-3.** $^1$H NMR (400 MHz, DMSO-$d_6$) $\delta$ 10.75 (s, 1H), 8.67 (d, *J*= 1.8 Hz, 1H), 7.56 (d, *J*= 1.8 Hz, 1H), 3.89 (s, 3H), 1.29 (s, 6H).

**Step 5: methyl 3,3-dimethyl-2-oxo-1-(thien-3-ylmethyl)-2,3-dihydro-1*H*-pyrrolo[3,2-*b*]pyrid-6-carboxylate (129-5)**

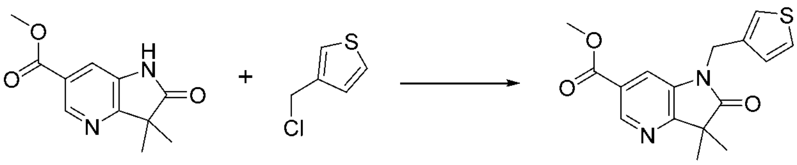

The preparation of methyl 3,3-dimethyl-2-oxo-1-(thien-3-ylmethyl)-2,3-dihydro-1*H*-pyrrolo[3,2-*b*]pyrid-6-carboxylate **(129-5)** was the same as that of compound **2-3.** $^1H$ NMR (400 MHz, DMSO-$d_6$) $\delta$ 8.72 (d, *J*= 1.7 Hz, 1H), 7.74 (d, *J*= 1.8 Hz, 1H), 7.52-7.55 (m, 1H), 7.42-7.45 (m, 1H), 7.01 (dd, *J* = 4.9, 1.3 Hz, 1H), 5.01 (s, 2H), 3.87 (s, 3H), 1.36 (s, 6H).

**Step 6: 3,3-dimethyl-2-oxo-1-(thien-3-ylmethyl)-2,3-dihydro-1*H*-pyrrolo[3,2-*b*]pyrid-6-carboxylic acid (129-6)**

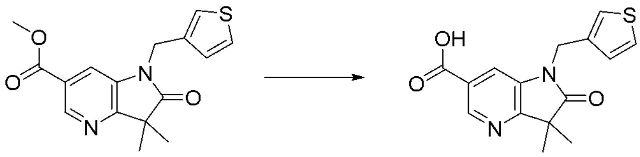

The preparation method of 3,3-dimethyl-2-oxo-1-(thien-3-ylmethyl)-2,3-dihydro-1*H*-pyrrolo[3,2-*b*]pyrid-6-carboxylic acid **(129-6)** was the same as that of compound **1-5.** $^1H$ NMR (400 MHz, DMSO-$d_6$) $\delta$ 13.40 (br s, 1H), 8.71 (d, *J* = 1.7 Hz, 1H), 7.71 (d, *J* = 1.7 Hz, 1H), 7.51-7.55 (m, 1H), 7.42-7.46 (m, 1H), 7.01 (dd, *J*= 4.9, 1.3 Hz, 1H), 5.00 (s, 2H), 1.36 (s, 6H).

**Step 7: *N*-(1*H*-indol-3-yl)-3,3-dimethyl-2-oxo-1-(thien-3-ylmethyl)-2,3-dihydro-1*H*-pyrrolo[3,2-*b*]pyrid-6-carboxamide (I-129)**

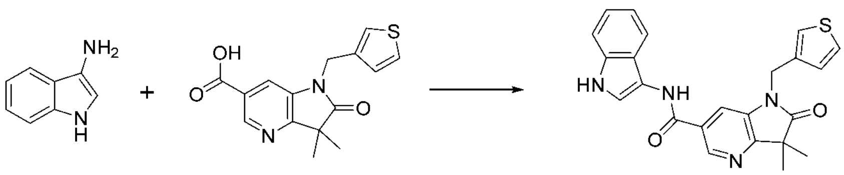

The preparation of *N*-(1*H*-indol-3-yl)-3,3-dimethyl-2-oxo-1-(thien-3-ylmethyl)-2,3-dihydro-1*H*-pyrrolo[3,2-b]pyrid-6-carboxamide **(I-129)** was the same as that of compound **I-1.** $^1H$ NMR (400 MHz, $CD_3OD$) $\delta$ 8.78 (d, *J*= 1.8 Hz, 1H), 7.84 (d, *J*= 1.8 Hz, 1H), 7.68-7.71 (m, 2H), 7.40-7.42 (m, 2H), 7.35-7.39 (m, 1H), 7.15 (ddd, *J* = 8.1, 7.0, 1.2 Hz, 1H), 7.05-7.09 (m, 2H), 5.04 (s, 2H), 1.46 (s, 6H). LCMS (ESI) *m/z* $[M+H]^+$: 417.2.

**Example 130** $IC_{50}$ of compounds as activity inhibitors for interferon stimulated gene (ISG) expression in human THP1-Blue-ISG cells

1. Experimental method:

10 μL of compound diluted with PBS/physiological saline was added to each well of a 96 well cell culture plate. The STING agonist was MSA-2, which was added to the counted cell suspension at a final concentration of 10 μM. For the control group without compounds, 10 μL of PBS/physiological saline containing 2% DMSO was added. 3 replicate wells were set in each group. THP1-Blue-ISG cells were counted and adjusted to a cell concentration of $4\times 10^5$/mL, and 190 μL of cells containing STING agonist were added to each well for incubation. Therefore, the final volume of each test well was 200 μL, the content of DMSO was 0.1%, and the final concentration of the compound tested was 10/3.3/1.1/0.4/0.13/0.04/0.013/0.004 μM, incubated for 24 hours. In addition, 190 μL of cells without STING agonist and 10 μL of physiological saline containing 2% DMSO were added to the blank group. 3 replicate wells were set in each group.

After 24 hours, 20 μL of culture medium was taken from each well and transferred to a new 96 well plate. 180 μL of

chromogenic solution Quanti-Blue was added. The plate was placed in a 37 °C incubator. After 2 hours, the OD650 values were measured.

Result analysis:

$$\text{Inhibition rate (\%)} = (\text{Con(OD650)-Treated(OD650)}) / \text{Con(OD650)} \times 100\%$$

The $IC_{50}$ calculation of inhibitors was implemented using the Graphpad Prism 8 software's calculation method log (inhibitor) vs. response - Variable slope (four parameters), with a constrain interval of 0-100.

2. Experimental results

The test results are shown in Table 1 below.

Table 1: Inhibition $IC_{50}$ of the compounds of the present invention on ISG gene expression induced by MSA-2 in human THP1-Blue-ISG cells

| Compound number | $IC_{50}$ (nM) | Compound number | $IC_{50}$ (nM) | Compound number | $IC_{50}$ (nM) | Compound number | $IC_{50}$ (nM) | Compound number | $IC_{50}$ (nM) |
|---|---|---|---|---|---|---|---|---|---|
| **A** | 2305 | **I-6** | 392 | **I-7** | 390 | **I-9** | 249 | **I-11** | 180 |
| **I-12** | 375 | **I-13** | 391 | **I-15** | 132 | **I-17** | 32 | **I-18** | 56 |
| **I-20** | 296 | **I-55** | 56 | **I-59** | 25 | **I-60** | 42 | **I-62** | 121 |
| **I-64** | 139 | **I-65** | 57 | **I-66** | 73 | **I-76** | 235 | **I-77** | 162 |
| **I-78** | 334 | **I-80** | 626 | **I-81** | 237 | **B** | >10000 | **C** | 1537 |

The experimental results indicate that the compounds of the present invention have the function of inhibiting MSA-2-induced ISG gene expression in human THP1-Blue-ISG cells, and the inhibitory activity of the compounds on STING protein are significantly better than other positive compounds. Among them, the structure of compound A is

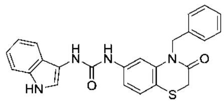,

which is derived from Example 18 of patent WO2021161230A1. The structure of compound B is

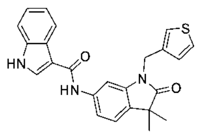;

The structure of compound C is

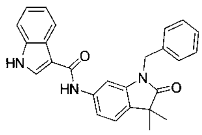;

The structure of H151 is

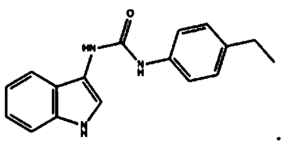.

**Example 131** $IC_{50}$ of inhibitory activity of compounds on ISG gene expression in mouse RAW-Lucia cells

1. Experimental method:

10 μL of compound diluted with PBS/physiological saline was added to each well of a 96 well cell culture plate. The STING agonist was DMXAA, which was added to the counted cell suspension at a final concentration of 50 μM. For the control group without compounds, 10 μL of PBS/physiological saline containing 2% DMSO was added. 3 replicate wells were set in each group. RAW-Lucia cells were counted and adjusted to a cell concentration of $4\times10^5$/mL, and 180 μL of cells were added to each well for incubation. Therefore, the final volume of each test well was 200 μL, the content of DMSO was 0.1%, and the final concentration of the compound tested was 10/3.3/1.1/0.4/0.13/0.04/0.013/0.004 μM. The final concentration of STING agonist DMXAA was 50 μM. The mixture was incubated for 24 hours. In addition, 200 μL of culture medium was added to the blank group. 3 replicate wells were set in each group.

After 24 hours, 20 μL of culture medium was taken from each well and transferred to a new 96 well plate with photopermeable bottom. 50 μL of the luciferase detection reagent QUANTI-Luc™ was added, and the fluorescence value was immediately measured (avoid light).

Result analysis:

$$\text{Inhibition rate (\%)} = (\text{Con(Lum)-Treated(Lum)}) / \text{Con(Lum)} \times 100\%$$

The $IC_{50}$ calculation of inhibitors was implemented using the Graphpad Prism 8 software's calculation method log (inhibitor) vs. response - Variable slope (four parameters), with a constrain interval of 0-100.

2. Experimental results

The experimental results are shown in Table 2 below.

Table 2: Inhibition $IC_{50}$ of the compounds of the present invention on DMXAA induced ISG gene expression in mouse RAW-Lucia cells

| Cmpd. No. | $IC_{50}$ (nM) | Cmpd. No. | $IC_{50}$ (nM) | Cmpd. No. | $IC_{50}$ (nM) | Cmpd. No. | $IC_{50}$ (nM) | Cmpd. No. | $IC_{50}$ (nM) |
|---|---|---|---|---|---|---|---|---|---|
| **I-6** | 33 | **I-11** | 23 | **I-15** | 61 | **I-17** | 6.8 | **I-18** | 19 |
| **I-20** | 16 | **I-28** | 62 | **I-55** | 80 | **I-62** | 38 | **I-76** | 2.24 |
| **I-77** | 2.28 | **I-78** | 7.4 | **I-80** | 24 | **I-81** | 0.42 | **A** | 161 |
| **I-84** | 4.69 | **I-85** | 19.4 | **I-92** | 94 | **I-97** | 17.1 | **I-100** | 15.1 |
| **I-101** | 38.5 | **I-102** | 10.6 | **I-103** | 42.6 | **I-104** | 8.69 | **I-105** | 3.38 |
| **I-106** | 0.24 | **I-107** | 2.72 | **I-108** | 1.74 | **I-109** | 11.8 | **I-110** | 14.3 |
| **I-111** | 29.1 | **I-112** | 41.9 | **I-113** | 27.9 | **I-115** | 7.51 | **I-117** | 76.7 |
| **I-118** | 45.2 | **I-119** | 45.5 | **I-120** | 8.7 | **I-121** | 31.7 | **I-122** | 15.9 |
| **I-123** | 5.71 | **I-124** | <3 | **I-125** | 5.26 | **I-126** | 58.5 | **I-128** | 39.4 |
| **B** | >1000 | **C** | 188 | **H151** | 271.2 | | | | |

The experimental results indicate that the compounds of the present invention have the function of inhibiting DMXAA induced ISG gene expression in mouse RAW-Lucia cells, and the inhibitory activity of the compounds on STING protein is significantly better than other positive compounds.

**Example 132** $IC_{50}$ of compounds as activity inhibitors for interferon stimulated gene (ISG) expression in human THP1-Dual cells

1. Experimental method:

10 μL of compound diluted with PBS/physiological saline was added to each well of a 96 well cell culture plate. The STING agonist was MSA-2, which was added to the counted cell suspension at a final concentration of 10 μM. For the

control group without compounds, 10 μL of PBS/physiological saline containing 2% DMSO was added. 3 replicate wells were set in each group. THP1-Dual cells were counted and adjusted to a cell concentration of $4\times10^5$/mL, and 190 μL of cells containing STING agonist were added to each well for incubation. Therefore, the final volume of each test well was 200 μL, the content of DMSO was 0.1%, and the final concentration of the compound tested was 10/3.3/1.1/0.4/0.13/0.04/0.013/0.004 μM, incubated for 24 hours. In addition, 190 μL of cells without STING agonist and 10 μL of physiological saline containing 2% DMSO were added to the blank group. 3 replicate wells were set in each group.

After 24 hours, 20 μL of culture medium for THP1-Dual cells was taken from each well and transferred to a new 96 well plate with photopermeable bottom. 50 μL of the luciferase detection reagent QUANTI-Luc™ was added, and the fluorescence value was immediately measured (avoid light).

$$\text{Result analysis: Inhibition rate (\%)} = (\text{Con(Lum)} - \text{Treated(Lum)}) / \text{Con(Lum)} \times 100\%$$

The $IC_{50}$ calculation of inhibitors was implemented using the Graphpad Prism 8 software's calculation method log (inhibitor) vs. response - Variable slope (four parameters), with a constrain interval of 0-100.

2. Experimental results

The test results are shown in Table 3 below.

Table 3: Inhibition $IC_{50}$ of the compounds of the present invention on ISG gene expression induced by MSA-2 in human THP1-Dual cells

| Cmpd. No. | $IC_{50}$ (nM) | Cmpd. No. | $IC_{50}$ (nM) | Cmpd. No. | $IC_{50}$ (nM) | Cmpd. No. | $IC_{50}$ (nM) | Cmpd. No. | $IC_{50}$ (nM) |
|---|---|---|---|---|---|---|---|---|---|
| **I-77** | 0.63 | **I-84** | 20.5 | **I-85** | 16.5 | **I-86** | 221 | **I-87** | 149 |
| **I-88** | 143 | **I-89** | 119 | **I-90** | 98 | **I-91** | 117 | **I-92** | 28.8 |
| **I-93** | 74.8 | **I-94** | 106 | **I-95** | 228 | **I-97** | 3.3 | **I-100** | 59.6 |
| **I-101** | 16.4 | **I-102** | 20.8 | **I-103** | 60.7 | **I-104** | 33.1 | **I-105** | 70.4 |
| **I-106** | 16 | **I-107** | 147 | **I-108** | 71.4 | **I-109** | 32.1 | **I-110** | 36.8 |
| **I-111** | 123 | **I-112** | 46.5 | **I-113** | 39.4 | **I-114** | 164 | **I-115** | 12.2 |
| **I-116** | 206 | **I-117** | 85 | **I-118** | 232 | **I-120** | 96.5 | **I-121** | 268 |
| **I-122** | 272 | **I-123** | 69 | **I-124** | 48 | **I-125** | 76.6 | **I-126** | 58.5 |
| **I-127** | 291 | **I-128** | 39.4 | H151 | 635 | | | | |

The experimental results show that the compounds of the present invention have the function of inhibiting MSA-2-induced ISG gene expression in human THP1-Dual cells, and some compounds have significantly better inhibitory activity on STING protein than the positive compound H151.

**Example 133 *In vivo* anti-inflammatory effect of injection administration of compound I-77 on STING agonist (MSA-2) stimulation**

The inflammatory response involved in STING activation stimulation include inflammatory bowel disease, Crohn's disease, ulcerative colitis, irritable bowel syndrome, uveitis, mucositis, etc.

1. Experimental methods

6-8 week C57BL6 female mice, purchased from Shanghai Jihui Experimental Animal Breeding Co., Ltd. Compound preparation method: 2 mg of **I-77** powder was taken, 12 μL of PEG400 was added thereto and ultrasonically dissolved, and then 8 μL of Tween 80 was added and ultrasonically mixed to prepare a 100 mg/mL stock solution.

Experimental grouping: The control group was administered vehicle solvent; The model group was administered MSA-2 (10mpk, dissolved in 40% PEG400); The treatment group (**I-77**) was administered MSA-2 and **I-77** (10mpk, IP). A final concentration of 10 mg/mL of **I-77** solution was prepared by dilution with double distilled water, and 200 μL of the solution was taken and injected intraperitoneally into the mice in treatment group; The control group and MSA-2 group

were injected intraperitoneally with 200 μL of vehicle solvent diluted 10 times. After 3 hours, the model group and **I-77** treatment group were intraperitoneally injected with 200 μL of MSA-2 at a concentration of 10 mg/mL, while the control group was injected with 200 μL of 40% PEG400 solvent. After 3 hours, the kidneys and heart were collected from the experimental endpoint samples. After high-speed centrifugation of blood samples, the plasma was taken and diluted by 2 times, and the levels of CXCL10 and IL6 in the plasma were detected using the Lianke ELISA kit. RNA was extracted from heart and kidney samples, and reversed to cDNA, and inflammation related cytokines (ISG15, ISG56, IL6, and IP10) in tissues were detected using qPCR technology. Statistical method: One way ANOVA, *P<0.05, **P<0.01, ***P<0.001, ***P<0.0001.

2. Experimental results

The above experimental results are shown in Figures 1, 2, and 3. **I-77** injection administration can significantly inhibit the increase in IL6 and IP10 levels in plasma stimulated by MSA-2, and suppress the inflammatory response of the heart and kidneys induced by MSA-2 stimulation.

**Example 134 *In vivo* anti-inflammatory effect of oral administration of compound I-77 on STING agonist (MSA-2) stimulation**

1. Experimental methods

6-8 week C57BL6 female mice, purchased from Shanghai Jihui Experimental Animal Breeding Co., Ltd. Compound preparation method: 2 mg of **I-77** powder was taken, 12 μL of PEG400 was added thereto and ultrasonically dissolved, and then 8 μL of Tween 80 was added and ultrasonically mixed to prepare a 100 mg/mL stock solution.

Experimental grouping: The control group was administered vehicle solvent; The model group was administered MSA-2 (10mpk, dissolved in 40% PEG400); The treatment group (**I-77**) was administered MSA-2 and **I-77** (20mpk, PO). A final concentration of 20 mg/mL of **I-77** solution was prepared by dilution with double distilled water, and 200 μL of the solution was taken and administered intragastrically into the mice in treatment group; The control group and MSA-2 group were administered intragastrically with 200 μL of vehicle solvent diluted 10 times. After 1 hour, the model group and **I-77** treatment group were intraperitoneally injected with 200 μL of 10 mg/mL of MSA-2 dissolved in 40% PEG400, while the control group was injected with 200 μL of 40% PEG400 solvent. After treated for 4 hours, the blood, kidneys and heart were collected from the experimental endpoint samples. After high-speed centrifugation of blood samples, the plasma was taken and diluted by 2 times, and the levels of CXCL10 and IL6 in the plasma were detected using the Lianke ELISA kit. RNA was extracted from heart and kidney samples, and reversed to cDNA, and inflammation related cytokines (ISG15, ISG56, IL6, and IP10) in tissues were detected using qPCR technology. Statistical method: One way ANOVA, *P<0.05, **P<0.01, ***P<0.001, ***P<0.0001.

2. Experimental results

The above experimental results are shown in Figures 4, 5, and 6. Oral administration of **I-77** significantly reduced the levels of IL6 and IP10 in plasma induced by MSA-2 injection, and inhibited the inflammatory response of the heart and kidneys induced by MSA-2 stimulation.

**Example 135 Inflammatory inhibitory effect of oral administration of compound I-77 on STING-V154M mutant autoimmune mouse disease model**

The STING-V154M autoimmune model mouse represents a disease model of constitutive activation of the STING pathway, including STING-associated vasculopathy with onset in infancy (SAVI), Singleton-Merten syndrome (SMS), autoimmune colitis, scleroderma, psoriasis, Sjogren's syndrome, rheumatoid arthritis, etc.

1. Experimental methods

The model mice were purchased from GEMPHARMATECH CO., LTD., and the wild-type C57/BL6 mice were also purchased from GEMPHARMATECH CO., LTD... Compound preparation method: 2 mg of **I-77** powder was taken, 12 μL of PEG400 was added thereto and ultrasonically dissolved, and then 8 μL of Tween 80 was added and ultrasonically mixed to prepare a 100 mg/mL stock solution.

Experimental grouping: Wild type mice control group (Control) was administered vehicle solvent; The model mice group (Model) was administered solvent; The treatment group (**I-77**) was administered **I-77** (20mpk, PO) to the model mice. The mice in the **I-77** treatment group were intragastrically administered 200 μL of **I-77** solution at a final concentration

of 20mg/mL which was prepared by dilution with double-distilled water; The same dose of solvent was administered to the remaining groups until the mortality rate of the model group exceeded 50%. The kidneys and heart were collected from the experimental endpoint samples. RNA extraction was performed on heart and kidneys samples, and the RNA was reversed to cDNA. Changes in gene expression of inflammation related cytokines (ISG15, CCL5, IP10, ISG56) in the tissues were detected using qPCR technology. Statistical method: One way ANOVA, *P<0.05, **P<0.01, ***P<0.001, ***P<0.0001.

2. Experimental results

The experimental results are shown in Figures 7, 8, and 9. Oral administration of **I-77** can significantly prolong the survival of STING V154M mutant model mice, effectively treat autoimmune diseases, and inhibit the cardiac and renal inflammatory responses of STING V154M mutant model mice.

**Example 136 Inflammatory inhibitory effect of oral administration of compound I-77 on Trex1-D18N autoimmune mouse model**

Trex1-D18N autoimmune disease model mice represents a disease model ofupstream activation of the STING pathway, such as arrhythmia syndrome (AGS), systemic lupus erythematosus (SLE), amyotrophic lateral sclerosis (ALS), multiple sclerosis (EAE), familial chilblain lupus erythematosus (FCL), neurodegenerative diseases, etc.

1. Experimental methods

The model mice were purchased from GEMPHARMATECH CO., LTD., and the wild-type C57/BL6 mice were also purchased from GEMPHARMATECH CO., LTD.. Compound preparation method: 2 mg of **I-77** powder was taken, 12 μL of PEG400 was added thereto and ultrasonically dissolved, and then 8 μL of Tween 80 was added and ultrasonically mixed to prepare a 100 mg/mL stock solution.

Experimental grouping: Wild type mice control group (Control) was administered vehicle solvent; The model mice group (Model) was administered vehicle solvent; The treatment group (**I-77**) was administered **I-77** (20mpk, PO) to the model mice. The mice in the **I-77** group were intragastrically administered 200 μL of **I-77** solution at a final concentration of 20mg/mL daily, which was prepared by dilution with double-distilled water; The same dose of vehicle solvent was administered to the remaining groups continuously for one month. The heart was collected from the experimental endpoint samples. RNA extraction was performed on heart samples, and the RNA was reversed to cDNA. Changes in gene expression of inflammation related cytokines (Cxcl10/IP10, ISG15, ISG56, IFN-β, IFN-γ, and STAT2) in the tissues were detected using qPCR technology. Statistical method: One way ANOVA, *P<0.05, **P<0.01, ***P<0.001, ***P<0.0001.

2. Experimental results

The experimental results are shown in Figure 10. Oral administration of **I-77** can significantly inhibit the upregulation of cardiac inflammation related cytokine expression caused by excessive autoimmunity in the Trex1-D18N model, and effectively suppress inflammation in the autoimmune model mice.

**Example 137 Preparation of Pharmaceutical Composition**

Tablet

| | |
|---|---|
| Compound **I-10** | 150g |
| Colloidal silica | 0.6 g |
| Magnesium stearate | 4.5g |
| Modified starch | 15g |
| Microcrystalline cellulose | 210 g |
| Lactose | 120g |

The above substances were mixed evenly and 1000 tablets were prepared through conventional processes. Suitable aqueous or non-aqueous coatings can be used to enhance palatability, improve appearance and stability, or delay

absorption.

Capsule

| | |
|---|---|
| Compound **I-10** | 150g |
| Starch | 140 g |
| Microcrystalline cellulose | 80g |

According to the conventional method, the above substances were mixed evenly and filled into ordinary gelatin capsules to prepare 1000 capsules.

All documents mentioned herein are incorporated by reference in this invention as if each document were individually incorporated by reference. In addition, it should be understood that after reading the above teaching content of the present invention, those skilled in the art can make various changes or modifications to the present invention, and these equivalent forms also fall within the scope defined by the appended claims of the present invention.

## Claims

**1.** A compound represented by formula (I), or a prodrug, an enantiomer, a diastereomer, a racemate, and the mixtures thereof, or a pharmaceutically acceptable salt thereof,

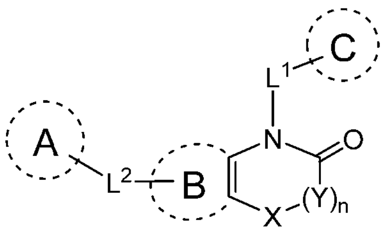

(I)

wherein, ring B is substituted or unsubstituted group selected from the group consisting of 6-membered aryl, 6-membered heteroaryl, and 5-membered heteroaryl, wherein the heteroatoms in the heteroaryl are selected from N, S and O; the number of heteroatoms is 1-3;

ring A and ring C are each independently substituted or unsubstituted group selected from the group consisting of 3-8-membered heterocyclyl, C3-C8 cycloalkyl, 5-membered heteroaryl, 6-membered aryl, 6-membered heteroaryl and

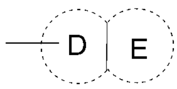

,

wherein the substituted refers to being substituted by 1-5 $R^1$ groups; wherein, each $R^1$ is independently selected from the group consisting of hydrogen, halogen, carboxyl, hydroxyl, cyano, amino, -COO-C1-C6 alkyl, nitro, -CO-(substituted or unsubstituted 3-8-membered heterocyclyl), -CO-(substituted or unsubstituted C3-C8 cycloalkyl), substituted or unsubstituted C6-C12 aryl-C1-C6-alkyl, substituted or unsubstituted 5-12 membered heteroaryl-C1-C6-alkyl, substituted or unsubstituted C1-C6 alkyl, substituted or unsubstituted C2-C6 alkenyl, substituted or unsubstituted C2-C6 alkynyl, substituted or unsubstituted C1-C6 alkoxy, substituted or unsubstituted C1-C6 alkylacyl, substituted or unsubstituted aminoacyl, substituted or unsubstituted C1-C6 alkylamido, substituted or unsubstituted C1-C4 alkylamino, substituted or unsubstituted C6-C12 arylamino, substituted or unsubstituted 3-8-membered heterocyclyl, substituted or unsubstituted C3-C8 cycloalkyl, substituted or unsubstituted 5-12 membered heteroaryl, and substituted or unsubstituted C6-C12 aryl; the substitution in $R^1$ refers to being substituted by one or more groups selected from the group consisting of halogen, carboxyl, hydroxyl, cyano, amino, nitro, benzyl, C1-C6 alkyl-OH, C1-C6 alkylamino, C1-C6 alkyl, halogenated C1-C6 alkyl, C1-C6

alkoxy, halogenated C1-C6 alkoxy, C3-C8 cycloalkyl, and 3-8-membered heterocyclyl;
ring D and ring E are each independently substituted or unsubstituted groups selected from the group consisting of: C5-C6 cycloalkane, benzene ring, 5-6 membered heterocycloalkane, or 5-6 membered heteroaromatic ring;
$L^1$ is selected from the group consisting of -substituted or unsubstituted C1-C6 alkylene-, - (substituted or unsubstituted C1-C6 alkylene)-sulfonyl-, substituted or unsubstituted monocyclic or bicyclic C3-C6 cycloalkyl, -substituted or unsubstituted C2-C6 alkenylene-, -substituted or unsubstituted C2-C6 alkynylene-, -substituted or unsubstituted C1-C6 alkylene-O-, -substituted or unsubstituted C1-C6 alkylene-OCO-, -substituted or unsubstituted monocyclic or bicyclic C6-C12 aryl, substituted or unsubstituted monocyclic or bicyclic 5-10 membered heteroaryl, substituted or unsubstituted monocyclic or bicyclic 3-8 membered heterocyclyl,

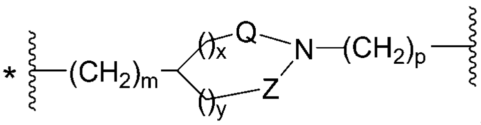, and 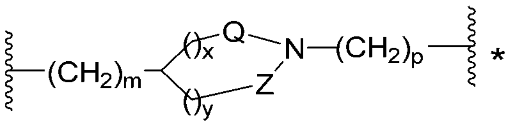;

wherein, m and p are each independently 0-3; x and y are each independently selected from 0-4; wherein "*" represents the connection to ring C;
$L^2$ is selected from the group consisting of

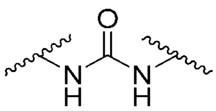, 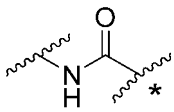, 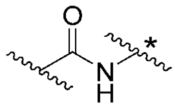,

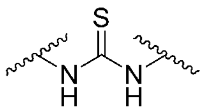, 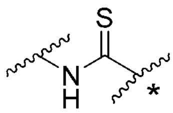, and 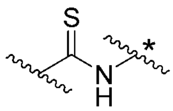,

wherein "*" represents the connection to ring B;
X and Y are each independently selected from the group consisting of S, SO, $SO_2$, O, $NR^2$, and $CR^3R^4$;
Q and Z are each independently selected from the group consisting of CO and a chemical bond;
with the proviso that when ring B is a 6-membered aryl, and $L^1$ is not

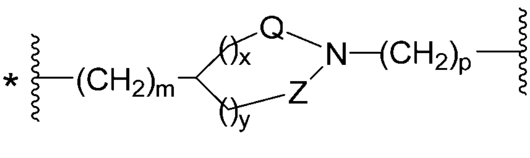 or 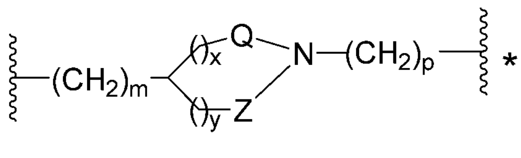,

$L^2$ is

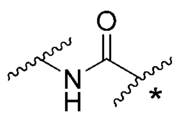 or 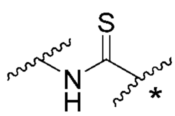,

wherein the "*" represents the connection to ring B;
$R^2$, $R^3$, and $R^4$ are each independently selected from the group consisting of hydrogen atom, halogen, carboxyl, hydroxyl, cyano, amino, nitro, benzyl, C1-C6 alkyl-OH, C1-C6 alkylamino, C1-C6 alkyl, halogenated C1-C6 alkyl, C1-C6 alkoxy, halogenated C1-C6 alkoxy, C3-C8 cycloalkyl, and 3-8-membered heterocyclyl; or $R^3$, $R^4$ and the C atom to which they are attached form a 3-5 membered cycloalkyl or heterocyclyl; or $R^3$, $R^4$ and the C atom to which they are attached form a C2-C6 alkenyl;
n is 0 or 1, wherein, when n is 0, X is directly connected to C=O;
the substituted refers to being substituted by one or more groups selected from the group consisting of halogen, carboxyl, hydroxyl, cyano, amino, nitro, benzyl, C1-C6 alkyl-OH, C1-C6 alkylamino, C1-C6 alkyl, halogenated C1-C6 alkyl, C1-C6 alkoxy, halogenated C1-C6 alkoxy, C3-C8 cycloalkyl, and 3-8-membered heterocyclyl.

**2.** The compound according to claim 1, or the prodrug, the enantiomer, the diastereomer, the racemate, and the mixtures thereof, or the pharmaceutically acceptable salt thereof, wherein ring B is selected from the following structures:

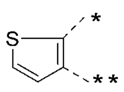 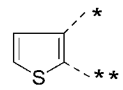 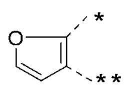 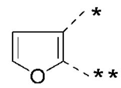 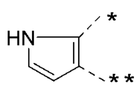 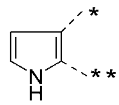

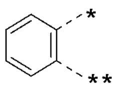 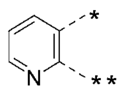 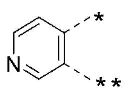 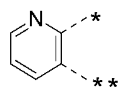 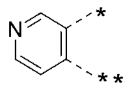 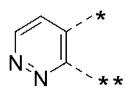

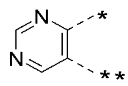 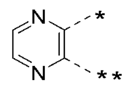

wherein, "*" represents the connection to C atom adjacent to N, and "* *" represents the connection to C atom adjacent to X; $L^2$ is connected to any unsubstituted site of ring B.

**3.** The compound according to claim 1, or the prodrug, the enantiomer, the diastereomer, the racemate, and the mixtures thereof, or the pharmaceutically acceptable salt thereof, wherein the compound has the structure shown in formulas II-VII:

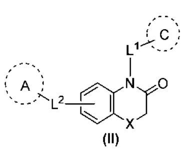
(II)
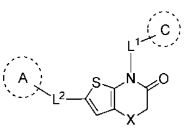
(III)
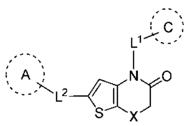
(IV)
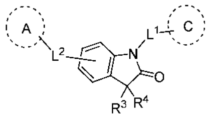
(V)
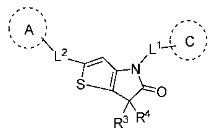
(VI)
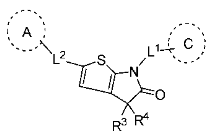
(VII)

wherein, the definitions of ring A, ring C, $R^3$, $R^4$, X, $L^1$, and $L^2$ are as described in claim 1.

**4.** The compound according to claim 3, or the prodrug, the enantiomer, the diastereomer, the racemate, and the mixtures thereof, or the pharmaceutically acceptable salt thereof, wherein the compound has the structure shown in formulas II-VII:

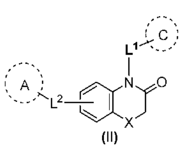
(II)
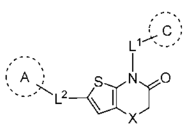
(III)
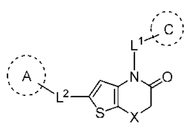
(IV)
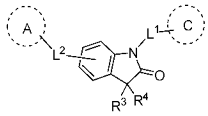
(V)
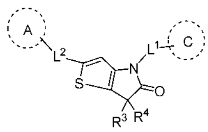
(VI)
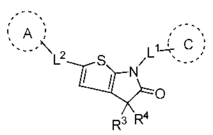
(VII)

wherein, ring A is

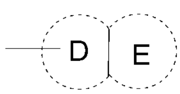,

wherein ring D and ring E are each independently substituted or unsubstituted groups selected from the group consisting of benzene ring and 5-6 membered heteroaromatic ring; the substituted refers to being substituted by 1-5 $R^1$ groups, wherein, $R^1$ is selected from the group consisting of hydrogen, halogen, hydroxyl, amino, C1-C6 alkyl, C2-C6 alkenyl, C1-C6 haloalkyl, C1-C6 alkoxy, C1-C6 alkylhydroxyl, and C1-C4 alkylamino;
ring C is substituted or unsubstituted group selected from the group consisting of 3-6-membered heterocyclyl, C3-C6 cycloalkyl, 6-membered aryl, 6-membered heteroaryl, 5-membered heteroaryl, and

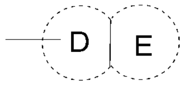,

wherein ring D and ring E are each independently substituted or unsubstituted groups selected from the group consisting of benzene ring, 5-6 membered heterocycloalkane, and 5-6 membered heteroaromatic ring; the substituted refers to being substituted by 1-5 $R^1$ groups, wherein, $R^1$ is selected from the group consisting of hydrogen, halogen, hydroxyl, amino, C1-C6 alkyl, C2-C6 alkenyl, C1-C6 haloalkyl, C1-C6 alkoxy, C1-C6 alkylhydroxyl, C1-C4 alkylamino, and C6 aryl;

$L^1$ is selected from the group consisting of -substituted or unsubstituted C1-C6 alkylene-, - substituted or unsubstituted C2-C6 alkenylene-, -substituted or unsubstituted C1-C6 alkylene-O-,

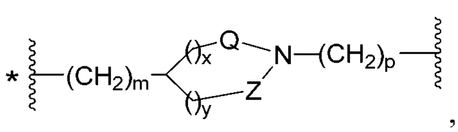, and 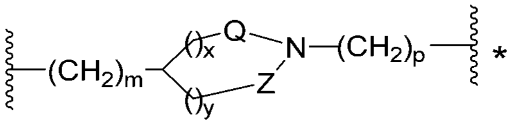;

wherein, m and p are each independently 0, 1, 2, or 3; x and y are each independently selected from 0, 1, 2, 3, or 4; Q and Z are each independently selected from the group consisting of CO and a chemical bond; wherein "*" represents the connection to ring C;

$L^2$ is selected from the group consisting of

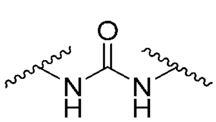, 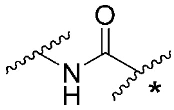, 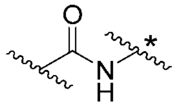,

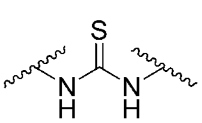, 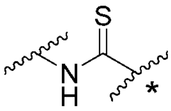, and 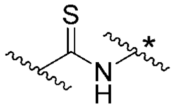,

wherein the "*" in

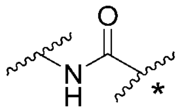,

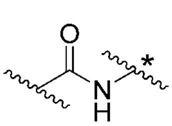, 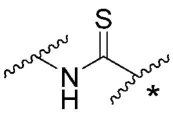, or 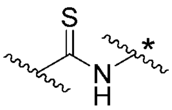

represents the connection to the mother nucleus and the other end is connected to ring A;

$R^3$ and $R^4$ are each independently selected from the group consisting of C1-C6 alkyl, and halogen; or $R^3$, $R^4$ and the C atom to which they are attached form a 3-5 membered cycloalkyl or heterocyclyl; or $R^3$, $R^4$ and the C atom to which they are attached form a C2-C6 alkenyl;

X is selected from the group consisting of O and S atoms;

with the proviso that, in formula II or formula V, when $L^1$ is not

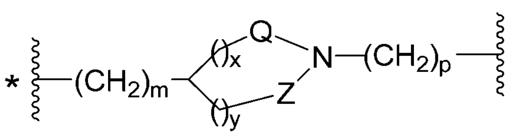 or 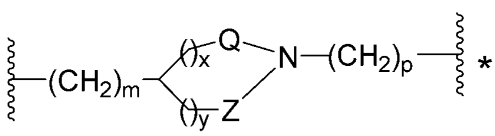, $L^2$

is

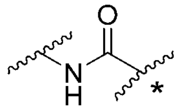
or
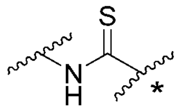,

wherein the "*" in

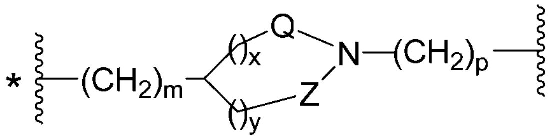

and

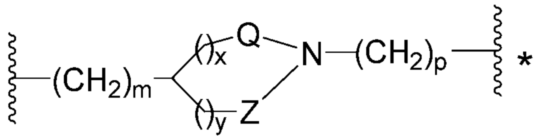

represents the connection to ring C, and the "*" in

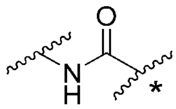
and
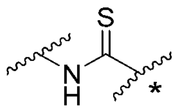

represents the connection to the mother nucleus, and the other end is connected to ring A;
the substituted refers to being substituted by one or more groups selected from the group consisting of halogen, carboxyl, hydroxyl, cyano, amino, nitro, benzyl, C1-C6 alkyl-OH, C1-C6 alkylamino, C1-C6 alkyl, halogenated C1-C6 alkyl, C1-C6 alkoxy, halogenated C1-C6 alkoxy, C3-C8 cycloalkyl, and 3-8-membered heterocyclyl.

**5.** The compound according to any one of claims 1-4, or the prodrug, the enantiomer, the diastereomer, the racemate, and the mixtures thereof, or the pharmaceutically acceptable salt thereof, wherein

in formula II or formula V,
$L^1$ is selected from the group consisting of -substituted or unsubstituted C1-C6 alkylene-, - substituted or unsubstituted C2-C6 alkenylene-, -substituted or unsubstituted C2-C6 alkynylene-, and -substituted or unsubstituted C1-C6 alkylene-O-; and $L^2$ is selected from the group consisting of

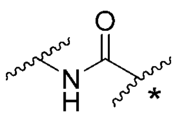
and
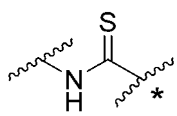,

wherein the "*" represents the connection to the mother nucleus, and the other end is connected to ring A;
or,
in formula II or formula V, $L^1$ is selected from the group consisting of

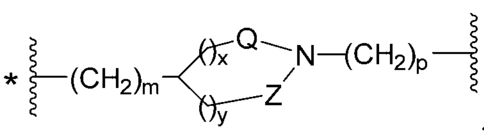
, and
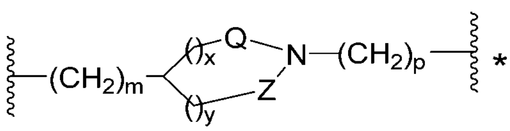,

wherein the "*" represents the connection to ring C;

or,

in formula III or formula IV or formula VI or formula VII, $L^1$ is selected from the group consisting of -substituted or unsubstituted C1-C6 alkylene-, -substituted or unsubstituted C2-C6 alkenylene-, -substituted or unsubstituted C2-C6 alkynylene-, and -substituted or unsubstituted C1-C6 alkylene-O-.

**6.** The compound according to any one of claims 1-5, or the prodrug, the enantiomer, the diastereomer, the racemate, and the mixtures thereof, or the pharmaceutically acceptable salt thereof, wherein ring A is

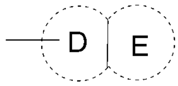,

ring C is substituted or unsubstituted group selected from the group consisting of C3-C6 cycloalkyl, 6-membered aryl, 6-membered heteroaryl, 5-membered heteroaryl, and

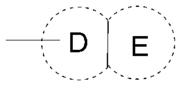.

**7.** The compound according to any one of claims 1-6, or the prodrug, the enantiomer, the diastereomer, the racemate, and the mixtures thereof, or the pharmaceutically acceptable salt thereof, wherein $L^1$ is selected from the group consisting of

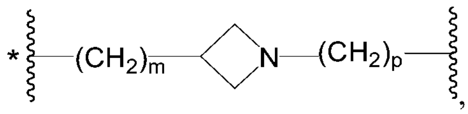,

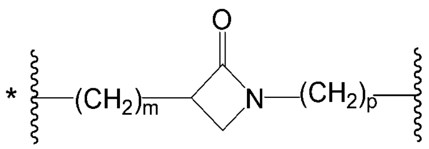, 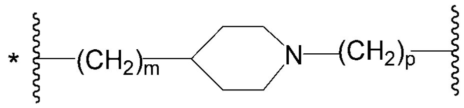,

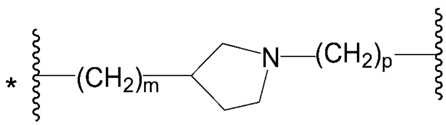, 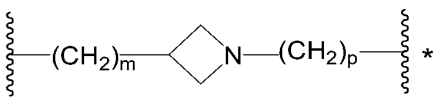,

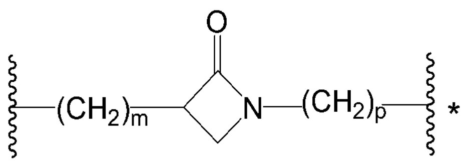, 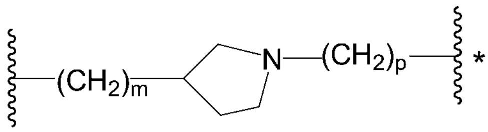,

and

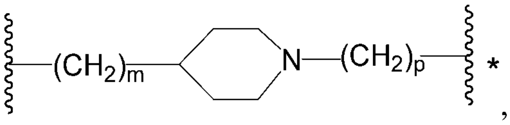,

wherein the "*" represents the connection to ring C.

**8.** The compound according to any one of claims 1-7, or the prodrug, the enantiomer, the diastereomer, the racemate, and the mixtures thereof, or the pharmaceutically acceptable salt thereof, wherein the compound has the structure

shown in formulas II-VII:

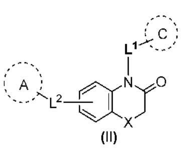
(II)
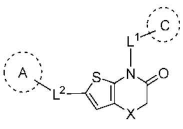
(III)
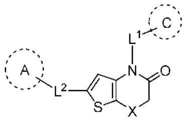
(IV)
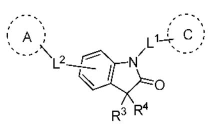
(V)
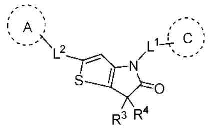
(VI)
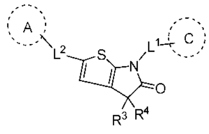
(VII)

wherein, the definitions of ring A, ring C, $L^1$, and $L^2$ are as described in any one of claims 1-7;
$R^3$ and $R^4$ are each independently methyl, ethyl, n-propyl, isopropyl, n-butyl, isobutyl, *tert*-butyl, fluorine, chlorine, or bromine, or $R^3$, $R^4$ and the C atom to which they are attached form cyclopropyl, cyclobutyl, cyclopentyl, or cyclohexyl; or $R^3$, $R^4$ and the C atom to which they are attached form vinyl, propenyl, or allyl;
X is selected from the group consisting of O and S atoms, preferably S atom.

**9.** The compound according to any one of claims 1-8, or the prodrug, the enantiomer, the diastereomer, the racemate, and the mixtures thereof, or the pharmaceutically acceptable salt thereof, wherein the compound has the structure shown in formulas II-VII:

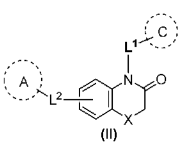
(II)
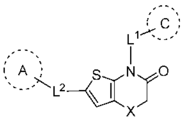
(III)
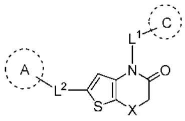
(IV)
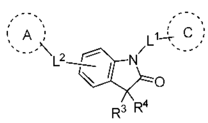
(V)
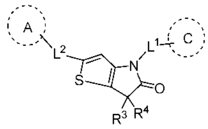
(VI)
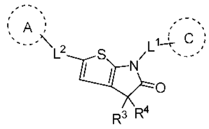
(VII)

wherein, ring A is

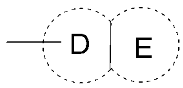,

wherein one of ring D and ring E is a substituted or unsubstituted benzene ring, and the other is a substituted or unsubstituted group selected from the group consisting of pyrrole ring, furan ring, and thiophene ring; the substituted refers to being substituted by 1-5 $R^1$ groups, wherein, $R^1$ is halogen, C1-C6 alkyl, C1-C6 haloalkyl, or C1-C6 alkoxy;
ring C is substituted or unsubstituted group selected from the group consisting of 3-6-membered heterocyclyl, C3-C6 cycloalkyl, 6-membered aryl, 6-membered heteroaryl, 5-membered heteroaryl, and

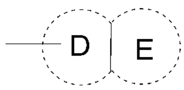,

wherein one of ring D and ring E is a substituted or unsubstituted benzene ring, and the other is a substituted or unsubstituted group selected from the group consisting of 5-membered heterocycloalkane, and 5-membered heteroaromatic ring; the substituted refers to being substituted by 1-5 $R^1$ groups, wherein, $R^1$ is selected from the group consisting of halogen, C1-C6 alkyl, C1-C6 haloalkyl, C1-C6 alkoxy, and C6 aryl;
$L^1$ is selected from the group consisting of C1-C6 alkylene-,

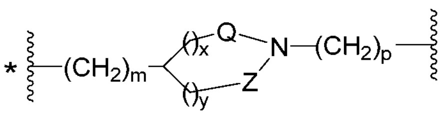, and 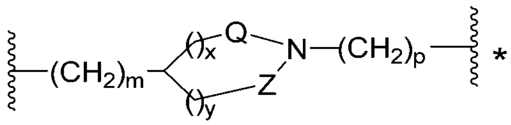;

wherein, m and p are each independently 0, 1, or 2; x and y are each independently selected from 0, 1, or 2; Q and Z are each independently selected from the group consisting of CO and a chemical bond; wherein "*" represents the connection to ring C;
$L^2$ is selected from the group consisting of

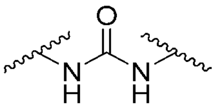, 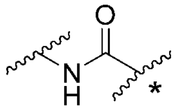, 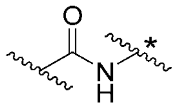,

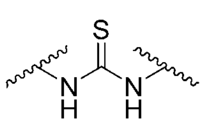, 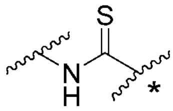, and 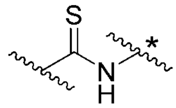,

wherein the "*" in

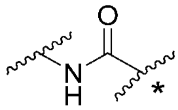,

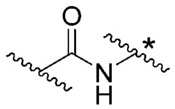, 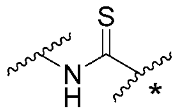, or 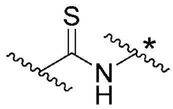

represents the connection to the mother nucleus, the other end is connected to ring A;
$R^3$ and $R^4$ are each independently C1-C6 alkyl;
X is S atom;
with the proviso that, in formula II or formula V, when $L^1$ is not

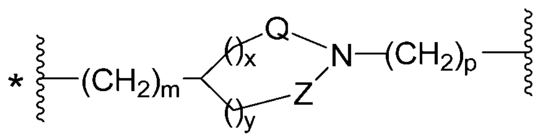 or 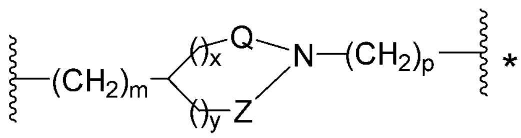,

$L^2$ is

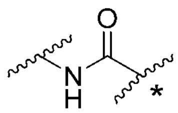 or 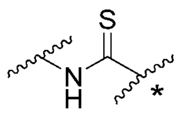,

wherein the "*" in

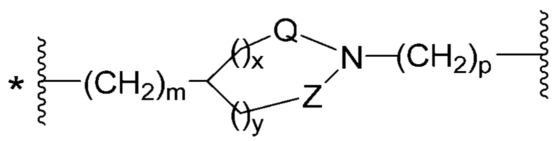

and

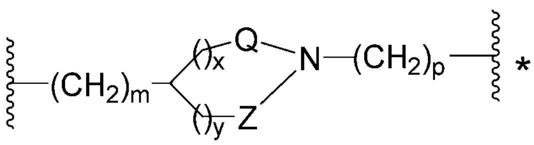

represents the connection to ring C, and the "*" in

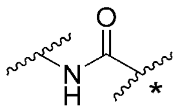
and
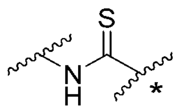

represents the connection to the mother nucleus and the other end is connected to ring A.

**10.** The compound according to any one of claims 1-9, or the prodrug, the enantiomer, the diastereomer, the racemate, and the mixtures thereof, or the pharmaceutically acceptable salt thereof, wherein the compound has the structure shown in formula II or formula IV or formula V or formula VI:

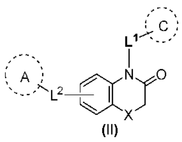
(II)
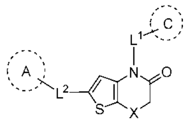
(IV)
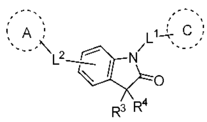
(V)
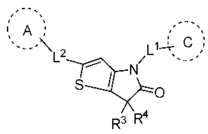
(VI)

wherein, ring A is substituted or unsubstituted group selected from the group consisting of

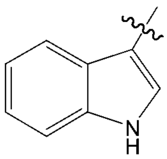
,
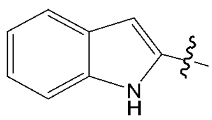
,
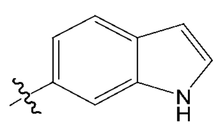
, and
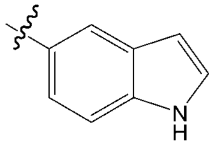
;

the substituted refers to being substituted by 1-5 $R^1$ groups, wherein, $R^1$ is selected from the group consisting of halogen, C1-C6 alkyl, C1-C6 haloalkyl, and C1-C6 alkoxy;
ring C is substituted or unsubstituted group selected from the group consisting of 3-6-membered heterocyclyl, C3-C6 cycloalkyl, 6-membered aryl, 6-membered heteroaryl, 5-membered heteroaryl, and

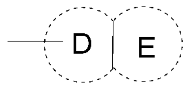
,

wherein one of ring D and ring E is a substituted or unsubstituted benzene ring, and the other is a substituted or unsubstituted group selected from the group consisting of 5-membered heterocycloalkane, and 5-membered heteroaromatic ring; the substituted refers to being substituted by 1-5 $R^1$ groups, wherein, $R^1$ is selected from the group consisting of halogen, C1-C6 alkyl, C1-C6 haloalkyl, C1-C6 alkoxy, and C6 aryl;
$L^1$ is selected from the group consisting of -C1-C6 alkylene-,

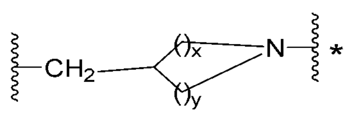
,

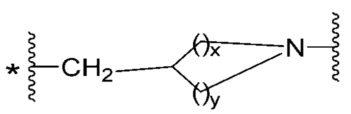
,
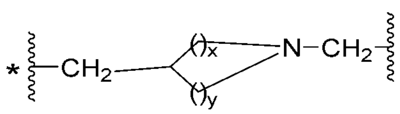
,

and

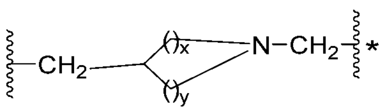
,

wherein, x and y are each independently selected from 0, 1, or 2; where "*" represents the connection to ring C; $L^2$ is selected from the group consisting of

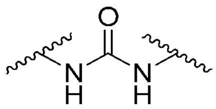 and 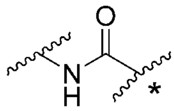,

the "*" in

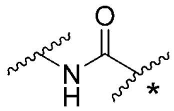

represents the connection to the mother nucleus, and the other end is connected to ring A;
$R^3$ and $R^4$ are each independently C1-C6 alkyl;
X is S atom;
with the proviso that, in formula II or formula V, when $L^1$ is not

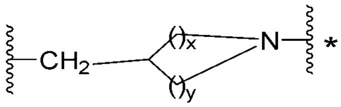,

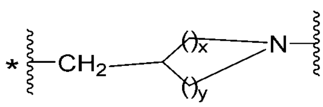, 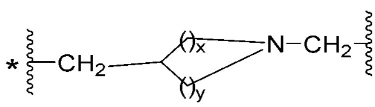, or 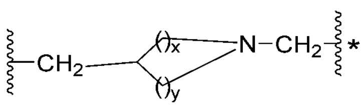,

$L^2$ is

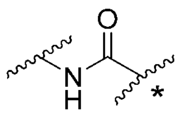,

wherein the "*" in

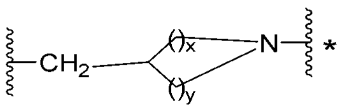, 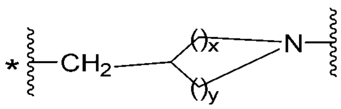,

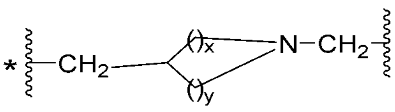, or 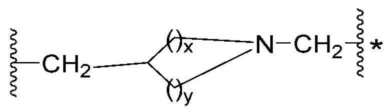

represents the connection to ring C, and the "*" in

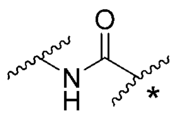

represents the connection to the mother nucleus, and the other end is connected to ring A.

**12.** The compound according to any one of claims 1-11, or the prodrug, the enantiomer, the diastereomer, the racemate, and the mixtures thereof, or the pharmaceutically acceptable salt thereof, wherein the compound is selected from the following compounds:

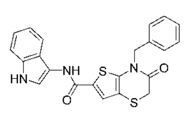 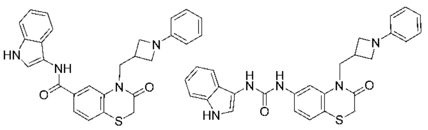 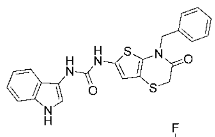 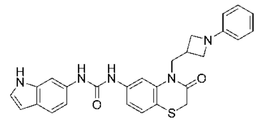

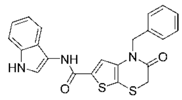 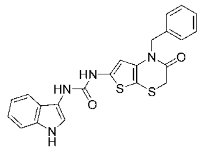 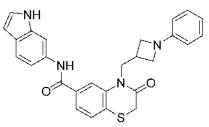 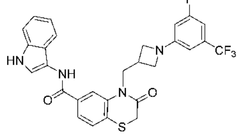 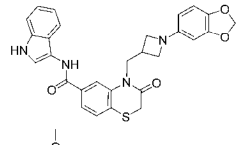

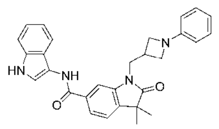 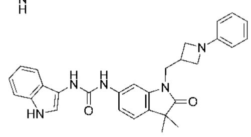 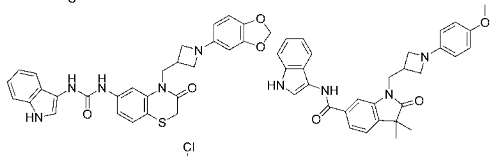 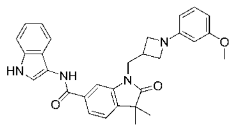

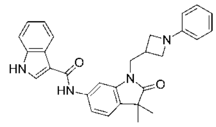 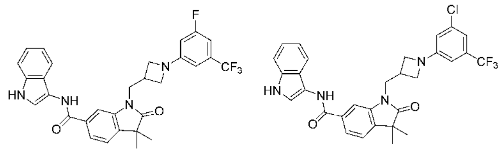 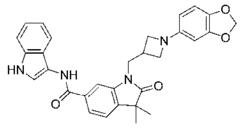 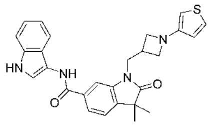

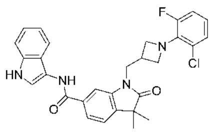 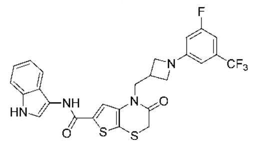 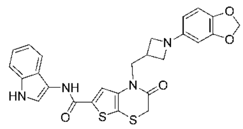 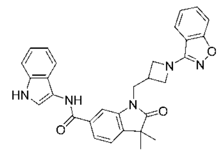 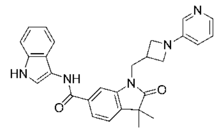

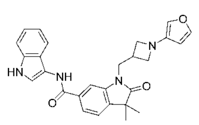 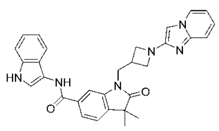 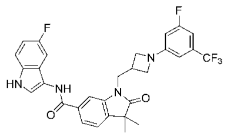 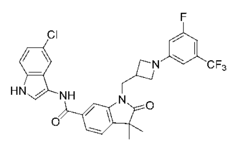 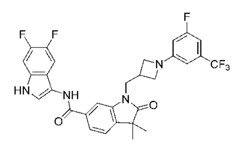

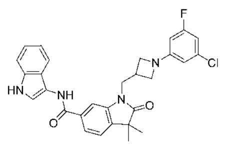 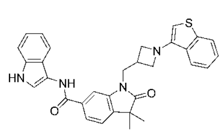 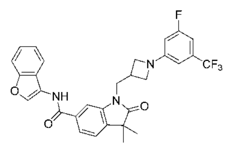 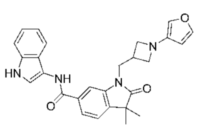 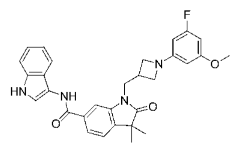

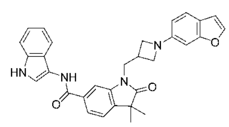 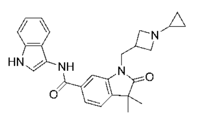 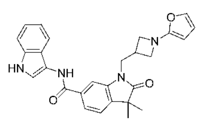 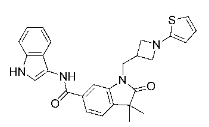 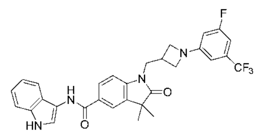

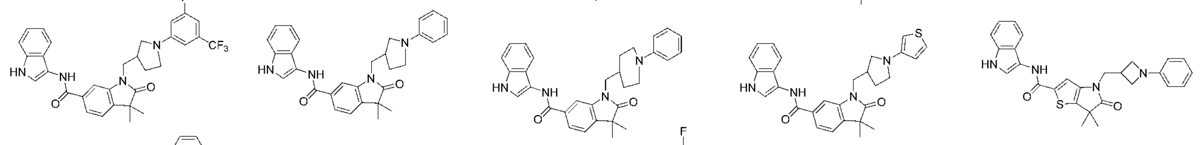

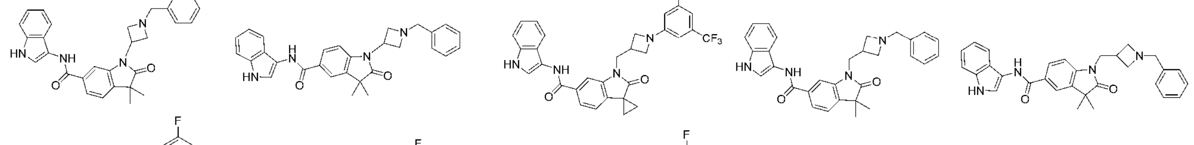

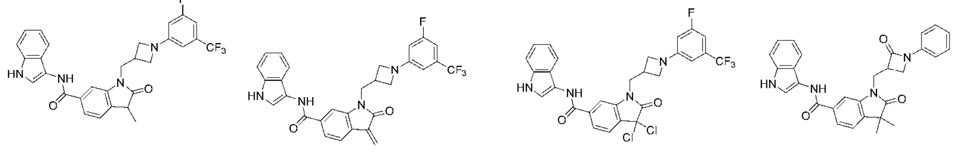

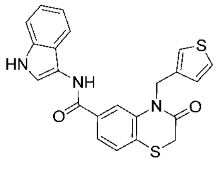
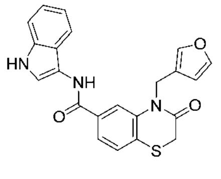
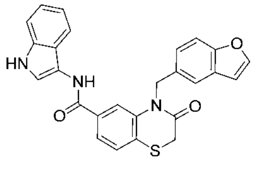
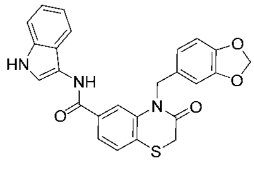
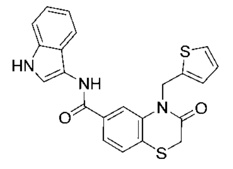

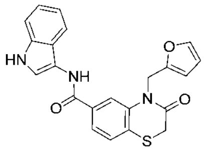
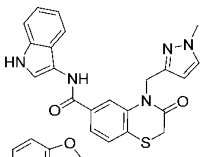
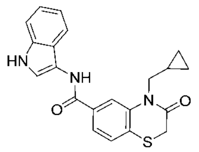
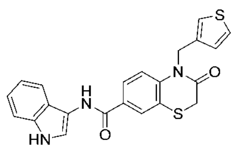
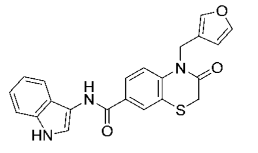

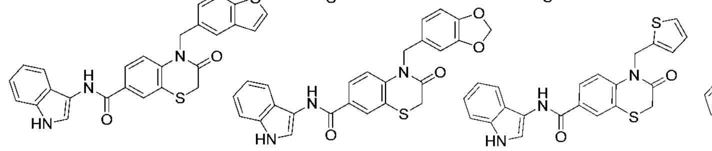
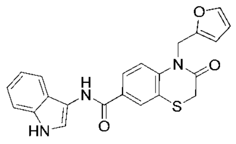
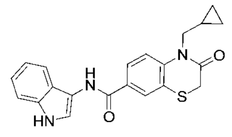

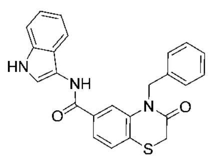
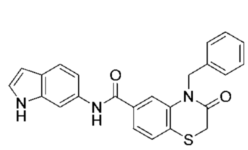
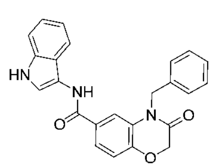
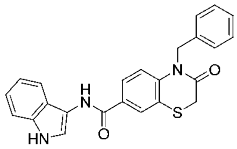
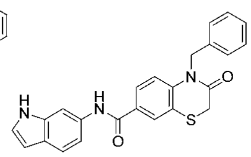

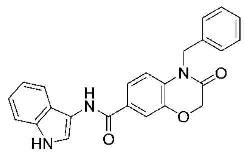
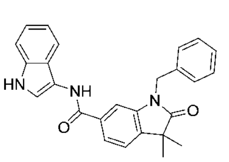
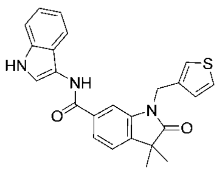
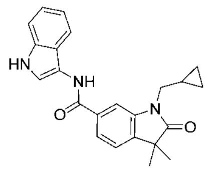
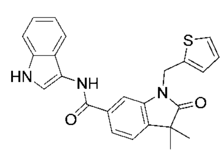

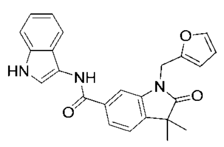
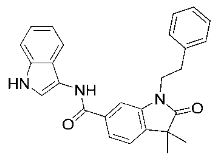
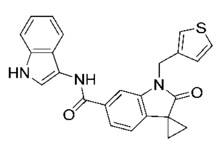
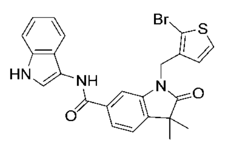
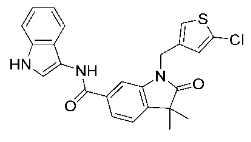

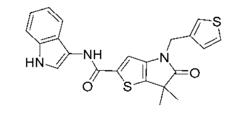
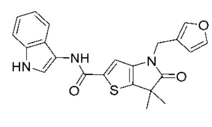
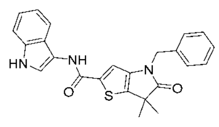
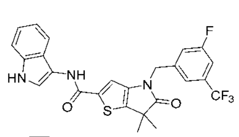
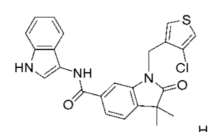

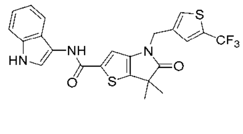
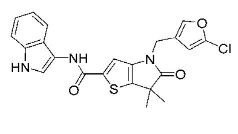
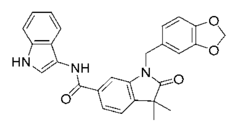
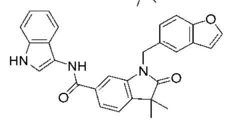
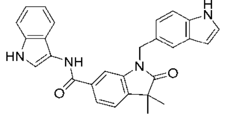

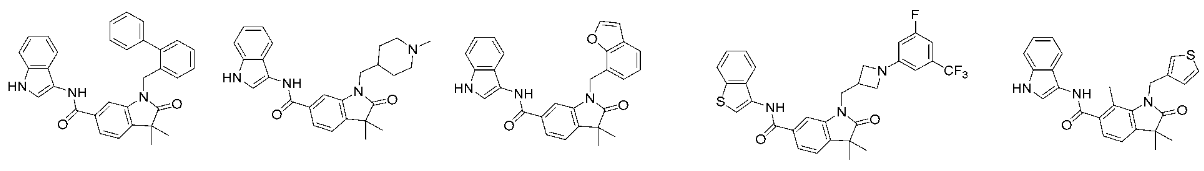

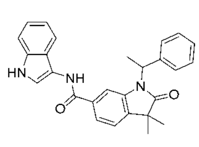 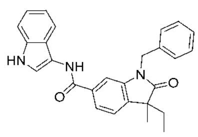 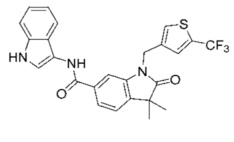 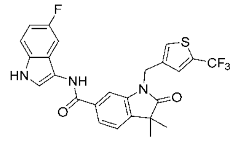 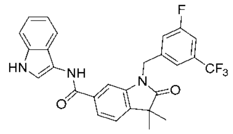

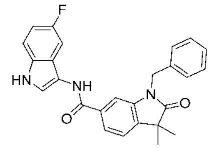 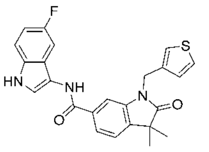 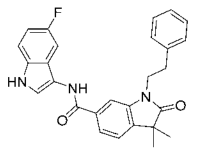 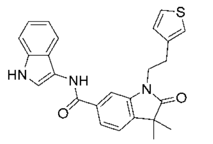 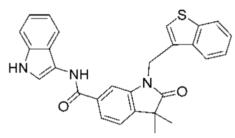

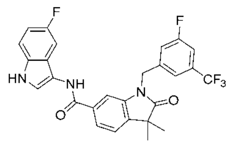 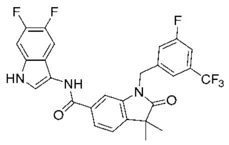 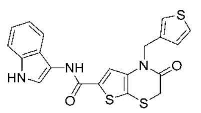 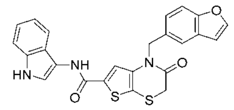 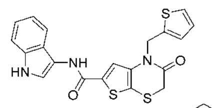

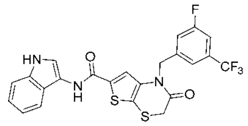 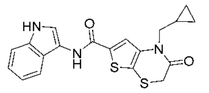 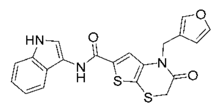 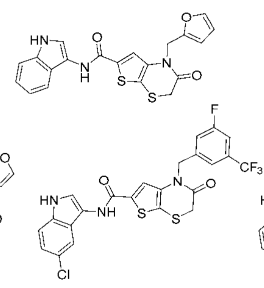

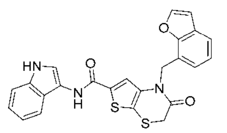 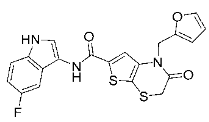 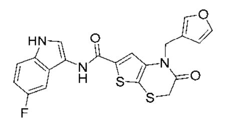

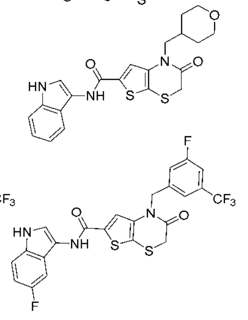 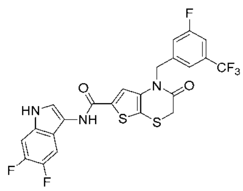 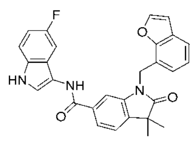 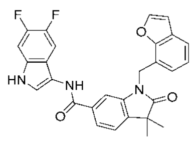 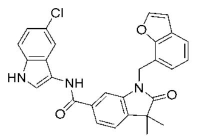 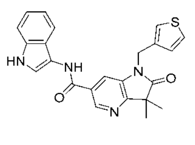.

**13.** A pharmaceutical composition comprising a therapeutically effective amount of one or more compounds selected from the group consisting of: the compound as described in any one of claims 1-12, or the prodrug, the enantiomer, the diastereomer, the racemate, and the mixtures thereof, or the pharmaceutically acceptable salt thereof; and optionally pharmaceutically acceptable carriers.

**14.** Use of the compound as described in any one of claims 1-12, or the prodrug, the enantiomer, the diastereomer, the racemate, and the mixtures thereof, or the pharmaceutically acceptable salt thereof, or the pharmaceutical composition as described in claim 13 for the preparation of cGAS/STING pathway targeted inhibitors; or
for the preparation of drugs for preventing and/or treating inflammatory diseases and/or autoimmune diseases.

**15.** The use according to claim 14, wherein the inflammatory disease is selected from the group consisting of: inflammatory bowel disease, Crohn's disease, ulcerative colitis, intestinal malabsorption syndrome, irritable bowel syndrome, uveitis, mucositis, diabetes, cardiovascular disease and neurodegenerative disease, and/or
the autoimmune disease is selected from the group consisting of: Singleton-Merten syndrome (SMS), Aicardi-Goutières syndrome (AGS), systemic lupus erythematosus (SLE), amyotrophic lateral sclerosis (ALS), familial chilblain lupus erythematosus (FCL), multiple sclerosis, autoimmune colitis, retinal vasculopathy with cerebral leukoencephalopathy (RVCL), STING-associated vasculopathy with onset in infancy (SAVI), scleroderma, psoriasis, Sjogren's syndrome, and rheumatoid arthritis.

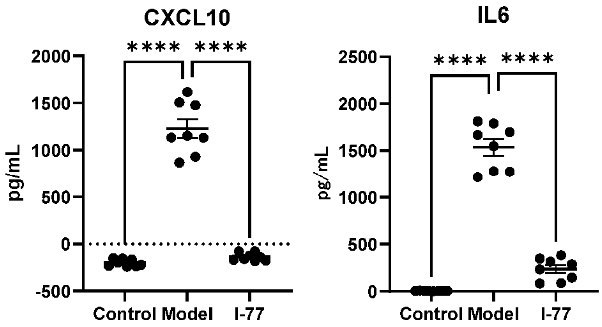


Figure 1

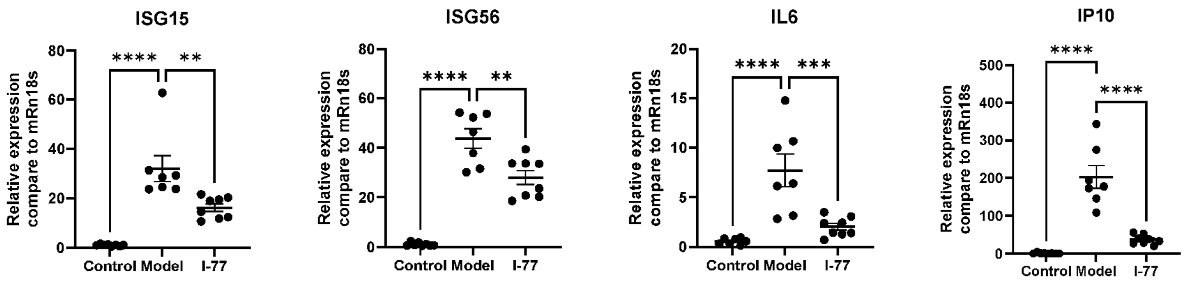


Figure 2

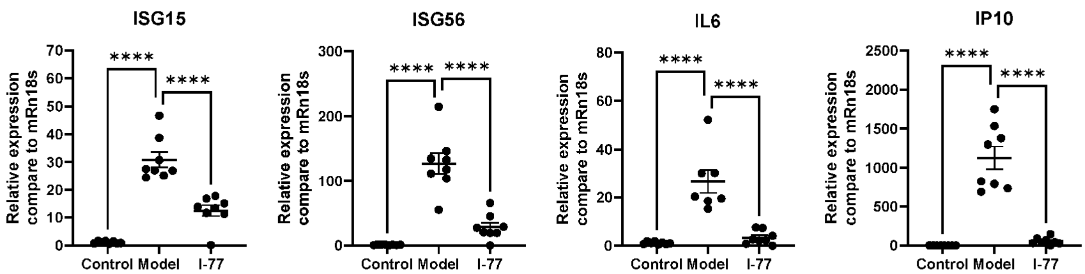


Figure 3

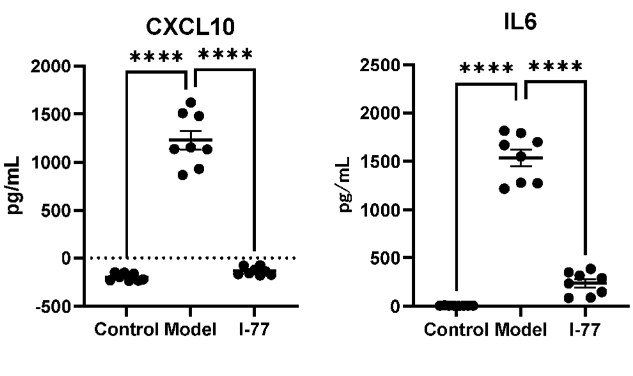


Figure 4

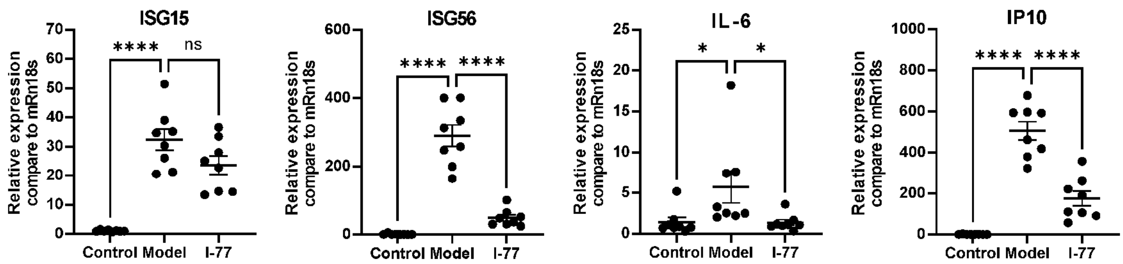


Figure 5

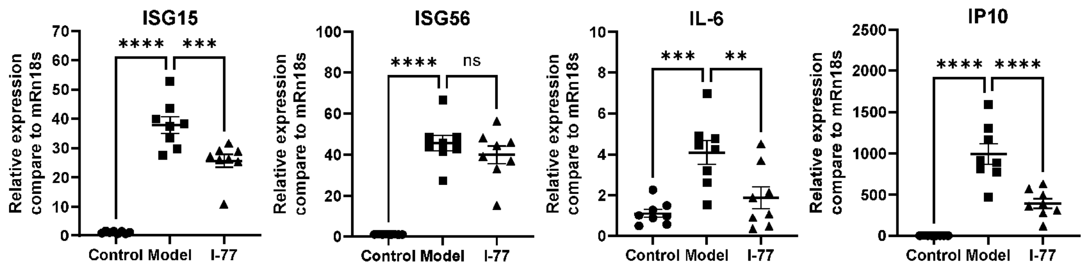


Figure 6

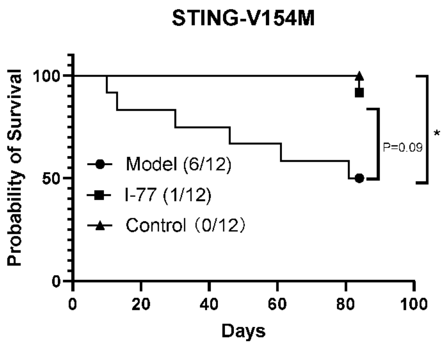


Figure 7

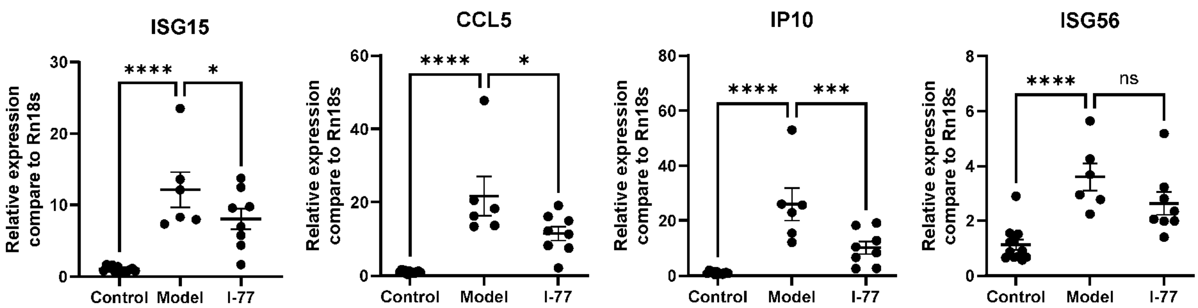


Figure 8

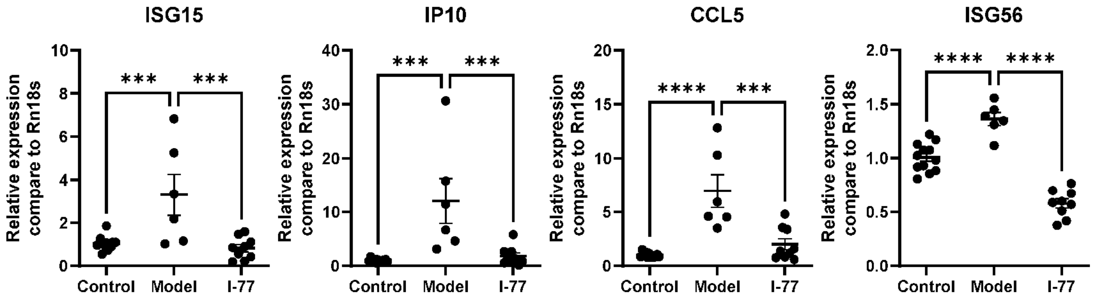


Figure 9

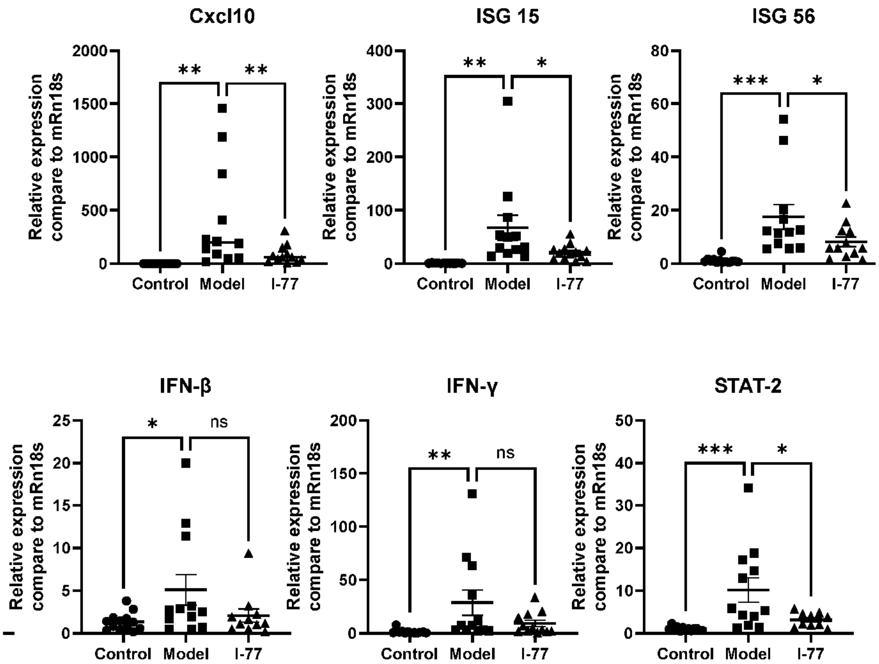


Figure 10

## INTERNATIONAL SEARCH REPORT

International application No.
**PCT/CN2024/105679**

**A. CLASSIFICATION OF SUBJECT MATTER**

C07D513/04(2006.01)i; A61K31/5415(2006.01)i; A61P3/10(2006.01)i

According to International Patent Classification (IPC) or to both national classification and IPC

**B. FIELDS SEARCHED**

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

CNTXT, WPABS, STN: 结构式, STING, 抑制剂, 噻嗪 , Inhibitor, Thiazine

**C. DOCUMENTS CONSIDERED TO BE RELEVANT**

| Category* | Citation of document, with indication, where appropriate, of the relevant passages | Relevant to claim No. |
|---|---|---|
| X | CN 115151304 A (CURADEV PHARMA PVT. LTD.) 04 October 2022 (2022-10-04) claims 1-41, and description, pages 58-144 | 1-15 |
| A | CN 114805309 A (CHINA PHARMACEUTICAL UNIVERSITY) 29 July 2022 (2022-07-29) entire document | 1-15 |
| A | WO 2023017451 A1 (CURADEV PHARMA PVT. LTD.) 16 February 2023 (2023-02-16) entire document | 1-15 |
| A | WO 2018234805 A1 (CURADEV PHARMA PVT. LTD.) 27 December 2018 (2018-12-27) entire document | 1-15 |
| A | WO 2018234808 A1 (CURADEV PHARMA PVT. LTD.) 27 December 2018 (2018-12-27) entire document | 1-15 |
| A | Registry. "RN 1216620-70-0" *STN,* 04 April 2010 (2010-04-04), pages 1-2 | 1-15 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **24 September 2024** | **08 October 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2024/105679**

| Patent document cited in search report | | | Publication date (day/month/year) | Patent family member(s) | | | Publication date (day/month/year) |
|---|---|---|---|---|---|---|---|
| CN | 115151304 | A | 04 October 2022 | CA | 3166358 | A1 | 19 August 2021 |
| | | | | IL | 295388 | A | 01 October 2022 |
| | | | | EP | 4103278 | A1 | 21 December 2022 |
| | | | | WO | 2021161230 | A1 | 19 August 2021 |
| | | | | WO | 2021161230 | A4 | 07 October 2021 |
| | | | | KR | 20220141328 | A | 19 October 2022 |
| | | | | JP | 2023513241 | A | 30 March 2023 |
| | | | | TW | 202140467 | A | 01 November 2021 |
| | | | | AU | 2021219370 | A1 | 25 August 2022 |
| | | | | US | 2023124361 | A1 | 20 April 2023 |
| CN | 114805309 | A | 29 July 2022 | | None | | |
| WO | 2023017451 | A1 | 16 February 2023 | TW | 202321232 | A | 01 June 2023 |
| | | | | JP | 2024529088 | A | 01 August 2024 |
| | | | | AU | 2022325543 | A1 | 15 February 2024 |
| | | | | CA | 3228528 | A1 | 16 February 2023 |
| | | | | EP | 4384267 | A1 | 19 June 2024 |
| | | | | KR | 20240046742 | A | 09 April 2024 |
| | | | | IL | 310705 | A | 01 April 2024 |
| WO | 2018234805 | A1 | 27 December 2018 | JP | 2020524717 | A | 20 August 2020 |
| | | | | US | 2020138827 | A1 | 07 May 2020 |
| | | | | EP | 3642184 | A1 | 29 April 2020 |
| WO | 2018234808 | A1 | 27 December 2018 | IL | 271522 | A | 27 February 2020 |
| | | | | IL | 271522 | B1 | 01 March 2023 |
| | | | | IL | 271522 | B2 | 01 July 2023 |
| | | | | CA | 3067257 | A1 | 27 December 2018 |
| | | | | SG | 11201912397 | RA | 30 January 2020 |
| | | | | PH | 12019502870 | A1 | 28 September 2020 |
| | | | | UA | 125730 | C2 | 25 May 2022 |
| | | | | ZA | 201908496 | B | 28 April 2021 |
| | | | | TW | 201920120 | A | 01 June 2019 |
| | | | | TWI | 799426 | B | 21 April 2023 |
| | | | | PE | 20200696 | A1 | 16 June 2020 |
| | | | | CL | 2019003793 | A1 | 07 August 2020 |
| | | | | AU | 2018288018 | A1 | 16 January 2020 |
| | | | | AU | 2018288018 | B2 | 14 April 2022 |
| | | | | AU | 2018288018 | C1 | 20 October 2022 |
| | | | | US | 2020147083 | A1 | 14 May 2020 |
| | | | | US | 11571423 | B2 | 07 February 2023 |
| | | | | JP | 2020524719 | A | 20 August 2020 |
| | | | | JP | 7296954 | B2 | 23 June 2023 |
| | | | | KR | 20200031616 | A | 24 March 2020 |
| | | | | KR | 102628892 | B1 | 24 January 2024 |
| | | | | BR | 112019027127 | A2 | 07 July 2020 |
| | | | | ECSP | 20004580 | A | 29 May 2020 |
| | | | | CO | 2020000562 | A2 | 31 January 2020 |
| | | | | EP | 3642198 | A1 | 29 April 2020 |
| | | | | EP | 3642198 | B1 | 16 March 2022 |
| | | | | AR | 114975 | A1 | 11 November 2020 |
| | | | | MX | 2019015468 | A | 03 August 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021161230 A1


### Non-patent literature cited in the description


- *Nature*, 2018, vol. 559, 269-273
- *Nat. Commun.*, 2019, vol. 10, 2261
- *Proc. Natl. Acad. Sci. USA*, 2021, vol. 118, e2105465118